(19) Europäisches Patentamt
European Patent Office
Office européen des brevets

(11) EP 3 967 762 A1

(12) # EUROPEAN PATENT APPLICATION
published in accordance with Art. 153(4) EPC

(43) Date of publication:
**16.03.2022 Bulletin 2022/11**

(21) Application number: **20806208.3**

(22) Date of filing: **08.05.2020**

(51) International Patent Classification (IPC):
***C12P 7/42*** (2006.01) ***C12P 7/44*** (2006.01)
***C12N 1/21*** (2006.01) ***C12N 15/53*** (2006.01)
***C12N 15/54*** (2006.01) ***C12N 9/04*** (2006.01)

(52) Cooperative Patent Classification (CPC):
**C12P 7/42; C12P 7/44**

(86) International application number:
**PCT/JP2020/018663**

(87) International publication number:
**WO 2020/230718 (19.11.2020 Gazette 2020/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.05.2019 JP 2019089770**

(71) Applicant: **Toray Industries, Inc.**
**Tokyo, 103-8666 (JP)**

(72) Inventors:
- **ISOBE, Kyohei**
**Kamakura-shi, Kanagawa 248-8555 (JP)**
- **KAWAMURA, Kenji**
**Kamakura-shi, Kanagawa 248-8555 (JP)**
- **YAMADA, Katsushige**
**Kamakura-shi, Kanagawa 248-8555 (JP)**

(74) Representative: **Kador & Partner PartG mbB**
**Corneliusstraße 15**
**80469 München (DE)**

(54) **GENETICALLY MODIFIED MICROORGANISM FOR PRODUCING 3-HYDROXYHEXANEDIOIC ACID, (E)-HEX-2-ENEDIOIC ACID AND/OR HEXANEDIOIC ACID, AND PRODUCTION METHOD FOR SAID CHEMICALS**

(57) Disclosed is a genetically modified microorganism that produces 3-hydroxyadipic acid, α-hydromuconic acid, or adipic acid in high yield. A nucleic acid encoding any one of the polypeptides described in (a) to (c) below is introduced or the expression of the polypeptide is enhanced and the function of pyruvate kinase is impaired in the genetically modified microorganism: (a) a polypeptide composed of an amino acid sequence represented by any one of SEQ ID NOs: 1 to 7; (b) a polypeptide having the same amino acid sequence as any one of those amino acid sequences, except that one or several amino acids are substituted, deleted, inserted, and/or added, and having an enzymatic activity that catalyzes a reaction to reduce 3-oxoadipyl-CoA to 3-hydroxyadipyl-CoA; (c) a polypeptide composed of an amino acid sequence with a sequence identity of not less than 70% to any one of those amino acid sequences and having an enzymatic activity that catalyzes a reaction to reduce 3-oxoadipyl-CoA to 3-hydroxyadipyl-CoA.

EP 3 967 762 A1

## Description

### Technical Field

The present invention relates to a genetically modified microorganism in which a nucleic acid encoding a polypeptide involved in the production of a substance of interest is introduced or the expression of the polypeptide is enhanced, and to a method of producing the substance by using the microorganism.

### Background Art

3-Hydroxyadipic acid (IUPAC name: 3-hydroxyhexanedioic acid), α-hydromuconic acid (IUPAC name: (E)-hex-2-enedioic acid), and adipic acid (IUPAC name: hexanedioic acid) are dicarboxylic acids containing six carbon atoms. These dicarboxylic acids can be polymerized with a polyhydric alcohol or a polyfunctional amine, to be used as raw materials for the production of polyesters or polyamides, respectively. Additionally, these dicarboxylic acids can be used alone after ammonia addition at a terminal position in these chemicals to form lactams as raw materials for the production of polyamides.

The following documents related to the production of 3-hydroxyadipic acid or α-hydromuconic acid using a microorganism are known.

Patent Document 1 describes a method of producing 1,3-butadiene by using a microorganism in which a relevant metabolic pathway is modified, wherein 3-hydroxyadipic acid (3-hydroxyadipate) is described to be a metabolic intermediate in the metabolic pathway for biosynthesis of 1,3-butadiene from acetyl-CoA and succinyl-CoA.

Patent Document 2 describes a method of producing muconic acid by using a microorganism in which a relevant metabolic pathway is modified, wherein α-hydromuconic acid (2,3-dehydroadipate) is described to be a metabolic intermediate in the metabolic pathway for biosynthesis of *trans*,*trans*-muconic acid from acetyl-CoA and succinyl-CoA.

Patent Documents 3 and 4 describe a method of producing adipic acid and hexamethylene diamine (HMDA) by using a non-natural microorganism, wherein the biosynthetic pathways for these substances are described to share a common reaction to synthesize 3-oxoadipyl-CoA from acetyl-CoA and succinyl-CoA but diverge after the synthesis of 3-oxoadipyl-CoA. Furthermore, Patent Document 3 describes the pyruvate kinase gene as a candidate gene that is additionally deleted from the metabolic pathway to improve the HMDA formation coupled with proliferation for the HMDA production, but a potential relationship between pyruvate kinase deficiency and increased adipic acid production is not mentioned in this document.

Additionally, all the biosynthetic pathways mentioned in Patent Documents 1 to 4 are described to share a common enzymatic reaction that reduces 3-oxoadipyl-CoA to 3-hydroxyadipyl-CoA.

Patent Documents 5 and 6 describe methods of producing 3-hydroxyadipic acid and α-hydromuconic acid by using a microorganism of the genus *Serratia*, respectively. The patent documents disclose that the efficiency of producing 3-hydroxyadipic acid and α-hydromuconic acid can be increased particularly by enhancing the activity of an acyl transferase that catalyzes a reaction to produce 3-oxoadipyl-CoA from acetyl-CoA and succinyl-CoA, but these documents have no description related to pyruvate kinase.

Moreover, a method of modifying a microorganism based on an *in silico* analysis is disclosed in Patent Document 7, in which the production of succinic acid is increased by deleting genes encoding pyruvate kinase and a phosphotransferase system enzyme in *Escherichia coli* (*E. coli*), *pykF*, *pykA*, and *ptsG*, and culturing the resulting *E. coli* bacteria under anaerobic conditions.

### Prior Art Documents

### Patent Documents

Patent Document 1: JP 2013-535203 A
Patent Document 2: US 2011/0124911 A1
Patent Document 3: JP 2015-146810 A
Patent Document 4: JP 2011-515111 A
Patent Document 5: WO 2017/209102
Patent Document 6: WO 2017/209103
Patent Document 7: JP 2008-527991 A

Summary of the Invention

Problems to be Solved by the Invention

Patent Documents 1 and 2 describe metabolic pathways by which the microorganisms can produce 3-hydroxyadipic acid and α-hydromuconic acid, but have no description about interruption of the metabolic pathways to allow the microorganisms to secrete 3-hydroxyadipic acid or α-hydromuconic acid into culture medium. Moreover, the prior studies described in Patent Documents 1 to 4 have not examined whether or not 3-hydroxyadipic acid, α-hydromuconic acid, or adipic acid can be actually produced by using a non-natural microorganism in which a nucleic acid encoding an enzyme that catalyzes a reaction to reduce 3-oxoadipyl-CoA to 3-hydroxyadipyl-CoA has been introduced. Accordingly, it is not known whether the enzyme that catalyzes a reaction to reduce 3-oxoadipyl-CoA to 3-hydroxyadipyl-CoA, as described in Patent Documents 1 to 4, also exhibits excellent activity in the production of 3-hydroxyadipic acid, α-hydromuconic acid, and/or adipic acid.

Accordingly, an object of the present invention is to provide a genetically modified microorganism for producing 3-hydroxyadipic acid, α-hydromuconic acid, and/or adipic acid in high yield and a method of producing a substance by using the modified microorganism, wherein the modified microorganism is based on a genetically modified microorganism in which a nucleic acid encoding an enzyme that exhibits excellent activity in 3-oxoadipyl-CoA reduction reaction is introduced or the expression of the enzyme is enhanced, and wherein the modified microorganism is further modified to have an altered metabolic pathway.

Means for Solving the Problem

The inventors intensively studied in order to achieve the above-described object and consequently found that 3-hydroxyadipic acid, α-hydromuconic acid, and/or adipic acid can be produced in high yield by a genetically modified microorganism in which a nucleic acid encoding an enzyme that exhibits excellent activity in 3-oxoadipyl-CoA reduction reaction is introduced or the expression of the enzyme is enhanced and the function of pyruvate kinase is further impaired, to complete the present invention.

That is, the present invention provides the following:

(1) A genetically modified microorganism in which a nucleic acid encoding any one of the polypeptides described in (a) to (c) below is introduced or the expression of the polypeptide is enhanced and the function of pyruvate kinase is impaired:

(a) a polypeptide composed of an amino acid sequence represented by any one of SEQ ID NOs: 1 to 7;
(b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;
(c) a polypeptide composed of an amino acid sequence with a sequence identity of not less than 70% to the sequence represented by any one of SEQ ID NOs: 1 to 7 and having an enzymatic activity that catalyzes a reaction to reduce 3-oxoadipyl-CoA to 3-hydroxyadipyl-CoA.

(2) The genetically modified microorganism according to (1), wherein a polypeptide selected from the above (b) and (c) contains a region composed of an amino acid sequence represented by SEQ ID NO: 173.
(3) The genetically modified microorganism according to (2), wherein the amino acid sequence represented by SEQ ID NO: 173 contains a phenylalanine or leucine residue at the 13th amino acid position from the N terminus, a leucine or glutamine residue at the 15th amino acid position from the N terminus, a lysine or asparagine residue at the 16th amino acid position from the N terminus, a glycine or serine residue at the 17th amino acid position from the N terminus, a proline or arginine residue at the 19th amino acid position from the N terminus, and a leucine, methionine, or valine residue at the 21 st amino acid position from the N terminus.
(4) The genetically modified microorganism according to any one of (1) to (3), which is a genetically modified microorganism belonging to a genus selected from the group consisting of *Escherichia*, *Serratia*, *Hafnia*, and *Pseudomonas.*
(5) The genetically modified microorganism according to any one of (1) to (4), which has an ability to generate 3-oxoadipyl-CoA and coenzyme A from acetyl-CoA and succinyl-CoA and an ability to generate 3-hydroxyadipic acid from 3-hydroxyadipyl-CoA.
(6) The genetically modified microorganism according to any one of (1) to (4), which has an ability to generate 3-oxoadipyl-CoA and coenzyme A from acetyl-CoA and succinyl-CoA, an ability to generate 2,3-dehydroadipyl-CoA from 3-hydroxyadipyl-CoA, and an ability to generate α-hydromuconic acid from 2,3-dehydroadipyl-CoA.

(7) The genetically modified microorganism according to any one of (1) to (4), which has an ability to generate 3-oxoadipyl-CoA and coenzyme A from acetyl-CoA and succinyl-CoA, an ability to generate 2,3-dehydroadipyl-CoA from 3-hydroxyadipyl-CoA, an ability to generate adipyl-CoA from 2,3-dehydroadipyl-CoA, and an ability to generate adipic acid from adipyl-CoA.

(8) The genetically modified microorganism according to any one of (1) to (7), wherein the function of a phosphotransferase system enzyme is further impaired.

(9) A method of producing 3-hydroxyadipic acid, comprising culturing the genetically modified microorganism according to any one of (1) to (5) and (8) in a culture medium containing a carbon source as a raw material for fermentation.

(10) A method of producing α-hydromuconic acid, comprising culturing the genetically modified microorganism according to any one of (1) to (4), (6) and (8) in a culture medium containing a carbon source as a raw material for fermentation.

(11) A method of producing adipic acid, comprising culturing the genetically modified microorganism according to any one of (1) to (4), (7) and (8) in a culture medium containing a carbon source as a raw material for fermentation.

(12) A method of producing one or more substances selected from the group consisting of 3-hydroxyadipic acid, α-hydromuconic acid, and adipic acid, comprising culturing a genetically modified microorganism in a culture medium containing a carbon source as a raw material for fermentation, wherein a nucleic acid encoding a polypeptide encoded by the 3-hydroxybutyryl-CoA dehydrogenase gene of a microorganism of the genus *Serratia*, which forms a gene cluster with 5-aminolevulinic acid synthase gene in the microorganism, is introduced or the expression of the polypeptide is enhanced and the function of pyruvate kinase is impaired in the genetically modified microorganism.

(13) The method according to (12), wherein the genetically modified microorganism is a microorganism in which the function of a phosphotransferase system enzyme is further impaired.

Effects of the Invention

The genetically modified microorganism according to the present invention, which expresses an enzyme that exhibits excellent activity in a reaction to reduce 3-oxoadipyl-CoA to 3-hydroxyadipyl-CoA and, furthermore, has an impaired pyruvate kinase function, can produce 3-hydroxyadipic acid, α-hydromuconic acid, and/or adipic acid in high yield compared to a parental strain of the microorganism in which pyruvate kinase is not impaired.

The method of producing a substance according to the present invention uses the genetically modified microorganism which is excellent in the production of 3-hydroxyadipic acid, α-hydromuconic acid, and/or adipic acid via production of 3-hydroxyadipyl-CoA and thus can greatly increase the production of those substances.

Brief Description of the Drawing

FIG. 1 shows a gene cluster composed of a 3-hydroxybutyryl-CoA dehydrogenase gene and a 5-aminolevulinic acid synthase gene.

Detailed Description of the Invention

The microorganism according to the present invention is a genetically modified microorganism in which a nucleic acid encoding a polypeptide described in (a) to (c) below is introduced or the expression of the polypeptide is enhanced and the function of pyruvate kinase is impaired:

(a) a polypeptide composed of an amino acid sequence represented by any one of SEQ ID NOs: 1 to 7;

(b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;

(c) a polypeptide composed of an amino acid sequence with a sequence identity of not less than 70% to the sequence represented by any one of SEQ ID NOs: 1 to 7 and having activity in reduction of 3-oxoadipyl-CoA to 3-hydroxyadipyl-CoA.

An enzyme that catalyzes the reaction of reducing 3-oxoadipyl-CoA to 3-hydroxyadipyl-CoA is hereinafter referred to as "3-oxoadipyl-CoA reductase" in the specification. Additionally, 3-hydroxyadipic acid, α-hydromuconic acid, and adipic acid may be abbreviated as 3HA, HMA, and ADA, respectively, in this specification.

In the present invention, introducing a nucleic acid means introducing a nucleic acid from the outside to the inside of a microorganism to give the microorganism an ability to produce a polypeptide encoded by the nucleic acid.

The method of introduction of a nucleic acid is not limited to a particular method, and examples of the method that can be used include a method in which a nucleic acid of interest is integrated into an expression vector capable of autonomous replication in a microorganism and then integrated into a host microorganism, and a method in which a nucleic acid of interest is integrated into the genome of a microorganism.

In the present invention, enhancing the expression of a polypeptide means enhancing the expression of a polypeptide which a microorganism originally has. The method of enhancement of expression is not limited to a particular method, and examples of the method include a method in which a nucleic acid encoding a polypeptide of interest is increased in copy number, and a method in which a promoter region or a ribosome-binding sequence upstream of the region coding for a polypeptide of interest is modified. These methods may be carried out individually or in combination.

Additionally, one or more of the above nucleic acids may be introduced. Moreover, the introduction of a nucleic acid and the enhancement of polypeptide expression may be combined.

For the polypeptide used in the present invention and composed of the same amino acid sequence as that represented by any one of SEQ ID NOs: 1 to 7, except that one or several amino acids are substituted, deleted, inserted, and/or added, and having 3-oxoadipyl-CoA reductase activity, the range represented by the phrase "one or several" is preferably 10 or less, more preferably 5 or less, especially preferably 4 or less, and most preferably 1 or 2 or less. In the case of amino acid substitution, the activity of the original polypeptide is more likely to be maintained when an amino acid(s) is/are replaced by an amino acid(s) with similar properties (so-called conservative substitution). That is, the physiological properties of the original polypeptide are often maintained when an amino acid(s) is/arc replaced by an amino acid(s) with similar properties. Therefore, in the case of substitution, a given amino acid is preferably replaced by another amino acid with similar properties. That is, the 20 amino acids that make up natural proteins can be divided into groups of amino acids with similar properties, such as neutral amino acids with a less polar side chain (Gly, Ile, Val, Leu, Ala, Met, Pro), neutral amino acids with a hydrophilic side chain (Asn, Gln, Thr, Ser, Tyr, Cys), acidic amino acids (Asp, Glu), and basic amino acids (Arg, Lys, His), and aromatic amino acids (Phe, Tyr, Trp). It is often the case that substitution between amino acids in the same group does not change the properties of the original polypeptide.

For the polypeptide used in the present invention and having an amino acid sequence with a sequence identity of not less than 70% to the sequence represented by any one of SEQ ID NOs: 1 to 7 and having 3-oxoadipyl-CoA reductase activity, the sequence identity is preferably not less than 80%, more preferably not less than 85%, further preferably not less than 90%, still further preferably not less than 95%, yet further preferably not less than 97%, and even further preferably not less than 99%.

In the present invention, the term "sequence identity" means a ratio (percentage) of the number of identical amino acid or nucleotide residues relative to the total number of amino acid or nucleotide residues over the overlapping portion of an amino acid sequence alignment (including an amino acid corresponding to the translation start site) or a nucleotide sequence alignment (including the start codon), which is obtained by aligning two amino acid or nucleotide sequences with or without introduction of gaps for an optimal match, and is calculated by the following formula (1). In the formula (1), the length of a shorter sequence being compared is not less than 400 amino acids; in cases where the length of the shorter sequence is less than 400 amino acids, the sequence identity is not defined. The sequence identity can be easily determined using BLAST (Basic Local Alignment Search Tool), an algorithm widely used in this field. For example, BLAST is publicly available on a website, such as that of NCBI (National Center for Biotechnology Information) or KEGG (Kyoto Encyclopedia of Genes and Genomes), on which the sequence identity can be easily determined using default parameters. Additionally, the sequence identity can also be determined using a similar function implemented in a software program such as Genetyx.

$$\text{Sequence identity (\%)} = \text{the number of matches (without counting the number of gaps) / the length of a shorter sequence (excluding the terminal gaps)} \times 100 \qquad (1)$$

By using a function of Genetyx (% Identity Matrix) to calculate sequence identities based on the formula (1) among the amino acid sequences represented by SEQ ID NOs: 1 to 7, the lowest sequence identity of 71.51% is found between the sequences represented by SEQ ID NOs: 2 and 4, and the sequence identities among the amino acid sequences represented by SEQ ID NOs: 1 to 7 are found to be at least not less than 70%. The results of calculation of sequence identity using Genetyx are presented in Table 1. In Tables 1 to 5 below, the numbers in the leftmost column represent SEQ ID NOs.

[Table 1] [GENETYX : %Identity Matrix] *Gaps are NOT taken into account.

[%]

| | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | * | | | | | | |
| 2 Serratia nematodiphila DSM21420 | 98.23 | * | | | | | |
| 3 Serratia plymuthica NBRC102599 | 72.10 | 71.51 | * | | | | |
| 4 Serratia proteamaculans 568 | 72.29 | 71.51 | 86.24 | * | | | |
| 5 Serratia ureilytica Lr5/4 | 90.76 | 90.76 | 72.88 | 73.28 | * | | |
| 6 Serratia sp. BW106 | 72.29 | 71.90 | 87.03 | 92.33 | 73.67 | * | |
| 7 Serratia liquefaciens FK01 | 72.29 | 71.70 | 84.67 | 86.83 | 73.47 | 87.81 | * |

[Match Count/Length]

| | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | * | | | | | | |
| 2 Serratia nematodiphila DSM21420 | 500/509 | * | | | | | |
| 3 Serratia plymuthica NBRC102599 | 367/509 | 364/509 | * | | | | |
| 4 Serratia proteamaculans 568 | 368/509 | 364/509 | 439/509 | * | | | |
| 5 Serratia ureilytica Lr5/4 | 462/509 | 462/509 | 371/509 | 373/509 | * | | |
| 6 Serratia sp. BW106 | 368/509 | 366/509 | 443/509 | 470/509 | 375/509 | * | |
| 7 Serratia liquefaciens FK01 | 368/509 | 365/509 | 431/509 | 442/509 | 374/509 | 447/509 | * |

When each of the amino acid sequences represented by SEQ ID NO: 1 to 7 as queries was compared using BLASTP to al the amino acid sequences registered in the NCBI amino acid database (non-redundant protein sequences) to determine sequence identities, all sequences with a sequence identity of not less than 70% were found to be from bacteria of the genus *Serratia.*

All the polypeptides represented by SEQ ID NOs: 1 to 7 as described above in (a) contain a common sequence 1 composed of 24 amino acid residues and represented by SEQ ID NO: 173 within a region from the 15th to the 38th amino acid residues from the N terminus (hereinafter, an amino acid residue at the n-th position from the N terminus may conveniently be represented by n "a.a."; for example, the region from the 15th to the 38th amino acid residues from the N terminus may be thus simply represented by "15 to 38 a.a."). In the common sequence 1, Xaa represents an arbitrary amino acid residue, and the 13 a.a. is preferably a phenylalanine or leucine, and the 15 a.a. is preferably a leucine or glutamine, and the 16 a.a. is preferably a lysine or asparagine, and the 17 a.a. is a glycine or serine, more preferably a glycine, and the 19 a.a. is preferably a proline or arginine, and the 21 a.a. is preferably a leucine, methionine, or valine. The common sequence 1 corresponds to the region including the $NAD^+$-binding residue and the surrounding amino acid residues. In the $NAD^+$-binding residues, the 24th amino acid residue in the common sequence 1 is an aspartic acid, as described in Biochimie., 2012 Feb, 94 (2): 471-8., but in the common sequence 1, the residue is an asparagine, which is characteristic. It is thought that the presence of the common sequence 1 causes the polypeptides represented by SEQ ID NOs: 1 to 7 to show excellent enzymatic activity as 3-oxoadipyl-CoA reductases.

The polypeptides as described above in (b) and (c) also preferably contain the common sequence 1 composed of 24 amino acid residues and represented by SEQ ID NO: 173 within a region from 1 to 200 a.a. The common sequence is more preferably located within a region from 1 to 150 a.a., and further preferably within a region from 1 to 100 a.a.

Specific examples of the polypeptides include those with the amino acid sequences represented by SEQ ID NOs: 8 to 86. The amino acid sequences represented by SEQ ID NOs: 8 to 86 contain the common sequence 1 composed of 24 amino acid residues and represented by SEQ ID NO: 173 within a region from 15 to 38 a.a. The amino acid sequences represented by SEQ ID NOs: 8 to 86 have a sequence identity of not less than 90% to the amino acid sequence represented by any one of SEQ ID NOs: 1 to 7. The results of calculation of sequence identity using Genetyx are presented in Tables 2-1 to 2-3 and Tables 3-1 to 3-3.

[Table 2-1]

[GENETYX : %Identity Matrix]
*Gaps are NOT taken into account.
[%]

| | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | * | | | | | | |
| 2 Serratia nematodiphila DSM21420 | 98.23 | * | | | | | |
| 3 Serratia plymuthica NBRC102599 | 72.10 | 71.51 | * | | | | |
| 4 Serratia proteamaculans 568 | 72.29 | 71.51 | 86.24 | * | | | |
| 5 Serratia ureilytica Lr5/4 | 90.76 | 90.76 | 72.88 | 73.28 | * | | |
| 6 Serratia sp. BW106 | 72.29 | 71.90 | 87.03 | 92.33 | 73.67 | * | |
| 7 Serratia liquefaciens FK01 | 72.29 | 71.70 | 84.67 | 86.83 | 73.47 | 87.81 | * |
| 8 Serratia sp. S119 | 94.89 | 94.30 | 72.88 | 72.49 | 91.55 | 73.08 | 72.88 |
| 9 Serratia sp. YD25 | 92.33 | 92.33 | 72.49 | 72.49 | 93.51 | 72.69 | 72.88 |
| 10 Serratia sp. FS14 | 98.62 | 99.60 | 71.70 | 71.70 | 91.15 | 72.10 | 72.10 |
| 11 Serratia sp. HMSC15F11 | 94.89 | 94.30 | 73.28 | 73.28 | 91.35 | 73.47 | 73.47 |
| 12 Serratia sp JKS000199 | 90.76 | 90.76 | 72.69 | 73.08 | 99.41 | 73.47 | 73.28 |
| 13 Serratia sp. TEL | 90.56 | 90.56 | 72.88 | 73.28 | 99.80 | 73.67 | 73.47 |
| 14 Serratia sp. ISTD04 | 90.56 | 90.56 | 72.49 | 73.08 | 99.41 | 73.47 | 73.28 |
| 15 Serratia sp. SCSI | 90.76 | 90.76 | 72.88 | 73.28 | 99.60 | 73.47 | 73.47 |
| 16 Serratia sp. S4 | 72.10 | 71.31 | 86.44 | 98.62 | 73.08 | 91.94 | 86.64 |
| 17 Serratia sp C-1 | 72.49 | 71.90 | 98.03 | 86.05 | 73.28 | 86.64 | 84.08 |
| 18 Serratia marcescens 532 | 99.80 | 98.03 | 72.29 | 72.10 | 90.56 | 72.10 | 72.10 |
| 19 Serratia marcescens 2880STDY5683033 | 99.60 | 97.83 | 72.10 | 72.29 | 90.37 | 72.10 | 72.29 |
| 20 Serratia marcescens WW4 | 98.42 | 99.41 | 71.90 | 71.90 | 90.96 | 72.29 | 71.90 |
| 21 Serratia marcescens K27 | 98.23 | 99.21 | 71.31 | 71.31 | 90.96 | 71.70 | 71.70 |
| 22 Serratia marcescens 280 | 98.42. | 99.41 | 71.70 | 71.70 | 90.96 | 72.10 | 72.10 |
| 23 Serratia marcescens 19F | 98. 42 | 99. 41 | 71.51 | 71.70 | 90.96 | 72.10 | 72.10 |
| 24 Serratia marcescens 1185 | 98.23 | 99.60 | 71.31 | 71.31 | 90.37 | 71.70 | 71.51 |

[Table 2-2]

| | | | | | | | |
|---|---|---|---|---|---|---|---|
| 25 Serratia marcescens S217 | 98.23 | 99.21 | 71.31 | 71.51 | 90.96 | 71.90 | 71.90 |
| 26 Serratia marcescens KHCo-24B | 98.03 | 99.60 | 71.31 | 71.31 | 90.56 | 71.70 | 71.90 |
| 27 Serratia marcescens Z6 | 98.03 | 99.01 | 71.70 | 71.90 | 90.56 | 72.29 | 71.90 |
| 28 Serratia marcescens 546 | 97.83 | 99.21 | 71.51 | 71.70 | 90.37 | 72.10 | 71.70 |
| 29 Serratia nematodiphila MB307 | 98.03 | 99.60 | 71.31 | 71.51 | 90.56 | 71.90 | 71.70 |
| 30 Serratia marcescens VGH107 | 98.03 | 99.01 | 71.31 | 71.51 | 90.56 | 71.90 | 71.90 |
| 31 Serratia marcescens MCB | 95.48 | 95.28 | 72.29 | 72.69 | 91.15 | 72.88 | 72.69 |
| 32 Serratia marcescens AH0660 | 95.67 | 95.48 | 72.29 | 72.69 | 90.76 | 73.28 | 72.69 |
| 33 Serratia marcescens UMH12 | 95.48 | 95.28 | 72.10 | 72.49 | 90.56 | 73.08 | 72.49 |
| 34 Serratia sp. OMLW3 | 95.48 | 95.28 | 72.29 | 72.49 | 90.76 | 73.28 | 72.69 |
| 35 Serratia marcescens UMH11 | 95.28 | 95.08 | 72.10 | 72.69 | 90.56 | 73.47 | 72.49 |
| 36 Serratia marcescens UMH1 | 95.08 | 94.89 | 72.29 | 72.49 | 90.17 | 73.08 | 72.29 |
| 37 Serratia marcescens 2880STDY568302D | 95.48 | 94.89 | 73.08 | 72.69 | 92.14 | 73.28 | 73.08 |
| 36 Serratia marcescens 99 | 95.48 | 94.69 | 73.28 | 72.88 | 91.55 | 73.67 | 73.28 |
| 39 Serratia marcescens 374 | 94.89 | 94.69 | 72.29 | 72.29 | 90.17 | 73.08 | 72.29 |
| 40 Serratia marcescens 2880STDY5683036 | 95.28 | 94.49 | 73.08 | 72.69 | 91.35 | 73.47 | 73.08 |
| 41 Serratia marcescens 2880STDY5683034 | 95.28 | 94.69 | 73.08 | 72.69 | 91.94 | 73.28 | 73.08 |
| 42 Serratia marceacens 2880STDY5682892 | 95.28 | 94.69 | 73.28 | 72.88 | 91.94 | 73.47 | 73.28 |
| 43 Serratia marcescens SM39 | 95.08 | 94.49 | 73.28 | 72.69 | 92.14 | 73.28 | 73.28 |
| 44 Serratia marcescens 189 | 95.08 | 94.49 | 73.28 | 72.88 | 92.14 | 73.47 | 73.28 |
| 45 Serratia marcescens SMB2099 | 95.08 | 94.49 | 73.47 | 72.69 | 91.74 | 73.67 | 73.47 |
| 46 Serratia marcescens 2880STDY5682862 | 94.89 | 94.30 | 73.47 | 72.88 | 91.55 | 73.47 | 73.47 |
| 47 Serratia marcescens SE4145 | 94.89 | 94.30 | 73.08 | 72.49 | 91.94 | 73.08 | 73.08 |
| 48 Serratia marcescens 2880STDY5682876 | 95.08 | 94.49 | 73.28 | 72.88 | 91.74 | 73.47 | 73.28 |
| 49 Serratia marcescens 709 | 95.08 | 94.49 | 73.08 | 72.69 | 91.74 | 73.28 | 73.08 |
| 50 Serratia marcescens MGH136 | 94.89 | 94.30 | 72.88 | 72.49 | 91.94 | 73.08 | 72.88 |
| 61 Serratia marcescens 2880STDY5682884 | 94.69 | 94.10 | 72.88 | 72.49 | 91.74 | 73.08 | 73.08 |
| 52 Serratia marcescens D-3 | 95.08 | 94.49 | 73.08 | 72.69 | 91.74 | 73.28 | 73.08 |
| 53 Serratia marcescens 2880STDY5682957 | 94.89 | 94.30 | 72.88 | 72.69 | 91.55 | 73.28 | 72.88 |
| 54 Serratia marcescens YDC563 | 94.69 | 94.10 | 72.88 | 72.69 | 91.35 | 73.28 | 72.88 |
| 55 Serratia marcescens 2880STDY5683035 | 94.89 | 94.30 | 73.08 | 72.69 | 91.55 | 73.28 | 73.08 |

[Table 2-3]

| | | | | | | | |
|---|---|---|---|---|---|---|---|
| 56 Serratia marcescens 28806STDY5682930 | 94.69 | 94.10 | 72.88 | 72.49 | 91.35 | 73.08 | 72.88 |
| 57 Serratia marcescens 790 | 94.49 | 94.30 | 73.28 | 72.88 | 91.35 | 73.47 | 73.28 |
| 58 Serratia marcescens UMH5 | 93.51 | 92.92 | 72.69 | 72.88 | 90.37 | 72.69 | 72.49 |
| 59 Sarratia marcescens 2880STDY5682988 | 93.32 | 92.73 | 72.69 | 72.88 | 90.17 | 72.69 | 72.49 |
| 60 Serratia marcescens 945154301 | 94.89 | 94.30 | 73.28 | 73.28 | 91.35 | 73.67 | 73.47 |
| 61 Serratia marcescens at10508 | 94.69 | 94.10 | 73.47 | 73.47 | 91.15 | 73.67 | 73.67 |
| 62 Serratia marcescens ML2637 | 94.49 | 93.90 | 73.28 | 73.47 | 90.96 | 73.67 | 73.67 |
| 63 Serratia marcescens SW1978 | 94.30 | 93.71 | 73.28 | 73.28 | 90.76 | 73.67 | 73.67 |
| 64 Serratia marcescens PWN146 | 94.10 | 93.51 | 72.88 | 72.88 | 90.96 | 72.88 | 73.28 |
| 65 Serratia marcescens H1q | 92.53 | 92.53 | 72.49 | 72.49 | 93.51 | 72.69 | 73.08 |
| 66 Serratia marcescens UMH6 | 91.15 | 91.15 | 72.69 | 73.08 | 99.60 | 73.47 | 73.28 |
| 67 Serratia nematodiphila WCU338 | 91.15 | 91.15 | 72.69 | 73.08 | 99.41 | 73.47 | 73.28 |
| 68 Serratia sp. 0LEL1 | 90.96 | 90.96 | 72.88 | 73.28 | 99.80 | 73.67 | 73.47 |
| 69 Serratia marcescens 7209 | 90.96 | 90.96 | 72.49 | 72.88 | 99.41 | 73.28 | 73.08 |
| 70 Serratia marcescens sicaria (Ss1) | 90.96 | 90.96 | 72.69 | 73.08 | 99.41 | 73.28 | 73.28 |
| 71 Serratia sp. 0LFL2 | 90.76 | 90.76 | 72.69 | 73.08 | 99.60 | 73.47 | 73.28 |

(continued)

| | | | | | | | |
|---|---|---|---|---|---|---|---|
| 72 Serratia marcescens BIDMC 81 | 90.76 | 90.76 | 72.88 | 73.28 | 99.60 | 73.67 | 73.47 |
| 73 Serratia marcescens BIDMC 50 | 90.76 | 90.76 | 72.69 | 73.08 | 99.21 | 73.47 | 73.28 |
| 74 Serratia marcescens UMH7 | 90.56 | 90.56 | 72.88 | 73.28 | 99.80 | 73.67 | 73.47 |
| 75 Serratia marcescens RSC-14 | 90.56 | 90.56 | 72.88 | 73.47 | 99.21 | 73.87 | 73.67 |
| 76 Serratia marcescens SM03 | 92.33 | 92.33 | 72.29 | 72.29 | 93.51 | 72.49 | 72.88 |
| 77 Serratia marcescens 90-166 | 90.17 | 89.78 | 72.49 | 73.47 | 96.66 | 73.67 | 73.08 |
| 78 Serratia marcescens UMH2 | 90.76 | 90.76 | 72.88 | 73.28 | 99.21 | 73.67 | 73.47 |
| 79 Serratia plymuthica AS9 | 72.49 | 71.90 | 96.66 | 85.06 | 73.47 | 86.05 | 83.69 |
| 80 Serratia plymuthica tumat 205 | 72.69 | 72.10 | 98.03 | 86.24 | 73.47 | 86.64 | 84.28 |
| 81 Serratia plymuthica A30 | 72.29 | 71.70 | 98.82 | 85.65 | 72.88 | 86.44 | 84.08 |
| 82 Serratia plymuthica 4Rx13 | 72.29 | 71.70 | 97.83 | 85.85 | 73.08 | 86.44 | 84.28 |
| 83 Serratia plymuthica V4 | 72.29 | 71.70 | 98.42 | 85.85 | 73.08 | 86.44 | 84.28 |
| 84 Serratia plymuthica 3Rp8 | 72.29 | 71.70 | 98.62 | 86.05 | 73.08 | 86.64 | 84.08 |
| 85 Serratia proteamaculans MFPA44A14 | 72.29 | 71.90 | 87.03 | 92.53 | 73.28 | 98.82 | 87.22 |
| 86 Serratia plymuthica A153 | 72.10 | 71.51 | 99.21 | 86.05 | 72.88 | 86.64 | 84.47 |

[Table 3-1]

| [Match Count/Length] | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | * | | | | | | |
| 2 Serratia nenatodiphila DSM21420 | 500/509 | * | | | | | |
| 3 Serratia plymuthica NBRC102599 | 367/509 | 364/509 | * | | | | |
| 4 Serratia proteamaculans 568 | 368/509 | 364/609 | 439/509 | * | | | |
| 5 Serratia ureilytica Lr5/4 | 462/509 | 462/509 | 371/509 | 373/509 | * | | |
| 6 Serratia sp. BW106 | 368/509 | 366/509 | 443/509 | 470/509 | 375/509 | * | |
| 7 Serratia liquefaciens FK01 | 368/50 | 9 365/509 | 431/509 | 442/509 | 374/509 | 447/509 | * |
| 8 Serratia sp. S119 | 483/509 | 480/509 | 371/509 | 369/509 | 466/509 | 372/509 | 371/509 |
| 9 Serratia sp. YD25 | 470/509 | 470/509 | 369/509 | 369/509 | 476/509 | 370/509 | 371/509 |
| 10 Serratia sp. FS14 | 502/509 | 507/509 | 365/509 | 365/509 | 464/509 | 367/509 | 367/509 |
| 11 Serratia sp. HMSC15F11 | 483/509 | 480/509 | 373/506 | 373/509 | 485/509 | 374/509 | 374/509 |
| 12 Serratia sp. JKS000199 | 482/509 | 462/509 | 370/506 | 372/509 | 506/509 | 374/509 | 373/509 |
| 13 Serratia sp. TEL | 461/509 | 461/509 | 371/509 | 373/509 | 508/509 | 375/509 | 374/509 |
| 14 Serratia sp. ISTD04 | 461/509 | 461/509 | 369/509 | 372/509 | 506/509 | 374/509 | 373/509 |
| 15 Serratia sp. SCBI | 462/509 | 462/509 | 371/509 | 373/509 | 507/509 | 374/509 | 374/509 |
| 16 Serratia sp. S4 | 367/509 | 363/509 | 440/509 | 502/509 | 372/509 | 468/509 | 441/509 |
| 17 Serratia sp. C-1 | 369/509 | 366/509 | 499/509 | 438/509 | 373/509 | 441/509 | 428/509 |
| 18 Serratia marcescens 532 | 508/506 | 499/509 | 368/509 | 367/509 | 461/509 | 367/509 | 367/509 |
| 19 Serratia marcescens 2880STDY5683033 | 507/509 | 498/509 | 367/509 | 368/509 | 460/509 | 367/509 | 368/509 |

(continued)

| [Match Count/Length] | 1 Serratia | 2 Serratia | 3 Serratia | 4 Serratia | 5 Serratia | 6 Serratia | 7 Serratia |
|---|---|---|---|---|---|---|---|
| 20 Serratia marcescens WW4 | 501/509 | 506/509 | 366/509 | 366/509 | 463/509 | 368/509 | 366/509 |
| 21 Serratia marcescens K27 | 500/509 | 506/509 | 363/509 | 363/509 | 463/509 | 365/509 | 365/509 |
| 22 Serratia marcescens 280 | 501/509 | 506/509 | 365/509 | 365/509 | 463/509 | 367/509 | 367/509 |
| 23 Serratia marcescens 19F | 501/509 | 506/509 | 364/509 | 365/509 | 463/509 | 367/509 | 367/509 |
| 24 Serratia marcescens 1185 | 500/509 | 507/509 | 383/509 | 363/509 | 460/509 | 365/509 | 364/509 |

[Table 3-2]

| | | | | | | | |
|---|---|---|---|---|---|---|---|
| 25 Serratia marcescens S217 | 500/509 | 505/509 | 363/509 | 364/509 | 463/509 | 366/509 | 366/509 |
| 26 Serratia marcescens KHCo-24B | 499/509 | 508/509 | 363/509 | 363/509 | 461/509 | 365/509 | 366/509 |
| 27 Serratia marcescens Z6 | 499/509 | 504/509 | 385/509 | 366/509 | 461/509 | 368/509 | 366/509 |
| 28 Serratia marcescens 546 | 498/509 | 506/509 | 364/509 | 365/509 | 460/509 | 367/509 | 365/509 |
| 29 Serratia nematodiphila MB307 | 499/509 | 508/509 | 363/609 | 364/509 | 461/509 | 366/509 | 365/509 |
| 30 Serratia marcescens VGH107 | 499/509 | 504/509 | 363/509 | 364/509 | 461/509 | 366/509 | 366/509 |
| 31 Serratia marcescens MC8 | 486/509 | 486/509 | 368/509 | 370/509 | 464/509 | 371/509 | 370/509 |
| 32 Serrata marcescens AH0650 | 487/509 | 486/509 | 368/509 | 370/509 | 462/509 | 373/509 | 370/509 |
| 33 Serratia marcescens UMH12 | 486/509 | 485/509 | 367/509 | 369/509 | 461/509 | 372/509 | 369/509 |
| 34 Serratia sp. OMLW3 | 486/509 | 486/509 | 368/509 | 369/509 | 462/509 | 373/509 | 370/509 |
| 35 Serratia marcescens UMH11 | 485/509 | 484/509 | 367/509 | 370/509 | 461/509 | 374/509 | 369/509 |
| 36 Serratia marcescens UMH1 | 484/509 | 483/509 | 368/509 | 369/509 | 459/509 | 372/509 | 368/509 |
| 37 Serratia marcescens 2880STDY5683020 | 486/509 | 483/509 | 372/509 | 370/509 | 469/509 | 373/509 | 372/509 |
| 38 Serratia marcescens 99 | 486/509 | 482/509 | 373/509 | 371/509 | 466/509 | 375/509 | 373/509 |
| 39 Serratia marcescens 374 | 483/509 | 482/509 | 368/509 | 368/509 | 459/509 | 372/509 | 368/509 |
| 40 Serratia marcescens 2880STDY5683036 | 485/509 | 481/509 | 372/509 | 370/509 | 465/509 | 374/509 | 372/509 |
| 41 Serratia, marcescens 2880STDY5683034 | 485/509 | 482/509 | 372/509 | 370/509 | 468/509 | 373/509 | 372/509 |
| 42 Serratia marcescens 2880STDY5682892 | 485/509 | 482/509 | 373/509 | 371/509 | 468/509 | 374/509 | 373/509 |
| 43 Serratia marcescens SM39 | 484/509 | 481/509 | 373/509 | 370/509 | 469/509 | 373/509 | 373/509 |
| 44 Serratia marcescens 189 | 484/509 | 481/509 | 373/509 | 371/509 | 469/509 | 374/509 | 373/509 |
| 45 Serratia marcescens SMB2099 | 484/509 | 481/509 | 374/509 | 370/509 | 467/509 | 375/509 | 374/509 |
| 46 Serratia marcescens 2880STDY5682862 | 483/509 | 480/509 | 374/509 | 371/509 | 466/509 | 374/509 | 374/509 |
| 47 Serratia marcescens SE4145 | 483/509 | 480/509 | 372/509 | 369/509 | 468/509 | 372/509 | 372/509 |

(continued)

| | | | | | | | |
|---|---|---|---|---|---|---|---|
| 48 Serratia, marcescens 2880STDY5682876 | 484/509 | 481/509 | 373/509 | 371/509 | 467/509 | 374/509 | 373/509 |
| 49 Serratia marcescens 709 | 484/509 | 481/509 | 372/509 | 370/509 | 467/509 | 373/509 | 372/509 |
| 50 Serratia marcescens MGH136 | 483/509 | 480/509 | 371/509 | 369/509 | 468/509 | 372/509 | 371/509 |
| 51 Serratia, marcescens 2880STDY5682884 | 482/509 | 479/509 | 371/509 | 369/509 | 467/509 | 372/509 | 372/509 |
| 52 Serratia marcescens D-3 | 484/509 | 481/509 | 372/509 | 370/509 | 467/509 | 373/509 | 372/509 |
| 53 Serratia marcescens 2880STDY5682957 | 483/509 | 480/509 | 371/509 | 370/509 | 466/509 | 373/509 | 371/509 |
| 54 Serratia marcescens YDC563 | 482/509 | 479/509 | 371/509 | 370/509 | 465/509 | 313/509 | 371/509 |
| 55 Serratia marcescens 2880STDY5683035 | 483/509 | 480/509 | 372/509 | 370/509 | 466/509 | 373/509 | 372/509 |

[Table 3-3]

| | | | | | | | |
|---|---|---|---|---|---|---|---|
| 56 Serratia marcescens 2880STDY5682930 | 482/509 | 479/509 | 371/509 | 369/509 | 465/509 | 372/509 | 371/509 |
| 57 Serratia marcescens 790 | 481/509 | 480/509 | 373/509 | 371/509 | 466/509 | 374/509 | 373/509 |
| 58 Serratia marcescens UMH5 | 476/509 | 473/509 | 370/509 | 371/509 | 460/509 | 370/509 | 369/509 |
| 59 Serratia marcescens 2880STDY5682988 | 475/509 | 472/509 | 370/509 | 371/509 | 460/509 | 370/509 | 369/509 |
| 60 Serratia marcescens 945154301 | 483/509 | 480/509 | 373/509 | 373/509 | 465/509 | 375/509 | 374/509 |
| 61 Serratia marcescens at10508 | 482/509 | 479/509 | 374/509 | 374/509 | 464/509 | 375/509 | 375/509 |
| 62 Serratia marcescens ML2637 | 481/509 | 478/509 | 373/509 | 374/509 | 463/509 | 375/509 | 375/509 |
| 63 Serratia mrcescens SM1978 | 480/509 | 477/509 | 373/509 | 373/509 | 462/509 | 375/509 | 375/509 |
| 64 Serratia marcescens PWN146 | 479/509 | 476/509 | 371/509 | 371/509 | 463/509 | 371/509 | 373/509 |
| 65 Serratia marcescens H1q | 471/509 | 471/509 | 369/509 | 369/509 | 476/509 | 370/509 | 372/509 |
| 66 Serratia marcescens UMH6 | 464/509 | 464/509 | 370/609 | 372/509 | 507/509 | 374/509 | 373/509 |
| 67 Serratia nematodiphila WCU338 | 464/509 | 464/509 | 370/509 | 372/509 | 506/509 | 374/509 | 373/509 |
| 68 Serratia sp. OLEL1 | 463/509 | 463/509 | 371/509 | 373/509 | 508/509 | 375/509 | 374/509 |
| 69 Serratia marcescens 7209 | 463/509 | 463/509 | 369/509 | 371/509 | 506/509 | 373/509 | 372/509 |
| 70 Serratia marcescens sicaria (Ss1) | 463/509 | 463/509 | 370/509 | 372/509 | 506/509 | 373/509 | 373/509 |
| 71 Serratia sp. OLFL2 | 462/509 | 462/509 | 370/509 | 372/509 | 507/509 | 374/509 | 373/509 |
| 72 Serratia marcescens BIDMC 81 | 462/509 | 462/509 | 371/509 | 373/509 | 507/509 | 375/509 | 374/509 |
| 73 Serratia marcescens BIDMC 50 | 462/509 | 462/509 | 370/509 | 372/509 | 505/509 | 374/509 | 373/509 |
| 74 Serratia marcescens UMH7 | 461/509 | 461/509 | 371/509 | 373/509 | 508/509 | 375/509 | 374/509 |
| 75 Serratia marcescens RSC-14 | 461/509 | 461/509 | 371/509 | 374/509 | 505/509 | 376/509 | 375/509 |
| 76 Serratia marcescens SM03 | 470/509 | 470/509 | 368/509 | 368/509 | 476/509 | 369/509 | 371/509 |
| 77 Serratia marcescens 90-166 | 459/509 | 457/509 | 369/509 | 374/509 | 492/509 | 375/509 | 372/509 |
| 78 Serratia marcescens UMH2 | 462/509 | 462/509 | 371/509 | 373/509 | 505/509 | 375/509 | 374/509 |

(continued)

| | | | | | | | |
|---|---|---|---|---|---|---|---|
| 79 Serratia plymuthica AS9 | 369/509 | 368/509 | 492/509 | 433/509 | 374/509 | 438/509 | 426/509 |
| 80 Serratia plymuthica tumat 205 | 370/509 | 367/509 | 499/509 | 439/509 | 374/509 | 441/509 | 429/509 |
| 81 Serratia plymuthica A30 | 368/509 | 365/509 | 503/509 | 436/509 | 371/509 | 440/509 | 428/509 |
| 82 Serratia plymuthica 4Rx13 | 368/509 | 365/509 | 408/509 | 437/509 | 372/509 | 440/509 | 429/509 |
| 83 Serratia plymuthica V4 | 368/509 | 365/509 | 501/509 | 437/509 | 372/509 | 440/509 | 429/509 |
| 84 Serratia plymuthica 3Rp8 | 368/509 | 366/509 | 502/509 | 438/509 | 372/509 | 441/509 | 428/509 |
| 85 Serratia proteamaculans MFPA44A14 | 368/509 | 366/509 | 443/509 | 471/509 | 373/509 | 503/509 | 444/509 |
| 86 Serratia plymuthica A153 | 367/509 | 364/509 | 505/509 | 438/509 | 371/509 | 441/509 | 430/509 |

The nucleic acids encoding the polypeptides described in (a) to (c) according to the present invention may contain an additional sequence that encodes a peptide or protein added to the original polypeptides at the N terminus and/or the C terminus. Examples of such a peptide or protein can include secretory signal sequences, translocation proteins, binding proteins, peptide tags for purification, and fluorescent proteins. Among those peptides or proteins, a peptide or protein with a desired function can be selected depending on the purpose and can be added to the polypeptides of the present invention by those skilled in the art. It should be noted that the amino acid sequence of such a peptide or protein is excluded from the calculation of sequence identity.

The nucleic acids encoding the polypeptides represented by SEQ ID NOs: 1 to 86 are not specifically limited, provided that the nucleic acids have nucleotide sequences that can be translated to the amino acid sequences represented by SEQ ID NOs: 1 to 86, and the nucleotide sequences can be determined considering the set of codons (standard genetic code) corresponding to each amino acid. In this respect, the nucleotide sequences may be redesigned using codons that are frequently used by a host microorganism used in the present invention.

Specific examples of the nucleotide sequences of the nucleic acids that encode the polypeptides with the amino acid sequences represented by SEQ ID NOs: 1 to 86 include the nucleotide sequences represented by SEQ ID NOs: 87 to 172.

In the present invention, whether or not a polypeptide encoded by a certain nucleic acid has 3-oxoadipyl-CoA reductase activity is determined as follows: transformants A and B below are produced and grown in a culture test; if 3-hydroxyadipic acid or α-hydromuconic acid is confirmed in the resulting culture medium, it is judged that the nucleic acid encodes a polypeptide having 3-oxoadipyl-CoA reductase activity. The determination method will be described using the scheme 1 below which shows a biosynthesis pathway.

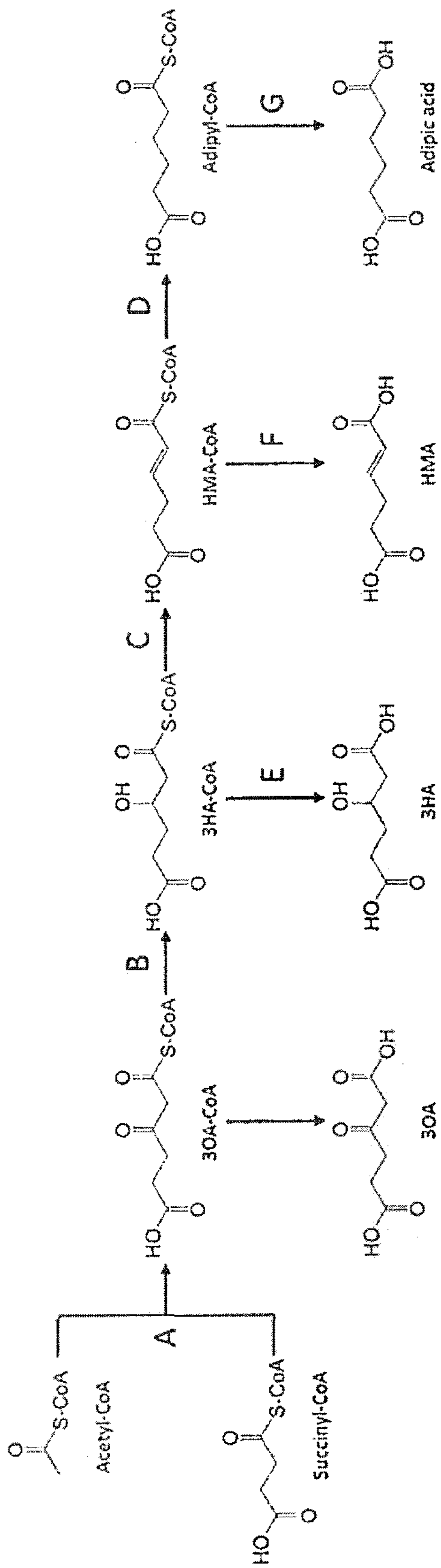


Acetyl-CoA
Succinyl-CoA
3OA-CoA
3OA
3HA-CoA
3HA
HMA-CoA
HMA
Adipyl-CoA
Adipic acid

Scheme 1

The above scheme 1 shows an exemplary reaction pathway required for the production of 3-hydroxyadipic acid, α-hydromuconic acid, and/or adipic acid. In this scheme, the reaction A represents a reaction that generates 3-oxoadipyl-CoA and coenzyme A from acetyl-CoA and succinyl-CoA. The reaction B represents a reaction that generates

3-hydroxyadipyl-CoA from 3-oxoadipyl-CoA. The reaction C represents a reaction that generates 2,3-dehydroadipyl-CoA from 3-hydroxyadipyl-CoA. The reaction D represents a reaction that generates adipyl-CoA from 2,3-dehydroadipyl-CoA. The reaction E represents a reaction that generates 3-hydroxyadipic acid from 3-hydroxyadipyl-CoA. The reaction F represents a reaction that generates α-hydromuconic acid from 2,3-dehydroadipyl-CoA. The reaction G represents a reaction that generates adipic acid from adipyl-CoA.

The transformant A has enzymes that catalyze the reactions A, E, and F. The transformant B has enzymes that catalyze the reactions A, C, E, and F.

The transformant A is first produced. Plasmids for the expression of the enzymes that catalyze the reactions A, E, and F, respectively, are produced. The reactions E and F can be catalyzed by an identical enzyme. The plasmids are introduced into *Escherichia coli* strain BL21 (DE3), which is a microorganism strain lacking abilities to produce all of 3-hydroxyadipic acid, α-hydromuconic acid, and adipic acid. Into the obtained transformant, an expression plasmid carrying a nucleic acid that encodes a polypeptide to be analyzed for the presence of the enzymatic activity of interest and is integrated downstream of an appropriate promoter is introduced to obtain the transformant A. The transformant A is cultured, and the post-culture fluid is examined for the presence of 3-hydroxyadipic acid. Once the presence of 3-hydroxyadipic acid in the culture fluid is confirmed, the transformant B is then produced. The transformant B is obtained by producing a plasmid for the expression of an enzyme that catalyzes the reaction C and introducing the resulting plasmid into the transformant A. The transformant B is cultured, and the post-culture fluid is examined for the presence of α-hydromuconic acid. When the presence of α-hydromuconic acid in the post-culture fluid is confirmed, it indicates that 3-hydroxyadipic acid produced in the transformant A and α-hydromuconic acid produced in the transformant B are generated via production of 3-hydroxyadipyl-CoA, and that the polypeptide of interest has 3-oxoadipyl-CoA reductase activity.

As the gene encoding the enzyme that catalyzes the reaction A, *pcaF* from *Pseudomonas putida* strain KT2440 (NCBI Gene ID: 1041755; SEQ ID NO: 174) is used.

As the genes encoding the enzyme that catalyzes the reactions E and F, a continuous sequence including the full lengths of *pcaI* and *pcaJ* from *Pseudomonas putida* strain KT2440 (NCBI Gene IDs: 1046613 and 1046612; SEQ ID NOs: 175 and 176) is used. The polypeptides encoded by *pcaI* and *pcaJ* forms a complex and then catalyze the reactions E and F.

As the nucleic acid encoding the enzyme that catalyzes the reaction C, the *paaF* gene from *Pseudomonas putida* strain KT2440 (NCBI Gene ID: 1046932, SEQ ID NO: 177) is used.

The method of culturing the transformant A and the transformant B is as follows. Antibiotics for stable maintenance of the plasmids and inducer substances for induction of expression of the polypeptides encoded by the incorporated nucleic acids may be added as appropriate to the culture. A loopful of either the transformant A or B is inoculated into 5 mL of the culture medium I (10 g/L Bacto Tryptone (manufactured by Difco Laboratories), 5 g/L Bacto Yeast Extract (manufactured by Difco Laboratories), 5 g/L sodium chloride) adjusted at pH 7 and is cultured at 30°C with shaking at 120 $min^{-1}$ for 18 hours to prepare a preculture fluid. Subsequently, 0.25 mL of the preculture fluid is added to 5 mL of the culture medium II (10 g/L succinic acid, 10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract) adjusted to pH 6.5 and is cultured at 30°C with shaking at 120 $min^{-1}$ for 24 hours. The obtained culture fluid is examined for the presence of 3-hydroxyadipic acid or α-hydromuconic acid.

The presence of 3-hydroxyadipic acid or α-hydromuconic acid in the culture fluid can be confirmed by centrifuging the culture fluid and analyzing the supernatant with LC-MS/MS. The analysis conditions are as described below:

- HPLC: 1290 Infinity (manufactured by Agilent Technologies, Inc.)

  Column: Synergi hydro-RP (manufactured by Phenomenex Inc.), length: 100 mm, internal diameter: 3 mm, particle size: 2.5 μm
  Mobile phase: 0.1% aqueous formic acid solution / methanol = 70/30
  Flow rate: 0.3 mL/min
  Column temperature: 40°C
  LC detector: DAD (210 nm)

- MS/MS: Triple-Quad LC/MS (manufactured by Agilent Technologies, Inc.) Ionization method: ESI in negative mode.

The 3-oxoadipyl-CoA reductase activity value can be calculated by quantifying 3-hydroxyadipyl-CoA generated from 3-oxoadipyl-CoA used as a substrate by using purified 3-oxoadipyl-CoA reductase, wherein the 3-oxoadipyl-CoA is prepared from 3-oxoadipic acid by an enzymatic reaction. The specific method is as follows.

3-Oxoadipic acid can be prepared by a known method (for example, a method described in Reference Example 1 of WO 2017/099209).

Preparation of 3-oxoadipyl-CoA solution: A PCR using the genomic DNA of *Pseudomonas putida* strain KT2440 as a template is performed in accordance with routine procedures, to amplify a nucleic acid encoding a CoA transferase (*pcaI* and *pcaJ*; NCBI-GeneIDs: 1046613 and 1046612) in the full-length form. The nucleotide sequences of primers used in this PCR are, for example, those represented by SEQ ID NOs: 194 and 195. The amplified fragment is inserted into the *Kpn*I site of pRSF-1b (manufactured by Novagen), an expression vector for *E. coli*, in-frame with the histidine-tag sequence. The plasmid is introduced into *E. coli* BL21 (DE3), and the enzyme is expressed from the plasmid under isopropyl-β-thiogalactopyranoside (IPTG) induction and is then purified using the histidine tag from the culture fluid in accordance with routine procedures to obtain a CoA transferase solution. The solution is used to prepare an enzymatic reaction solution for 3-oxoadipyl-CoA preparation with the following composition, and the enzymatic reaction solution is kept at 25°C for 3 minutes to allow the reaction to proceed and is then filtered through a UF membrane (Amicon Ultra-0.5mL 10K; manufactured by Merck Millipore) to remove the enzyme, and the obtained filtrate is designated as 3-oxoadipyl-CoA solution.

(Enzymatic Reaction Solution)

100 mM Tris-HCl (pH 8.2)
10 mM $MgCl_2$
0.5 mM succinyl-CoA
5 mM 3-oxoadipic acid sodium salt
2 μM CoA transferase.

Identification of 3-oxoadipyl-CoA reductase activity: A PCR using the genomic DNA of a microorganism strain as a template is performed in accordance with routine procedures, to amplify a nucleic acid encoding 3-oxoadipyl-CoA reductase in the full-length form. The nucleotide sequences of primers used in this PCR are, for example, those represented by SEQ ID NOs: 196 and 197. The amplified fragment is inserted into the *Bam*HI site of pACYCDuet-1 (manufactured by Novagen), an expression vector for *E. coli*, in-frame with the histidine-tag sequence. The plasmid is introduced into *E. coli* BL21 (DE3), and the enzyme is expressed from the plasmid under isopropyl-p-thiogalactopyranoside (IPTG) induction and is then purified using the histidine tag from the culture fluid in accordance with routine procedures to obtain a 3-oxoadipyl-CoA reductase solution. The 3-oxoadipyl-CoA reductase activity can be determined by using the enzyme solution to prepare an enzymatic reaction solution with the following composition and quantifying 3-hydroxyadipyl-CoA generated at 25°C.

(Enzymatic Reaction Solution)

100 mM Tris-HCl (pH 8.2)
10 mM $MgCl_2$
150 μL/mL 3-oxoadipyl-CoA solution
0.5 mM NADH
1 mM dithiothreitol
10 μM 3-oxoadipyl-CoA reductase.

In the present invention, the genetically modified microorganism in which the expression of any one of the polypeptides described in (a) to (c) is enhanced is a microorganism as a host which originally has a nucleic acid encoding any one of the polypeptides described in (a) to (c) and is genetically modified for increased expression of any one of the polypeptides described in (a) to (c) which are owned by the host microorganism.

Specific examples of the microorganism which originally has a nucleic acid encoding any one of the polypeptides described in (a) to (c) include the following microorganisms of the genus *Serratia,* including *Serratia marcescens* (a microorganism having the sequences represented by SEQ ID NOs: 1, 18 to 28, 30 to 33, 35 to 66, 69, 70, 72 to 78, and 79), *Serratia nematodiphila* (a microorganism having the sequences represented by SEQ ID NOs: 2, 29, and 67), *Serratia plymuthica* (a microorganism having the sequences represented by SEQ ID NOs: 3, 79 to 84, and 86), *Serratia proleamaculans* (a microorganism having the sequences represented by SEQ ID NOs: 4 and 85), *Serratia ureilytica* (a microorganism having the sequence represented by SEQ ID NO: 5), *Serratia* sp. BW106 (a microorganism having the sequence represented by SEQ ID NO: 6), *Serratia liquefaciens* (a microorganism having the sequence represented by SEQ ID NO: 7), *Serratia* sp. S119 (a microorganism having the sequence represented by SEQ ID NO: 8), *Serratia* sp. YD25 (a microorganism having the sequence represented by SEQ ID NO: 9), *Serratia* sp. FS14 (a microorganism having the sequence represented by SEQ ID NO: 10), *Serratia* sp. HMSC15F11 (a microorganism having the sequence represented by SEQ ID NO: 11), *Serratia* sp. JKS000199 (a microorganism having the sequence represented by SEQ ID NO: 12), *Serratia* sp. TEL (a microorganism having the sequence represented by SEQ ID NO: 13), *Serratia* sp. ISTD04 (a microorganism having the sequence represented by SEQ ID NO: 14), *Serratia* sp. SCBI (a microorganism having the sequence represented by SEQ ID NO: 15), *Serratia* sp. S4 (a microorganism having the sequence represented by SEQ ID NO: 16), *Serratia* sp. C-1 (a microorganism having the sequence represented by SEQ ID NO: 17), *Serratia* sp. OMLW3 (a microorganism having the sequence represented by SEQ ID NO: 34), *Serratia* sp. OLEL1 (a microorganism having the sequence represented by SEQ ID NO: 68), *Serratia* sp. OLEL2 (a microorganism having the sequence represented by SEQ ID NO: 71), and the like.

Each of the polypeptides as described above in (a), (b), and (c) also has 3-hydroxybutyryl-CoA dehydrogenase activity, and the 3-hydroxybutyryl-CoA dehydrogenase is encoded by a 3-hydroxybutyryl-CoA dehydrogenase gene, which forms a gene cluster with the 5-aminolevulinic acid synthase gene in the microorganisms of the genus *Serratia.*

As used herein, the term "gene cluster" in the phrase "the 3-hydroxybutyryl-CoA dehydrogenase gene, which forms a gene cluster with 5-aminolevulinic acid synthase gene in the microorganisms of the genus *Serratia*" refers to a region in which a set of nucleic acids encoding proteins with related functions are located in close proximity to each other. Specific components in a gene cluster include, for example, nucleic acids which are transcribed under the control of a single transcription regulator, and those in an operon which are transcribed under the control of a single transcription promoter. Whether or not a certain nucleic acid is a nucleic acid component of a gene cluster can also be investigated using an online gene cluster search program, such as antiSMASH. Additionally, whether or not a certain polypeptide is classified as a 3-hydroxybutyryl-CoA dehydrogenase or a 5-aminolevulinic acid synthase can be determined by BLAST (Basic Local Alignment Search Tool) searching on a website, such as that of NCBI (National Center for Biotechnology Information) or KEGG (Kyoto Encyclopedia of Genes and Genomes), to find any enzyme with a high degree of homology to the polypeptide in amino acid sequence. For example, the amino acid sequence represented by SEQ ID NO: 4 is registered in an NCBI database under Protein ID: ABV40935.1, which is annotated as a putative protein with 3-hydroxybutyryl-CoA dehydrogenase activity, as judged from the amino acid sequence. A gene encoding the amino acid sequence represented by SEQ ID NO: 4 is registered in an NCBI database under Gene ID: CP000826.1 and can be identified through a database search as conserved in the genome of *Serratia proteamaculans* strain 568 or as conserved in the region from 2015313 to 2016842 bp on the sequence of Gene ID: CP000826.1. Furthermore, the positional information of the gene can lead to identification of the sequences of flanking genes, from which the gene can be found to form a gene cluster with the 5-aminolevulinic acid synthase gene (Protein ID: ABV40933.1), as shown in FIG. 1. Similarly, for the amino acid sequences represented by SEQ ID NOs: 1 to 3, 6 to 20, 22 to 30, 32 to 35, 37, 38, 40, 42 to 48, 51 to 56, 59 to 63, 65, 66, 68 to 73, 75 to 81, and 83 to 85, the information can be checked on the NCBI site with the Protein IDs and Gene IDs presented in Tables 3-4 and 3-5.

[Table 3-4]

| SEQ ID NO: | Gene ID: position (from..to) | Protein ID |
|---|---|---|
| 1 | JMPQ01000047.1:133194..134723 | KFD11732.1 |
| 2 | JPUX00000000.1:4202615..4204144 | WP 033633399.1 |
| 3 | BCTU01000013.1:85647..87176 | WP 063199278.1 |
| 4 | CP000826.1:2015313..2016842 | ABV40935.1 |
| 6 | MCGS01000002.1:43811..45340 | WP 099061672.1 |
| 7 | CP006252.1:1825868..1827397 | AGQ30498.1 |
| 8 | MSFH01000022.1:147976..149505 | ONK16968.1 |
| 9 | CP016948.1:1213474..1215003 | AOE98783.1 |
| 10 | CP005927.1 :4244665..4246194 | WP 044031504.1 |
| 11 | LWNG01000196.1:83086..84615 | OFS85208.1 |
| 12 | LT907843.1:1172733..1174262 | SNY82966.1 |
| 13 | LDEG01000005.1:19627..21156 | KLE40298.1 |

(continued)

| SEQ ID NO: | Gene ID: position (from..to) | Protein ID |
|---|---|---|
| 14 | MBDW01000089.1 :53478..55007 | ODJ15373.1 |
| 15 | CP003424.1:1869825..1871300 | AIM21329.1 |
| 16 | APLA01000003.1:1964823..1966352 | WP 017892361.1 |
| 17 | CAQO01000118.1:101692..103221 | WP 062792820.1 |
| 18 | JVDI01000070.1:19399..20928 | WP 049300487.1 |
| 19 | FCGF01000001.1:938090..939619 | WP 060444298.1 |
| 20 | NC 020211.1:1963542..1965071 | WP 015377392.1 |
| 22 | JVNC01000043.1 :47711..49240 | WP 049187553.1 |
| 23 | MCNK01000010.1:591271..592800 | WP 076740355.1 |
| 24 | JVZV01000138.1 :53080..54609 | WP 049277247.1 |
| 25 | CP021984.1:1963542..1965071 | WP 088381461.1 |
| 26 | NERL01000025.1:86571..88100 | WP 060559176.1 |
| 27 | MTEH01000001.1:215863..217392 | WP 085336366.1 |
| 28 | JVCS01000001.1:19397..20926 | WP_049239700.1 |
| 29 | MTBJ01000002.1 :216232..217761 | WP 082996863.1 |
| 30 | AORJ01000010.1:70272..71801 | WP 033645451.1 |
| 32 | LFJS01000012.1:944087..945616 | WP 025302345.1 |
| 33 | CP018930.1:1161338..1162867 | WP 060447438.1 |
| 34 | MSTK01000013.1:54046..55575 | WP 099817374.1 |
| 35 | CP018929.1 :1167577..1170106 | WP 089180755.1 |
| 37 | FCGS01000006.1:98915..100444 | WP 060438851.1 |
| 38 | MQRI01000002.1:585500..587029 | WP 060387554.1 |
| 40 | FCFE01000001.1:962839..964368 | WP 060435888.1 |
| 42 | FCI001000002.1:145369146898.. | WP 033637938.1 |
| 43 | AP013063.1:1329259..1330788 | WP 041034581.1 |
| 44 | MQRJ01000O04.1:178926..180455 | WP 074026553.1 |
| 45 | HG738868.1:1928329..1929858 | WP 060437960.1 |

[Table 3-5]

| SEQ ID NO: | Gene ID: position (from..to) | Protein ID |
|---|---|---|
| 46 | FCHQ01000006.1:51377..52906 | WP 060420535.1 |
| 47 | NPGG01000001.1:301231..302760 | WP 047568134.1 |
| 48 | FCME01000002.1:205632..207161 | WP 060443161.1 |
| 51 | FCIH01000014.1:52403..53932 | WP 060429049.1 |
| 52 | NBWV01000007.1:110621..112150 | WP 039566649.1 |
| 53 | FCKI01000001.1:594106..595635 | WP 060429902.1 |
| 54 | JPOB01000010.1:81351..82880 | WP 033654196.1 |

(continued)

| SEQ ID NO: | Gene ID: position (from..to) | Protein ID |
|---|---|---|
| 55 | FCFI01000001.1:582222..583751 | WP 060443342.1 |
| 56 | FCML01000001.1:1005802.. 1007331 | WP 060456892.1 |
| 59 | FCMR01000001.1:1873566..1875095 | WP 060440240.1 |
| 60 | LJEV02000002.1:115432..116961 | WP 047727865.1 |
| 61 | NP1X01000027.1 :38249..39778 | WP 094461128.1 |
| 62 | NDXU01000091.1:70343..71872 | WP 048233299.1 |
| 63 | FNXW01000055.1:13619..15148 | WP 080490898.1 |
| 65 | AYM001000023.1:23978..25507 | WP 025160335.1 |
| 66 | CP018926.1:1215941..1217470 | WP 089191486.1 |
| 68 | MORG01000026.1:13723..15252 | WP 099782744.1 |
| 69 | PEHC01000008.1:57274..58803 | PHY81681.1 |
| 70 | MEDA01000063.1:13491..15020 | WP 072627918.1 |
| 71 | MORII01000030.1:13633..15162 | WP 099789708.1 |
| 72 | KK214286.1:392757..394286 | WP 033650708.1 |
| 73 | KI929259.1:1574567..1576096 | WP 033642621.1 |
| 75 | CP012639.1:230596..232125 | WP 060659686.1 |
| 76 | LZOB01000011.1:1613417..1614946 | WP 074054551.1 |
| 77 | LCWI01000024.1:46336..47865 | WP 046899223.1 |
| 78 | CP018924.1:1213305..1214834 | WP 089194521.1 |
| 79 | NC 015567.1:1930552..1932081 | WP 013812379.1 |
| 80 | MQML01000205.1:9362..10891 | WP 073439751.1 |
| 81 | AMSV01000032.1:251478..253007 | WP 006324610.1 |
| 83 | CP007439.1:1991332..1992861 | AHY06789.1 |
| 84 | CP012096.1:319897..321426 | WP 037432641.1 |
| 85 | FWWG01000018.1:38528..40057 | WP 085116175.1 |

A nucleic acid encoding a polypeptide encoded by the 3-hydroxybutyryl-CoA dehydrogenase gene of a microorganism of the genus *Serratia*, which forms a gene cluster with the 5-aminolevulinic acid synthase gene, is hereinafter referred to as "the 3-hydroxybutyryl-CoA dehydrogenase gene used in the present invention," and the polypeptide encoded by the 3-hydroxybutyryl-CoA dehydrogenase gene is referred as "the 3-hydroxybutyryl-CoA dehydrogenase used in the present invention."

A gene cluster including the 3-hydroxybutyryl-CoA dehydrogenase gene used in the present invention may include other nucleic acids, provided that the gene cluster includes at least the 3-hydroxybutyryl-CoA dehydrogenase gene and the 5-aminolevulinic acid synthase gene. FIG. 1 shows a specific example of the gene cluster including the 3-hydroxybutyryl-CoA dehydrogenase gene used in the present invention.

Specific examples of the microorganisms of the genus *Serratia* that contain the above gene cluster include *S. marcescens*, *S. nematodiphila*, *S. plymuthica*, *S. proleamaculans*, *S. ureilytica*, *S. liquefaciens*, *Serratia* sp. BW106, *Serratia* sp. S119, *Serratia* sp. YD25, *Serratia* sp. FS14, Serratia sp. HMSC15F11, *Serratia* sp. JKS000199, *Serratia* sp. TEL, *Serratia* sp. ISTD04, Serratia sp. SCBI, *Serratia* sp. S4, *Serratia* sp. C-1, *Serratia* sp. OMLW3, *Serratia* sp. OLEL1, *Serratia* sp. OLEL2, and *S. liquefaciens.*

The 3-hydroxybutyryl-CoA dehydrogenase used in the present invention has an excellent 3-oxoadipyl-CoA reductase activity. Whether or not a 3-hydroxybutyryl-CoA dehydrogenase-encoding nucleic acid has a 3-oxoadipyl-CoA reductase activity can be determined by the same method as described above.

The polypeptide encoded by the 3-hydroxybutyryl-CoA dehydrogenase gene used in the present invention is characterized by containing the common sequence 1. Specific examples of amino acid sequences of such polypeptides include the amino acid sequences represented by SEQ ID NOs: 1 to 86.

In the present invention, a nucleic acid encoding a polypeptide composed of the same amino acid sequence as that represented by any one of SEQ ID NOs: 8 to 86, except that one or several amino acids are substituted, deleted, inserted, and/or added, and having an enzymatic activity that catalyzes a reaction to reduce 3-oxoadipyl-CoA to 3-hydroxyadipyl-CoA can also be suitable for use, provided that the common sequence 1 is contained in the polypeptide. In this respect, the range represented by the phrase "one or several" is preferably 10 or less, more preferably 5 or less, especially preferably 4 or less, and most preferably one or two. In the case of amino acid substitution, the activity of the original polypeptide is more likely to be maintained when an amino acid(s) is/are replaced by an amino acid(s) with similar properties (*i.e.*, conservative substitution as described above). A nucleic acid encoding a polypeptide composed of an amino acid sequence with a sequence identity to not less than 70%, preferably not less than 80%, more preferably not less than 85%, further preferably not less than 90%, still further preferably not less than 95%, yet further preferably not less than 97%, even further preferably not less than 99%, to the sequence represented by any one of SEQ ID NOs: 8 to 86 and having an enzymatic activity that catalyzes a reaction to reduce 3-oxoadipyl-CoA to 3-hydroxyadipyl-CoA can also be suitably used.

On the other hand, examples of a polypeptide that is not the 3-hydroxybutyryl-CoA dehydrogenase used in the present invention but has 3-oxoadipyl-CoA reductase activity include PaaH from *Pseudomonas putida* strain KT2440 (SEQ ID NO: 178), PaaH from *Escherichia coli* strain K-12 substrain MG1655 (SEQ ID NO: 179), DcaH from *Acinetobacter baylyi* strain ADP1 (SEQ ID NO: 180), and PaaH from *Serratia plymuthica* strain NBRC102599 (SEQ ID NO: 181). As shown in Tables 4 and 5, these polypeptides are found not to contain the common sequence 1. It should be noted that those polypeptides are neither (b) polypeptides composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA, nor (c) polypeptides having an amino acid sequence with a sequence identity of not less than 70% to the sequence represented by any one of SEQ ID NOs: 1 to 7 and having an enzymatic activity that catalyzes a reaction to reduce 3-oxoadipyl-CoA to 3-hydroxyadipyl-CoA.

[Table 4]

| | Consensus sequence1 (positions 5 10 15 20 25 30 35 40): GAGTMGRGIAYLXAXXXIXTXLYN |
|---|---|
| 1. 1 Serratia marcescens ATCC13880 | MAESNAAIQSAAIIGAGTMGRGIAYLFAQKGIRTVLYNRN |
| 2. 2 Serratia nematodiphila DSM21420 | MAESNAAIQSAAIIGAGTMGRGIAYLFAQKGIPTMLYNRN |
| 3. 3 Serratia plymuthica NBRC102599 | MAENNSAIRSAAVIGAGTMGRGIAYLLALNGIRTVLYNRN |
| 4. 4 Serratia proteamaculans 568 | MAENNSAIHSVAVIGAGTMGRGIAYLLAQNGIRTLLYNRS |
| 5. 5 Serratia ureilytica Lr5/4 | MAESNAAIQSAAIIGAGTMGRGIAYLLAQKSIRTVLYNRN |
| 6. 6 Serratia sp. BW106 | MAENNSAIHSVAVIGAGTMGRGIAYLLAQNGIRTLLYNRS |
| 7. 7 Serratia liquefaciens FK01 | MAENNTAIDSVAVIGAGTMGRGIAYLLALNGIRTLLYNRN |
| 8. 8 Serratia sp. S119 | MAESNAAIQSAAIIGAGTMGRGIAYLFAQKGIRTVLYNRN |
| 9. 9 Serratia sp. YD25 | MAERNAAIQSAAIIGAGTMGRGIAYLFAQKGIRTVLYNRN |
| 10. 10 Serratia sp. FS14 | MAESNAAIQSAAIIGAGTMGRGIAYLFAQKGIPTMLYNRN |
| 11. 11 Serratia sp. HMSC15F11 | MAESNAAIQSAAIIGAGTMGRGIAYLFAQKGIRTVLYNRN |
| 12. 12 Serratia sp. JKS000199 | MAESNAAIQSAAIIGAGTMGRGIAYLLAQKSIRTVLYNRN |
| 13. 13 Serratia sp. TEL | MAESNAAIQSAAIIGAGTMGRGIAYLLAQKSIRTVLYNRN |
| 14. 14 Serratia sp. ISTD04 | MAESNAAIQSAAIIGAGTMGRGIAYLLAQKSIRTVLYNRN |
| 15. 15 Serratia sp. SCBI | MAESNAAIQSAAIIGAGTMGRGIAYLLAQKSIRTVLYNRN |
| 16. 16 Serratia sp. S4 | MAENNSAIHSVAVIGAGTMGRGIAYLLAQNGIRTLLYNRS |
| 17. 17 Serratia sp. C-1 | MAENNSAIRSAAVIGAGTMGRGIAYLLALNGIRTVLYNRN |
| 18. 18 Serratia marcescens 532 | MAESNAAIQSAAIIGAGTMGRGIAYLFAQKGIRTVLYNRN |
| 19. 19 Serratia marcescens 2880STDY5683033 | MAESNAAIQSAAIIGAGTMGRGIAYLFAQKGIRTVLYNRN |
| 20. 20 Serratia marcescens WW4 | MAESNAAIQSAAIIGAGTMGRGIAYLFAQKGIPTMLYNRN |
| 21. 21 Serratia marcescens K27 | MAESNAAIQSAAIIGAGTMGRGIAYLFAQKGIPTMLYNRN |
| 22. 22 Serratia marcescens 280 | MAESNAAIQSAAIIGAGTMGRGIAYLFAQKGIPTMLYNRN |
| 23. 23 Serratia marcescens 19F | MAESNAAIQSAAIIGAGTMGRGIAYLFAQKGIPTMLYNRN |
| 24. 24 Serratia marcescens 1185 | MAESNAAIQSAAIIGAGTMGRGIAYLFAQKGIPTMLYNRN |
| 25. 25 Serratia marcescens S217 | MAESNAAIQSAAIIGAGTMGRGIAYLFAQKGIPTMLYNRN |
| 26. 26 Serratia marcescens KHCo-24B | MAESNAAIQSAAIIGAGTMGRGIAYLFAQKGIPTMLYNRN |
| 27. 27 Serratia marcescens Z6 | MAESNAAIQSAAIIGAGTMGRGIAYLFAQKGIPTMLYNRN |
| 28. 28 Serratia marcescens 546 | MAESNAAIQSAAIIGAGTMGRGIAYLFAQKGIPTMLYNRN |
| 29. 29 Serratia nematodiphila MB307 | MAESNAAIQSAAIIGAGTMGRGIAYLFAQKGIPTMLYNRN |
| 30. 30 Serratia marcescens VGH107 | MAESNAAIQSAAIIGAGTMGRGIAYLFAQKGIPTMLYNRN |
| 31. 31 Serratia marcescens MCB | MAESNAAIQSAAIIGAGTMGRGIAYLFAQKGIRTVLYNRN |
| 32. 32 Serratia marcescens AH0650 | MAESNAAIQSAAIIGAGTMGRGIAYLFAQKGIRTVLYNRN |
| 33. 33 Serratia marcescens UMH12 | MAESNAEIQSAAIIGAGTMGRGIAYLFAQKGIRTVLYNRN |
| 34. 34 Serratia sp. OMLW3 | MAESNAAIQSAAIIGAGTMGRGIAYLFAQKGIRTVLYNRN |
| 35. 35 Serratia marcescens UMH11 | MAESNAAIQSAAIIGAGTMGRGIAYLFAQKGIRTVLYNRS |
| 36. 36 Serratia marcescens UMH1 | MAESNAAIQSAAIIGAGTMGRGIAYLFAQKGIRTVLYNRN |
| 37. 37 Serratia marcescens 2880STDY5683020 | MAESNAAIQSAAIIGAGTMGRGIAYLFAQKGIRTVLYNRN |
| 38. 38 Serratia marcescens 99 | MAESNAAIQSAAIIGAGTMGRGIAYLFAQKGIRTVLYNRN |
| 39. 39 Serratia marcescens 374 | MAESNAAIQSAAIIGAGTMGRGIAYLFAQKGIRTVLYNRN |
| 40. 40 Serratia marcescens 2880STDY5683036 | MAESNAAIQSAAIIGAGTMGRGIAYLFAQKGIRTVLYNRN |
| 41. 41 Serratia marcescens 2880STDY5683034 | MAESNAAIQSAAIIGAGTMGRGIAYLFAQKGIRTVLYNRN |
| 42. 42 Serratia marcescens 2880STDY5682892 | MAESNAAIQSAAIIGAGTMGRGIAYLFAQKGIRTVLYNRN |
| 43. 43 Serratia marcescens SM39 | MAESNAAIQSAAIIGAGTMGRGIAYLFAQKGIRTVLYNRN |

[Table 5]

| | | | | 5 10 15 20 25 30 35 40<br>Consensus sequencel<br>GAGTMGRGIAYLXAXXXIXTXLYN |
|---|---|---|---|---|
| 44. | 44 | Serratia marcescens | 189 | MAESNAA QSAA IGAG MGRG I AYLFAQKG I RTVLYHRM |
| 45. | 45 | Serrat a marcescens | SMB2099 | MAESNAA QSAA GAG MGRG I AYLFAQKG I R VLYNRN |
| 46. | 46 | Serrat a marcescens | 2880STDY5882862 | MAESNAA QSAA GAG MGRGIAYLFAQKGIR VLYNRN |
| 47. | 47 | Serratia marcescens | SE4145 | MAESNAE I QSAA I GAGTMGRG AYLFAOKQ IR VLYNRN |
| 48. | 48 | Serratia marcescens | 2880STDY5682876 | MAESNAA I QSAA GAGTMGRG AYLFAQKG I RTVLYNRN |
| 40. | 49 | Serrat a marcescens | 709 | MAESNAA I QSAA GAGTMGRG I AYLFAQKG I RTVLYNRN |

(continued)

| | | | | 5 10 15 20 25 30 35 40<br>Consensus sequencel |
|---|---|---|---|---|
| 50. | 50 | Serrat a marcescens | MGH136 | MAESNAA I QSAA I I GAG MGRGIAYLFAQKGIR VLYNRN |
| 51. | 51 | Serratia marcescens | 2880STDY5682884 | MAESNAA I QSAA I I GAG MGRG I AYLFAQKG I R VLYNRN |
| 52 | 52 | Serratia marcescens | D-3 | MAESNAA I QSAA I GAG MGRG AYLFAQKG I RTVLYNRN |
| 58 | 53 | Serratia macescens | 2880STDY5882957 | MAESNAA QSAA I GAGTMGRG AYLFAQKG RTVLYNRN |
| 54. 54 | | Serratia marcescens | YDC563 | MAESNAA QSAA IGAGTMGRGIAYLFAQKG RTVLYNRN |
| 55. | 55 | Serratia macescens | 2880STDY5683035 | MAESNAA QSAA I GAGTMGRG I AYLFAQKG R VLYNRN |
| 56. | 56 | Serrat a marcescens | 2880STDY5882930 | MAESNAA QSAA GAGTMGRGIAYLFAQKGIR VLYNRN |
| 57. | 57 | Serrat a marcescens | 790 | MAESNAA QSAA GAG MORGI AYLFAQKG I R VLYNRN |
| 58. | 58 | Serrat a marcescens | UMH5 | MAESNAA I QSAA GAG MGRGIAYLFAQKGIR VLYNRN |
| 59. | 59 | Serrat a macescens | 2880STDY5682088 I | MAESNAA QSAA GAGTMGRG I AYLFAQKG I R VLYNRN |
| 60. | 60 | Serrat a macescens | 945154301 | MAESNAA QSAA GAGTMGRGIAYLFAQKGIR VLYNRN |
| 61. | 61 | Serratia marcescens | at00508 | MAESNAA I QSAA GAGTMGRGI AYLFAQKGI RTVLYNRN |
| 62. | 62 | Serratia marcescens | WL2637 | MAESNAA I QSAA I I GAGT MGRGI AYLFAQKG I RTVLYNRN |
| 63. | 63 | Serrat a macescens | SM1978 | -MAESNAA QSAA I GAG MGRGIAYLFAQKGIR VLYNRN |
| 64. | 64 | Serrat a mrcescens | PWN146 | MAESNAA QSAA GAGTMGRG AYLFAQKG I R VLYNRN |
| 65. | 65 | Serrat a macescens | Hlq | MAERNAA QSAA I GAGTMGRG AYLFAQKG IR VLYNRN |
| 66. | 66 | Serrat a marcescens | UMH6 | MAESNAA QSAA GAG MGRGI AYLFAQKS I R VLYNRN |
| 67. | 67 | Serrat a nematodlphila | WCU338 | MAESNAA QSAA GAG MGRGAYLFAQKS IR VLYNRN |
| 68. | 68 | Serrat a sp. OLEL1 | | MAESNAA QSAA GAG IIGRG AYLLAQKS IR VLYNRN |
| 69. | 69 | Serrat a marcescens | 7209 | MAESNAA QSAA GAG MGRGAYLFAQKS IR VLYNRN |
| 70. | 70 | Serrat a marcescens | sicar ia (Ss1) | MAESNAA QSAA GAG IGRSI AYLFAQKS RTVLYNRN |
| 71. | 71 | Serrat a sp. OLFL2 | | MAESNAA QSAA GABTMGRG AYLLAQKS RTVLYNRN |
| 72. | 72 | Serratia marcescens | BIDMC 81 | MAESNAA QSAA I GAGTMGRG AYLLAQKS I RTVLYNRN |
| 73. | 73 | Serrat a macescens | BIDMC 50 | MAESNAA QSAA' I GAG MGRGIAYLFAQKS I RTVLNRN |
| 74. | 74 | Serrat a macescens | UH7 | MAESNAA QSAA I GAG MGRGIAYLLAQKSIR VLYNRN |

(continued)

| | | | | 5 10 15 20 25 30 35 40 Consensus sequencel |
|---|---|---|---|---|
| 75. | 75 | Serratia macescens | RSC-14 | MAESNAA QSAA I GAG MGRGI AYLLAQKS I R VLYNRN |
| 76 | 76 | Serratia macescens | SM03 | MAERNAA I QSAA GAG MGRGAYLFAQKG I RTVLYNRN |
| 77. | 77 | Serrat a macescens | 90-166 | MAESNAA IQSAA GAG MGRGAYLFAQKG I R VLYNRN |
| 78. | 78 | Serrat a marcescens | UMH2 | MAESNAA QSAA GAGTMGRGIAYLLAQKS R VLYNRN |
| 79. | 79 | Serrat a plynuthca | AS9 | MAENNSA RSAAV GAGTMGRG IAYLLALNG RTVLYNRN |
| 80. | 80 | Serrat a plynuthica | tumat 205 | MAENNSA RSAAV GAGMGRGAYLLALNG RTVLYNRN |
| 81. | 81 | Serratia plynuthca | A30 | MAENNSA RSAAV GAG MGRG AYLLALNG RTVLYNRN |
| 82. | 82 | Serrat a plymuthica | 4Rx13 | MAENNSA I RSAAV GAGTNGRG IAYLLALNG RTVLYNRN |
| 83. | 83 | Serrat a plynuthica | V4 | MAENNSA RSAAV GAGTMGRG I AYLLALNG RTVLYNRN |
| 84. 84 | | Serrat | 3Rp8 | AENNSA RSAAV I GAGTMGRG I AYLLALNG I RTVLYNRN |
| 85. 85 | | a plynuthca Serrat a proteanculans | NFPA44A14 | MAENNSA HSVAV GAG MGRGIAYLLAQNGIRTLLYNRS |
| 86. | 86 | Serratia plymthica | A153 | MAENNSA I RSAAV GAG MGRGI AYLLALNG I RTVLYNRN |

In the present invention, impairing the function of pyruvate kinase or a phosphotransferase system enzyme means impairing the enzymatic activity of the enzyme. The method of impairment of the function is not limited to a particular method, but the function can be impaired, for example, by disrupting a gene that encodes the enzyme, such as via partial or complete deletion of the gene by mutagenesis with a chemical mutagen, ultraviolet irradiation, or the like, or by site-directed mutagenesis or the like, or via introduction of a frame-shift mutation or a stop codon into the nucleotide sequence of the gene. Alternatively, recombinant DNA technologies can be used to disrupt the gene by partial or complete deletion of the nucleotide sequence or by partial or complete substitution of the nucleotide sequence with another nucleotide sequence. Among those, the methods for partial or complete deletion of the nucleotide sequence are preferred.

Pyruvate kinase is classified as EC 2.7.1.40 and is an enzyme that catalyzes a reaction to dephosphorylate phosphoenolpyruvic acid (in this specification, also referred to as PEP) to pyruvic acid and ATP. Specific examples of pyruvate kinase include pykF (NCBI-Protein ID: NP_416191, SEQ ID NO: 182) and pykA (NCBI-Protein ID: NP_416368, SEQ ID NO: 183) from *Escherichia coli strain* K-12 substrain MG1655, and pykF (SEQ ID NO: 184) and pykA (SEQ ID NO: 185) from *Serratia grimesii* strain NBRC13537.

In cases where a microorganism used in the present invention has two or more genes that each encode a pyruvate kinase, as illustrated in the metabolic pathway shown in the scheme 2 below, it is desirable to impair the function of all the pyruvate kinases. Whether or not a polypeptide encoded by a certain gene of a microorganism used in the present invention is a pyruvate kinase may be determined by BLAST (Basic Local Alignment Search Tool) searching on a website, such as that of NCBI (National Center for Biotechnology Information) or KEGG (Kyoto Encyclopedia of Genes and Genomes).

In the genetically modified microorganism of the present invention, it is desirable to further impair the function of a phosphotransferase system enzyme. The phosphotransferase system enzyme is relevant to the phosphoenolpyruvate (PEP)-dependent phosphotransferase system (PTS) (in this specification, also referred to as a PTS enzyme). PTS is a major mechanism for the uptake of carbohydrates such as hexose, hexitol, and disaccharide into a cell, as illustrated in the metabolic pathway shown in the scheme 2 below. PTS involves uptake of carbohydrates into a cell and simultaneous conversion of the carbohydrates to a phosphate ester, while converting a phosphate donor, PEP, to pyruvic acid. Therefore, the conversion reaction from PEP to pyruvic acid is inhibited in a mutant microorganism with a disrupted PTS

enzyme gene.

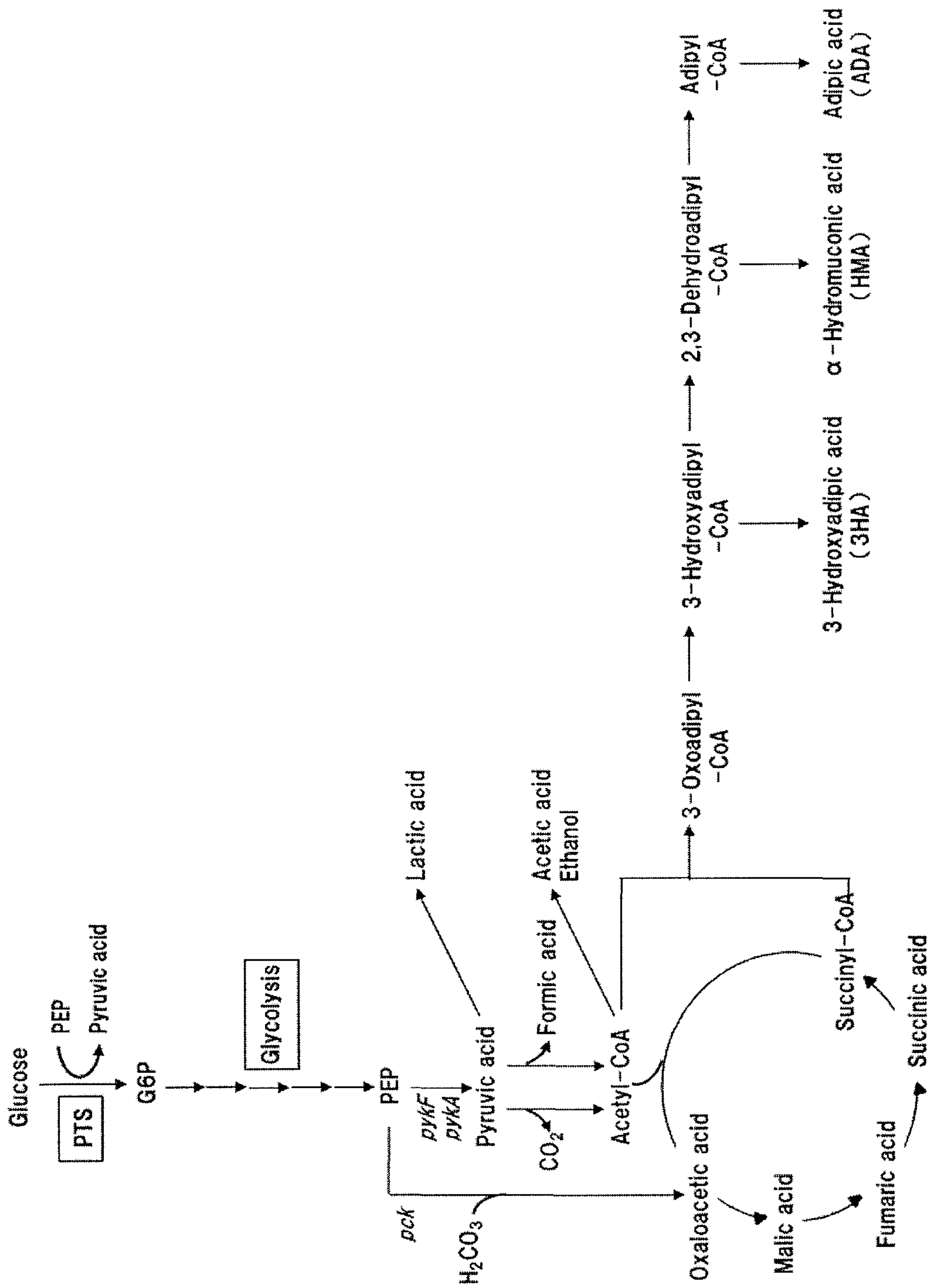


Scheme 2

PTS enzymes are composed of two common enzymes that exert their functions on any type of carbohydrate, phosphoenolpyruvate sugar phosphotransferase enzyme I and phospho carrier protein HPr, and membrane-bound sugar specific permeases (enzymes II) that are specific for particular carbohydrates. The enzymes II are further composed of sugar-specific components IIA, IIB, and IIC. The enzymes II exist as independent proteins or as fused domains in a single protein, and this depends on the organism which those enzymes are originated from. In microorganisms, phosphoenolpyruvate sugar phosphotransferase enzyme I is encoded by the *ptsI* gene, and phospho carrier protein HPr is encoded by the *ptsH* gene, and glucose-specific enzyme IIA is encoded by the *crr* gene, and glucose-specific enzymes IIB and IIC are encoded by the *ptsG* gene. The enzyme encoded by the *ptsG* gene is classified as EC 2.7.1.199 and is called protein-Npi-phosphohistidine-D-glucose phosphotransferase.

In the present invention, one or more of the above PTS enzyme genes may be disrupted. Although any of the

above PTS enzyme genes may be disrupted, it is desirable to impair an enzyme gene that is involved in glucose uptake, particularly the *ptsG* gene. Specific examples of the *ptsG* gene include *ptsG* from *Escherichia coli* strain K-12 substrain MG1655 (NCBI-Gene ID: 945651) and *ptsG* from *Serratia grimesii* strain NBRC13537 (SEQ ID NO: 238).

Whether or not a polypeptide encoded by a certain gene of a microorganism used in the present invention is a protein-Npi-phosphohistidine-D-glucose phosphotransferase may be determined by BLAST searching on a website, such as that of NCBI or KEGG.

As described below, *E. coli* is a microorganism that has an ability to produce 3-hydroxyadipic acid and α-hydromuconic acid, and JP 2008-527991 A describes production of a genetically modified *E. coli* strain with defects in *the pykF* and *pykA* genes, which each encode a pyruvate kinase, and in the *ptsG* gene, which encodes a phosphotransferase system enzyme, wherein the yield of succinic acid is increased, and the yields of acetic acid and ethanol are decreased, by culturing the genetically modified strain under anaerobic conditions. In this respect, acetic acid and ethanol are compounds generated from the metabolism of acetyl-CoA, as illustrated in the metabolic pathway shown in the above scheme 2. That is, in JP 2008-527991 A, it is presumed that the defects of the *ptsG, pykF*, and *pykA* genes in *E. coli* resulted in a reduced supply of acetyl-CoA and in turn a lower yield of acetic acid and ethanol.

The 3-hydroxyadipic acid, α-hydromuconic acid, and/or adipic acid produced by the method of the present invention are compounds generated through reactions in the metabolism of 3-oxoadipyl-CoA, which is produced from acetyl-CoA and succinyl-CoA by the reaction A, as described above. Accordingly, from the description in JP 2008-527991 A, it is expected that disruption of genes encoding pyruvate kinase and a phosphotransferase system enzyme also results in a decreased yields of 3-hydroxyadipic acid, α-hydromuconic acid, and/or adipic acid due to the reduced supply of acetyl-CoA. However, in the present invention, disruption of genes encoding pyruvate kinase and a phosphotransferase system enzyme increases the yields of 3-hydroxyadipic acid, α-hydromuconic acid, and/or adipic acid and also the yields of acetic acid and ethanol in a genetically modified microorganism expressing an enzyme that exhibits excellent activity in a reaction to reduce 3-oxoadipyl-CoA to 3-hydroxyadipyl-CoA, which is contrary to the above expectation.

In the present invention, examples of the microorganism that can be used as a host to obtain the genetically modified microorganism include microorganisms belonging to the genera *Escherichia, Serratia*, *Hafnia*, *Pseudomonas, Corynebacterium, Bacillus, Streptomyces, Cupriavidus*, *Acinetobacter, Alcaligenes, Brevibacterium, Delftia*, *Shimwellia, Aerobacter, Rhizobium, Thermobifida, Clostridium, Schizosaccharomyces, Kluyveromyces, Pichia,* and *Candida.* Among those, microorganisms belonging to the genera *Escherichia, Serratia*, *Hafnia*, and *Pseudomonas* are preferred.

The method of producing 3-hydroxyadipic acid, α-hydromuconic acid, and/or adipic acid by using a genetically modified microorganism of the present invention will be described.

As a microorganism that has an ability to produce 3-hydroxyadipic acid, a microorganism that has an ability to generate 3-oxoadipyl-CoA and coenzyme A from acetyl-CoA and succinyl-CoA (the reaction A), and an ability to generate 3-hydroxyadipic acid from 3-hydroxyadipyl-CoA (the reaction E) is used. The microorganism with these production abilities can be used as a host microorganism to obtain a genetically modified microorganism according to the present invention with an ability to abundantly produce 3-hydroxyadipic acid.

Microorganisms that are speculated to originally have abilities to catalyze the above reactions A and E include microorganisms belonging to the following species:

species of the genus *Escherichia,* such as *Escherichia fergusonii* and *Escherichia coli;*
species of the genus *Pseudomonas,* such as *Pseudomonas chlororaphis*, *Pseudomonas putida, Pseudomonas azotoformans*, and *Pseudomonas chlororaphis* subsp. *aureofaciens;*
species of the genus *Hafnia*, such as *Hafnia alvei;*
species of the genus *Corynebacterium,* such as *Corynebacterium acetoacidophilum*, *Corynebacterium acetoglutamicum*, *Corynebacterium ammoniagenes,* and *Corynebacterium glutamicum;*
species of the genus *Bacillus,* such as *Bacillus badius, Bacillus magaterium*, and *Bacillus roseus;*
species of the genus *Streptomyces,* such as *Streptomyces vinaceus*, *Streptomyces karnatakensis*, and *Streptomyces olivaceus;*
species of the genus *Cupriavidus*, such as *Cupriavidus metallidurans*, *Cupriavidus necator*, and *Cupriavidus oxalaticus*;
species of the genus *Acinetobacter*, such as *Acinetobacter baylyi* and *Acinetobacter radioresistens*;
species of the genus *Alcaligenes,* such as *Alcaligenes faecalis*;
species of the genus *Nocardioides*, such as *Nocardioides albus;*
species of the genus *Brevibacterium,* such as *Brevibacterium iodinum*;
species of the genus *Delftia*, such as *Delftia acidovorans;*
species of the genus *Shimwellia*, such as *Shimwellia blattae*;
species of the genus *Aerobacter,* such as *Aerobacter cloacae;*
species of the genus *Rhizobium,* such as *Rhizobium radiobacter*;
species of the genus *Serratia,* such as *Serratia grimesii, Serratia ficaria*, *Serratia fonticola*, *Serratia odorifera*, *Serratia*

*plymuthica, Serratia entomophila*, and *Serratia nematodiphila.*

Even a microorganism that originally has no abilities to catalyze the reactions A and/or E can also be used as the aforementioned host microorganism when an appropriate combination of nucleic acids that encode enzymes catalyzing the reactions A and E is introduced into the microorganism to impart those production abilities.
As a microorganism that has an ability to produce α-hydromuconic acid, a microorganism that has an ability to generate 3-oxoadipyl-CoA and coenzyme A from acetyl-CoA and succinyl-CoA (the reaction A), an ability to generate 2,3-dehydroadipyl-CoA from 3-hydroxyadipyl-CoA through dehydration (the reaction C), and an ability to generate α-hydromuconic acid from 2,3-dehydroadipyl-CoA (the reaction F) is used. The microorganism with these production abilities can be used as a host microorganism to obtain a genetically modified microorganism according to the present invention with an ability to abundantly produce α-hydromuconic acid.
Microorganisms that are speculated to originally have abilities to catalyze the above reactions A, C, and F include microorganisms belonging to the following species:

species of the genus *Escherichia*, such as *Escherichia fergusonii* and *Escherichia coli;*
species of the genus *Pseudomonas,* such as *Pseudomonas fluoresceins*, *Pseudomonas putida, Pseudomonas azotoformans*, and *Pseudomonas chlororaphis* subsp. *aureofaciens*;
species of the genus *Hafnia*, such as *Hafnia alvei*;
species of the genus *Bacillus,* such as *Bacillus badius*;
species of the genus *Cupriavidus*, such as *Cupriavidus metallidurans, Cupriavidus numazuensis*, and *Cupriavidus oxalaticus*;
species of the genus *Acinetobacter,* such as *Acinetobacter baylyi* and *A cinetobacter radioresistens*;
species of the genus *Alcaligenes,* such as *Alcaligenes faecalis*;
species of the genus *Delftia*, such as *Delftia acidovorans*;
species of the genus *Shimwellia*, such as *Shimwellia blattae*;
species of the genus *Serratia,* such as *Serratia grimesii*, *Serratia ficaria*, *Serratia fonticola*, *Serratia odorifera*, *Serratia plymuthica*, *Serratia entomophila*, and *Serratia nematodiphila.*

Even a microorganism that originally has no abilities to catalyze the reactions A, C, and/or F can also be used as the aforementioned host microorganism when an appropriate combination of nucleic acids that encode enzymes catalyzing the reactions A, C, and F is introduced into the microorganism to impart those production abilities.
As a microorganism that has an ability to produce adipic acid, a microorganism that has an ability to generate 3-oxoadipyl-CoA and coenzyme A from succinyl-CoA (the reaction A), an ability to generate 2,3-dehydroadipyl-CoA from 3-hydroxyadipyl-CoA through dehydration (the reaction C), an ability to reduce 2,3-dehydroadipyl-CoA to adipyl-CoA (the reaction D), and an ability to generate adipic acid from adipyl-CoA (the reaction G) is used. The microorganism with these production abilities can be used as a host microorganism to obtain a genetically modified microorganism with an ability to abundantly produce adipic acid.
Microorganisms that are speculated to originally have abilities to catalyze the above reactions A, C, D, and G include microorganisms of the genus *Thermobifida*, such as *Thermobifida fusca.*
Even a microorganism that originally has no abilities to catalyze the reactions A, C, D, and G can also be used as the aforementioned host microorganism when an appropriate combination of nucleic acids that encode enzymes catalyzing the reactions A, C, D, and G is introduced into the microorganism to impart those production abilities.
Specific examples of the enzymes that catalyze the reactions A and C to G are presented below.
As an enzyme that catalyzes the reaction A to generate 3-oxoadipyl-CoA, for example, an acyl transferase (β-ketothiolase) can be used. The acyl transferase is not limited to a particular number in the EC classification but is preferably an acyl transferase classified into EC 2.3.1.-, specifically including an enzyme classified as 3-oxoadipyl-CoA thiolase and classified into EC number 2.3.1.174, an enzyme classified as acetyl-CoA C-acetyltransferase and classified into EC number 2.3.1.9, and an enzyme classified as acetyl-CoA C-acyl transferase and classified into EC number 2.3.1.16. Among these, PaaJ from *Escherichia coli* strain MG1655 (NCBI-Protein ID: NP_415915), PcaF from *Pseudomonas putida* strain KT2440 (NCBI-Protein ID: NP_743536), and the like can be suitably used.
Whether or not the above acyl transferases can generate 3-oxoadipyl-CoA from succinyl-CoA and acetyl-CoA as substrates can be determined by measuring a decrease in NADH coupled with reduction of 3-oxoadipyl-CoA in a combination of the reaction catalyzed by purified acyl transferase to generate 3-oxoadipyl-CoA and a reaction catalyzed by purified 3-oxoadipyl-CoA reductase to reduce 3-oxoadipyl-CoA as a substrate. The specific measurement method is, for example, as follows.
Identification of acyl transferase activity: A PCR using the genomic DNA of a subject microorganism strain as a template is performed in accordance with routine procedures, to amplify a nucleic acid encoding an acyl transferase in the full-length form. The amplified fragment is inserted into the *Sac*I site of pACYCDuet-1 (manufactured by Novagen),

an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain an acyl transferase solution. The acyl transferase activity can be determined by using the enzyme solution to prepare an enzymatic reaction solution with the following composition and measuring a decrease in absorbance at 340 nm coupled with oxidation of NADH at 30°C.

(Enzymatic Reaction Solution)

100 mM Tris-HCl (pH 8.0)
10 mM $MgCl_2$
0.1 mM succinyl-CoA
0.2 mM acetyl-CoA
0.2 mM NADH
1 mM dithiothreitol
10 μg/mL 3-oxoadipyl-CoA reductase
5 μg/mL acyl transferase.

Whether or not an enzyme originally expressed in a host microorganism used in the present invention has acyl transferase activity can be determined by performing the above-described measurement using cell homogenate (cell free extract: CFE) instead of purified acyl transferase. The specific measurement method targeted to *E. coli* is, for example, as follows.

Preparation of CFE: A loopful *of E. coli* strain MG1655 to be subjected to the measurement of the activity is inoculated into 5 mL of a culture medium (culture medium composition: 10 g/L tryptone, 5 g/L yeast extract, 5 g/L sodium chloride) adjusted to pH 7, and incubated at 30°C with shaking for 18 hours. The obtained culture fluid is added to 5 mL of a culture medium (culture medium composition: 10 g/L tryptone, 5 g/L yeast extract, 5 g/L sodium chloride, 2.5 mM ferulic acid, 2.5 mM *p*-coumaric acid, 2.5 mM benzoic acid, 2.5 mM cis,cis-muconic acid, 2.5 mM protocatechuic acid, 2.5 mM catechol, 2.5 mM 3OA, 2.5 mM 3-hydroxyadipic acid, 2.5 mM α-hydromuconic acid, 2.5 mM adipic acid, 2.5 mM phenylethylamine) adjusted to pH 7, and incubated at 30°C with shaking for 3 hours.

The obtained culture fluid is supplemented with 10 mL of 0.9% sodium chloride and then centrifuged to remove the supernatant from bacterial cells, and this operation is repeated three times in total to wash the bacterial cells. The washed bacterial cells are suspended in 1 mL of a Tris-HCl buffer composed of 100 mM Tris-HCl (pH 8.0) and 1 mM dithiothreitol, and glass beads (with a diameter of 0.1 mm) are added to the resulting suspension to disrupt the bacterial cells at 4°C with an ultrasonic disruptor. The resulting bacterial homogenate is centrifuged to obtain the supernatant, and 0.5 mL of the supernatant is filtered through a UF membrane (Amicon Ultra-0.5mL 10K; manufactured by Merck Millipore) to remove the resulting filtrate, followed by application of 0.4 mL of the Tris-HCl buffer to the UF membrane, and this operation is repeated three times in total to remove lowmolecular-weight impurities, and the resulting supernatant is then resuspended in the Tris-HCl buffer to a final volume of 0.1 mL, which is designated as CFE. Instead of purified enzyme, 0.05 mL of the CFE is added to a total of 0.1 mL of the enzymatic reaction solution to determine the enzymatic activity.

As an enzyme that catalyzes the reaction C to generate 2,3-dehydroadipyl-CoA, for example, an enoyl-CoA hydratase can be used. The enoyl-CoA hydratase is not limited by a particular number in the EC classification, and is preferably an enoyl-CoA hydratase classified into EC 4.2.1.-, specifically including an enzyme classified as enoyl-CoA hydratase or 2,3-dehydroadipyl-CoA hydratase and classified into EC 4.2.1.17. Among them, PaaF from *Escherichia coli* strain MG1655 (NCBI-ProteinID: NP_415911), PaaF from *Pseudomonas putida* strain KT2440 (NCBI-ProteinID: NP_745427), and the like can be suitably used.

Since the reaction catalyzed by enoyl-CoA hydratase is generally reversible, whether or not an enoyl-CoA hydratase has an activity to catalyze a reaction that generates 2,3-dehydroadipyl-CoA from 3-hydroxyadipyl-CoA used as a substrate can be determined by detecting 3-hydroxyadipyl-CoA generated using purified enoyl-CoA hydratase with 2,3-dehydroadipyl-CoA used as a substrate thereof, which is prepared from α-hydromuconic acid through an enzymatic reaction. The specific measurement method is, for example, as follows.

The α-hydromuconic acid used in the above reaction can be prepared by a known method (for example, a method described in Reference Example 1 of WO 2016/199858 A1).

Preparation of 2,3-dehydroadipyl-CoA solution: A PCR using the genomic DNA of *Pseudomonas putida* strain KT2440 as a template is performed in accordance with routine procedures, to amplify a nucleic acid encoding a CoA transferase (including *pcaI* and *pcaJ*; NCBI-GeneIDs: 1046613 and 1046612) in the full-length form. The amplified

fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution for 2,3-dehydroadipyl-CoA preparation with the following composition, which is allowed to react at 30°C for 10 minutes and then filtered through a UF membrane (Amicon Ultra-0.5mL 10K; manufactured by Merck Millipore) to remove the enzyme, and the obtained filtrate is designated as 2,3-dehydroadipyl-CoA solution.

(Enzymatic Reaction Solution)

100 mM Tris-IICl (pH 8.0)
10 mM $MgCl_2$
0.4 mM succinyl-CoA
2 mM α-hydromuconic acid sodium salt
20 μg/mL CoA transferase.

Identification of enoyl-CoA hydratase activity: A PCR using the genomic DNA of a subject microorganism strain as a template is performed in accordance with routine procedures, to amplify a nucleic acid encoding an enoyl-CoA hydratase in the full-length form. The amplified fragment is inserted into the *Nde*I site of pET-16b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain an enoyl-CoA hydratase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The enoyl-CoA hydratase activity can be confirmed by detecting 3-hydroxyadipyl-CoA in the resulting filtrate on high-performance liquid chromatograph-tandem mass spectrometer (LC-MS/MS) (Agilent Technologies, Inc.).

(Enzymatic Reaction Solution)

100 mM Tris-HCl (pH 8.0)
10 mM $MgCl_2$
300 μL/mL 2,3-dehydroadipyl-CoA solution
1 mM dithiothreitol
20 μg/mL enoyl-CoA hydratase.

Whether or not an enzyme originally expressed in a host microorganism used in the present invention has enoyl-CoA hydratase activity can be determined by adding 0.05 mL of the CFE, instead of purified enoyl-CoA hydratase, to a total of 0.1 mL of the enzymatic reaction solution and performing the above-described measurement. The specific CFE preparation method targeted to *E. coli* is as described for that used in determination of acyl transferase activity.

As an enzyme that catalyzes the reaction D to generate adipyl-CoA, for example, an enoyl-CoA reductase can be used. The enoyl-CoA reductase is not limited by a particular number in the EC classification, and is preferably an enoyl-CoA reductase classified into EC 1.3.-.-, specifically including an enzyme classified as trans-2-enoyl-CoA reductase and classified into EC 1.3.1.44, and an enzyme classified as acyl-CoA dehydrogenase and classified into EC 1.3.8.7. These specific examples are disclosed in, for example JP 2011-515111 A, J Appl Microbiol. 2015 Oct;l 19 (4): 1057-63., and the like; among them, TER from *Euglena gracilis* strain Z (UniProtKB: Q5EU90), Tfu_1647 from *Thermobifida fusca* strain YX (NCBI-ProteinID: AAZ55682), DcaA from *Acinetobacter baylyi* strain ADP1 (NCBI-ProteinID: AAL09094.1), and the like can be suitably used.

Whether or not an enoyl-CoA reductase has an activity to generate adipyl-CoA from 2,3-dehydroadipyl-CoA used as a substrate can be determined by measuring a decrease in NADH coupled with reduction of 2,3-dehydroadipyl-CoA in a reaction using purified enoyl-CoA reductase with 2,3-dehydroadipyl-CoA used as a substrate thereof, which is prepared from α-hydromuconic acid through another enzymatic reaction.

Preparation of α-hydromuconic acid and of 2,3-dehydroadipyl-CoA solution can be performed in the same manner as described above.

Identification of enoyl-CoA reductase activity: A PCR using the genomic DNA of a subject microorganism strain as a template is performed in accordance with routine procedures, to amplify a nucleic acid encoding an enoyl-CoA reductase in the full-length form. The amplified fragment is inserted into the *Nde*I site of pET-16b (manufactured by Novagen), an expression vector for *E. coli,* in-frame with the histidine-tag sequence. The plasmid is introduced into *E. coli* BL21 (DE3), and expression of the enzyme is induced with isopropyl-p-thiogalactopyranoside (IPTG) in accordance with routine procedures and the enzyme is purified using the histidine tag from the culture fluid to obtain an enoyl-CoA reductase solution. The enoyl-CoA reductase activity can be determined by using the enzyme solution to prepare an enzymatic reaction solution with the following composition and measuring a decrease in absorbance at 340 nm coupled with oxidation of NADH at 30°C.

(Enzymatic Reaction Solution)

100 mM Tris-HCl (pH 8.0)
10 mM $MgCl_2$
300 μL/mL 2,3-dehydroadipyl-CoA solution
0.2 mM NADH
1 mM dithiothreitol
20 μg/mL enoyl-CoA reductase.

Whether or not an enzyme originally expressed in a host microorganism used in the present invention has enoyl-CoA reductase activity can be determined by adding 0.05 mL of the CFE, instead of purified enoyl-CoA reductase, to a total of 0.1 mL of the enzymatic reaction solution and performing the above-described measurement. The specific CFE preparation method targeted to *E. coli* is as described for that used in determination of acyl transferase activity.

As an enzyme that catalyzes the reaction E to generate 3-hydroxyadipic acid, the reaction F to generate α-hydromuconic acid, and the reaction G to generate adipic acid, for example, a CoA transferase or an acyl-CoA hydrolase, preferably a CoA transferase, can be used.

The CoA transferase is not limited by a particular number in the EC classification, and is preferably a CoA transferase classified into EC 2.8.3.-, specifically including an enzyme classified as CoA transferase or acyl-CoA transferase and classified into EC 2.8.3.6, and the like.

In the present invention, the term "CoA transferase" refers to an enzyme with activity (CoA transferase activity) to catalyze a reaction that generates carboxylic acid and succinyl-CoA from acyl-CoA and succinic acid used as substrates.

As an enzyme that catalyzes the reaction E to generate 3-hydroxyadipic acid and the reaction F to generate α-hydromuconic acid, PcaI and PcaJ from *Pseudomonas putida* strain KT2440 (NCBI-ProteinIDs: NP 746081 and NP_746082), and the like can be suitably used, among others.

As an enzyme that catalyzes the reaction G to generate adipic acid, DcaI and DcaJ from *Acinetobacter baylyi* strain ADP1 (NCBI-ProteinIDs: CAG68538 and CAG68539), and the like can be suitably used.

Since the above enzymatic reactions are reversible, the CoA transferase activity against 3-hydroxyadipyl-CoA, 2,3-dehydroadipyl-CoA, or adipyl-CoA used as a substrate can be determined by detecting 3-hydroxyadipyl-CoA, 2,3-dehydroadipyl-CoA, or adipyl-CoA generated respectively using purified CoA transferase with 3-hydroxyadipic acid and succinyl-CoA, α-hydromuconic acid and succinyl-CoA, or adipic acid and succinyl-CoA used as substrates thereof. The specific measurement method is, for example, as follows.

Preparation of 3-hydroxyadipic acid: Preparation of 3-hydroxyadipic acid is performed according to the method described in Reference Example 1 of WO 2016/199856 A1.

Identification of CoA transferase activity using 3-hydroxyadipic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can be confirmed by detecting 3-hydroxyadipyl-CoA in the resulting filtrate on high-performance liquid chromatograph-tandem mass spectrometer (LC-MS/MS) (Agilent Technologies, Inc.).

(Enzymatic Reaction Solution)

100 mM Tris-HCl (pH 8.0)
10 mM $MgCl_2$
0.4 mM succinyl-CoA
2 mM 3-hydroxyadipic acid sodium salt
20 μg/mL CoA transferase.

Preparation of α-hydromuconic acid: Preparation of α-hydromuconic acid is performed according to the method described in Reference Example 1 of WO 2016/199858 A1.

Identification of CoA transferase activity using α-hydromuconic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can be confirmed by detecting 2,3-dehydroadipyl-CoA in the resulting filtrate on high-performance liquid chromatograph-tandem mass spectrometer (LC-MS/MS) (Agilent Technologies, Inc.).

(Enzymatic Reaction Solution)

100 mM Tris-HCl (pH 8.0)
10 mM $MgCl_2$
0.4 mM succinyl-CoA
2 mM α-hydromuconic acid sodium salt
20 μg/mL CoA transferase.

Identification of CoA transferase activity using adipic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can be confirmed by detecting adipyl-CoA in the resulting filtrate on high-performance liquid chromatograph-tandem mass spectrometer (LC-MS/MS) (Agilent Technologies, Inc.).

(Enzymatic Reaction Solution)

100 mM Tris-HCl (pH 8.0)
10 mM $MgCl_2$
0.4 mM succinyl-CoA
2 mM adipic acid sodium salt
20 μg/mL CoA-transferase.

Whether or not an enzyme originally expressed in a host microorganism used in the present invention has CoA transferase activity can be determined by adding 0.05 mL of the CFE, instead of purified CoA transferase, to a total of

0.1 mL of the enzymatic reaction solution and performing the above-described measurement. The specific CFE preparation method targeted to *E. coli* is as described for that used in determination of acyl transferase activity.

Either the polypeptides described in (a) to (c) or the 3-hydroxybutyryl-CoA dehydrogenase in the present invention is characterized by having higher activity than 3-oxoadipyl-CoA reductases used in conventional techniques. In this respect, the phrase "higher activity" refers to production of 3-hydroxyadipic acid, $\alpha$-hydromuconic acid, or adipic acid with a higher yield in a genetically modified microorganism expressing any one of the polypeptides than in a genetically modified microorganism expressing a conventional 3-oxoadipyl-CoA reductase when those microorganisms are derived from the same host microorganism species and are cultured under the same expression conditions in a culture medium containing a carbon source as a material for fermentation. In this respect, the yield of 3-hydroxyadipic acid is calculated according to the formula (2). The yield of $\alpha$-hydromuconic acid or adipic acid is calculated according to the formula (2), where 3-hydroxyadipic acid is replaced by $\alpha$-hydromuconic acid or adipic acid, respectively.

$$\text{Yield (\%)} = \text{amount of generated 3-hydroxyadipic acid (mol)} / \text{amount of consumed carbon source (mol)} \times 100 \qquad \text{Formula (2)}$$

The specific method to confirm the higher activity of either the polypeptides described in (a) to (c) or the 3-hydroxybutyryl-CoA dehydrogenase in the present invention compared to the activity of 3-oxoadipyl-CoA reductases used in conventional techniques is as follows. The pBBR1MCS-2 vector, which is able to self-replicate in *E. coli* (ME Kovach, (1995), Gene 166: 175-176), is cleaved with *Xho*I to obtain pBBR1MCS-2/XhoI. To integrate a constitutive expression promoter into the vector, an upstream 200-b region (SEQ ID NO: 186) of *gapA* (NCBI Gene ID: NC_000913.3) is amplified by PCR using the genomic DNA of *Escherichia coli* K-12 MG1655 as a template in accordance with routine procedures (for example, primers represented by SEQ ID NOs: 187 and 188 are used), and the obtained fragment and the pBBR1MCS-2/XhoI are ligated together using the In-Fusion HD Cloning Kit (manufactured by Takara Bio Inc.) to obtain the plasmid pBBR1MCS-2::Pgap. The pBBR1MCS-2::Pgap is cleaved with *Sca*I to obtain pBBR1MCS-2::Pgap/ScaI. A nucleic acid encoding an acyl transferase in the full length form is amplified by PCR in accordance with routine procedures (for example, primers represented by SEQ ID NOs: 190 and 191 are used), and the obtained fragment and pBBR1MCS-2::Pgap/ScaI are ligated together using the In-Fusion HD Cloning Kit to obtain the plasmid pBBR1MCS-2::AT. The pBBR1MCS-2::AT is cleaved with *Hpa*I to obtain pBBR1MCS-2::AT/HpaI. A nucleic acid encoding a CoA transferase in the full length form is amplified by PCR in accordance with routine procedures (for example, primers represented by SEQ ID NOs: 194 and 195 are used), and the obtained fragment and pBBR1MCS-2::AT/HpaI are ligated together using "the In-Fusion HD Cloning Kit" to obtain the plasmid pBBR1MCS-2::ATCT.

On the other hand, the pACYCDuet-1 expression vector (manufactured by Novagen), which is able to self-replicate in *E. coli,* is cleaved with *Bam*HI to obtain pACYCDuet-1/BamHI. A nucleic acid encoding a polypeptide represented by any one of SEQ ID NOs: 1 to 86 or encoding a conventionally used 3-oxoadipyl-CoA reductase, is amplified by PCR in accordance with routine procedures (for example, primers represented by SEQ ID NOs: 196 and 197 are used), and the obtained fragment and pACYCDuet-1/BamHI are ligated together using the In-Fusion HD Cloning Kit (manufactured by Takara Bio Inc.) to obtain a plasmid that expresses the polypeptide represented by any one of SEQ ID NOs: 1 to 86 or expresses the conventionally used 3-oxoadipyl-CoA reductase.

The obtained plasmid and the pBBR1MCS-2::ATCT are introduced into *E. coli* strain BL21 (DE3) by electroporation (NM Calvin, PC Hanawalt. J. Bacteriol, 170 (1988), pp. 2796-2801). A loopful of the strain after the introduction is inoculated into 5 mL of the culture medium I (10 g/L Bacto Tryptone (manufactured by Difco Laboratories), 5 g/L Bacto Yeast Extract (manufactured by Difco Laboratories), 5 g/L sodium chloride, 25 $\mu$g/mL kanamycin, and 15 $\mu$g/mL chloramphenicol) adjusted to pH 7, and incubated at 30°C with shaking at 120 $min^{-1}$ for 18 hours. Subsequently, 0.25 mL of the culture fluid is added to 5 mL of the culture medium II (10 g/L succinic acid, 10 g/L glucose, 1 g/L ammonium sulfate, 50 mM potassium phosphate,0.025 g/L magnesium sulfate,0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 25 $\mu$g/mL kanamycin, 15 $\mu$g/mL chloramphenicol, and 0.01 mM IPTG) adjusted to pII 6.5, and incubated at 30°C with shaking at 120 $min^{-1}$ for 24 hours. The supernatant separated from bacterial cells by centrifugation of the culture fluid is processed by membrane treatment using Millex-GV (0.22 $\mu$m; PVDF; manufactured by Merck KGaA), and the resulting filtrate is analyzed to measure the 3-hydroxyadipic acid and carbon source concentrations in the culture supernatant. Quantitative analysis of 3-hydroxyadipic acid on LC-MS/MS is performed under the following conditions.

- HPLC: 1290 Infinity (manufactured by Agilent Technologies, Inc.)

  Column: Synergi hydro-RP (manufactured by Phenomenex Inc.), length: 100 mm, internal diameter: 3 mm,

particle size: 2.5 μm
Mobile phase: 0.1% aqueous formic acid solution / methanol = 70/30
Flow rate: 0.3 mL/min
Column temperature: 40°C
LC detector: DAD (210 nm)

- MS/MS: Triple-Quad LC/MS (manufactured by Agilent Technologies, Inc.) Ionization method: ESI in negative mode.

Quantitative analysis of carbon sources, such as sugars and succinic acid, on HPLC is performed under the following conditions.
- HPLC: Shimazu Prominence (manufactured by Shimadzu Corporation)

Column: Shodex Sugar SH1011 (manufactured by Showa Denko K.K.), length: 300 mm, internal diameter: 8 mm, particle size: 6 μm
Mobile phase: 0.05M aqueous sulfuric acid solution
Flow rate: 0.6 mL/min
Column temperature: 65°C
Detector: RI.

When a nucleic acid encoding any one selected from the group of the acyl transferase, the CoA transferase, the enoyl-CoA hydratase, and the enoyl-CoA reductase is introduced into a host microorganism in the present invention, the nucleic acid may be artificially synthesized based on the amino acid sequence information of the enzyme in a database, or isolated from the natural environment. In cases where the nucleic acid is artificially synthesized, the usage frequency of codons corresponding to each amino acid in the nucleic acid sequence may be changed depending on the host microorganism into which the nucleic acid is introduced.
In the present invention, the method of introducing a nucleic acid encoding any one selected from the group of the acyl transferase, the CoA transferase, the enoyl-CoA hydratase, and the enoyl-CoA reductase into the host microorganism method is not limited to a particular method; for example, a method in which the nucleic acid is integrated into an expression vector capable of autonomous replication in the host microorganism and then introduced into the host microorganism, a method in which the nucleic acid is integrated into the genome of the host microorganism, and the like can be used.
In cases where a nucleic acid encoding any one of the enzymes is isolated from the natural environment, the sources of the genes are not limited to particular organisms, and examples of the organisms include those of the genus *Acinetobacter,* such as *Acinetobacter baylyi* and *Acinetobacter radioresistens*; the genus *Aerobacter,* such as *Aerobacter cloacae;* the genus *Alcaligenes,* such as *Alcaligenes faecalis*; the genus *Bacillus*, such as *Bacillus badius*, *Bacillus magaterium,* and *Bacillus roseus*; the genus *Brevibacterium*, such as *Brevibacterium iodinum*; the genus *Corynebacterium,* such as *Corynebacterium acetoacidophilum, Corynebacterium acetoglutamicum, Corynebacterium ammoniagenes,* and *Corynebacterium glutamicum;* the genus *Cupriavidus*, such as *Cupriavidus metallidurans*, *Cupriavidus necator, Cupriavidus numazuensis*, and *Cupriavidus oxalaticus;* the genus *Delftia*, such as *Delftia acidovorans;* the genus *Escherichia,* such as *Escherichia coli* and *Escherichia fergusonii*; the genus *Hafnia,* such as *Hafnia alvei*; the genus *Microbacterium,* such as *Microbacterium ammoniaphilum*; the genus *Nocardioides*, such as *Nocardioides albus;* the genus *Planomicrobium*, such as *Planomicrobium okeanokoites*; the genus *Pseudomonas,* such as *Pseudomonas azotoformans, Pseudomonas chlororaphis, Pseudomonas fluorescens*, *Pseudomonas fragi*, *Pseudomonas putida,* and *Pseudomonas reptilivora;* the genus *Rhizobium,* such as *Rhizobium radiobacter;* the genus *Rhodosporidium*, such as *Rhodosporidium toruloides*; the genus *Saccharomyces,* such as *Saccharomyces cerevisiae;* the genus *Serratia*, such as *Serratia entomophila, Serratia ficaria*, *Serratia fonticola*, *Serratia grimesii, Serratia nematodiphila*, *Serratia odorifera*, and *Serratia plymuthica;* the genus *Shimwellia,* such as *Shimwellia blattae*; the genus *Streptomyces,* such as *Streptomyces vinaceus, Streptomyces karnatakensis*, *Streptomyces olivaceus*, and *Streptomyces vinaceus;* the genus *Yarrowia*, such as *Yarrowia lipolytica*; the genus *Yersinia,* such as *Yersinia ruckeri*; the genus *Euglena*, such as *Euglena gracilis*; and the genus *Thermobifida,* such as *Thermobifida fusca.* Preferably, the organisms are those of the genera *Acinetobacter, Corynebacterium, Escherichia, Pseudomonas, Serratia*, *Euglena*, and *Thermobifida.*
When a nucleic acid encoding a polypeptide expressed in the present invention is integrated into an expression vector or the genome of a host microorganism, the nucleic acid being integrated into the expression vector or the genome is preferably composed of a promoter, a ribosome-binding sequence, a nucleic acid encoding the polypeptide to be expressed, and a transcription termination sequence, and may additionally contain a gene that controls the activity of the promoter.

The promoter used in the present invention is not limited to a particular promoter, provided that the promoter drives expression of the enzyme in the host microorganism; examples of the promoter include *gap* promoter, *trp* promoter, *lac* promoter, *tac* promoter, and T7 promoter.

In cases where an expression vector is used in the present invention to introduce the nucleic acid or to enhance the expression of the polypeptide, the expression vector is not limited to a particular vector, provided that the vector is capable of autonomous replication in the microorganism; examples of the vector include pBBR1MCS vector, pBR322 vector, pMW vector, pET vector, pRSF vector, pCDF vector, pACYC vector, and derivatives of the above vectors.

In cases where a nucleic acid for genome integration is used in the present invention to introduce the nucleic acid or to enhance the expression of the polypeptide, the nucleic acid for genome integration is introduced by site-specific homologous recombination. The method for site-specific homologous recombination is not limited to a particular method, and examples of the method include a method in which λ Red recombinase and FLP recombinase are used (Proc Natl Acad Sci U.S.A. 2000 Jun 6; 97 (12): 6640-6645.), and a method in which λ Red recombinase and the *sacB* gene are used (Biosci Biotechnol Biochem. 2007 Dec;71 (12):2905-11.).

The method of introducing the expression vector or the nucleic acid for genome integration is not limited to a particular method, provided that the method is for introduction of a nucleic acid into a microorganism; examples of the method include the calcium ion method (Journal of Molecular Biology, 53, 159 (1970)), and electroporation (NM Calvin, PC Hanawalt. J. Bacteriol, 170 (1988), pp. 2796-2801).

In the present invention, a genetically modified microorganism in which a nucleic acid encoding a 3-oxoadipyl-CoA reductase is introduced or expression of the corresponding polypeptide is enhanced is cultured in a culture medium, preferably a liquid culture medium, containing a carbon source as a material for fermentation which can be used by ordinary microorganisms. The culture medium used contains, in addition to the carbon source that can be used by the genetically modified microorganism, appropriate amounts of a nitrogen source, inorganic salts, and, if necessary, organic trace nutrients such as amino acids and vitamins. Any of natural and synthetic culture media can be used as long as the medium contains the above-described nutrients.

The material for fermentation is a material that can be metabolized by the genetically modified microorganism. The term "metabolize" refers to conversion of a chemical substance, which a microorganism has taken up from the extracellular environment or intracellularly generated from a different chemical substance, to another chemical substance through an enzymatic reaction. Sugars can be suitably used as the carbon source. Specific examples of the sugars include monosaccharides, such as glucose, sucrose, fructose, galactose, mannose, xylose, and arabinose; disaccharides and polysaccharides formed by linking these monosaccharides; and saccharified starch solution, molasses, and saccharified solution from cellulose-containing biomass, each containing any of those saccharides.

Other than the above sugars, succinic acid, a substrate of the CoA transferase, can also be added to the culture medium for efficient production of 3-hydroxyadipic acid, α-hydromuconic acid, and/or adipic acid.

The above-listed carbon sources may be used individually or in combination. When a carbon source is added, the concentration of the carbon source in the culture medium is not particularly limited, and can be appropriately selected depending on the type of the carbon source; in the case of sugars, the concentration is preferably from 5 g/L to 300 g/L; in the case of succinic acid, the concentration is preferably from 0.1 g/L to 100 g/L.

As the nitrogen source used for culturing the genetically modified microorganism, for example, ammonia gas, aqueous ammonia, ammonium salts, urea, nitric acid salts, other supportively used organic nitrogen sources, such as oil cakes, soybean hydrolysate, casein degradation products, other amino acids; vitamins, corn steep liquor, yeast or yeast extract, meat extract, peptides such as peptone, and bacterial cells and hydrolysate of various fermentative bacteria can be used. The concentration of the nitrogen source in the culture medium is not particularly limited, and is preferably from 0.1 g/L to 50 g/L.

As the inorganic salts used for culturing the genetically modified microorganism, for example, phosphoric acid salts, magnesium salts, calcium salts, iron salts, and manganese salts can be appropriately added to the culture medium and used.

The culture conditions for the genetically modified microorganism to produce 3-hydroxyadipic acid, α-hydromuconic acid, and/or adipic acid are set by appropriately adjusting or selecting, for example, the culture medium with the above composition, culture temperature, stirring speed, pH, aeration rate, and inoculation amount, depending on, for example, the species of the genetically modified microorganism and external conditions.

The pH range of the culture is not specifically limited, provided that the genetically modified microorganism can be grown in the pH range. However, the pH range is preferably from pH 5 to 8, more preferably from pH 5.5 to 6.8.

Although the range of aeration rates in the culture is not specifically limited, as long as 3-hydroxyadipic acid, α-hydromuconic acid, and/or adipic acid can be produced under the aeration conditions. It is desired that oxygen remain in the gaseous phase and/or liquid phase in a culture container for good growth of the mutant microorganism at least at the start of incubation.

In cases where foam is formed in a liquid culture, an antifoaming agent such as a mineral oil, silicone oil, or surfactant may be appropriately added to the culture medium.

After a recoverable amount of 3-hydroxyadipic acid, α-hydromuconic acid, and/or adipic acid is produced during culturing of the microorganism, the produced products can be recovered. The produced products can be recovered, for example isolated, according to a commonly used method, in which the culturing is stopped once a product of interest is accumulated to an appropriate level, and the fermentation product is collected from the culture. Specifically, the products can be isolated from the culture by separation of bacterial cells through, for example, centrifugation or filtration prior to, for example, column chromatography, ion exchange chromatography, activated charcoal treatment, crystallization, membrane separation, or distillation. More specifically, examples include, but are not limited to, a method in which an acidic component is added to salts of the products, and the resulting precipitate is collected; a method in which water is removed from the culture by concentration using, for example, a reverse osmosis membrane or an evaporator to increase the concentrations of the products and the products and/or salts of the products are then crystallized and precipitated by cooling or adiabatic crystallization to recover the crystals of the products and/or salts of the products by, for example, centrifugation or filtration; and a method in which an alcohol is added to the culture to produce esters of the products and the resulting esters of the products are subsequently collected by distillation and then hydrolyzed to recover the products. These recovery methods can be appropriately selected and optimized depending on, for example, physical properties of the products.

Examples

The present invention will be specifically described below with reference to examples.

Reference Example 1

Production of plasmids each expressing an enzyme catalyzing a reaction to generate 3-oxoadipyl-CoA and coenzyme A (the reaction A), an enzyme catalyzing a reaction to generate 3-hydroxyadipic acid from 3-hydroxyadipyl-CoA (the reaction E) and a reaction to generate α-hydromuconic acid from 2,3-dehydroadipyl-CoA (the reaction F), and a polypeptidc represented by SEQ ID NO: 1, 2, 3, 4, 5, 6, or 7

The pBBR1MCS-2 vector, which is capable of autonomous replication in *E. coli* (ME Kovach, (1995), Gene 166: 175-176), was cleaved with *Xho*I to obtain pBBR1 MCS-2/XhoI. To integrate a constitutive expression promoter into the vector, primers (SEQ ID NOs: 187 and 188) were designed to amplify the upstream 200-b region (SEQ ID NO: 186) of *gapA* (NCBI Gene ID: NC_000913.3) by PCR using the genomic DNA of *Escherichia coli* K-12 MG1655 as a template, and a PCR reaction was performed in accordance with routine procedures. The obtained fragment and pBBR1 MCS-2/XhoI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant *E. coli* strain was confirmed in accordance with routine procedures, and the plasmid was designated as pBBR1MCS-2::Pgap. Then, the pBBR1 MCS-2::Pgap was cleaved with *Sca*I to obtain pBBR1MCS-2::Pgap/ScaI. For amplification of a gene encoding an enzyme catalyzing the reaction A, primers (SEQ ID NOs: 190 and 191) were designed to amplify the full length of the acyl transferase gene *pcaF* (NCBI Gene ID: 1041755, SEQ ID NO: 189) by PCR using the genomic DNA of *Pseudomonas putida* strain KT2440 as a template, and a PCR reaction was performed in accordance with routine procedures. The obtained fragment and the pBBR1 MCS-2::Pgap/ScaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::AT. Then, the pBBR1MCS-2::AT was cleaved with *Hpa*I to obtain pBBR1MCS-2::AT/HpaI. For amplification of a gene encoding an enzyme catalyzing the reactions D and E, primers (SEQ ID NOs: 194 and 195) were designed to amplify a continuous sequence including the full lengths of genes together encoding a CoA transferase, *pcaI* and *pcaJ* (NCBI Gene IDs: 1046613 and 1046612, SEQ ID NOs: 192 and 193) by PCR using the genomic DNA of *Pseudomonas putida* strain KT2440 as a template, and a PCR reaction was performed in accordance with routine procedures. The obtained fragment and the pBBRIMCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCT.

The pBBR1MCS-2::ATCT was cleaved with *Sca*I to obtain pBBR1MCS-2::ATCT/ScaI. For amplification of a nucleic acid encoding a polypeptide represented by SEQ ID NO: 1, primers (SEQ ID NOs: 196 and 197) were designed to amplify the nucleic acid represented by SEQ ID NO: 87 through PCR using the genomic DNA of *Serratia marcescens* strain ATCC13880 as a template, and a PCR reaction was performed in accordance with routine procedures. For amplification of a nucleic acid encoding a polypeptide represented by SEQ ID NO: 2, primers (SEQ ID NOs: 198 and 199) were designed to amplify the nucleic acid represented by SEQ ID NO: 88 through PCR using the genomic DNA of *Serratia nematodiphila* strain DSM21420 as a template, and a PCR reaction was performed in accordance with routine procedures. For amplification of a nucleic acid encoding a polypeptide represented by SEQ ID NO: 3, primers (SEQ ID

NOs: 200 and 201) were designed to amplify the nucleic acid represented by SEQ ID NO: 89 through PCR using the genomic DNA of *Serratia plymuthica* strain NBRC102599 as a template, and a PCR reaction was performed in accordance with routine procedures. For amplification of a nucleic acid encoding a polypeptide represented by SEQ ID NO: 4, primers (SEQ ID NOs: 202 and 203) were designed to amplify the nucleic acid represented by SEQ ID NO: 90 through PCR using the genomic DNA of *Serratia proteamaculans* strain 568 as a template, and a PCR reaction was performed in accordance with routine procedures. For amplification of a nucleic acid encoding a polypeptide represented by SEQ ID NO: 5, primers (SEQ ID NOs: 204 and 205) were designed to amplify the nucleic acid represented by SEQ ID NO: 91 through PCR using the genomic DNA of *Serratia ureilytica* strain Lr5/4 as a template, and a PCR reaction was performed in accordance with routine procedures. For amplification of a nucleic acid encoding a polypeptide represented by SEQ ID NO: 6, primers (SEQ ID NOs: 206 and 207) were designed to amplify the nucleic acid represented by SEQ ID NO: 92 through PCR using the genomic DNA of *Serratia* sp. strain BW106 as a template, and a PCR reaction was performed in accordance with routine procedures. For amplification of a nucleic acid encoding a polypeptide represented by SEQ ID NO: 7, primers (SEQ ID NOs: 208 and 209) were designed to amplify the nucleic acid represented by SEQ ID NO: 93 through PCR using the genomic DNA of *Serratia liquefaciens* strain FK01 as a template, and a PCR reaction was performed in accordance with routine procedures. Each of the obtained fragments and the pBBR1MCS-2::ATCT/ScaI were ligated together using the In-Fusion HD Cloning Kit (manufactured by Takara Bio Inc.), and each of the resulting plasmids was introduced into *E. coli* strain DH5$\alpha$. The nucleotide sequence on the plasmid isolated from each of the obtained recombinant strains was confirmed in accordance with routine procedures.

The plasmid for expression of the polypeptide represented by SEQ ID NO: 1 was designated as "pBBR1MCS-2::ATCTOR1"; the plasmid for expression of the polypeptide represented by SEQ ID NO: 2 was designated as "pBBR1MCS-2::ATCTOR2"; the plasmid for expression of the polypeptide represented by SEQ ID NO: 3 was designated as "pBBR1MCS-2::ATCTOR3"; the plasmid for expression of the polypeptide represented by SEQ ID NO: 4 was designated as "pBBR1MCS-2::ATCTOR4"; the plasmid for expression of the polypeptide represented by SEQ ID NO: 5 was designated as "pBBR1MCS-2::ATCTOR5"; the plasmid for expression of the polypeptide represented by SEQ ID NO: 6 was designated as "pBBR 1MCS-2::ATCTOR6"; and the plasmid for expression of the polypeptide represented by SEQ ID NO: 7 was designated as "pBBR1MCS-2::ATCTOR7"; and these plasmids are listed in Table 6.

[Table 6]

| Plasmid | Originating organism | Gene ID | SEQ ID NO: |
|---|---|---|---|
| pBBR1MCS-2::ATCTOR1 | *Serratia marcescens* ATCC 13880 | JMPQ01000047.1 | 87 |
| pBBR1MCS-2::ATCTOR2 | *Serratia nematodiphila* DSM21420 | JPUX00000000.1 | 88 |
| pBBR1MCS-2::ATCTOR3 | *Serratia plymuthica* NBRC102599 | BCTU01000013.1 | 89 |
| pBBR1MCS-2::ATCTOR4 | *Serratia proteamaculans* 568 | CP000826.1 | 90 |
| pBBR1MCS-2::ATCTOR5 | *Serratia ureilytica* Lr5/4 | JSFB01000001 | 91 |
| pBBR1MCS-2::ATCTOR6 | *Serratia* sp. BW106 | MCGS01000002.1 | 92 |
| pBBR1MCS-2::ATCTOR7 | *Serratia liquefaciens* FK01 | CP006252.1 | 93 |

Reference Example 2

Production of a plasmid for expression of an enzyme catalyzing a reaction to generate 2,3-dehydroadipyl-CoA from 3-hydroxyadipyl-CoA (the reaction C)

The pMW119 expression vector (manufactured by Nippon Gene Co., Ltd.), which is capable of autonomous replication in *E. coli,* was cleaved with *Sac*I to obtain pMW119/SacI. To integrate a constitutive expression promoter into the vector, primers (SEQ ID NOs: 210 and 211) were designed to amplify the upstream 200-b region (SEQ ID NO: 186) of *gapA* (NCBI Gene ID: NC_000913.3) by PCR using the genomic DNA of *Escherichia coli* K-12 MG1655 as a template, and a PCR reaction was performed in accordance with routine procedures. The obtained fragment and the pMW119/SacI were ligated together using the In-Fusion HD Cloning Kit (manufactured by Takara Bio Inc.), and the resulting plasmid was introduced into *E. coli* strain DH5$\alpha$. The nucleotide sequence on the plasmid isolated from the obtained recombinant *E. coli* strain was confirmed in accordance with routine procedures, and the plasmid was designated as pMW119::Pgap. Then, the pMW119::Pgap was cleaved with *Sph*I to obtain pMW119::Pgap/SphI. For amplification of a gene encoding an enzyme catalyzing the reaction C, primers (SEQ ID NOs: 212 and 213) were designed to amplify the full length of the enoyl-CoA hydratase gene *paaF* (NCBI Gene ID: 1046932, SEQ ID NO: 176) by PCR using the genomic DNA of *Pseudomonas putida* strain KT2440 as a template, and a PCR reaction was performed in accordance with routine

procedures. The obtained fragment and the pMW119::Pgap/SphI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures. The obtained plasmid was designated as "pMW119::EH".

Reference Example 3

Production of plasmids each expressing an enzyme catalyzing a reaction to generate 3-oxoadipyl-CoA and coenzyme A from acetyl-CoA and succinyl-CoA (the reaction A), an enzyme catalyzing a reaction to generate adipic acid from adipyl-CoA (the reaction G), and a polypeptide represented by SEQ ID NO: 1, 2, 3, 4, 5, 6, or 7

For amplification of a gene encoding an enzyme catalyzing the reaction G, primers (SEQ ID NOs: 216 and 217) were designed to amplify a continuous sequence including the full lengths of genes together encoding a CoA transferase, *dcaI* and *dcaJ* (NCBI Gene ID: CR543861.1, SEQ ID NOs: 214 and 215) by PCR using the genomic DNA of *Acinetobacter baylyi* strain ADP1 as a template, and a PCR reaction was performed in accordance with routine procedures. The obtained fragment and each of the fragments obtained by cleaving the pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, and pBBR1MCS-2::ATCTOR7 with *Hpa*I, which were produced in Reference Example 1, were ligated together using the In-Fusion HD Cloning Kit, and each of the resulting plasmids was introduced into *E. coli* strain DH5α. The nucleotide sequences on the plasmids isolated from the obtained recombinant strains were confirmed in accordance with routine procedures, and the plasmids were designated as pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, and pBBR1MCS-2::ATCT2OR7.

Reference Example 4

Production of a plasmid for expression of enzymes catalyzing a reaction to generate 2,3-dehydroadipyl-CoA from 3-hydroxyadipyl-CoA (the reaction C) and a reaction to generate adipyl-CoA from 2,3-dehydroadipyl-CoA (the reaction D)

The pMW119::EH was cleaved with *Hind*III to obtain pMW119::EH/HindIII. For amplification of a gene encoding an enzyme catalyzing the reaction D, primers (SEQ ID NOs: 219 and 220) were designed to amplify the full length of *dcaA* (NCBI-Protein ID: AAL09094.1, SEQ ID NO: 218) from *Acinetobacter baylyi* strain ADP1 by PCR, and a PCR reaction was performed in accordance with routine procedures. The obtained fragment and the pMW119::EH/HindIII were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pMW119::EHER.

Example 1

Generation of a mutant microorganism of the genus *Serratia* with impaired pyruvate kinase function

Genes encoding the pyruvate kinase of a microorganism of the genus *Serratia, pykF* and *pykA*, were disrupted to generate a mutant microorganism of the genus *Serratia* with impaired pyruvate kinase function.

The procedure for disrupting *pykF* and *pykA* followed the method described in Proc Natl Acad Sci U S A., 2000 Jun 6, 97(12): 6640-6645.

Generation of a mutant microorganism of the genus *Serratia* deficient in *pykF*

A PCR reaction was performed using pKD4 as a template and oligo DNAs represented by SEQ ID NOs: 221 and 222 as primers to obtain a PCR fragment of 1.6 kb in length for disruption of *pykF.* A FRT recombinase expression plasmid, pKD46, was introduced into *Serratia grimesii* strain NBRC13537, and an ampicillin-resistant strain was obtained. The obtained strain was inoculated into 5 mL of LB medium containing 500 μg/mL ampicillin and was cultured at 30°C with shaking for 1 day. Subsequently, 0.5 mL of the culture fluid was inoculated into 50 mL of LB medium containing 500 μg/mL ampicillin and 50 mM arabinose and was cultured in rotation at 30°C for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 5 μL of the PCR fragment, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the

electroporation cuvette immediately after the electroporation, and the bacterial cells in the cuvette were incubated at 30°C with shaking for 2 hours. The total volume of the culture was applied to LB agar medium containing 25 μg/mL kanamycin and was incubated at 30°C for 1 day. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 225 were used.

Subsequently, one of the kanamycin-resistant strains was inoculated into 5 mL of LB medium and was cultured at 37°C and passaged twice to segregate away the pKD46 and to obtain an ampicillin-sensitive strain. The plasmid pCP20 was introduced into the ampicillin-sensitive strain, and ampicillin-resistant strains were again obtained. After culturing the obtained strains at 40°C, colony direct PCR was performed on the resulting strains to confirm the deletion of the kanamycin resistance gene from the length of the amplified band. Oligo DNA primers represented by SEQ ID NOs: 224 and 225 were used. Subsequently, one of the kanamycin-sensitive strains was inoculated into 5 mL of LB medium and was cultured at 37°C and passaged twice to segregate away the pCP20. The obtained strain was designated as *Serratia grimesii* NBRC13537 *ΔpykF.*

Generation of a mutant microorganism of the genus *Serratia* deficient in *pykA*

A PCR reaction was performed using pKD4 as a template and oligo DNAs represented by SEQ ID NOs: 226 and 227 as primers to obtain a PCR fragment of 1.6 kb in length for disruption of *pykA.*

By the same method as used for the generation of the *pykF*-deficient strain, *pykA* was disrupted in the *Serratia grimesii* NBRC13537 *ΔpykF* strain. After the plasmid pKD46 was introduced into the above strain, the PCR fragment used for disruption of *pykA* was introduced to the resulting strain. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 229 were used.

Subsequently, an ampicillin-sensitive strain was obtained by segregating away the pKD46. The plasmid pCP20 was introduced into the ampicillin-sensitive strain, and ampicillin-resistant strains were again obtained. Colony direct PCR was performed on the obtained strains to confirm the deletion of the kanamycin resistance gene from the length of the amplified band. Oligo DNA primers represented by SEQ ID NOs: 228 and 229 were used. The pCP20 was segregated away from one of the kanamycin-sensitive strains. The obtained strain was designated as SgΔPP.

Example 2

Generation of mutant microorganisms of the genus *Serratia* with impaired pyruvate kinase function and carrying a plasmid expressing enzymes that catalyze the reactions A, B, E, and F

Each of the plasmids produced in Reference Example 1 was introduced into the SgΔPP produced in Example 1 to generate mutant microorganisms of the genus *Serratia.* Additionally, a mutant microorganism of the genus *Serratia* was generated as a control by introducing the pBBR1MCS-2 empty vector into the SgΔPP.

The SgΔPP was inoculated into 5 mL of LB medium and cultured at 30°C with shaking for 1 day. Subsequently, 0.5 mL of the culture fluid was inoculated into 5 mL of LB medium and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2 (control), pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 25 μg/mL kanamycin and was incubated at 30°C for 1 day. The obtained strains were designated as SgΔPP/pBBR (negative control), SgΔPP/3HA1, SgΔPP/3HA2, SgΔPP/3HA3, SgΔPP/3HA4, SgΔPP/3HA5, SgΔPP/3HA6, and SgΔPP/3HA7.

Reference Example 5

Generation of mutant microorganisms of the genus *Serratia* with intact pyruvate kinase function and carrying a plasmid expressing enzymes that catalyze the reactions A, B, E, and F

By the same method as in Example 2, the pBBR1MCS-2 (control), pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into *Serratia grimesii* NBRC13537. The obtained strains

were designated as Sg/pBBR (negative control), Sg/3HA1, Sg/3HA2, Sg/3HA3, Sg/3HA4, Sg/3HA5, Sg/3HA6, and Sg/3HA7.

Example 3

Production test of 3-hydroxyadipic acid and α-hydromuconic acid using mutant microorganisms of the genus *Serratia* with impaired pyruvate kinase function

The production test of 3-hydroxyadipic acid and α-hydromuconic acid was conducted using the mutant microorganisms of the genus *Serratia* produced in Example 2.

A loopful of each mutant produced in Example 2 was inoculated into 5 mL (in a glass test tube of 18-mm diameter with aluminum cap) of the culture medium I (10 g/L Bacto Tryptone (manufactured by Difco Laboratories), 5 g/L Bacto Yeast Extract (manufactured by Difco Laboratories), 5 g/L sodium chloride, 25 μg/mL kanamycin) adjusted to pH 7 and was cultured at 30°C with shaking at 120 $min^{-1}$ for 24 hours. Subsequently, 0.25 mL of the culture fluid was added to 5 mL (in a glass test tube of 18-mm diameter with aluminum cap) of the culture medium II (50g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 25 μg/mL kanamycin) adjusted to pH 6.5 and was cultured at 30°C with shaking at 120 $min^{-1}$ for 24 hours.

Quantitative analysis of substrate and product

The supernatant separated from bacterial cells by centrifugation of each culture fluid was processed by membrane treatment using Millex-GV (0.22 μm; PVDF; manufactured by Merck KGaA), and the resulting filtrate was analyzed by the following methods to quantify the concentrations of 3-hydroxyadipic acid, α-hydromuconic acid, and other products accumulated in the culture supernatant and the concentration of sugars remaining unused in the culture medium. The yields of 3-hydroxyadipic acid and α-hydromuconic acid calculated using the above formula (2) from the measurement results are shown in Table 7. However, a concentration of not more than 0.1 mg/L is considered to be below the detection limit in the quantitative LC-MS/MS analysis and is hereinafter denoted in each table as N.D.

Quantitative analysis of 3-hydroxyadipic acid and α-hydromuconic acid by LC-MS/MS

- HPLC: 1290 Infinity (manufactured by Agilent Technologies, Inc.)

    Column: Synergi hydro-RP (manufactured by Phenomenex Inc.), length: 100 mm, internal diameter: 3 mm, particle size: 2.5 μm
    Mobile phase: 0.1% aqueous formic acid solution / methanol = 70/30
    Flow rate: 0.3 mL/min
    Column temperature: 40°C
    LC detector: 1260DAD VL+ (210 nm)

- MS/MS: Triple-Quad LC/MS (manufactured by Agilent Technologies, Inc.) Ionization method: ESI in negative mode.

Quantitative analysis of organic acids by HPLC

- HPLC:LC-10A (manufactured by Shimadzu Corporation)

    Column: Shim-pack SPR-H (manufactured by Shimadzu GLC Ltd.), length: 250 mm, internal diameter: 7.8 mm, particle size: 8 μm
    Shim-pack SCR-101H (manufactured by Shimadzu GLC Ltd.) length: 250 mm, internal diameter: 7.8 mm, particle size: 10 μm
    Mobile phase: 5 mM *p*-toluenesulfonic acid
    Reaction solution: 5 mM *p*-toluenesulfonic acid, 0.1 mM EDTA, 20 mM Bis-Tris Flow rate: 0.8 mL/min
    Column temperature: 45°C
    Detector: CDD-10Avp (manufactured by Shimadzu Corporation)

Quantitative analysis of sugars and alcohol by HPLC

- HPLC: Shimazu Prominence (manufactured by Shimadzu Corporation)

  Column: Shodex Sugar SH1011 (manufactured by Showa Denko K.K.), length: 300 mm, internal diameter: 8 mm, particle size: 6 μm
  Mobile phase: 0.05M aqueous sulfuric acid solution
  Flow rate: 0.6 mL/min
  Column temperature: 65°C
  Detector: RID-10A (manufactured by Shimadzu Corporation).

Comparative Example 1

Production test of 3-hydroxyadipic acid and α-hydromuconic acid using mutant microorganisms of the genus *Serratia* with intact pyruvate kinase function

The mutant microorganisms of the genus *Serratia* produced in Reference Example 5 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measurement results are shown in Table 7.
By comparing the results of Comparative Example 1 and Example 3, it was found that the yields of 3-hydroxyadipic acid and α-hydromuconic acid were increased by impairing the function of pyruvate kinase in the microorganism of the genus *Serratia.*

[Table 7]

| | Strain | Yield of 3HA (%) | Yield of HMA (%) |
|---|---|---|---|
| Example 3 | SgΔPP/pBBR | 0.0362 | 0.0113 |
| | SgΔPP/3HA1 | 3.47 | 0.0782 |
| | SgΔPP/3HA2 | 5.78 | 0.0960 |
| | SgΔPP/3HA3 | 5.24 | 0.0846 |
| | SgΔPP/3HA4 | 5.10 | 0.0909 |
| | SgΔPP/3HA5 | 6.21 | 0.107 |
| | SgΔPP/3HA6 | 6.28 | 0.103 |
| | SgΔPP/3HA7 | 4.96 | 0.0638 |
| Comparative Example 1 | Sg/pBBR | N.D. | N.D. |
| | Sg/3HA1 | 0.784 | 0.0293 |
| | Sg/3HA2 | 1.15 | 0.0470 |
| | Sg/3HA3 | 0.942 | 0.0461 |
| | Sg/3HA4 | 0.875 | 0.0418 |
| | Sg/3HA5 | 1.01 | 0.0529 |
| | Sg/3HA6 | 1.03 | 0.0366 |
| | Sg/3HA7 | 0.943 | 0.0237 |

Example 4

Generation of an *E. coli* mutant with impaired pyruvate kinase function

Genes encoding the pyruvate kinase *of E. coli*, *pykF* and *pykA*, were disrupted to generate an *E. coli* mutant with impaired pyruvate kinase function. The procedure for disrupting *pykF* and *pykA* followed the method described in Proc Natl Acad Sci USA., 2000 Jun 6, 97(12): 6640-6645.

Generation of an *E. coli* mutant deficient in *pykF*

A PCR reaction was performed using pKD4 as a template and oligo DNAs represented by SEQ ID NOs: 230 and 231 as primers to obtain a PCR fragment of 1.6 kb in length for disruption of *pykF.*

A FRT recombinase expression plasmid, pKD46, was introduced into *Escherichia coli* strain MG1655, and an ampicillin-resistant strain was obtained. The obtained strain was inoculated into 5 mL of LB medium containing 100 μg/mL ampicillin and cultured at 30°C with shaking for 1 day. Subsequently, 0.5 mL of the culture fluid was inoculated into 50 mL of LB medium containing 100 μg/mL ampicillin and 50 mM arabinose, and was cultured in rotation at 30°C for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 5 μL of the PCR fragment, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 2 hours. The total volume of the culture was applied to LB agar medium containing 25 μg/mL kanamycin and was incubated at 30°C for 1 day. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 233 were used.

Subsequently, one of the kanamycin-resistant strains was inoculated into 5 mL of LB medium and was cultured at 37°C and passaged twice to segregate away the pKD46 and to obtain an ampicillin-sensitive strain. The plasmid pCP20 was introduced into the ampicillin-sensitive strain, and ampicillin-resistant strains were again obtained. After culturing the obtained strains at 40°C, direct colony PCR was performed on the resulting strains to confirm the deletion of the kanamycin resistance gene from the length of the amplified band. Oligo DNA primers represented by SEQ ID NOs: 232 and 233 were used. Subsequently, one of the kanamycin-sensitive strains was inoculated into 5 mL of LB medium and was cultured at 37°C and passaged twice to segregate away the pCP20. The obtained strain was designated as *Escherichia coli* MG1655 *ΔpykF.*

Generation of an *E. coli* mutant deficient in *pykA*

A PCR reaction was performed using pKD4 as a template and oligo DNAs represented by SEQ ID NOs: 234 and 235 as primers to obtain a PCR fragment of 1.6 kb in length for disruption of *pykA.*

By the same method as used for the generation of the *pykF*-deficient strain, *pykA* was disrupted in the *Escherichia coli* MG1655 *ΔpykF* strain. After the plasmid pKD46 was introduced into the above strain, the PCR fragment used for disruption of *pykA* was introduced to the resulting strain. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 224 were used.

Subsequently, an ampicillin-sensitive strain was obtained by segregating away the pKD46. The plasmid pCP20 was introduced into the ampicillin-sensitive strain, and ampicillin-resistant strains were again obtained. Direct colony PCR was performed on the obtained strains to confirm the deletion of the kanamycin resistance gene from the length of the amplified band. Oligo DNA primers represented by SEQ ID NOs: 236 and 237 were used. The pCP20 was segregated away from one of the kanamycin-sensitive strains. The obtained strain was designated as EcΔPP.

Example 5

Generation *of E. coli* mutants with impaired pyruvate kinase function and carrying a plasmid expressing enzymes that catalyze the reactions A, B, E, and F

Each of the plasmids produced in Reference Example 1 was introduced into the EcΔPP produced in Example 4 to generate *E. coli* mutants.

The EcΔPP was inoculated into 5 mL of LB medium and cultured at 30°C with shaking for 1 day. Subsequently,

0.5 mL of the culture fluid was inoculated into 5 mL of LB medium and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2 (negative control), pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1 MCS-2::ATCTOR6, or pBBR1MCS-2::ATCTOR7, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 25 μg/mL kanamycin and was incubated at 30°C for 1 day. The obtained strains were designated as EcΔPP/pBBR (negative control), EcΔPP/3HA1, EcΔPP/3HA2, EcΔPP/3HA3, EcΔPP/3HA4. EcΔPP/3HA5, EcΔPP/3HA6, and EcΔPP/3HA7.

Reference Example 6

Generation *of E. coli* mutants with intact pyruvate kinase function and carrying a plasmid expressing enzymes that catalyze the reactions A, B, E, and F

By the same method as in Example 5, the pBBR1MCS-2 (control), pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into *Escherichia coli* MG1655. The obtained strains were designated as Ec/pBBR (negative control), Ec/3HA1, Ec/3HA2, Ec/3HA3, Ec/3HA4, Ec/3HA5, Ec/3HA6, and Ec/3HA7.

Example 6

Production test of 3-hydroxyadipic acid and α-hydromuconic acid using *E. coli* mutants with impaired pyruvate kinase function

The mutants produced in Example 5 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 8.

Comparative Example 2

Production test of 3-hydroxyadipic acid and α-hydromuconic acid using *E. coli* mutants with intact pyruvate kinase function

The mutants produced in Reference Example 6 were cultured in the same manner as in Example 6. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 8.

By comparing the results of Comparative Example 2 and Example 6, it was found that the yields of 3-hydroxyadipic acid and α-hydromuconic acid were increased by impairing the function of pyruvate kinase in *E. coli.*

[Table 8]

| | Strain | Yield of 3HA (%) | Yield of HMA (%) |
|---|---|---|---|
| Example 6 | EcΔPP/pBBR | 0.0427 | 0.0132 |
| | EcΔPP/3HA1 | 2.54 | 0.0292 |
| | EcΔPP/3HA2 | 3.97 | 0.0333 |
| | EcΔPP/3HA3 | 3.64 | 0.0273 |
| | EcΔPP/3HA4 | 2.86 | 0.0257 |
| | EcΔPP/3HA5 | 3.67 | 0.0269 |
| | EcΔPP/3HA6 | 3.57 | 0.0348 |
| | EcΔPP/3HA7 | 3.13 | 0.0274 |

(continued)

| | Strain | Yield of 3HA (%) | Yield of HMA (%) |
|---|---|---|---|
| Comparative Example 2 | Ec/pBBR | N.D. | N.D. |
| | Ec/3HA1 | 1.48 | 0.0172 |
| | Ec/3HA2 | 2.59 | 0.0160 |
| | Ec/3HA3 | 2.64 | 0.0167 |
| | Ec/3HA4 | 1.82 | 0.0186 |
| | Ec/3HA5 | 2.47 | 0.0166 |
| | Ec/3HA6 | 2.66 | 0.0228 |
| | Ec/3HA7 | 1.78 | 0.0172 |

Example 7

Generation of mutant microorganisms of the genus *Serratia* with impaired pyruvate kinase function and carrying plasmids expressing enzymes that catalyze the reactions A, B, C, E, and F

The plasmid pMW119::EH produced in Reference Example 2 was introduced into each mutant microorganism of the genus *Serratia* produced in Example 2 to generate mutant microorganisms of the genus *Serratia.* Additionally, a mutant microorganism of the genus *Serratia* was generated as a control by introducing the pMW119 empty vector into the SgΔPP/pBBR produced in Example 2.

The SgΔPP/pBBR, SgΔPP/3HA1, SgΔPP/3HA2, SgΔPP/3HA3, SgΔPP/3HA4, SgΔPP/3HA5, SgΔPP/3HA6, or SgΔPP/3HA7 was inoculated into 5 mL of LB medium containing 25 μg/mL kanamycin and cultured at 30°C with shaking for 1 day. Subsequently, 0.5 mL of the culture fluid was inoculated into 5 mL of LB medium containing 25 μg/mL kanamycin and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2 (control) or pMW119::EH, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 500 μg/mL ampicillin and 25 μg/mL kanamycin and was incubated at 30°C for 1 day. The obtained strains were designated as SgΔPP/pBBRpMW (negative control), SgΔPP/HMA1, SgΔPP/HMA2, SgΔPP/HMA3, SgΔPP/HMA4, SgΔPP/HMA5, SgΔPP/HMA6, and SgΔPP/HMA7.

Reference Example 7

Generation of mutant microorganisms of the genus *Serratia* with intact pyruvate kinase function and carrying plasmids expressing enzymes that catalyze the reactions A, B, C, E, and F

By the same method as in Example 7, the pMW119 (control) or pMW119::EH was introduced into Sg/pBBR, Sg/3HA1, Sg/3HA2, Sg/3HA3, Sg/3HA4, Sg/3HA5, Sg/3IIA6, and Sg/3HA7. The obtained strains were designated as Sg/pBBRpMW (negative control), Sg/HMA1, Sg/HMA2, Sg/HMA3, Sg/HMA4, Sg/HMA5, Sg/HMA6, and Sg/HMA7.

Example 8

Production test of α-hydromuconic acid using mutant microorganisms of the genus *Serratia* with impaired pyruvate kinase function

The mutants produced in Example 7 were cultured in the same manner as in Example 3, except that ampicillin was added to the culture medium to a final concentration of 500 μg/mL. The concentrations of α-hydromuconic acid and other products accumulated in the culture supernatant and the concentration of sugars remaining unused in the culture medium were quantified. The yield of α-hydromuconic acid calculated using the above formula (2) from the measured values is shown in Table 9.

Comparative Example 3

Production test of α-hydromuconic acid using mutant microorganisms of the genus *Serratia* with intact pyruvate kinase function

The mutants produced in Reference Example 7 were cultured in the same manner as in Example 8. The concentrations of α-hydromuconic acid and other products accumulated in the culture supernatant and the concentration of sugars remaining unused in the culture medium were quantified. The yield of a-hydromuconic acid calculated using the above formula (2) from the measured values is shown in Table 9.

By comparing the results of Comparative Example 3 and Example 8, it was found that the yield of α-hydromuconic acid was increased by impairing the function of pyruvate kinase in the microorganism of the genus *Serralia.*

[Table 9]

| | Strain | Yield of HMA (%) |
|---|---|---|
| Example 8 | SgΔPP/pBBRpMW | 0.0119 |
| | SgΔPPHMA1 | 0.156 |
| | SgΔPP/HMA2 | 0.179 |
| | SgΔPP/HMA3 | 0.153 |
| | SgΔPP/HMA4 | 0.118 |
| | SgΔPP/HMA5 | 0.217 |
| | SgΔPP/HMA6 | 0.241 |
| | SgΔPP/HMA7 | 0.140 |
| Comparative Example 3 | Sg/pBBRpMW | N.D. |
| | Sg/HMA1 | 0.0495 |
| | Sg/HMA2 | 0.0584 |
| | Sg/HMA3 | 0.0434 |
| | Sg/HMA4 | 0.0524 |
| | Sg/HMA5 | 0.0587 |
| | Sg/HMA6 | 0.0618 |
| | Sg/HMA 7 | 0.0519 |

Example 9

Generation of *E. coli* mutants with impaired pyruvate kinase function and carrying plasmids expressing enzymes that catalyze the reactions A, B, C, E, and F

The plasmid pMW119::EH produced in Reference Example 2 was introduced into each of the *E. coli* mutants produced in Example 5 to generate *E. coli* mutants. Additionally, an *E. coli* mutant was generated as a control by introducing the pMW119 empty vector into the EcΔPP/pBBR produced in Example 5.

The EcΔPP/pBBR, EcΔPP/3HA1, EcΔPP/3HA2, EcΔPP/3HA3, EcΔPP/3HA4, EcΔPP/3HA5, EcΔPP/3IIA6, or EcΔPP/3HA7 was inoculated into 5 mL of LB medium containing 25 μg/mL kanamycin and cultured at 30°C with shaking for 1 day. Subsequently, 0.5 mL of the culture fluid was inoculated into 5 mL of LB medium containing 25 μg/mL kanamycin and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2 (control) or pMW119::EH, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 100 μg/mL ampicillin and 25 μg/mL kanamycin and was incubated at 30°C for 1 day. The obtained strains were designated as EcΔPP/pBBRpMW (negative control),

EcΔPP/HMA1, EcΔPP/HMA2, EcΔPP/HMA3, EcΔPP/HMA4, EcΔPP/HMA5, EcΔPP/HMA6, and EcΔPP/HMA7.

Reference Example 8

Generation of *E. coli* mutants with intact pyruvate kinase function and carrying plasmids expressing enzymes that catalyze the reactions A, B, C, E, and F

By the same method as in Example 9, the pMW119 (control) or pMW119::EH was introduced into the Ec/pBBR, Ec/3HA1, Ec/3HA2, Ec/3HA3, Ec/3HA4, Ec/3HA5, Ec/3HA6, and Ec/3HA7. The obtained strains were designated as Ec/pBBRpMW (negative control), Ec/HMA1, Ec/HMA2, Ec/HMA3, Ec/HMA4, Ec/HMA5, Ec/HMA6, and Ec/HMA7.

Example 10

Production test of α-hydromuconic acid using *E. coli* mutants with impaired pyruvate kinase function

The mutants produced in Reference Example 9 were cultured in the same manner as in Example 6, except that ampicillin was added to the culture medium to a concentration of 100 μg/mL. The concentrations of α-hydromuconic acid and other products accumulated in the culture supernatant and the concentration of sugars remaining unused in the culture medium were quantified. The yield of a-hydromuconic acid calculated using the above formula (2) from the measured values is shown in Table 10.

Comparative Example 4

Production test of α-hydromuconic acid using *E. coli* mutants with intact pyruvate kinase function

The mutants produced in Reference Example 8 were cultured in the same manner as in Example 10. The concentrations of α-hydromuconic acid and other products accumulated in the culture supernatant and the concentration of sugars remaining unused in the culture medium were quantified. The yield of a-hydromuconic acid calculated using the above formula (2) from the measured values is shown in Table 10.

By comparing the results of Comparative Example 4 and Example 10, it was found that the yield of α-hydromuconic acid was increased by impairing the function of pyruvate kinase in *E. coli.*

[Table 10]

| | Strain | Yield of HMA (%) |
|---|---|---|
| Example 10 | EcΔPP/pBBRpMW | 0.0167 |
| | EcΔPP/HMA1 | 0.0511 |
| | EcΔPP/HMA2 | 0.0818 |
| | EcΔPP/HMA3 | 0.0717 |
| | EcΔPP/HMA4 | 0.0688 |
| | EcΔPP/HMA5 | 0.0765 |
| | EcΔPP/HMA6 | 0.0761 |
| | EcΔPP/HMA7 | 0.0599 |
| Comparative Example 4 | Ec/pBBRpMW | N.D. |
| | Ec/HMA1 | 0.0362 |
| | Ec/HMA2 | 0.0636 |
| | Ec/HMA3 | 0.0569 |
| | Ec/HMA4 | 0.0624 |
| | Ec/HMA5 | 0.0621 |
| | Ec/HMA6 | 0.0640 |
| | Ec/HMA7 | 0.0491 |

Example 11

Generation of mutant microorganisms of the genus *Serratia* with impaired pyruvate kinase function and carrying plasmids expressing enzymes that catalyze the reactions A, B, C, D, and G

By the same method as in Example 2, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into the SgΔPP. By the same method as in Example 7, the plasmid pMW119::EHER produced in Reference Example 4 was introduced into each of the obtained mutants to generate mutant microorganisms of the genus *Serratia*. The obtained strains were designated as SgΔPP/ADA1, SgΔPP/ADA2, SgΔPP/ADA3, SgΔPP/ADA4, SgΔPP/ADA5, SgΔPP/ADA6, and SgΔPP/ADA7.

Reference Example 9

Generation of mutant microorganisms of the genus *Serratia* with intact pyruvate kinase function and carrying plasmids expressing enzymes that catalyze the reactions A, B, C, D, and G

By the same method as in Example 11, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into *Serratia grimesii* NBRC13537. By the same method as in Example 7, the plasmid pMW119::EHER produced in Reference Example 4 was introduced into each of the obtained mutants to generate mutant microorganisms of the genus *Serratia.* The obtained strains were designated as Sg/ADA1, Sg/ADA2, Sg/ADA3, Sg/ADA4, Sg/ADA5, Sg/ADA6, and Sg/ADA7.

Example 12

Production test of adipic acid using mutant microorganisms of the genus *Serratia* with impaired pyruvate kinase function

The mutants produced in Example 11 and the SgΔPP/pBBRpMW (negative control) were cultured in the same manner as in Example 8. The concentrations of adipic acid and other products accumulated in the culture supernatant and the concentration of sugars remaining unused in the culture medium were quantified. The quantification of adipic acid was performed using LC-MS/MS under the same conditions for the quantification of 3-hydroxyadipic acid and α-hydromuconic acid. The yield of adipic acid calculated using the above formula (2) from the measured values is shown in Table 11.

Comparative Example 5

Production test of adipic acid using mutant microorganisms of the genus *Serratia* with intact pyruvate kinase function

The mutants produced in Reference Example 9 and the Sg/pBBRpMW were cultured in the same manner as in Example 8. The concentrations of adipic acid and other products accumulated in the culture supernatant and the concentration of sugars remaining unused in the culture medium were quantified. The yield of adipic acid calculated using the above formula (2) from the measured values is shown in Table 11.

By comparing the results of Comparative Example 5 and Example 12, it was found that the yield of adipic acid was increased by impairing the function of pyruvate kinase in the microorganism of the genus *Serratia.*

[Table 11]

| | Strain | Yield of ADA (%) |
|---|---|---|
| Example 12 | SgΔPP/pBBRpMW | N.D. |
| | SgΔPP/ADA1 | 0.0783 |
| | SgΔPP/ADA2 | 0.110 |
| | SgΔPP/ADA3 | 0.0861 |
| | SgΔPP/ADA4 | 0.116 |
| | SgΔPP/ADA5 | 0.108 |
| | SgΔPP/ADA6 | 0.136 |
| | SgΔPP/ADA7 | 0.0958 |
| Comparative Example 5 | Sg/pBBRpMW | N.D. |
| | Sg/ADA1 | 0.0244 |
| | Sg/ADA2 | 0.0325 |
| | Sg/ADA3 | 0.0254 |
| | Sg/ADA4 | 0.0246 |
| | Sg/ADA5 | 0.0314 |
| | Sg/ADA6 | 0.0264 |
| | Sg/ADA7 | 0.0289 |

Example 13

Generation of *E. coli* mutants with impaired pyruvate kinase function and carrying plasmids expressing enzymes that catalyze the reactions A, B, C, D, and G

By the same method as in Example 5, the PBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into the EcΔPP. By the same method as in Example 9, the plasmid pMW119::EHER produced in Reference Example 4 was introduced into each of the obtained mutants to generate *E. coli* mutants. The obtained strains were designated as EcΔPP/ADA1, EcΔPP/ADA2, EcΔPP/ADA3, EcΔPP/ADA4, EcΔPP/ADA5, EcΔPP/ADA6, and EcΔPP/ADA7.

Reference Example 10

Generation of *E. coli* mutants with intact pyruvate kinase function and carrying plasmids expressing enzymes that catalyze the reactions A, B, C, D, and G

By the same method as in Example 13, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into *Escherichia coli* MG1655. By the same method as in Example 9, the plasmid pMW119::EHER produced in Reference Example 4 was introduced into each of the obtained mutants to generate *E. coli* mutants. The obtained strains were designated as Ec/ADA1, Ec/ADA2, Ec/ADA3, Ec/ADA4, Ec/ADA5, Ec/ADA6, and Ec/ADA7.

Example 14

Production test of adipic acid using *E. coli* mutants with impaired pyruvate kinase function

The mutants produced in Example 13 and the EcΔPP/pBBRpMW were cultured in the same manner as in Example 10. The concentrations of adipic acid and other products accumulated in the culture supernatant and the concentration of sugars remaining unused in the culture medium were quantified. The quantification of adipic acid was

performed using LC-MS/MS under the same conditions for the quantification of 3-hydroxyadipic acid and α-hydromuconic acid. The yield of adipic acid calculated using the above formula (2) from the measured values is shown in Table 12.

Comparative Example 6

Production test of adipic acid using *E. coli* mutants with intact pyruvate kinase function

The mutants produced in Reference Example 10 and the Ec/pBBRpMW were cultured in the same manner as in Example 10. The concentrations of adipic acid and other products accumulated in the culture supernatant and the concentration of sugars remaining unused in the culture medium were quantified. The yield of adipic acid calculated using the above formula (2) from the measured values is shown in Table 12.
By comparing the results of Comparative Example 6 and Example 14, it was found that the yield of adipic acid was increased by impairing the function of pyruvate kinase in *E. coli.*

[Table 12]

| | Strain | Yield of ADA (%) |
|---|---|---|
| Example 14 | EcΔPP/pBBRpMW | N.D. |
| | EcΔPP/ADA1 | 0.0213 |
| | EcΔPP/ADA2 | 0.0338 |
| | EcΔPP/ADA3 | 0.0293 |
| | EcΔPP/ADA4 | 0.0315 |
| | EcΔPP/ADA5 | 0.0382 |
| | EcΔPP/ADA6 | 0.0407 |
| | EcΔPP/ADA7 | 0.0235 |
| Comparative Example 6 | Ec/pBBRpMW | N.D. |
| | Ec/ADA1 | 0.0148 |
| | Ec/ADA2 | 0.0153 |
| | Ec/ADA3 | 0.0107 |
| | Ec/ADA4 | 0.0192 |
| | Ec/ADA5 | 0.0139 |
| | Ec/ADA6 | 0.0147 |
| | Ec/ADA7 | 0.0167 |

Example 15

Production test 2 of 3-hydroxyadipic acid and α-hydromuconic acid using mutant microorganisms of the genus *Serratia* with impaired pyruvate kinase function

The production test of 3-hydroxyadipic acid and α-hydromuconic acid was conducted using the mutant microorganisms of the genus *Serratia* produced in Example 2 under anaerobic conditions.
The mutant microorganisms of the genus *Serratia* produced in Example 2 were cultured in the same manner as in Example 3, except that the mutant microorganisms were cultured statically using the culture medium II. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 13.

Comparative Example 7

Production test 2 of 3-hydroxyadipic acid and α-hydromuconic acid using mutant microorganisms of the genus *Serratia* with intact pyruvate kinase function

The mutants produced in Reference Example 5 were cultured in the same manner as in Example 15. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 13.

By comparing the results of Comparative Example 7 and Example 15, it was found that the yields of 3-hydroxyadipic acid and α-hydromuconic acid were increased even under anaerobic conditions by impairing the function of pyruvate kinase in the microorganism of the genus *Serratia.*

[Table 13]

| | Strain | Yield of 3HA (%) | Yield of HMA (%) |
|---|---|---|---|
| Example 15 | SgΔPP/pBBR | 0.0485 | 0.0224 |
| | SgΔPP/3HA1 | 4.84 | 0.159 |
| | SgΔPP/3HA2 | 6.07 | 0.171 |
| | SgΔPP/3HA3 | 5.99 | 0.143 |
| | SgΔPP/3HA4 | 5.30 | 0.195 |
| | SgΔPP/3HA5 | 5.84 | 0.180 |
| | SgΔPP/3HA6 | 6.02 | 0.202 |
| | SgΔPP/3HA7 | 5.98 | 0.160 |
| Comparative Example 7 | Sg/pBBR | N.D. | N.D. |
| | Sg/3HA1 | 1.68 | 0.0482 |
| | Sg/3HA2 | 2.46 | 0.0577 |
| | Sg/3HA3 | 1.94 | 0.0471 |
| | Sg/3HA4 | 1.99 | 0.0527 |
| | Sg/3HA5 | 2.29 | 0.0523 |
| | Sg/3HA6 | 2.95 | 0.0627 |
| | Sg/3HA7 | 1.66 | 0.0595 |

Example 16

Production test 2 of 3-hydroxyadipic acid and α-hydromuconic acid using *E. coli* mutants with impaired pyruvate kinase function

The production test of 3-hydroxyadipic acid and α-hydromuconic acid was conducted under anaerobic conditions using the *E. coli* mutants produced in Example 5.

The *E. coli* mutants produced in Example 5 were cultured in the same manner as in Example 6, except that the mutants were cultured statically using the culture medium II. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 14.

Comparative Example 8

Production test 2 of 3-hydroxyadipic acid and α-hydromuconic acid using *E. coli* mutants with intact pyruvate kinase function

The mutants produced in Reference Example 6 were cultured in the same manner as in Example 16. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 14.
By comparing the results of Comparative Example 8 and Example 16, it was found that the yields of 3-hydroxyadipic acid and α-hydromuconic acid were increased even under anaerobic conditions by impairing the function of pyruvate kinase in *E. coli.*

[Table 14]

| | Strain | Yield of 3HA (%) | Yield of HMA (%) |
|---|---|---|---|
| Example 16 | EcΔPP/pBBR | 0.0669 | 0.0113 |
| | EcΔPP/3HA1 | 13.2 | 0.0213 |
| | EcΔPP/3HA2 | 14.9 | 0.0277 |
| | EcΔPP/3HA3 | 13.9 | 0.0268 |
| | EcΔPP/3HA4 | 14.1 | 0.0224 |
| | EcΔPP/3HA5 | 14.3 | 0.0259 |
| | EcΔPP/3HA6 | 14.7 | 0.0226 |
| | EcΔPP/3HA7 | 13.2 | 0.0213 |
| Comparative Example 8 | Ec/pBBR | N.D. | N.D. |
| | Ec/3HA1 | 1.32 | 0.0171 |
| | Ec/3HA2 | 2.00 | 0.0154 |
| | Ec/3HA3 | 1.82 | 0.0130 |
| | Ec/3HA4 | 1.47 | 0.0136 |
| | Ec/3HA5 | 2.17 | 0.0138 |
| | Ec/3HA6 | 1.77 | 0.0166 |
| | Ec/3HA7 | 1.14 | 0.0179 |

Example 17

Production test 2 of adipic acid using mutant microorganisms of the genus *Serratia* with impaired pyruvate kinase function

The production test of adipic acid was conducted using the mutant microorganisms of the genus *Serratia* produced in Example 11 under anaerobic conditions.
The mutant microorganisms of the genus *Serratia* produced in Example 11 were cultured in the same manner as in Example 12, except that the mutant microorganisms were cultured statically using the culture medium II. The concentrations of adipic acid and other products accumulated in the culture supernatant and the concentration of sugars remaining unused in the culture medium were quantified. The yield of adipic acid calculated using the above formula (2) from the measured values is shown in Table 15.

Comparative Example 9

Production test 2 of adipic acid using mutant microorganisms of the genus *Serratia* with intact pyruvate kinase function

The mutants produced in Reference Example 9 were cultured in the same manner as in Example 17. The concentrations of adipic acid and other products accumulated in the culture supernatant and the concentration of sugars

remaining unused in the culture medium were quantified. The yield of adipic acid calculated using the above formula (2) from the measured values is shown in Table 15.

By comparing the results of Comparative Example 9 and Example 17, it was found that the yield of adipic acid was increased even under anaerobic conditions by impairing the function of pyruvate kinase in the microorganism of the genus *Serratia.*

[Table 15]

| | Strain | Yield of ADA (%) |
|---|---|---|
| Example 17 | SgΔPP/pBBRpMW | N.D. |
| | SgΔPP/ADA1 | 0.0359 |
| | SgΔPP/ADA2 | 0.0480 |
| | SgΔPP/ADA3 | 0.0379 |
| | SgΔPP/ADA4 | 0.0395 |
| | SgΔPP/ADA5 | 0.0431 |
| | SgΔPP/ADA6 | 0.0490 |
| | SgΔPP/ADA7 | 0.0375 |
| Comparative Example 9 | Sg/pBBRpMW | N.D. |
| | Sg/ADA1 | 0.0152 |
| | Sg/ADA2 | 0.0181 |
| | Sg/ADA3 | 0.0188 |
| | Sg/ADA4 | 0.0179 |
| | Sg/ADA5 | 0.0135 |
| | Sg/ADA6 | 0.0130 |
| | Sg/ADA7 | 0.0093 |

Example 18

Production test 2 of adipic acid using *E. coli* mutants with impaired pyruvate kinase function

The production test of adipic acid was conducted using the *E. coli* mutants produced in Example 13 under anaerobic conditions.

The *E. coli* mutants produced in Example 13 were cultured in the same manner as in Example 14, except that the mutants were cultured statically using the culture medium II. The concentrations of adipic acid and other products accumulated in the culture supernatant and the concentration of sugars remaining unused in the culture medium were quantified. The yield of adipic acid calculated using the above formula (2) from the measured values is shown in Table 16.

Comparative Example 10

Production test 2 of adipic acid using *E. coli* mutants with intact pyruvate kinase function

The mutants produced in Reference Example 10 were cultured in the same manner as in Example 18. The concentrations of adipic acid and other products accumulated in the culture supernatant and the concentration of sugars remaining unused in the culture medium were quantified. The yield of adipic acid calculated using the above formula (2) from the measured values is shown in Table 16.

By comparing the results of Comparative Example 10 and Example 18, it was found that the yield of adipic acid was increased even under anaerobic conditions by impairing the function of pyruvate kinase in *E. coli.*

[Table 16]

| | Strain | Yield of ADA (%) |
|---|---|---|
| Example 18 | EcΔPP/pBBRpMW | N.D. |
| | EcΔPP/ADA1 | 0.0255 |
| | EcΔPP/ADA2 | 0.0254 |
| | EcΔPP/ADA3 | 0.0269 |
| | EcΔPP/ADA4 | 0.0248 |
| | EcΔPP/ADA5 | 0.0212 |
| | EcΔPP/ADA6 | 0.0278 |
| | EcΔPP/ADA7 | 0.0212 |
| Comparative Example 10 | Ec/pBBRpMW | N.D. |
| | Ec/ADA1 | 0.0166 |
| | Ec/ADA2 | 0.0180 |
| | Ec/ADA3 | 0.0140 |
| | Ec/ADA4 | 0.0161 |
| | Ec/ADA5 | 0.0148 |
| | Ec/ADA6 | 0.0219 |
| | Ec/ADA7 | 0.0123 |

Example 19

Generation of a mutant microorganism of the genus *Serratia* with defects in genes encoding pyruvate kinase and a phosphotransferase system enzyme

A mutant microorganism of the genus *Serratia* with impaired function of both pyruvate kinase and a phosphotransferase system enzyme was generated by disrupting a gene encoding a phosphotransferase, *ptsG,* in the SgΔPP strain produced in Example 1.

A PCR reaction was performed using pKD4 as a template and oligo DNAs represented by SEQ ID NOs: 239 and 240 as primers to obtain a PCR fragment of 1.6 kb in length for disruption of *ptsG.* The introduction of pKD46 into the above strain was followed by the introduction of the PCR fragment for disruption of *ptsG* into the resulting strain. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 242 were used.

Subsequently, an ampicillin-sensitive strain was obtained by segregating away the pKD46. The plasmid pCP20 was introduced into the ampicillin-sensitive strain, and ampicillin-resistant strains were again obtained. Direct colony PCR was performed on the obtained strains to confirm the deletion of the kanamycin resistance gene from the length of the amplified band. Oligo DNA primers represented by SEQ ID NOs: 241 and 242 were used. The pCP20 was segregated away from one of the kanamycin-sensitive strains. The obtained strain is hereinafter referred to as SgΔPPG.

Example 20

Generation of a mutant microorganism of the genus *Serratia* with defects in genes encoding pyruvate kinase and a phosphotransferase system enzyme and carrying a plasmid expressing enzymes that catalyze the reactions A, B, E, and F

By the same method as in Example 2, a plasmid produced in Reference Example 1, pBBR1MCS-2::ATCTOR1, was introduced into the SgΔPPG strain produced in Example 19, and the obtained mutant microorganism of the genus *Serratia* was designated as SgΔPPG/3HA1.

Example 21

Production test of 3-hydroxyadipic acid and α-hydromuconic acid using a mutant microorganism of the genus *Serratia* with impaired function of both pyruvate kinase and a phosphotransferase system enzyme and carrying a plasmid expressing enzymes that catalyze the reactions A, B, E, and F

By the same method as in Example 15, the production test of 3-hydroxyadipic acid and α-hydromuconic acid was conducted using the mutant microorganism of the genus *Serratia* produced in Example 20.

Comparative Example 11

Production test of 3-hydroxyadipic acid and α-hydromuconic acid using a mutant microorganism of the genus *Serratia* with intact pyruvate kinase function and intact phosphotransferase system enzyme function and carrying a plasmid expressing enzymes that catalyze the reactions A, B, E, and F

By the same method as in Comparative Example 7, the production test of 3-hydroxyadipic acid and α-hydromuconic acid was conducted using the Sg/3HA1 strain produced in Reference Example 5.

By comparing the results of Example 21 and Example 15, it was found that the yields of 3-hydroxyadipic acid and α-hydromuconic acid were further increased in the mutant microorganism of the genus *Serratia* with defects in the genes encoding pyruvate kinase and the phosphotransferase system enzyme and with enhanced activity of an enzyme that catalyzes the reaction of reducing 3-oxoadipyl-CoA to 3-hydroxyadipyl-CoA. Additionally, by comparing the results of Example 21 and Comparative Example 11, it was found that the yields of acetic acid and ethanol, both of which were generated by conversion of acetyl-CoA, were also increased in the mutant with defects in the genes encoding pyruvate kinase and the phosphotransferase system enzyme.

[Table 17]

| | Strain | Yield of 3HA (%) | Yield of HMA (%) | Yield of succinic acid (%) | Yield of acetic acid (%) | Yield of ethanol (%) |
|---|---|---|---|---|---|---|
| Example 21 | SgΔPPG/ 3HA1 | 6.06 | 0.180 | 60.6 | 36.8 | 52.2 |
| Comparative Example 11 | Sg/3HA1 | 1.68 | 0.0482 | 7.26 | 35.1 | 38.8 |

Example 22

Generation of an *E. coli* mutant with defects in genes encoding pyruvate kinase and a phosphotransferase system enzyme

An *E. coli* mutant with impaired function of both pyruvate kinase and a phosphotransferase system enzyme was generated by disrupting a gene encoding a phosphotransferase, *ptsG,* in the EcΔPP produced in Example 4.

A PCR reaction was performed using pKD4 as a template and oligo DNAs represented by SEQ ID NOs: 243 and 244 as primers to obtain a PCR fragment of 1.6 kb in length for disruption of *ptsG.* The introduction of pKD46 into the above strain was followed by the introduction of the PCR fragment for disruption of *ptsG* into the resulting strain. Direct colony PCR was performed on the resulting kanamycin-resistant strains to confirm the deletion of the gene of interest and the insertion of a kanamycin resistance gene from the length of the amplified band. Oligo DNA primers represented by SEQ ID NOs: 223 and 246 were used.

Subsequently, an ampicillin-sensitive strain was obtained by segregating away the pKD46. The plasmid pCP20 was introduced into the ampicillin-sensitive strain, and ampicillin-resistant strains were again obtained. Direct colony PCR was performed on the obtained strains to confirm the deletion of the kanamycin resistance gene from the length of the amplified band. Oligo DNA primers represented by SEQ ID NOs: 245 and 246 were used. The pCP20 was segregated away from one of the kanamycin-sensitive strains. The obtained strain is hereinafter referred to as EcΔPPG.

Example 23

Generation of an *E. coli* mutant with defects in genes encoding pyruvate kinase and a phosphotransferase system enzyme and carrying a plasmid expressing enzymes that catalyze the reactions A, B, E, and F

By the same method as in Example 5, the pBBR1MCS-2::ATCTOR1 produced in Reference Example 1 was introduced into the EcΔPPG strain produced in Example 22, and the obtained *E. coli* mutant was designated as EcΔPPG/3HA1.

Example 24

Production test of 3-hydroxyadipic acid and α-hydromuconic acid using an *E. coli* mutant with impaired function of both pyruvate kinase and a phosphotransferase system enzyme and carrying a plasmid expressing enzymes that catalyze the reactions A, B, E, and F

By the same method as in Example 16, the production test of 3-hydroxyadipic acid and α-hydromuconic acid was conducted using the *E. coli* mutant produced in Example 23.

Comparative Example 12

Production test of 3-hydroxyadipic acid and α-hydromuconic acid using an *E. coli* mutant with intact pyruvate kinase function and intact phosphotransferase system enzyme function and carrying a plasmid expressing enzymes that catalyze the reactions A, B, E, and F

By the same method as in Comparative Example 8, the production test of 3-hydroxyadipic acid and α-hydromuconic acid was conducted using the Ec/3HA1 produced in Reference Example 6.

By comparing the results of Example 24 and Example 16, it was found that the yields of 3-hydroxyadipic acid and α-hydromuconic acid were further increased in the *E. coli* mutant with defects in the genes encoding pyruvate kinase and the phosphotransferase system enzyme and with enhanced activity of an enzyme that catalyzes the reaction of reducing 3-oxoadipyl-CoA to 3-hydroxyadipyl-CoA. Additionally, by comparing the results of Example 24 and Comparative Example 12, it was found that the yields of acetic acid and ethanol, both of which were generated by conversion of acetyl-CoA, were also increased in the mutant with defects in the genes encoding pyruvate kinase and the phosphotransferase system enzyme.

[Table 18]

| | Strain | Yield of 3HA (%) | Yield of HMA (%) | Yield of succinic acid (%) | Yield of acetic acid (%) | Yield of ethanol (%) |
|---|---|---|---|---|---|---|
| Example 24 | EcΔPPG/3HA1 | 15.4 | 0.0439 | 60.2 | 51.3 | 58.0 |
| Comparative Example 12 | Ec/3HA1 | 1.32 | 0.0171 | 12.6 | 36.7 | 40.7 |

## Claims

1. A genetically modified microorganism in which a nucleic acid encoding any one of the polypeptides described in (a) to (c) below is introduced or the expression of the polypeptide is enhanced and the function of pyruvate kinase is impaired:

   (a) a polypeptide composed of an amino acid sequence represented by any one of SEQ ID NOs: 1 to 7;
   (b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;
   (c) a polypeptide composed of an amino acid sequence with a sequence identity of not less than 70% to the sequence represented by any one of SEQ ID NOs: 1 to 7 and having an enzymatic activity that catalyzes a reaction to reduce 3-oxoadipyl-CoA to 3-hydroxyadipyl-CoA.

2. The genetically modified microorganism according to claim 1, wherein a polypeptide selected from the above (b) and (c) contains a region composed of an amino acid sequence represented by SEQ ID NO: 173.

3. The genetically modified microorganism according to claim 2, wherein the amino acid sequence represented by SEQ ID NO: 173 contains a phenylalanine or leucine residue at the 13th amino acid position from the N terminus, a leucine or glutamine residue at the 15th amino acid position from the N terminus, a lysine or asparagine residue at the 16th amino acid position from the N terminus, a glycine or serine residue at the 17th amino acid position from the N terminus, a proline or arginine residue at the 19th amino acid position from the N terminus, and a leucine, methionine, or valine residue at the 21st amino acid position from the N terminus.

4. The genetically modified microorganism according to any one of claims 1 to 3, which is a genetically modified microorganism belonging to a genus selected from the group consisting of *Escherichia, Serralia, Hafnia,* and *Pseudomonas.*

5. The genetically modified microorganism according to any one of claims 1 to 4, which has an ability to generate 3-oxoadipyl-CoA and coenzyme A from acetyl-CoA and succinyl-CoA and an ability to generate 3-hydroxyadipic acid from 3-hydroxyadipyl-CoA.

6. The genetically modified microorganism according to any one of claims 1 to 4, which has an ability to generate 3-oxoadipyl-CoA and coenzyme A from acetyl-CoA and succinyl-CoA, an ability to generate 2,3-dehydroadipyl-CoA from 3-hydroxyadipyl-CoA, and an ability to generate $\alpha$-hydromuconic acid from 2,3-dehydroadipyl-CoA.

7. The genetically modified microorganism according to any one of claims 1 to 4, which has an ability to generate 3-oxoadipyl-CoA and coenzyme A from acetyl-CoA and succinyl-CoA, an ability to generate 2,3-dehydroadipyl-CoA from 3-hydroxyadipyl-CoA, an ability to generate adipyl-CoA from 2,3-dehydroadipyl-CoA, and an ability to generate adipic acid from adipyl-CoA.

8. The genetically modified microorganism according to any one of claims 1 to 7, wherein the function of a phosphotransferase system enzyme is further impaired.

9. A method of producing 3-hydroxyadipic acid, comprising culturing the genetically modified microorganism according to any one of claims 1 to 5 and 8 in a culture medium containing a carbon source as a raw material for fermentation.

10. A method of producing $\alpha$-hydromuconic acid, comprising culturing the genetically modified microorganism according to any one of claims 1 to 4, 6 and 8 in a culture medium containing a carbon source as a raw material for fermentation.

11. A method of producing adipic acid, comprising culturing the genetically modified microorganism according to any one of claims 1 to 4, 7 and 8 in a culture medium containing a carbon source as a raw material for fermentation.

12. A method of producing one or more substances selected from the group consisting of 3-hydroxyadipic acid, $\alpha$-hydromuconic acid, and adipic acid, comprising culturing a genetically modified microorganism in a culture medium containing a carbon source as a raw material for fermentation, wherein a nucleic acid encoding a polypeptide encoded by the 3-hydroxybutyryl-CoA dehydrogenase gene of a microorganism of the genus *Serratia,* which forms a gene cluster with 5-aminolevulinic acid synthase gene in the microorganism, is introduced or the expression of

the polypeptide is enhanced and the function of pyruvate kinase is impaired in the genetically modified microorganism.

**13.** The method according to claim 12, wherein the genetically modified microorganism is a microorganism in which the function of a phosphotransferase system enzyme is further impaired.

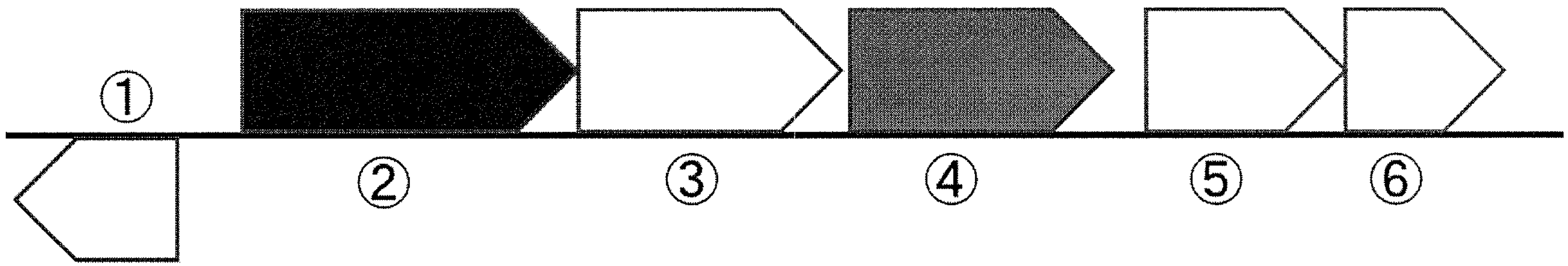


| | Definition(BLAST search result) | Protein ID (Serratia proteamaculans 568) | Gene Position (Gene ID:CP000826.1) |
|---|---|---|---|
| ① | LuxR family transcriptional regulator or quorum-sensing transcriptional activator | ABV40936.1 | 2017190..2017906 |
| ② | 3-hydroxybutyryl-CoA dehydrogenase | ABV40935.1 | 2015313..2016842 (Complement) |
| ③ | acetyl-CoA C-acyltransferase or beta-ketoadipyl CoA thiolase | ABV40934.1 | 2014092..2015303 (Complement) |
| ④ | 5-aminolevulinate synthase | ABV40933.1 | 2012865..2014076 (Complement) |
| ⑤ | MFS transporter or Purine efflux pump PbuE | ABV40932.1 | 2011696..2012868 (Complement) |
| ⑥ | enoyl-CoA hydratase or Carnitinyl-CoA dehydratase | ABV40931.1 | 2010981..2011703 (Complement) |

Fig.1

## INTERNATIONAL SEARCH REPORT

International application No. PCT/JP2020/018663

A. CLASSIFICATION OF SUBJECT MATTER
C12P 7/42(2006.01)i; C12P 7/44(2006.01)i; C12N 1/21(2006.01)i; C12N 15/53(2006.01)i; C12N 15/54(2006.01)i; C12N 9/04(2006.01)i
FI: C12N1/21; C12P7/42; C12P7/44; C12N15/53 ZNA; C12N15/54; C12N9/04 Z
According to International Patent Classification (IPC) or to both national classification and IPC

B. FIELDS SEARCHED

Minimum documentation searched (classification system followed by classification symbols)
C12P7/42; C12P7/44; C12N1/21; C12N15/53; C12N15/54; C12N9/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); GenBank/EMBL/DDBJ/ GeneSeq; UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | JP 2011-515111 A (GENOMATICA, INC.) 19.05.2011 (2011-05-19) claims, paragraph [0057], example II, fig. 2 | 1-13 |
| Y | WO 2017/033965 A1 (RIKEN, INSTITUTE OF PHYSICAL AND CHEMICAL RESEARCH) 02.03.2017 (2017-03-02) claims, paragraphs [0010], [0039]-[0040], [0103] | 1-13 |
| Y | WO 2019/022083 A1 (RIKEN, INSTITUTE OF PHYSICAL AND CHEMICAL RESEARCH) 31.01.2019 (2019-01-31) paragraph [0110] | 1-13 |
| Y | JP 2017-184746 A (MYRIANT CORPORATION) 12.10.2017 (2017-10-12) claims, paragraphs [0003], [0045] | 1-13 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 July 2020 (06.07.2020) | 14 July 2020 (14.07.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/018663

C (Continuation). DOCUMENTS CONSIDERED TO BE RELEVANT

| Category* | Citation of document, with indication, where appropriate, of the relevant passages | Relevant to claim No. |
|---|---|---|
| Y | Database GenBank [online], Accession No. ALL37524.1, "3-hydroxybutyryl-CoA dehydrogenase [Serratia marcescens]", 28 February 2017 [retrieval date 06 February 2019], <URL:https//www.ncbi.nlm.nih.gov/protein/943365850> in particular, section "DEFINITION", "ORIGIN" | 1-13 |
| Y | Database GenBank [online], Accession No. KFF90258.1, "3-hydroxybutyryl-CoA dehydrogenase [Serratia nernatodiphila DZ0503SBS1]", 07 August 2014 [retrieval date 06 February 2019], <URL: https://www.ncbi.nlm.gov/protein/KFF90258.1> in particular, section "DEFINITION", "ORIGIN" | 1-13 |
| Y | Database GenBank [online], Accession No. AGP43956.1, "3-hydroxybutyryl-CoA dehydrogenase [Serratia plyrnuthica S13]", 30 January 2014 [retrieval date 06 February 2019], <URL: https://www.ncbi.nlm.gov/protein/AGP43956.1> in particular, section "DEFINITION", "ORIGIN" | 1-13 |
| Y | Database GenBank [online], Accession No. ABV40935.1, "3-hydroxybutyryl-CoA dehydrogenase [Serratia protearnaculans 568]", 28 January 2014 [retrieval date 06 February 2019], <URL: https://www.ncbi.nlm.gov/protein/ABV40935.1> in particular, section "DEFINITION", "ORIGIN" | 1-13 |
| Y | Database GenBank [online], Accession No. ASM11476.1, "3-hydroxybutyryl-CoA dehydrogenase [Serratia rnarcescens]", 18 July 2017 [retrieval date 06 February 2019], <URL:https://www.ncbi.n;m.gov/protein/ASM11476.1> in particular, section "DEFINITION", "ORIGIN" | 1-13 |
| Y | Database GenBank [online], Accession No. SMB31239.1, "3-hydroxybutyryl-CoA dehydrogenase [Serratia protearnaculans]", 14 April 2017 [retrieval date 06 February 2019], <URL: https://www.ncbi.nlm.gov/pratein/SMB31239.1> in particular, section "DEFINITION", "ORIGIN" | 1-13 |
| Y | Database GenBank [online], Accession No. AMG98270.1, "3-hydroxybutyryl-CoA dehydrogenase [Serratia liquefaciens]", 11 February 2016 [retrieval date 06 February 2019], <URL: https://www.ncbi.nlrn.gov/protein/991935067?sat=4&satkey=158110186> in particular, section "DEFINITION", "ORIGIN" | 1-13 |
| Y | JP 2018-500911 A (GENOMATICA, INC.) 18.01.2018 (2018-01-18) paragraphs [0282], [0343], fig. 12, 24 | 1-13 |

Form PCT/ISA/210 (continuation of second sheet) (January 2015)

**INTERNATIONAL SEARCH REPORT**

International application No.
PCT/JP2020/018663

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2013-535203 A (GENOMATICA, INC.) 12.09.2013 (2013-09-12) paragraphs [0586], [0600], [0628]-[0645], fig. 20 | 1-13 |
| Y | WO 2017/209102 A1 (TORAY INDUSTRIES, INC.) 07.12.2017 (2017-12-07) claims | 1-13 |
| Y | WO 2017/209103 A1 (TORAY INDUSTRIES, INC.) 07.12.2017 (2017-12-07) claims | 1-13 |
| P, Y | WO 2019/107516 A1 (TORAY INDUSTRIES, INC.) 06.06.2019 (2019-06-06) claims | 1-13 |

Form PCT/ISA/210 (continuation of second sheet) (January 2015)

**INTERNATIONAL SEARCH REPORT**
Information on patent family members

International application No.
PCT/JP2020/018663

| Patent Documents referred in the Report | Publication Date | Patent Family | Publication Date |
|---|---|---|---|
| JP 2011-515111 A | 19 May 2011 | US 2009/0305364 A1 claims, paragraph [0065], example II, fig. 2<br>EP 2265709 A1<br>WO 2009/151728 A2 | |
| WO 2017/033965 A1 | 02 Mar. 2017 | US 2018/0237813 A1 claims, paragraphs [0010], [0065]-[0066], [0116]<br>EP 3342874 A1 | |
| WO 2019/022083 A1 | 31 Jan. 2019 | (Family: none) | |
| JP 2017-184746 A | 12 Oct. 2017 | US 2015/0044755 A1 claims, paragraphs [0003], [0062]<br>EP 2809771 A1<br>WO 2013/116244 A1 | |
| JP 2018-500911 A | 18 Jan. 2018 | US 2017/0369913 A1 paragraphs [0368], [0400], fig. 12, 24<br>EP 3237629 A1<br>WO 2016/106367 A1 | |
| JP 2013-535203 A | 12 Sep. 2013 | US 2012/0021478 A1 paragraphs [0529], [0538], [0554]-[0564], fig. 20<br>EP 2607340 A1<br>WO 2012/018624 A2 | |
| WO 2017/209102 A1 | 07 Dec. 2017 | US 2019/0276860 A1 claims<br>EP 3498852 A1 | |
| WO 2017/209103 A1 | 07 Dec. 2017 | EP 3467113 A1 claims | |
| WO 2019/107516 A1 | 06 Jun. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013535203 A
- US 20110124911 A1
- JP 2015146810 A
- JP 2011515111 A
- WO 2017209102 A
- WO 2017209103 A
- JP 2008527991 A
- WO 2017099209 A
- WO 2016199858 A1
- WO 2016199856 A1


### Non-patent literature cited in the description


- *Biochimie.,* 09 February 2012, vol. 4 (2), 471-8
- *J Appl Microbiol.,* October 2015, vol. 19 (4), 1057-63
- **ME KOVACH.** *Gene,* 1995, vol. 166, 175-176
- **NM CALVIN ; PC HANAWALT.** *J. Bacteriol,* 1988, vol. 170, 2796-2801
- *Proc Natl Acad Sci U.S.A.,* 06 June 2000, vol. 97 (12), 6640-6645
- *Biosci Biotechnol Biochem.,* December 2007, vol. 71 (12), 2905-11
- *Journal of Molecular Biology,* 1970, vol. 53, 159
- *Proc Natl Acad Sci U S A.,* 06 June 2000, vol. 97 (12), 6640-6645
- *Proc Natl Acad Sci USA.,* 06 June 2000, vol. 97 (12), 6640-6645

SEQUENCE LISTING

<110> Toray Industries, Inc.

<120> Microorganisms and method for production of 3-hydroxyadipate, alfa-hydromuconic acid, and/or adipic acid

<130> PF669-PCT

<160> 246

<170> PatentIn version 3.5

<210> 1
<211> 509
<212> PRT
<213> Serratia marcescens ATCC13880

<400> 1

```
Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Gly Ala Val Leu Ala Asn Leu Met Phe Thr Ser
65                  70                  75                  80

Val Phe Glu Ala Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175
```

```
Ala Ala Leu Gly Lys His Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Ser Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ser Ala Val Gln Ile Asn Asp Ala Phe Thr Val
            340                 345                 350

Ser Ile Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Thr
    370                 375                 380

Tyr Leu Ala Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430
```

```
Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505


<210>  2
<211>  509
<212>  PRT
<213>  Serratia nematodiphila DSM21420

<400>  2

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Pro Thr Met Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Arg Asp Lys Asp Ala Val Leu Ala Asn Leu Met Phe Thr Ser
65                  70                  75                  80

Val Phe Glu Ala Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140
```

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Leu Ala Leu Asn Tyr Ala Asp Thr Thr
370 375 380

Tyr Leu Val Ala Ala His Ser Arg His Ala Ser Ala Ala Asn Lys Ala

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 3
<211> 509
<212> PRT
<213> Serratia plymuthica NBRC102599

<400> 3

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Asp
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr

100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser Arg Ala Thr Ser Leu Arg Cys Gln Gln Leu Val
165 170 175

Thr Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Asn Pro Thr Thr Glu Gly Asp Thr Pro Thr
290 295 300

Ser Leu His Phe Tyr Gly Glu His Ala Leu Phe Asp His Leu Gln Gln
305 310 315 320

Arg Ala Leu Ala Thr Trp Pro Ala Leu Arg Val Gln Arg Leu Pro Glu
325 330 335

Arg Pro Glu Leu Gly Arg Phe Ile Leu Val Asn Asn Arg Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Thr Asn Lys Ala
385 390 395 400

Leu Phe Leu Thr Leu Leu Gln Thr Leu Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Thr Arg Tyr Tyr Pro His Tyr Ser
485 490 495

Leu Leu Asn Ala Ala Arg Pro Glu Leu Ala Val Ala Pro
500 505

<210> 4
<211> 509
<212> PRT
<213> Serratia proteamaculans 568

<400> 4

Met Ala Glu Asn Asn Ser Ala Ile His Ser Val Ala Val Ile Gly Ala
1 5 10 15

Gly Thr Met Gly Arg Gly Ile Ala Tyr Leu Leu Ala Gln Asn Gly Ile
20 25 30

Arg Thr Leu Leu Tyr Asn Arg Ser Gly Asn Asn Leu Asn Gln Ala Arg
35 40 45

Asp Tyr Ile Ile Arg Asp Leu Asp Lys Lys Ile Asp Gly Gly Lys Ile
50 55 60

```
Ser Pro Gln Lys Lys Gly Glu Ile Leu Ala Asn Leu Val Phe Ser Pro
65                  70                  75                  80

Ile Phe Glu Ala Ile Ala Asp Ser Asp Leu Val Ile Glu Thr Ile Ala
                85                  90                  95

Glu His Glu Ala Thr Lys His Glu Ile Leu Ala Ala Ile Ala Ala Thr
            100                 105                 110

Val Lys Lys Glu Ala Ile Ile Ala Thr Asn Thr Ser Ser Leu Ser Leu
        115                 120                 125

Asn Lys Leu Ala Ala Gly Val Glu Asn Asn Ala Arg Phe Ile Gly Leu
    130                 135                 140

His Phe Phe Asn Pro Ala Pro Leu Met Lys Leu Ile Glu Ile Ile Pro
145                 150                 155                 160

Ser Tyr Phe Thr Ser Arg Ala Thr Ser Leu Arg Cys Gln Gln Leu Val
                165                 170                 175

Thr Ala Ile Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Ser Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Phe Phe Thr Pro
        275                 280                 285

Ser Ser Ala Glu Pro Ser Ser Ala Asp Ala Gly Ser Gly Thr Pro Thr
    290                 295                 300

Ser Leu Asn Phe Tyr Gly Glu His Pro Leu Phe Asp Leu Leu Gln Gln
305                 310                 315                 320
```

Arg Ala Leu Ala Leu Trp Pro Arg Val Gln Ile Asn Arg Gln Ser Glu
325 330 335

Gln Pro Thr Leu Gly Arg Phe Ile Arg Val Asn Asp Ala Met Ala Ile
340 345 350

Lys Ile Thr Asp Gly Arg Thr Ala Asn Leu Leu Ala Glu Leu Thr Glu
355 360 365

Leu Asp Thr Phe Val Ile Asp Ala Ala Leu Asn Tyr Ala Asp Thr Ala
370 375 380

Tyr Leu Ala Ala Ala His Ser Gln Asp Ala Ser Ala Ala Asn Lys Ala
385 390 395 400

Leu Phe Leu Thr Leu Leu Gln Thr Leu Ile Pro Gln Val Glu Phe Ile
405 410 415

Lys Asp Ser Pro Gly Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ser Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Thr Pro Arg Pro Glu Leu Ala Val Ala Pro
500 505

<210> 5
<211> 509
<212> PRT
<213> Serratia ureilytica Lr5/4

<400> 5

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
    290                 295                 300

Thr Leu Arg Ile Tyr Gly Glu His Pro Leu Phe Thr Leu Leu Gln Gln
305                 310                 315                 320

Arg Ala Thr Leu Gln Trp Pro Arg Leu Arg Val Glu Gln Arg Pro Thr
                325                 330                 335

Leu Pro Gly Leu Gly Ala Ala Ile Gln Val Asn Asp Ala Phe Thr Val
            340                 345                 350

Ser Val Thr Asp Gly Arg Thr Ala Asn Gln Leu Ala Glu Gln Thr Ala
        355                 360                 365

Ala Asp Ala Phe Val Val Asp Val Ala Leu Asn Tyr Gly Asp Thr Ala
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Thr Arg Leu Gly Glu Glu Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 6
<211> 509
<212> PRT
<213> Serratia sp. BW106

<400> 6

```
Met Ala Glu Asn Asn Ser Ala Ile His Ser Val Ala Val Ile Gly Ala
1               5                   10                  15

Gly Thr Met Gly Arg Gly Ile Ala Tyr Leu Leu Ala Gln Asn Gly Ile
            20                  25                  30

Arg Thr Leu Leu Tyr Asn Arg Ser Gly Asn Asn Leu Asp Gln Ala Arg
        35                  40                  45

Asp Tyr Ile Ile Arg Asp Leu Asp Lys Lys Ile Asp Asn Gly Lys Ile
    50                  55                  60

Ser Gln Gln Lys Lys Gly Glu Val Leu Ala Asn Leu Val Phe Ser Pro
65                  70                  75                  80

Ile Phe Asp Ala Ile Ala Asp Ser Asp Leu Val Ile Glu Thr Ile Ala
                85                  90                  95

Glu His Glu Thr Thr Lys His Glu Ile Leu Ala Ala Ile Ala Ala Thr
            100                 105                 110

Val Lys Gln Glu Ala Ile Ile Ala Thr Asn Thr Ser Ser Leu Ser Leu
        115                 120                 125

Asn Lys Leu Ala Ala Gly Val Glu Asn Asn Ala Arg Phe Ile Gly Leu
    130                 135                 140

His Phe Phe Asn Pro Ala Pro Leu Met Lys Leu Ile Glu Ile Ile Pro
145                 150                 155                 160

Ser Tyr Phe Thr Ser Arg Ala Thr Ser Leu Arg Cys Gln Gln Leu Val
                165                 170                 175

Thr Ala Leu Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Ser Gln Ile Trp Gln Asp Met
```

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Phe Phe Ala Ala
275 280 285

Pro Ser Ser Glu Ser Asn Pro Leu Asp Ala Gly Asn Gly Thr Leu Thr
290 295 300

Ser Leu His Phe Tyr Gly Glu His Thr Leu Phe Asp Leu Leu Gln Gln
305 310 315 320

Arg Ala Leu Ala Ile Trp Pro Thr Leu Gln Ile Ile His Gln Pro Glu
325 330 335

Arg Pro Thr Leu Gly Arg Phe Ile Arg Val Asn Asp Ala Leu Ala Val
340 345 350

Lys Ile Thr Asp Gly Arg Thr Ala Asn Leu Leu Ala Glu Leu Thr Asp
355 360 365

Leu Asp Thr Phe Val Ile Asp Ala Ala Leu Asn Tyr Ser Asp Thr Ala
370 375 380

Tyr Leu Val Ala Ala His Asn Gln Asp Ala Ala Glu Ala Asn Lys Ala
385 390 395 400

Leu Phe Leu Ser Leu Leu Gln Thr Leu Ile Pro Gln Val Glu Phe Ile
405 410 415

Lys Asp Ser Pro Gly Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Thr Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Pro Arg Pro Glu Leu Ala Val Ala Pro
500 505

<210> 7
<211> 509
<212> PRT
<213> Serratia liquefaciens FK01

<400> 7

Met Ala Glu Asn Asn Thr Ala Ile Asp Ser Val Ala Val Ile Gly Ala
1 5 10 15

Gly Thr Met Gly Arg Gly Ile Ala Tyr Leu Leu Ala Leu Asn Gly Ile
20 25 30

Arg Thr Leu Leu Tyr Asn Arg Asn Gly Asn His Leu Asn Gln Ala Arg
35 40 45

Asp Tyr Ile Val Gly Asp Leu Asp Lys Lys Ile Asp Asn Gly Lys Ile
50 55 60

Ser Gln Gln Lys Lys Gly Glu Val Leu Ala Asn Leu Val Phe Ser Pro
65 70 75 80

Val Phe Asp Ala Ile Ala Asp Ser Asp Leu Val Ile Glu Thr Ile Ala
85 90 95

Glu His Glu Pro Thr Lys His Glu Ile Leu Ala Ala Ile Ala Ala Thr
100 105 110

Val Lys Lys Glu Ala Ile Ile Ala Thr Asn Thr Ser Ser Leu Ser Leu
115 120 125

Asn Lys Leu Ala Ala Gly Ile Glu Asn Asn Ser Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ala Arg Ala Thr Thr Leu Arg Cys Gln Gln Leu Val
165 170 175

Thr Ala Ile Gly Lys Lys Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Ser Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Phe Phe Ser Pro
        275                 280                 285

Ser Ala Asp Ala Ala Asn Pro Pro Ala Thr Gly Gly Gly Thr Leu Ser
    290                 295                 300

Ser Leu His Phe Phe Gly Glu His Pro Leu Phe Asp Leu Leu Gln Gln
305                 310                 315                 320

His Ala Phe Ala Thr Trp Ala Pro Leu Ala Ile Thr Arg Gln Pro Glu
                325                 330                 335

His Pro Val Leu Gly Arg Phe Ile Gln Val Asn Asp Ser Leu Ala Val
            340                 345                 350

Lys Ile Thr Asp Gly Arg Thr Ala Asn Gln Leu Ala Glu Leu Ala Gly
        355                 360                 365

Leu Asp Thr Phe Val Val Asp Val Ala Leu Asn Tyr Ala Asn Thr Ala
    370                 375                 380

Phe Leu Val Ala Ala His Ser Gln Gln Ala Thr Glu Ala Asn Lys Glu
385                 390                 395                 400

Leu Phe Leu Thr Leu Leu Gln Thr Val Ile Pro Gln Val Glu Phe Val
                405                 410                 415

Lys Asp Ser Pro Gly Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Ile Ala Ala Val Ala Gly Val Asn Tyr Ala Asp Gly Ile Phe
    450                 455                 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu His Ala Thr Arg Pro Glu Leu Ala Val Ala Pro
500 505

<210> 8
<211> 509
<212> PRT
<213> Serratia sp. S119

<400> 8

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

```
Asn Glu Ser Val Ile Met Val Glu Ser Gly Val Cys Ser Arg Glu Asp
        435                 440                 445

Ile Asn Val Ala Ala Val Ala Gly Val Asn Tyr Ala Asp Gly Ile Phe
    450                 455                 460

Gly Leu Leu Asp Ser Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505


<210>  9
<211>  509
<212>  PRT
<213>  Serratia sp. YD25

<400>  9

Met Ala Glu Arg Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Ile Gln Ala Arg
        35                  40                  45

Glu Tyr Ile Val Gln Asp Leu Asp Lys Lys Val Glu Gln Gly Arg Leu
    50                  55                  60

Ala Pro Gln Asp Lys Asp Ala Val Leu Ala Asn Leu Gln Phe Ser Ser
65                  70                  75                  80

Val Phe Glu Ala Ile Val Asp Ser Asp Leu Val Leu Glu Thr Ile Ala
                85                  90                  95

Glu Gln Glu Gln Ala Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140
```

His Phe Phe Asn Pro Ala Pro Leu Met Lys Leu Ile Glu Ile Ile Pro
145 150 155 160

Ala Tyr Phe Thr Thr Gln Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Thr Ala Pro Pro Val Glu Ala Ala Ala Glu Ala Asp Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Gln Leu Arg Val Glu Gln Arg Pro Ala
325 330 335

Leu Ser Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala

385 390 395 400

Leu Phe Leu Arg Leu Leu His Thr Ala Leu Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 10
<211> 509
<212> PRT
<213> Serratia sp. FS14

<400> 10

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val

```
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Asn Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ala Ala Val Gln Ile Asn Asp Ala Phe Thr Val
            340                 345                 350
```

Ser Ile Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Thr
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 11
<211> 509
<212> PRT
<213> Serratia sp. HMSC15F11

<400> 11

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Asp Tyr Ile Glu Leu Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Val
275 280 285

Glu Glu Asn Ala Pro Pro Val Met Ala Ala Thr Asp Ala Asp Ile Glu
290 295 300

Thr Leu His Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Pro Ala Val Arg Ile Asn Asp Ala Phe Thr Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 12
<211> 509
<212> PRT
<213> Serratia sp. JKS000199

<400> 12

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Thr
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Leu Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 13
<211> 509
<212> PRT
<213> Serratia sp. TEL

<400> 13

```
Met Ala Glu Ser Asn Ala Ala Ile Gln Ser Ala Ala Ile Ile Gly Ala
1               5                   10                  15

Gly Thr Met Gly Arg Gly Ile Ala Tyr Leu Leu Ala Gln Lys Ser Ile
            20                  25                  30

Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

Ala Leu Gln Asp Lys Asp Ala Val Leu Ala Asn Leu Thr Phe Ser Ala
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Val Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
```

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Thr Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Ala Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 14
<211> 509
<212> PRT
<213> Serratia sp. ISTD04

<400> 14

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Val Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Ser Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285

Glu Glu Ser Pro Pro Pro Leu Ala Ala Ala Val Asp Ala Glu Val Glu
    290                 295                 300

Thr Leu Arg Ile Tyr Gly Glu His Pro Leu Phe Thr Leu Leu Gln Gln
305                 310                 315                 320

Arg Ala Ala Leu Gln Trp Pro Arg Leu Arg Val Glu Gln Arg Pro Thr
                325                 330                 335

Leu Pro Gly Leu Gly Ala Ala Ile Gln Val Asn Asp Ala Phe Thr Val
            340                 345                 350

Ser Val Thr Asp Gly Arg Thr Ala Asn Gln Leu Ala Glu Gln Thr Ala
        355                 360                 365

Ala Asp Ala Phe Val Val Asp Val Ala Leu Asn Tyr Gly Asp Thr Ala
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 15
<211> 509
<212> PRT
<213> Serratia sp. SCBI

<400> 15

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Thr
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 16
<211> 509
<212> PRT
<213> Serratia sp. S4

<400> 16

Met Ala Glu Asn Asn Ser Ala Ile His Ser Val Ala Val Ile Gly Ala
1 5 10 15

Gly Thr Met Gly Arg Gly Ile Ala Tyr Leu Leu Ala Gln Asn Gly Ile
20 25 30

Arg Thr Leu Leu Tyr Asn Arg Ser Gly Asn Asn Leu Asn Gln Ala Arg
35 40 45

Asp Tyr Ile Ile Arg Asp Leu Asp Lys Lys Ile Asp Gly Gly Lys Ile
50 55 60

Ser Leu Gln Lys Lys Gly Glu Ile Leu Ala Asn Leu Val Phe Ser Pro
65 70 75 80

Ile Phe Glu Ala Ile Ala Asp Ser Asp Leu Val Ile Glu Thr Ile Ala
85 90 95

Glu His Glu Ser Thr Lys His Glu Ile Leu Ser Ala Ile Ala Ala Thr
100 105 110

Val Lys Lys Glu Ala Ile Ile Ala Thr Asn Thr Ser Ser Leu Ser Leu
115 120 125

Asn Lys Leu Ala Ala Gly Val Glu Asn Asn Ala Arg Phe Ile Gly Leu
130 135 140

```
His Phe Phe Asn Pro Ala Pro Leu Met Lys Leu Ile Glu Ile Ile Pro
145                 150                 155                 160

Ser Tyr Phe Thr Ser Arg Ala Thr Ser Leu Arg Cys Gln Gln Leu Val
                165                 170                 175

Thr Val Ile Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Ser Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Phe Phe Thr Pro
        275                 280                 285

Ser Ser Ala Thr Pro Ser Ser Ala Asp Ala Gly Ser Gly Thr Pro Thr
    290                 295                 300

Ser Leu Asn Phe Tyr Gly Glu His Pro Leu Phe Asp Leu Leu Gln Gln
305                 310                 315                 320

Arg Ala Leu Ala Leu Trp Pro Arg Leu Gln Ile Asn Arg Gln Ser Glu
                325                 330                 335

Gln Pro Thr Leu Gly Arg Phe Ile Arg Val Asn Asp Ala Met Ala Ile
            340                 345                 350

Lys Ile Thr Asp Gly Arg Thr Ala Asn Leu Leu Ala Glu Leu Thr Glu
        355                 360                 365

Leu Asp Thr Phe Val Ile Asp Ala Ala Leu Asn Tyr Ala Asp Thr Ala
    370                 375                 380

Tyr Leu Ala Ala Ala His Ser Gln Asp Ala Ser Thr Ala Asn Lys Ala
```

385 390 395 400

Leu Phe Leu Thr Leu Leu Gln Thr Leu Ile Pro Gln Val Glu Phe Ile
405 410 415

Lys Asp Ser Pro Gly Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ser Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Thr Pro Arg Pro Glu Leu Ala Val Ala Pro
500 505

<210> 17
<211> 509
<212> PRT
<213> Serratia sp. C-1

<400> 17

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr

100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser His Ala Thr Ser Leu Arg Cys Gln Lys Leu Val
165 170 175

Ile Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Thr Pro Thr Thr Glu Ser Asp Thr Pro Thr
290 295 300

Ser Leu His Phe Tyr Gly Glu His Ala Leu Phe Asp His Leu Gln Gln
305 310 315 320

Arg Ala Leu Ala Ala Trp Pro Ala Leu Arg Val Gln Arg Leu Pro Glu
325 330 335

Arg Pro Glu Leu Gly Arg Phe Ile Leu Val Asn Asn Ala Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Thr Asn Lys Ala
385 390 395 400

Leu Phe Leu Thr Leu Leu Gln Thr Val Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Ala Arg Pro Glu Leu Ala Val Ala Pro
500 505

<210> 18
<211> 509
<212> PRT
<213> Serratia marcescens 532

<400> 18

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

```
Ala Leu Gln Asp Lys Gly Ala Val Leu Ala Asn Leu Met Phe Thr Ser
65                  70                  75                  80

Val Phe Glu Ala Ile Thr Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys His Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Ser Asn Ala Asp Val Glu
    290                 295                 300

Thr Leu Arg Val Tyr Gly Glu His Pro Phe Phe Thr Leu Leu Gln Gln
305                 310                 315                 320
```

Arg Ala Ala Leu Gln Trp Pro Gln Leu Arg Val Glu Gln Arg Pro Ala
325 330 335

Leu Pro Gly Leu Gly Ser Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Val Ala Leu Asn Tyr Ala Asp Thr Thr
370 375 380

Tyr Leu Ala Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 19
<211> 509
<212> PRT
<213> Serratia marcescens 2880STDY5683033

<400> 19

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala His Asp Leu Asn Lys Lys Val Glu Gln Gly Lys Ile
50 55 60

Ala Leu Gln Asp Lys Gly Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys His Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Thr
275 280 285

Glu Glu Thr Ala Pro Pro Val Thr Ala Ala Ser Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ser Ala Val Gln Ile Asn Asp Ala Phe Thr Val
            340                 345                 350

Ser Ile Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Thr
    370                 375                 380

Tyr Leu Ala Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 20
<211> 509
<212> PRT
<213> Serratia marcescens WW4

<400> 20

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Ile Asp Val Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

```
Leu Leu His Ala Ala Gln Pro Ala Leu Thr Thr Thr Pro
            500                 505


<210>  21
<211>  509
<212>  PRT
<213>  Serratia marcescens K27

<400>  21

Met Ala Glu Ser Asn Ala Ala Ile Gln Ser Ala Ala Ile Ile Gly Ala
1               5                   10                  15


Gly Thr Met Gly Arg Gly Ile Ala Tyr Leu Phe Ala Gln Lys Gly Ile
            20                  25                  30


Pro Thr Met Leu Tyr Asn Arg Asn Gly Asn Thr Leu Asn Gln Ala Arg
        35                  40                  45


Glu Tyr Ile Ala Gln Asp Leu Asn Lys Lys Val Glu Gln Gly Lys Ile
    50                  55                  60


Ala Leu Gln Asp Lys Asp Ala Val Leu Ala Asn Leu Met Phe Thr Ser
65                  70                  75                  80


Val Phe Glu Ala Ile Ala Asp Ser Glu Leu Val Ile Glu Thr Ile Ala
                85                  90                  95


Glu Gln Glu Gln Thr Lys Leu Glu Val Leu Ala Ala Ile Ala Ala Val
            100                 105                 110


Val Lys Pro Asp Thr Leu Ile Ala Thr Asn Thr Ser Ser Leu Ser Leu
        115                 120                 125


Asn Lys Leu Ala Thr Ala Val Thr His Ser Glu Arg Phe Ile Gly Leu
    130                 135                 140


His Phe Phe Asn Pro Ala Pro Leu Met Lys Leu Ile Glu Ile Ile Pro
145                 150                 155                 160


Ala Tyr Phe Thr Ala His Ala Thr Thr Glu Arg Cys Arg Gln Leu Val
                165                 170                 175


Ala Ala Leu Gly Lys Arg Asp Val Val Cys Gln Ala Thr Pro Gly Phe
            180                 185                 190


Ile Val Asn Arg Met Ala Arg Pro Tyr Tyr Leu Glu Gly Phe Arg Leu
        195                 200                 205
```

Leu Glu Glu His Val Ala Arg Ser Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Val Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Arg Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 22
<211> 509
<212> PRT
<213> Serratia marcescens 280

<400> 22

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Val Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Leu Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 23
<211> 509
<212> PRT
<213> Serratia marcescens 19F

<400> 23

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys His Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Val Ala Leu Asn Tyr Ala Asp Thr Thr
370 375 380

Tyr Leu Val Ala Ala His Ser Arg His Ala Ser Ala Ala Asn Lys Ala

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 24
<211> 509
<212> PRT
<213> Serratia marcescens 1185

<400> 24

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Arg Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys His Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
        355                 360                 365

Ala Asp Ala Phe Val Val Asp Leu Ala Leu Asn Tyr Ala Asp Thr Thr
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Ser Ala Leu Thr Thr Thr Pro
            500                 505

<210>  25
<211>  509
<212>  PRT
<213>  Serratia marcescens S2I7

<400>  25

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Pro Thr Met Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Cys Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Val Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Arg Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 26
<211> 509
<212> PRT
<213> Serratia marcescens KHCo-24B

<400> 26

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

```
Pro Thr Met Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Arg Asp Lys Asp Ala Val Leu Ala Asn Leu Met Phe Thr Ser
65                  70                  75                  80

Val Phe Glu Ala Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285
```

Glu Glu Thr Ala Pro Pro Val Thr Ala Ala Asn Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ala Ala Val Gln Ile Asn Asp Ala Phe Thr Val
            340                 345                 350

Ser Ile Thr Asp Gly Arg Thr Ala Ser Gln Leu Ala Glu Gln Ala Ala
        355                 360                 365

Ala Asp Ala Phe Val Val Asp Leu Ala Leu Asn Tyr Ala Asp Thr Thr
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 27
<211> 509
<212> PRT
<213> Serratia marcescens Z6

<400> 27

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Pro Thr Met Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Asp Ala Val Leu Ala Asn Leu Met Phe Thr Ser
65                  70                  75                  80

Val Phe Glu Ala Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Arg Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Cys Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Ile Asp Val Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 28
<211> 509
<212> PRT
<213> Serratia marcescens 546

<400> 28

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Arg Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ser Thr Ala Val Thr His Asn Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

```
Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Asn Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ala Ala Val Arg Ile Asn Asp Ala Phe Thr Val
            340                 345                 350

Ser Ile Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Thr
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460
```

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 29
<211> 509
<212> PRT
<213> Serratia nematodiphila MB307

<400> 29

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Pro Thr Met Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Arg Asp Lys Asp Ala Val Leu Ala Asn Leu Met Phe Thr Ser
65 70 75 80

Val Phe Glu Ala Ile Ala Asp Ser Glu Leu Val Ile Glu Thr Ile Ala
85 90 95

Glu Gln Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Ala Gln Ile Asp Arg Ala Leu Lys
210 215 220

Ala Gly Gly Arg Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Gln Val Thr Ala Ala Asn Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Ala Ala Val Gln Ile Asn Asp Ala Phe Thr Val
340 345 350

Ser Ile Thr Asp Gly Arg Thr Ala Ser Gln Leu Ala Glu Gln Thr Ala
355 360 365

Ala Asp Ala Phe Val Val Asp Leu Ala Leu Asn Tyr Ala Asp Thr Thr
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

```
Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505


<210>  30
<211>  509
<212>  PRT
<213>  Serratia marcescens VGH107

<400>  30

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Pro Thr Met Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Asp Ala Val Leu Ala Asn Leu Met Phe Thr Ser
65                  70                  75                  80

Val Phe Glu Ala Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Val
            100                 105                 110

Val Lys His Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140
```

```
His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Ala Gln Ile Asp Arg Ala Leu Lys
    210                 215                 220

Ala Gly Gly Arg Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Asn Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Ala Ala Val Gln Ile Asn Asp Ala Phe Thr Val
            340                 345                 350

Ser Ile Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Thr
    370                 375                 380

Tyr Leu Val Ala Ala His Ser Arg His Ala Ser Ala Ala Asn Lys Ala
```

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Met Leu Ser
465 470 475 480

Asn Leu Ala Gln Leu Leu His Ala Ala Arg Tyr Ala Pro His Tyr Asn
485 490 495

Leu Leu His Ala Ala Gln Pro Ala Leu Thr Thr Thr Pro
500 505

<210> 31
<211> 509
<212> PRT
<213> Serratia marcescens MCB

<400> 31

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Ser Ser Val Leu Ala Asn Leu Val Phe Ser Thr
65 70 75 80

Ala Leu Glu Ala Ile Ala Asp Ser Glu Leu Val Ile Glu Thr Ile Ala
85 90 95

Glu His Glu Gln Thr Lys Leu Glu Val Leu Ala Ala Ile Ala Ala Val

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Thr Ala
275 280 285

Glu Glu Thr Ala Pro Pro Val Thr Ala Ala Ile Gly Ala Asp Val Glu
290 295 300

Thr Leu Arg Ile Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Val Gly Pro Ala Val Gln Ile Asn Asp Ala Phe Thr Val
340 345 350

Ser Ile Thr Asp Gly Arg Thr Ala Ser Gln Leu Ala Glu Gln Thr Thr
355 360 365

Ala Asp Ala Phe Val Val Asp Ile Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Ser Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 32
<211> 509
<212> PRT
<213> Serratia marcescens AH0650

<400> 32

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Ser Ser Val Leu Ala Asn Leu Val Phe Ser Thr
65 70 75 80

Ala Leu Glu Ala Ile Ala Asp Ser Glu Leu Val Ile Glu Thr Ile Ala
85 90 95

Glu His Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Ile Asp Ala Asp Val Glu
290 295 300

Thr Leu Arg Val Tyr Gly Glu His Pro Phe Phe Thr Leu Leu Gln Gln
305 310 315 320

Arg Ala Ala Arg Gln Trp Pro Gln Leu Arg Val Glu Gln Arg Pro Ala
325 330 335

Leu Pro Ser Leu Gly Pro Ala Val Gln Ile Asn Asp Ala Phe Thr Val
340 345 350

Ser Ile Thr Asp Gly Arg Thr Ala Ser Gln Leu Ala Glu Gln Thr Thr
355 360 365

Ala Asp Ala Phe Val Val Asp Ile Ala Leu Asn Tyr Ala Asp Thr Ala
370 375 380

Tyr Leu Val Ala Ala His Asn Arg Tyr Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 33
<211> 509
<212> PRT
<213> Serratia marcescens UMH12

<400> 33

Met Ala Glu Ser Asn Ala Glu Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Ser Ser Val Leu Ala Asn Leu Val Phe Ser Thr
65 70 75 80

Ala Leu Glu Ala Ile Ala Asp Ser Glu Leu Val Ile Glu Thr Ile Ala
85 90 95

Glu His Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Ile Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Arg Gln Trp Pro Gln Leu Arg Val Glu Gln Arg Pro Ala
                325                 330                 335

Leu Pro Ser Leu Gly Pro Ala Val Gln Ile Asn Asp Ala Phe Thr Val
            340                 345                 350

Ser Ile Thr Asp Gly Arg Thr Ala Ser Gln Leu Ala Glu Gln Thr Thr
        355                 360                 365

Ala Asp Ala Phe Val Val Asp Ile Ala Leu Asn Tyr Ala Asp Thr Ala
    370                 375                 380

Tyr Leu Val Ala Ala His Asn Arg Tyr Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460

Asp Trp Leu Gly Lys Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 34
<211> 509
<212> PRT
<213> Serratia sp. OMLW3

<400> 34

```
Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Ser Ser Val Leu Ala Asn Leu Val Phe Ser Thr
65                  70                  75                  80

Ala Leu Val Ala Ile Ala Asp Ser Glu Leu Val Ile Glu Thr Ile Ala
                85                  90                  95

Glu His Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
```

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Ile Asp Ala Asp Val Glu
290 295 300

Thr Leu Arg Val Tyr Gly Glu His Pro Phe Phe Thr Leu Leu Gln Gln
305 310 315 320

Arg Ala Ala Arg Gln Trp Pro Gln Leu Arg Val Glu Gln Arg Pro Ala
325 330 335

Leu Pro Ser Leu Gly Pro Ala Val Gln Ile Asn Asp Ala Phe Thr Val
340 345 350

Ser Ile Thr Asp Gly Arg Thr Ala Ser Gln Leu Ala Glu Gln Thr Thr
355 360 365

Ala Asp Ala Phe Val Val Asp Ile Ala Leu Asn Tyr Ala Asp Thr Ala
370 375 380

Tyr Leu Val Ala Ala His Asn Arg Tyr Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

```
Leu Leu His Ala Ala Gln Pro Ala Leu Thr Thr Thr Pro
            500                 505


<210>  35
<211>  509
<212>  PRT
<213>  Serratia marcescens UMH11

<400>  35

Met Ala Glu Ser Asn Ala Ala Ile Gln Ser Ala Ala Ile Ile Gly Ala
1               5                   10                  15


Gly Thr Met Gly Arg Gly Ile Ala Tyr Leu Phe Ala Gln Lys Gly Ile
            20                  25                  30


Arg Thr Val Leu Tyr Asn Arg Ser Gly Asn Thr Leu Asn Gln Ala Arg
        35                  40                  45


Glu Tyr Ile Ala Gln Asp Leu Asn Lys Lys Val Glu Gln Gly Lys Ile
    50                  55                  60


Ala Leu Gln Asp Lys Ser Ser Val Leu Ala Asn Leu Val Phe Ser Thr
65                  70                  75                  80


Ala Leu Val Ala Ile Ala Asp Ser Glu Leu Val Ile Glu Thr Ile Ala
                85                  90                  95


Glu His Glu Gln Thr Lys Leu Glu Val Leu Ala Ala Ile Ala Ala Val
            100                 105                 110


Val Lys Pro Asp Thr Leu Ile Ala Thr Asn Thr Ser Ser Leu Ser Leu
        115                 120                 125


Asn Lys Leu Ala Thr Ala Val Thr His Ser Glu Arg Phe Ile Gly Leu
    130                 135                 140


His Phe Phe Asn Pro Ala Pro Leu Met Lys Leu Ile Glu Ile Ile Pro
145                 150                 155                 160


Ala Tyr Phe Thr Ala His Ala Thr Thr Glu Arg Cys Arg Gln Leu Val
                165                 170                 175


Ala Ala Leu Gly Lys Arg Asp Val Val Cys Gln Ala Thr Pro Gly Phe
            180                 185                 190


Ile Val Asn Arg Met Ala Arg Pro Tyr Tyr Leu Glu Gly Phe Arg Leu
        195                 200                 205
```

```
Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Ile Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Arg Gln Trp Pro Gln Leu Arg Val Glu Gln Arg Pro Ala
                325                 330                 335

Leu Pro Ser Leu Gly Pro Ala Val Gln Ile Asn Asp Ala Phe Thr Val
            340                 345                 350

Ser Ile Thr Asp Gly Arg Thr Ala Ser Gln Leu Ala Glu Gln Thr Thr
        355                 360                 365

Ala Asp Ala Phe Val Val Asp Ile Ala Leu Asn Tyr Ala Asp Thr Ala
    370                 375                 380

Tyr Leu Val Ala Ala His Asn Arg Tyr Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Gly Gly Ile Phe
    450                 455                 460
```

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 36
<211> 509
<212> PRT
<213> Serratia marcescens UMH1

<400> 36

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Ser Ser Val Leu Ala Asn Leu Val Phe Ser Thr
65 70 75 80

Ala Leu Glu Ala Ile Ala Asp Ser Glu Leu Val Ile Glu Thr Ile Ala
85 90 95

Glu His Glu Gln Thr Lys Leu Glu Val Leu Ala Ala Ile Ala Ala Val
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Thr Ala Pro Pro Val Thr Ala Ala Ile Asp Ala Asp Val Glu
290 295 300

Thr Leu Arg Val Tyr Gly Glu His Pro Phe Phe Thr Leu Leu Gln Gln
305 310 315 320

Gln Ala Ala Arg Gln Trp Pro Gln Leu Arg Val Glu Gln Arg Pro Ala
325 330 335

Leu Pro Ser Leu Gly Pro Ala Val Gln Ile Asn Gly Ala Phe Thr Val
340 345 350

Ser Ile Thr Asp Gly Arg Thr Ala Ser Gln Leu Ala Glu Gln Thr Thr
355 360 365

Ala Asp Ala Phe Val Ile Asp Ile Ala Leu Asn Tyr Ala Asp Thr Ala
370 375 380

Tyr Leu Val Ala Ala His Asn Arg Tyr Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 37
<211> 509
<212> PRT
<213> Serratia marcescens 2880STDY5683020

<400> 37

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

```
His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
```

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 38
<211> 509
<212> PRT
<213> Serratia marcescens 99

<400> 38

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Ser Asn Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Val Leu Thr Thr Thr Pro
500 505

<210> 39
<211> 509
<212> PRT
<213> Serratia marcescens 374

<400> 39

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

```
Ala Leu Gln Asp Lys Ser Ser Val Leu Ala Asn Leu Val Phe Ser Thr
65                  70                  75                  80

Ala Leu Val Ala Ile Ala Asp Ser Glu Leu Val Ile Glu Thr Ile Ala
                85                  90                  95

Glu His Glu Gln Thr Lys Leu Glu Val Leu Ala Ala Ile Ala Ala Val
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Thr Ala Pro Pro Val Thr Ala Ala Ile Asp Ala Asp Val Glu
    290                 295                 300

Thr Leu Arg Val Tyr Gly Glu His Pro Phe Phe Thr Leu Leu Gln Gln
305                 310                 315                 320
```

Gln Ala Ala Arg Gln Trp Pro Gln Leu Arg Val Glu Gln Arg Pro Ala
325 330 335

Leu Pro Ser Leu Gly Pro Ala Val Gln Ile Asn Gly Ala Phe Thr Val
340 345 350

Ser Ile Thr Asp Gly Arg Thr Ala Ser Gln Leu Ala Glu Gln Thr Thr
355 360 365

Ala Asp Ala Phe Val Ile Asp Ile Ala Leu Asn Tyr Ala Asp Thr Ala
370 375 380

Tyr Leu Val Ala Ala His Asn Arg Tyr Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Gly Gly Ile Phe
450 455 460

Asp Trp Leu Gly Lys Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 40
<211> 509
<212> PRT
<213> Serratia marcescens 2880STDY5683036

<400> 40

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Leu Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Ser Asn Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Leu Thr Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Ser Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Val Leu Thr Thr Thr Pro
            500                 505

<210>  41
<211>  509
<212>  PRT
<213>  Serratia marcescens 2880STDY5683034

<400> 41

```
Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65                  70                  75                  80

Val Phe Glu Thr Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
```

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Ile Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Asn Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 42
<211> 509
<212> PRT
<213> Serratia marcescens 2880STDY5682892

<400> 42

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 43
<211> 509
<212> PRT
<213> Serratia marcescens SM39

<400> 43

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Val Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Ile Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Asn Leu Gly Glu Lys Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 44
<211> 509
<212> PRT
<213> Serratia marcescens 189

<400> 44

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65                  70                  75                  80

Val Phe Glu Thr Ile Ala Asp Ser Asp Leu Val Ile Glu Thr Ile Ala
                85                  90                  95

Glu Gln Glu Gln Thr Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

```
His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
    290                 295                 300

Thr Leu Arg Ile Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Leu Thr Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
```

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Asn Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 45
<211> 509
<212> PRT
<213> Serratia marcescens SMB2099

<400> 45

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala

```
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350
```

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Glu Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 46
<211> 509
<212> PRT
<213> Serratia marcescens 2880STDY5682862

<400> 46

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Val Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Met Ala Gln Leu Leu His Ala Ala Arg Tyr Ala Pro His Tyr Thr
485 490 495

Leu Leu His Ala Ala Gln Pro Ala Leu Met Thr Thr Pro
500 505

<210> 47
<211> 509
<212> PRT
<213> Serratia marcescens SE4145

<400> 47

Met Ala Glu Ser Asn Ala Glu Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
        35                  40                  45

Glu Tyr Ile Val Gln Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65                  70                  75                  80

Val Phe Glu Thr Ile Ala Asp Ser Glu Leu Val Ile Glu Thr Ile Ala
                85                  90                  95

Glu Gln Glu Gln Thr Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
290 295 300

Thr Leu Arg Ile Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Asn Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 48
<211> 509
<212> PRT
<213> Serratia marcescens 2880STDY5682876

<400> 48

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Gly Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 49
<211> 509
<212> PRT
<213> Serratia marcescens 709

<400> 49

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Asp Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 50
<211> 509
<212> PRT
<213> Serratia marcescens MGH136

<400> 50

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Asp Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 51
<211> 509
<212> PRT
<213> Serratia marcescens 2880STDY5682884

<400> 51

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

```
His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
    290                 295                 300

Thr Leu Arg Ile Tyr Gly Glu His Pro Phe Phe Thr Leu Leu Gln Gln
305                 310                 315                 320

Arg Ala Thr Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Leu Met Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
```

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Met Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 52
<211> 509
<212> PRT
<213> Serratia marcescens D-3

<400> 52

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Ile Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Leu Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 53
<211> 509
<212> PRT
<213> Serratia marcescens 2880STDY5682957

<400> 53

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Ala Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 54
<211> 509
<212> PRT
<213> Serratia marcescens YDC563

<400> 54

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Ala Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Gln Thr Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Ser Leu Gly Glu Lys Asn Val Arg Thr Thr Leu Ser
465                 470                 475                 480

Asn Met Ala Gln Leu Leu His Ala Ala Arg Tyr Ala Pro His Tyr Thr
                485                 490                 495

Leu Leu His Ala Ala Gln Pro Ala Leu Met Thr Thr Pro
            500                 505

<210> 55
<211> 509
<212> PRT
<213> Serratia marcescens 2880STDY5683035

<400> 55

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Asn Gln Ala Arg
        35                  40                  45

Glu Tyr Ile Ala Gln Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65                  70                  75                  80

Val Phe Glu Thr Ile Ala Asp Ser Asp Leu Val Ile Glu Thr Ile Ala
                85                  90                  95

Glu Gln Glu Gln Thr Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Arg Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Ile Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Ile Asn Glu Ala Phe Thr Val
340 345 350

Ser Val Thr Asp Gly Arg Thr Ala Ser Gln Leu Ala Glu Leu Thr Ala
355 360 365

Ala Asp Val Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Leu Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 56
<211> 509
<212> PRT
<213> Serratia marcescens 2880STDY5682930

<400> 56

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Asp Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Leu Pro Val Thr Ala Ala Thr Asp Ala Asp Val Glu
    290                 295                 300

Thr Leu Arg Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Pro Ala
                325                 330                 335

Leu Pro Gly Leu Gly Pro Ala Val Gln Ile Asn Glu Ala Phe Thr Val
            340                 345                 350

Ser Val Thr Asp Gly Arg Thr Ala Ser Gln Leu Ala Glu Leu Thr Ala
        355                 360                 365

Ala Asp Ala Phe Val Val Asp Val Ala Leu Asn Tyr Ala Asp Thr Ala
    370                 375                 380

Tyr Leu Val Ala Ala His Asn Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asn Val Ala Ala Val Ala Gly Val Asn Tyr Ala Asp Gly Ile Phe
    450                 455                 460

Gly Leu Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Val Leu Thr Thr Thr Pro
500 505

<210> 57
<211> 509
<212> PRT
<213> Serratia marcescens 790

<400> 57

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Glu Tyr Ile Ala Gln Asp Leu Asn Lys Lys Val Glu Gln Gly Lys Ile
50 55 60

Ala Leu Arg Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Arg Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Leu Pro Val Thr Ala Ala Thr Asp Ala Asp Val Glu
290 295 300

Thr Leu Arg Val Tyr Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Gln Thr Asn Glu Ala Phe Thr Val
340 345 350

Ser Val Thr Asp Gly Arg Thr Ala Asn Gln Leu Ala Glu Leu Thr Ala
355 360 365

Ala Asp Ala Phe Val Val Asp Val Ala Leu Asn Tyr Ala Asp Thr Ala
370 375 380

Tyr Leu Val Ala Ala His Asn Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Ser Leu Gly Glu Lys Asn Val Arg Thr Thr Leu Ser
465                 470                 475                 480

Asn Met Ala Gln Leu Leu His Ala Ala Arg Tyr Ala Pro His Tyr Thr
                485                 490                 495

Leu Leu His Ala Ala Gln Pro Ala Leu Met Thr Thr Pro
            500                 505

<210>  58
<211>  509
<212>  PRT
<213>  Serratia marcescens UMH5

<400>  58

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Ser Gln Ala Arg
        35                  40                  45

Asp Tyr Ile Glu Leu Asp Leu Asn Lys Lys Val Glu Gln Gly Lys Ile
    50                  55                  60

Ala Leu Gln Asp Lys Gly Ala Val Leu Ala Asn Leu Val Phe Thr Ser
65                  70                  75                  80

Val Phe Glu Thr Ile Ala Asp Ser Glu Leu Val Ile Glu Thr Ile Ala
                85                  90                  95

Glu Gln Glu Gln Thr Lys Leu Glu Val Leu Ala Thr Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Cys Thr Ala His Ala Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Ala Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Val
        275                 280                 285

Glu Glu Asn Ala Pro Pro Val Met Ala Ala Thr Asp Ala Asp Ile Glu
    290                 295                 300

Thr Leu His Val Tyr Gly Glu His Pro Phe Phe Thr Leu Leu Gln Gln
305                 310                 315                 320

Arg Ala Ala Leu Gln Trp Pro Arg Leu Arg Val Glu Gln Arg Gln Ala
                325                 330                 335

Leu Pro Gly Leu Gly Pro Ala Val Arg Ile Asn Asp Ala Phe Thr Ile
            340                 345                 350

Ser Ile Thr Asp Gly Arg Thr Ala Asn Gln Leu Ala Glu Gln Thr Ala
        355                 360                 365

Ala Asp Ala Phe Val Val Asp Val Ala Leu Asn Tyr Ala Asp Thr Ala
    370                 375                 380

Tyr Leu Val Ala Ala His Ser Arg His Ala Ser Gly Ala Asn Lys Ala

385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 59
<211> 509
<212> PRT
<213> Serratia marcescens 2880STDY5682988

<400> 59

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Ser Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Thr Ile Ala Ala Ala

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Cys Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Val
275 280 285

Glu Glu Asn Ala Pro Pro Val Met Ala Ala Thr Asp Ala Asp Ile Glu
290 295 300

Thr Leu His Val Tyr Gly Glu His Pro Phe Phe Thr Leu Leu Gln Gln
305 310 315 320

Arg Ala Ala Leu Gln Trp Pro Arg Leu Arg Val Glu Gln Arg Gln Ala
325 330 335

Leu Pro Gly Leu Gly Pro Ala Val Arg Ile Asn Asp Ala Phe Thr Ile
340 345 350

Ser Ile Thr Asp Gly Arg Thr Ala Asn Gln Leu Ala Glu Gln Thr Ala
355 360 365

Thr Asp Ala Phe Val Val Asp Val Ala Leu Asn Tyr Ala Asp Thr Ala
370 375 380

Tyr Leu Val Ala Ala His Ser Arg His Ala Ser Gly Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 60
<211> 509
<212> PRT
<213> Serratia marcescens 945154301

<400> 60

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Ala
275 280 285

Glu Glu Asn Ala Pro Pro Val Met Ala Ala Thr Asp Ala Asp Ile Glu
290 295 300

Thr Leu His Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Pro Ala Val Arg Ile Asn Asp Ala Phe Ser Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 61
<211> 509
<212> PRT
<213> Serratia marcescens at10508

<400> 61

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Thr Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Val
275 280 285

Glu Glu Asn Ala Pro Pro Val Met Ala Ala Thr Asp Ala Asp Ile Glu
290 295 300

Thr Leu His Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Pro Ala Val Arg Ile Asn Asp Ala Phe Thr Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 62
<211> 509
<212> PRT
<213> Serratia marcescens ML2637

<400> 62

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Thr Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met

245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Val
275 280 285

Glu Glu Asn Ala Pro Pro Val Met Ala Ala Thr Asp Ala Asp Ile Glu
290 295 300

Thr Leu His Val Tyr Gly Glu His Pro Phe Phe Thr Leu Leu Gln Gln
305 310 315 320

Arg Ala Ala Leu Gln Trp Pro Gln Leu Arg Val Glu Gln Arg Pro Ala
325 330 335

Leu Pro Gly Leu Gly Pro Ala Val Arg Ile Asn Asp Ala Phe Thr Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Val Gln Pro Ala Leu Thr Thr Thr Pro
500 505

<210> 63
<211> 509
<212> PRT
<213> Serratia marcescens SM1978

<400> 63

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Thr Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Ala Arg Tyr Thr Pro Gly His Leu Gln Arg Ser Leu Val
            260                 265                 270

Asp Ala Gly Leu Leu Gly Lys Lys Asn Gly Arg Ser Tyr Phe Ala Ala
        275                 280                 285

Glu Glu Asn Ala Pro Pro Val Met Ala Ser Thr Asp Ala Asp Ile Glu
    290                 295                 300

Thr Leu His Val Tyr Gly Glu His Pro Phe Phe Thr Leu Leu Gln Gln
305                 310                 315                 320

Arg Ala Ala Leu Gln Trp Pro Gln Leu Arg Val Glu Gln Arg Pro Ala
                325                 330                 335

Leu Pro Gly Leu Gly Pro Ala Val Arg Ile Asn Asp Ala Phe Ser Val
            340                 345                 350

Ser Ile Thr Asp Gly Arg Thr Ala Asn Gln Leu Ala Glu Gln Thr Thr
        355                 360                 365

Ala Asp Ala Phe Val Val Asp Val Ala Leu Asn Tyr Ala Asp Thr Ala
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Ser Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 64
<211> 509
<212> PRT
<213> Serratia marcescens PWN146

<400> 64

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Asn Gln Ala Arg
35 40 45

Asp Tyr Ile Glu Leu Asp Leu Asn Lys Lys Val Glu Gln Gly Lys Ile
50 55 60

Ala Leu Gln Asp Lys Gly Ala Val Leu Ala Asn Leu Val Phe Thr Ser
65 70 75 80

Val Phe Glu Thr Ile Ala Asp Ser Glu Leu Val Ile Glu Thr Ile Ala
85 90 95

Glu Gln Glu Gln Thr Lys Leu Glu Val Leu Ala Thr Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala His Ala Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Ala Arg Tyr Thr Pro Gly His Leu Gln Arg Ser Leu Val
260 265 270

Asp Ala Gly Leu Leu Gly Lys Lys Asn Gly Arg Ser Tyr Phe Ala Val
275 280 285

Glu Glu Asn Ala Pro Pro Val Met Ala Ala Thr Asp Ala Asp Ile Glu
290 295 300

Thr Leu His Val Cys Gly Glu His Pro Phe Phe Thr Leu Leu Gln Gln
305 310 315 320

Arg Ala Ala Leu Gln Trp Pro Arg Leu Arg Val Glu Gln Arg Pro Ala
325 330 335

Leu Pro Gly Leu Gly Pro Ala Val Trp Ile Asn Asp Ala Phe Thr Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

```
Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Asp Ser Leu Gly Glu Lys Asn Val Arg Ala Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505


<210>  65
<211>  509
<212>  PRT
<213>  Serratia marcescens H1q

<400>  65

Met Ala Glu Arg Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Gly Ile
            20                  25                  30

Arg Thr Val Leu Tyr Asn Arg Asn Gly Asn Thr Leu Ile Gln Ala Arg
        35                  40                  45

Glu Tyr Ile Val Gln Asp Leu Asp Lys Lys Val Glu Gln Gly Arg Leu
    50                  55                  60

Ala Pro Gln Asp Lys Asp Ala Val Leu Ala Asn Leu Gln Phe Ser Ser
65                  70                  75                  80

Val Phe Glu Ala Ile Val Asp Ser Asp Leu Val Leu Glu Thr Ile Ala
                85                  90                  95

Glu Gln Glu Gln Ala Lys Leu Glu Val Leu Ala Ala Ile Ala Ala Ala
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140
```

His Phe Phe Asn Pro Ala Pro Leu Met Lys Leu Ile Glu Ile Ile Pro
145 150 155 160

Ala Tyr Phe Thr Ala Gln Ala Thr Thr Glu Thr Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Thr Ala Pro Pro Val Glu Ala Ala Val Glu Ala Asp Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Gln Leu Arg Val Glu Gln Arg Pro Ala
325 330 335

Leu Ser Gly Leu Gly Ala Ala Ile Gln Val Asn Asp Ala Phe Thr Val
340 345 350

Ser Ile Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala

385 390 395 400

Leu Phe Leu Arg Leu Leu His Thr Ala Leu Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 66
<211> 509
<212> PRT
<213> Serratia marcescens UMH6

<400> 66

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 67
<211> 509
<212> PRT
<213> Serratia nematodiphila WCU338

<400> 67

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Ile Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285

Glu Glu Ser Pro Pro Pro Leu Ala Ala Ala Val Asp Ala Glu Val Glu
    290                 295                 300

Thr Leu Arg Ile Tyr Gly Glu His Pro Leu Phe Thr Leu Leu Gln Gln
305                 310                 315                 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 68
<211> 509
<212> PRT
<213> Serratia sp. OLEL1

<400> 68

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

```
Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

Ala Leu Gln Asp Lys Asp Ala Val Leu Ala Asn Leu Thr Phe Ser Ala
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Val Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285
```

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 69
<211> 509
<212> PRT
<213> Serratia marcescens 7209

<400> 69

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Ser Ile
            20                  25                  30

Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

Ala Leu Gln Asp Lys Asp Ala Val Leu Ala Asn Leu Thr Phe Ser Ala
65                  70                  75                  80

Glu Phe Gly Val Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Val Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 70
<211> 509
<212> PRT
<213> Serratia marcescens sicaria (Ss1)

<400> 70

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Thr
        275                 280                 285

Glu Glu Ser Pro Pro Pro Leu Ala Ala Ala Val Asp Ala Glu Val Glu
    290                 295                 300

Thr Leu Arg Ile Tyr Gly Glu His Pro Leu Phe Thr Leu Leu Gln Gln
305                 310                 315                 320

Arg Ala Ala Leu Gln Trp Pro Arg Leu Arg Val Glu Gln Arg Pro Thr
                325                 330                 335

Leu Pro Gly Leu Gly Ala Ala Ile Gln Val Asn Asp Ala Phe Thr Val
            340                 345                 350

Ser Val Thr Asp Gly Arg Thr Ala Asn Gln Leu Ala Glu Gln Thr Ala
        355                 360                 365

Ala Asp Ala Phe Val Val Asp Val Ala Leu Asn Tyr Gly Asp Thr Ala
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 71
<211> 509
<212> PRT
<213> Serratia sp. OLFL2

<400> 71

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Asp Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 72
<211> 509
<212> PRT
<213> Serratia marcescens BIDMC 81

<400> 72

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Ala
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Asp Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala

```
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Thr Arg Leu Gly Glu Glu Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505


<210>  73
<211>  509
<212>  PRT
<213>  Serratia marcescens BIDMC 50

<400>  73

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Phe Ala Gln Lys Ser Ile
            20                  25                  30

Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

Ala Leu Gln Asp Lys Asp Val Val Leu Ala Asn Leu Thr Phe Ser Ala
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
```

100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Asp Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
        355                 360                 365

Ala Asp Ala Phe Val Val Asp Val Ala Leu Asn Tyr Gly Asp Thr Ala
    370                 375                 380

Tyr Leu Val Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460

Gly Trp Leu Thr Arg Leu Gly Glu Glu Asn Val Arg Thr Thr Leu Ser
465                 470                 475                 480

Asn Leu Ala Gln Leu Leu His Ala Ala Arg Tyr Ala Pro His Tyr Thr
                485                 490                 495

Leu Leu His Ala Ala Gln Pro Ala Leu Thr Thr Thr Pro
            500                 505

<210> 74
<211> 509
<212> PRT
<213> Serratia marcescens UMH7

<400> 74

Met Ala Glu Ser Asn Ala Ala Ile Gln Ser Ala Ala Ile Ile Gly Ala
1               5                   10                  15

Gly Thr Met Gly Arg Gly Ile Ala Tyr Leu Leu Ala Gln Lys Ser Ile
            20                  25                  30

Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

```
Ala Leu Gln Asp Lys Asp Thr Val Leu Ala Asn Leu Thr Phe Ser Ala
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Thr Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Val Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285

Glu Glu Ser Pro Pro Pro Leu Ala Ala Ala Val Asp Ala Glu Val Glu
    290                 295                 300

Thr Leu Arg Ile Tyr Gly Glu His Pro Leu Phe Thr Leu Leu Gln Gln
305                 310                 315                 320
```

Arg Ala Thr Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 75
<211> 509
<212> PRT
<213> Serratia marcescens RSC-14

<400> 75

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

```
Arg Thr Val Leu Tyr Asn Arg Asn Gly Asn Thr Leu Asn Gln Ala Arg
        35                  40                  45

Asp Ala Ile Val Gln Asp Leu Asn Lys Lys Val Glu Gln Gly Lys Leu
    50                  55                  60

Ala Leu Gln Asp Lys Asp Ala Val Leu Ala Asn Leu Thr Phe Ser Thr
65                  70                  75                  80

Glu Phe Gly Ala Ile Ala Asp Ser Asp Leu Val Ile Glu Thr Ile Ala
                85                  90                  95

Glu His Glu Gln Ala Lys Leu Glu Val Leu Ala Ala Ile Ala Ala Thr
            100                 105                 110

Val Lys Pro Asp Arg Leu Ile Ala Thr Asn Thr Ser Ser Leu Ser Leu
        115                 120                 125

Asn Lys Leu Ala Thr Ala Val Thr His Ser Glu Arg Phe Ile Gly Leu
    130                 135                 140

His Phe Phe Asn Pro Ala Pro Leu Met Lys Leu Ile Glu Ile Ile Pro
145                 150                 155                 160

Ala Tyr Phe Thr Ala Gln Val Thr Thr Glu Arg Cys Arg Gln Leu Val
                165                 170                 175

Ala Ala Leu Gly Lys Arg Asp Val Val Cys Gln Ala Thr Pro Gly Phe
            180                 185                 190

Ile Val Asn Arg Met Ala Arg Pro Tyr Tyr Leu Glu Gly Phe Arg Leu
        195                 200                 205

Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285
```

Glu Glu Ser Pro Pro Pro Leu Ala Ala Ala Val Asp Ala Glu Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Gln Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 76
<211> 509
<212> PRT
<213> Serratia marcescens SM03

<400> 76

Met Ala Glu Arg Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Ile Gln Ala Arg
35 40 45

Glu Tyr Ile Val Gln Asp Leu Asp Lys Lys Val Glu Gln Gly Arg Leu
50 55 60

Ala Pro Gln Asp Lys Asp Ala Val Leu Ala Asn Leu Gln Phe Ser Ser
65 70 75 80

Val Phe Glu Ala Ile Val Asp Ser Asp Leu Val Leu Glu Thr Ile Ala
85 90 95

Glu Gln Glu Gln Ala Lys Leu Glu Val Leu Ala Ala Ile Ala Ala Ala
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Ala Thr Thr Glu Thr Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met

245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Thr Ala Pro Pro Val Glu Ala Ala Ala Glu Ala Asp Val Glu
290 295 300

Thr Leu Arg Ile Tyr Gly Glu His Pro Leu Phe Thr Leu Leu Gln Gln
305 310 315 320

Arg Ala Ala Leu Gln Trp Pro Gln Leu Arg Val Glu Gln Arg Pro Ala
325 330 335

Leu Ser Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Ala Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Leu Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr Arg Leu Gly Glu Lys Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 77
<211> 509
<212> PRT
<213> Serratia marcescens 90-166

<400> 77

Met Ala Glu Ser Asn Ala Ala Ile Gln Ser Ala Ala Ile Ile Gly Ala
1 5 10 15

Gly Thr Met Gly Arg Gly Ile Ala Tyr Leu Phe Ala Gln Lys Gly Ile
20 25 30

Arg Thr Val Leu Tyr Asn Arg Asn Gly Asn Thr Leu Ile Gln Ala Arg
35 40 45

Glu Tyr Ile Val Gln Asp Leu Asp Lys Lys Ile Glu Gln Gly Arg Val
50 55 60

Ala Pro Gln Asp Lys Asp Ala Val Leu Ala Asn Leu Thr Phe Ser Ala
65 70 75 80

Glu Phe Gly Ala Ile Val Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

```
Leu Glu Glu His Val Ala Arg Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Arg Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Tyr Phe Ser Ala
        275                 280                 285

Glu Glu Ser Pro Pro Pro Leu Ala Ala Ala Val Asp Ala Glu Ile Glu
    290                 295                 300

Thr Leu Arg Ile Tyr Gly Glu His Pro Leu Phe Thr Leu Leu Gln Gln
305                 310                 315                 320

Arg Ala Ala Leu Gln Trp Pro Arg Leu Arg Val Glu His Arg Pro Thr
                325                 330                 335

Leu Pro Gly Leu Gly Ala Ala Ile Gln Val Asn Asp Ala Phe Thr Val
            340                 345                 350

Ser Val Thr Asp Gly Arg Thr Ala Asn Gln Leu Ala Glu Gln Thr Ala
        355                 360                 365

Ala Asp Ala Phe Val Val Asp Val Ala Leu Asn Tyr Gly Asp Thr Ala
    370                 375                 380

Tyr Leu Ala Ala Ala His Ser Arg His Ala Ser Ala Ala Asn Lys Ala
385                 390                 395                 400

Leu Phe Leu Arg Leu Leu His Thr Ala Ile Pro Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Glu Asp
        435                 440                 445

Ile Asp Val Ala Ala Val Ala Gly Val Asn Tyr Ala Asp Gly Ile Phe
    450                 455                 460
```

Val Trp Leu Thr Arg Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Ser Thr Pro
500 505

<210> 78
<211> 509
<212> PRT
<213> Serratia marcescens UMH2

<400> 78

Met Ala Glu Ser Asn Ala Ala Ile Gln Ser Ala Ala Ile Ile Gly Ala
1 5 10 15

Gly Thr Met Gly Arg Gly Ile Ala Tyr Leu Leu Ala Gln Lys Ser Ile
20 25 30

Arg Thr Val Leu Tyr Asn Arg Asn Gly Asn Thr Leu Asn Gln Ala Arg
35 40 45

Asp Ala Ile Val Gln Asp Leu Asn Lys Lys Val Glu Gln Gly Lys Leu
50 55 60

Ala Leu Gln Asp Lys Asp Ala Val Leu Ala Asn Leu Thr Phe Ser Thr
65 70 75 80

Glu Phe Gly Ala Ile Ala Asp Ser Asp Leu Val Ile Glu Thr Ile Ala
85 90 95

Glu His Glu Gln Ala Lys Leu Glu Val Leu Ala Ala Ile Ala Ala Thr
100 105 110

Val Lys Pro Asp Thr Leu Ile Ala Thr Asn Thr Ser Ser Leu Ser Leu
115 120 125

Asn Lys Leu Ala Thr Ala Val Thr His Ser Glu Arg Phe Ile Gly Leu
130 135 140

His Phe Phe Asn Pro Ala Pro Leu Met Lys Leu Ile Glu Ile Ile Pro
145 150 155 160

Ala Tyr Phe Thr Ala Gln Val Thr Thr Glu Arg Cys Arg Gln Leu Val
165 170 175

Ala Ala Leu Gly Lys Arg Asp Val Val Cys Gln Ala Thr Pro Gly Phe
180 185 190

Ile Val Asn Arg Met Ala Arg Pro Tyr Tyr Leu Glu Gly Phe Arg Leu
195 200 205

Leu Glu Glu His Val Ala Arg Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Arg Gln Ile Trp Gln Asp Met
245 250 255

Gln Tyr Asp Pro Arg Tyr Thr Pro Gly His Leu Gln Arg Ser Leu Val
260 265 270

Asp Ala Gly Leu Leu Gly Lys Lys Asn Gly Arg Ser Tyr Phe Ser Ala
275 280 285

Glu Glu Ser Pro Pro Pro Leu Ala Ala Ala Val Asp Ala Glu Val Glu
290 295 300

Ala Leu Arg Ile Tyr Gly Glu His Pro Leu Phe Thr Leu Leu Gln Gln
305 310 315 320

Arg Ala Ala Leu Gln Trp Pro Arg Leu Arg Val Glu Gln Arg Pro Thr
325 330 335

Leu Pro Gly Leu Gly Ala Ala Ile Gln Val Asn Asp Ala Phe Thr Val
340 345 350

Ser Val Thr Asp Gly Arg Thr Ala Asn Gln Leu Ala Glu Gln Thr Ala
355 360 365

Ala Asp Ala Phe Val Val Asp Val Ala Leu Asn Tyr Gly Asp Thr Ala
370 375 380

Tyr Leu Val Ala Ala His Ser Arg His Ala Ser Ala Ala Asn Lys Ala
385 390 395 400

Leu Phe Leu Arg Leu Leu His Thr Ala Ile Pro Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Glu Asp
435 440 445

Ile Asp Val Ala Ala Val Ala Gly Val Asn Tyr Ala Asp Gly Ile Phe
450 455 460

Gly Trp Leu Thr His Leu Gly Glu Glu Asn Val Arg Thr Thr Leu Ser
465 470 475 480

Asn Leu Ala Gln Leu Leu His Ala Ala Arg Tyr Ala Pro His Tyr Thr
485 490 495

Leu Leu His Ala Ala Gln Pro Ala Leu Thr Thr Thr Pro
500 505

<210> 79
<211> 509
<212> PRT
<213> Serratia plymuthica AS9

<400> 79

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Met Asp Asn Gly Lys Ile
50 55 60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr
100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145                 150                 155                 160

Ser Tyr Phe Thr Ser His Thr Thr Ser Leu Arg Cys Gln Gln Leu Val
                165                 170                 175

Ile Ala Leu Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Gln Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Lys Gln Ile Trp Gln Asp Met
                245                 250                 255

Gln Phe Asp Ser Arg Tyr Thr Pro Gly His Leu Gln Arg Ser Leu Val
            260                 265                 270

Asp Ala Gly Leu Leu Gly Arg Lys Asn Gly Arg Ser Phe Phe Ala Ser
        275                 280                 285

Leu Pro Ala Thr Pro Pro Thr Pro Ala Thr Glu Ser Asp Thr Pro Thr
    290                 295                 300

Ser Leu His Phe Tyr Gly Glu His Ala Leu Phe Asp His Leu Gln Gln
305                 310                 315                 320

Arg Ala Leu Ala Thr Trp Pro Ala Leu Arg Val Gln Arg Leu Pro Glu
                325                 330                 335

Arg Pro Glu Leu Gly Arg Leu Ile Leu Val Asn Asn Thr Leu Ala Ile
            340                 345                 350

Lys Ile Thr Asp Gly Arg Thr Ala Asn Leu Leu Ala Gly Leu Thr Ala
        355                 360                 365

Leu Asp Thr Phe Val Ile Asp Ala Ala Leu Asn Tyr Ala Asp Thr Ala
    370                 375                 380

Tyr Leu Val Ala Ala His Asn Gln His Ala Thr Glu Ala Asn Lys Ala

385 390 395 400

Leu Phe Leu Ser Leu Leu Gln Thr Val Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Ala Arg Pro Glu Leu Ala Val Ala Pro
500 505

<210> 80
<211> 509
<212> PRT
<213> Serratia plymuthica tumat 205

<400> 80

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr

100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser His Ala Thr Ser Leu Arg Cys Gln Gln Leu Val
165 170 175

Ile Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Val Thr Pro Pro Thr Pro Thr Thr Glu Ser Asp Thr Pro Thr
290 295 300

Ser Leu His Phe Tyr Gly Glu His Ala Leu Phe Asp His Leu Gln Gln
305 310 315 320

Arg Ala Leu Ala Thr Trp Pro Ala Leu Arg Val Gln Arg Leu Pro Glu
325 330 335

Arg Pro Glu Leu Gly Arg Phe Ile Leu Val Asn Asn Ala Met Ala Ile
340 345 350

Lys Ile Thr Asp Gly Arg Thr Ala Asn Leu Leu Ala Gly Leu Thr Ala
        355                 360                 365

Leu Asp Thr Phe Val Ile Asp Ala Ala Leu Asn Tyr Ala Asp Thr Ala
    370                 375                 380

Tyr Leu Val Ala Ala His Asn Gln His Ala Thr Glu Thr Asn Lys Ala
385                 390                 395                 400

Leu Phe Leu Thr Leu Leu Gln Thr Val Ile Ala Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Ala Asp
        435                 440                 445

Ile Asp Ile Ala Ala Val Ala Gly Val Asn Tyr Ala Asp Gly Ile Phe
    450                 455                 460

Ala Trp Leu Ala Gln Leu Gly Gln Lys Asn Val Lys Ser Thr Leu Asp
465                 470                 475                 480

Asn Met Ala Gln Leu Leu His Ser Ala Arg Tyr Tyr Pro His Tyr Ser
                485                 490                 495

Leu Leu Asn Ala Ala Arg Pro Glu Leu Ala Val Ala Pro
            500                 505

<210> 81
<211> 509
<212> PRT
<213> Serratia plymuthica A30

<400> 81

Met Ala Glu Asn Asn Ser Ala Ile Arg Ser Ala Ala Val Ile Gly Ala
1               5                   10                  15

Gly Thr Met Gly Arg Gly Ile Ala Tyr Leu Leu Ala Leu Asn Gly Ile
            20                  25                  30

Arg Thr Val Leu Tyr Asn Arg Asn Gly Asn Asn Leu Asn Gln Ala Arg
        35                  40                  45

Asp Tyr Ile Val Ser Asp Leu Asp Arg Lys Ile Asp Asn Gly Lys Ile
    50                  55                  60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr
100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser His Ala Thr Ser Leu Arg Cys Gln Lys Leu Val
165 170 175

Ile Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Asn Pro Thr Thr Glu Gly Asp Thr Pro Thr
290 295 300

Ser Leu His Phe Tyr Gly Glu His Ala Leu Phe Asp His Leu Gln Gln
305 310 315 320

```
Arg Ala Leu Ala Thr Trp Pro Ala Leu Arg Val Gln Arg Leu Pro Glu
                325                 330                 335

Arg Pro Glu Leu Gly Arg Phe Ile Leu Met Asn Asn Arg Leu Ala Ile
            340                 345                 350

Lys Ile Thr Asp Gly Arg Thr Ala Asn Leu Leu Ala Gly Leu Thr Ala
        355                 360                 365

Leu Asp Thr Phe Val Ile Asp Ala Ala Leu Asn Tyr Ala Asp Thr Ala
    370                 375                 380

Tyr Leu Val Ala Ala His Asn Gln His Ala Thr Glu Thr Asn Lys Ala
385                 390                 395                 400

Leu Phe Leu Thr Leu Leu Gln Thr Leu Ile Ala Gln Val Glu Phe Ile
                405                 410                 415

Lys Asp Ser Pro Ala Leu Ile Val Ala Arg Val Leu Ser Ser Leu Ile
            420                 425                 430

Asn Glu Ser Val Ile Met Val Glu Ser Gly Val Cys Ser Arg Ala Asp
        435                 440                 445

Ile Asp Ile Ala Ala Val Ala Gly Val Asn Tyr Ala Asp Gly Ile Phe
    450                 455                 460

Ala Trp Leu Ala Gln Leu Gly Gln Lys Asn Val Lys Ser Thr Leu Asp
465                 470                 475                 480

Asn Met Ala Gln Leu Leu His Ser Ala Arg Tyr Tyr Pro His Tyr Ser
                485                 490                 495

Leu Leu Asn Ala Ala Arg Pro Glu Leu Ala Val Ala Pro
            500                 505
```

<210> 82
<211> 509
<212> PRT
<213> Serratia plymuthica 4Rx13

<400> 82

```
Met Ala Glu Asn Asn Ser Ala Ile Arg Ser Ala Ala Val Ile Gly Ala
1               5                   10                  15

Gly Thr Met Gly Arg Gly Ile Ala Tyr Leu Leu Ala Leu Asn Gly Ile
            20                  25                  30
```

Arg Thr Val Leu Tyr Asn Arg Asn Gly Asn Ser Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr
100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser His Ala Thr Ser Leu Arg Cys Gln Lys Leu Val
165 170 175

Ile Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Thr Pro Thr Thr Glu Ser Asp Thr Pro Thr
290 295 300

Ser Leu His Phe Tyr Gly Glu His Ala Leu Phe Asp His Leu Gln Gln
305 310 315 320

Arg Ala Leu Ala Thr Trp Pro Ala Leu Ser Val Gln Arg Leu Pro Glu
325 330 335

Arg Pro Glu Leu Gly Arg Phe Ile Leu Val Asn Asn Ala Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Thr Asn Lys Ala
385 390 395 400

Leu Phe Leu Thr Leu Leu Gln Thr Val Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Ala Arg Pro Glu Leu Ala Val Ala Pro
500 505

<210> 83
<211> 509
<212> PRT
<213> Serratia plymuthica V4

<400> 83

Met Ala Glu Asn Asn Ser Ala Ile Arg Ser Ala Ala Val Ile Gly Ala
1               5                   10                  15

Gly Thr Met Gly Arg Gly Ile Ala Tyr Leu Leu Ala Leu Asn Gly Ile
            20                  25                  30

Arg Thr Val Leu Tyr Asn Arg Asn Gly Asn Asn Leu Asn Gln Ala Arg
        35                  40                  45

Asp Tyr Ile Val Ser Asp Leu Asp Arg Lys Ile Asp Asn Gly Lys Ile
    50                  55                  60

Thr Leu Gln Lys Lys Gly Gln Ile Leu Ala Asn Ile Ile Phe Ser Ala
65                  70                  75                  80

Val Phe Asp Ala Ile Thr Asp Ser Asp Leu Val Ile Glu Thr Ile Ala
                85                  90                  95

Glu Asp Glu Gln Thr Lys His Glu Ile Leu Ala Ala Ile Ala Ala Thr
            100                 105                 110

Val Lys Pro Glu Ala Ile Ile Ala Thr Asn Thr Ser Ser Leu Ser Leu
        115                 120                 125

Asn Lys Leu Ala Ala Gly Val Glu Asn Asn Pro Arg Phe Ile Gly Leu
    130                 135                 140

His Phe Phe Asn Pro Ala Pro Leu Met Lys Leu Ile Glu Ile Ile Pro
145                 150                 155                 160

Ser Tyr Phe Thr Ser His Ala Thr Ser Leu Arg Cys Gln Lys Leu Val
                165                 170                 175

Ile Ala Leu Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Lys Gln Ile Trp Gln Asp Met

245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Thr Pro Thr Thr Glu Ser Asp Thr Pro Thr
290 295 300

Ser Leu His Phe Tyr Gly Glu His Ala Leu Phe Asp His Leu Gln Gln
305 310 315 320

Arg Ala Leu Ala Thr Trp Pro Ala Leu Arg Val Gln Arg Leu Pro Glu
325 330 335

Arg Pro Glu Leu Gly Arg Phe Ile Leu Val Asn Asn Arg Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Thr Asn Lys Ala
385 390 395 400

Leu Phe Leu Thr Leu Leu Gln Thr Val Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Ala Arg Pro Glu Leu Ala Val Ala Pro
500 505

<210> 84
<211> 509
<212> PRT
<213> Serratia plymuthica 3Rp8

<400> 84

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Ala
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr
100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser His Ala Thr Ser Leu Arg Cys Gln Lys Leu Val
165 170 175

Ile Ala Leu Gly Lys Gln Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Thr Pro Thr Thr Glu Ser Asp Thr Pro Thr
290 295 300

Ser Leu His Phe Tyr Gly Glu His Ala Leu Phe Asp His Leu Gln Gln
305 310 315 320

Arg Ala Leu Ala Thr Trp Pro Ala Leu Arg Val Gln Arg Leu Pro Glu
325 330 335

Arg Pro Glu Leu Gly Arg Phe Ile Leu Val Asn Asn Arg Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Thr Asn Lys Ala
385 390 395 400

Leu Phe Leu Thr Leu Leu Gln Thr Leu Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ala Ala Arg Pro Glu Leu Ala Val Ala Pro
500 505

<210> 85
<211> 509
<212> PRT
<213> Serratia proteamaculans MFPA44A14

<400> 85

Met Ala Glu Asn Asn Ser Ala Ile His Ser Val Ala Val Ile Gly Ala
1 5 10 15

Gly Thr Met Gly Arg Gly Ile Ala Tyr Leu Leu Ala Gln Asn Gly Ile
20 25 30

Arg Thr Leu Leu Tyr Asn Arg Ser Gly Asn Asn Leu Asp Gln Ala Arg
35 40 45

Asp Tyr Ile Ile Arg Asp Leu Asp Lys Lys Ile Asp Asn Gly Lys Ile
50 55 60

Ser Pro Gln Lys Lys Gly Glu Val Leu Ala Asn Leu Val Phe Ser Pro
65 70 75 80

Ile Phe Asp Ala Ile Ala Asp Ser Asp Leu Val Ile Glu Thr Ile Ala
85 90 95

Glu His Glu Thr Thr Lys His Glu Ile Leu Ala Ala Ile Ala Ala Thr
100 105 110

Val Lys Gln Glu Ala Ile Ile Ala Thr Asn Thr Ser Ser Leu Ser Leu
115 120 125

Asn Lys Leu Ala Ala Gly Val Glu Asn Asn Ala Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser Arg Ala Thr Ser Leu Arg Cys Gln Gln Leu Val
165 170 175

```
Thr Ala Leu Gly Lys Gln Phe Val Val Cys Lys Ala Thr Pro Gly Phe
            180                 185                 190

Ile Val Asn Arg Met Ala Arg Pro Phe Tyr Leu Glu Gly Phe Arg Leu
        195                 200                 205

Leu Glu Glu Asn Val Ala Leu Ala Pro Gln Ile Asp Arg Ala Leu Lys
    210                 215                 220

Ala Gly Gly His Phe Arg Met Gly Pro Leu Glu Leu Thr Asp Phe Ile
225                 230                 235                 240

Gly Gln Asp Ile Asn Tyr Gln Val Ser Ser Gln Ile Trp Gln Asp Met
                245                 250                 255

Gln Tyr Asp Pro Arg Tyr Thr Pro Gly His Leu Gln Arg Ser Leu Val
            260                 265                 270

Asp Ala Gly Leu Leu Gly Lys Lys Asn Gly Arg Ser Phe Phe Ala Ala
        275                 280                 285

Pro Ser Ala Glu Ser Asn Leu Leu Asp Ala Gly Asn Gly Thr Leu Thr
    290                 295                 300

Ser Leu His Phe Tyr Gly Glu His Thr Leu Phe Asp Leu Leu Gln Gln
305                 310                 315                 320

Arg Ala Leu Ala Thr Trp Pro Thr Leu Gln Ile Ile His Gln Pro Glu
                325                 330                 335

Arg Pro Thr Leu Gly Arg Phe Ile Arg Val Asn Asp Ala Leu Ala Val
            340                 345                 350

Lys Ile Thr Asp Gly Arg Thr Ala Asn Leu Leu Ala Glu Leu Thr Asp
        355                 360                 365

Leu Asp Thr Phe Val Ile Asp Ala Ala Leu Asn Tyr Ser Asp Thr Thr
    370                 375                 380

Tyr Leu Val Ala Ala His Asn Gln Asp Ala Ala Glu Ala Asn Lys Ala
385                 390                 395                 400

Leu Phe Leu Ser Leu Leu Gln Thr Leu Ile Pro Gln Val Glu Phe Ile
                405                 410                 415

Lys Asp Ser Pro Gly Leu Ile Val Ala Arg Val Leu Ser Ser Leu Ile
            420                 425                 430
```

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Thr Gln Leu Gly Gln Lys Asn Val Lys Ser Thr Leu Asp
465 470 475 480

Asn Met Ala Gln Leu Leu His Ser Ala Arg Tyr Tyr Pro His Tyr Ser
485 490 495

Leu Leu Asn Ile Pro Arg Pro Glu Leu Ala Val Ala Pro
500 505

<210> 86
<211> 509
<212> PRT
<213> Serratia plymuthica A153

<400> 86

Met Ala Glu Asn Asn Ser Ala Ile Arg Ser Ala Ala Val Ile Gly Ala
1 5 10 15

Gly Thr Met Gly Arg Gly Ile Ala Tyr Leu Leu Ala Leu Asn Gly Ile
20 25 30

Arg Thr Val Leu Tyr Asn Arg Asn Gly Asn Asn Leu Asn Gln Ala Arg
35 40 45

Asp Tyr Ile Val Ser Asp Leu Asp Arg Lys Ile Asp Asn Gly Lys Ile
50 55 60

Thr Leu Gln Lys Lys Gly Gln Ile Leu Ala Asn Ile Ile Phe Ser Asp
65 70 75 80

Val Phe Asp Ala Ile Thr Asp Ser Asp Leu Val Ile Glu Thr Ile Ala
85 90 95

Glu Asp Glu Gln Thr Lys His Glu Ile Leu Ala Ala Ile Ala Ala Thr
100 105 110

Val Lys Pro Glu Ala Ile Ile Ala Thr Asn Thr Ser Ser Leu Ser Leu
115 120 125

Asn Lys Leu Ala Ala Gly Val Glu Asn Asn Pro Arg Phe Ile Gly Leu
130 135 140

His Phe Phe Asn Pro Ala Pro Leu Met Lys Leu Ile Glu Ile Ile Pro
145 150 155 160

Ser Tyr Phe Thr Ser Arg Ala Thr Ser Leu Arg Cys Gln Gln Leu Val
165 170 175

Thr Ala Leu Gly Lys Leu Phe Val Val Cys Lys Ala Thr Pro Gly Phe
180 185 190

Ile Val Asn Arg Met Ala Arg Pro Phe Tyr Leu Glu Gly Phe Arg Leu
195 200 205

Leu Glu Glu Asn Val Ala Leu Ala Pro Gln Ile Asp Arg Ala Leu Lys
210 215 220

Ala Gly Gly His Phe Arg Met Gly Pro Leu Glu Leu Thr Asp Phe Ile
225 230 235 240

Gly Gln Asp Ile Asn Tyr Gln Val Ser Lys Gln Ile Trp Gln Asp Met
245 250 255

Gln Phe Asp Pro Arg Tyr Thr Pro Gly His Leu Gln Arg Ser Leu Val
260 265 270

Asp Ala Gly Leu Leu Gly Arg Lys Asn Gly Arg Ser Phe Phe Ala Ser
275 280 285

Gln Pro Ala Thr Pro Pro Asn Pro Thr Thr Glu Gly His Thr Pro Thr
290 295 300

Ser Leu Leu Phe Tyr Gly Glu His Ala Leu Phe Asp His Leu Gln Gln
305 310 315 320

Arg Ala Leu Ala Thr Trp Pro Ala Leu Arg Val Gln Arg Leu Pro Glu
325 330 335

Arg Pro Glu Leu Gly Arg Phe Ile Leu Val Asn Asn Arg Leu Ala Ile
340 345 350

Lys Ile Thr Asp Gly Arg Thr Ala Asn Leu Leu Ala Gly Leu Thr Ala
355 360 365

Leu Asp Thr Phe Val Ile Asp Ala Ala Leu Asn Tyr Ala Asp Thr Ala
370 375 380

Tyr Leu Val Ala Ala His Asn Gln His Ala Thr Glu Pro Asn Lys Ala

385 390 395 400

Leu Phe Leu Thr Leu Leu Gln Thr Leu Ile Ala Gln Val Glu Phe Ile
405 410 415

Lys Asp Ser Pro Ala Leu Ile Val Ala Arg Val Leu Ser Ser Leu Ile
420 425 430

Asn Glu Ser Val Ile Met Val Glu Ser Gly Val Cys Ser Arg Ala Asp
435 440 445

Ile Asp Ile Ala Ala Val Ala Gly Val Asn Tyr Ala Asp Gly Ile Phe
450 455 460

Ala Trp Leu Ala Gln Leu Gly Gln Lys Asn Val Lys Ser Thr Leu Asp
465 470 475 480

Asn Met Ala Gln Leu Leu His Ser Thr Arg Tyr Tyr Pro His Tyr Ser
485 490 495

Leu Leu Asn Ala Ala Arg Pro Glu Leu Ala Val Ala Pro
500 505

<210> 87
<211> 1530
<212> DNA
<213> Serratia marcescens ATCC13880

<400> 87
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcggtgctgg ccaatctaat gttcacttca 240

gtgtttgagg ccatcgccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggctactg cggtgacgca cagcgaacgc 420

tttatcggtt tgcatttttt caaccccgcg ccgctgatga agctgattga aatcattccg 480

gcctacttta ccgcgcacgc caccaccgaa cgctgccgcc aactggtggc ggcgttgggg 540

aaacacgatg tcgtctgcca ggccacgccg ggtttcatcg tcaatcgcat ggcccgcccc 600

tactacctgg aagggttccg cctgttggaa gaacacgtgg cgcgcgcggc tcagatcgac 660

cgcgccctca aggccggcgg gcgcttccgc atggggccgc tcgagctgac cgattttatc 720

ggccaagaca tcaactatca ggtcagtcgg caaatctggc aggatatgca atacgacccg 780

cgctataccc ccggtcatct gcagcgttca ctggtcgatg ccggtctgtt ggggaaaaag 840

aacggccgct cctattttgc cgccgaagaa accgccccgc cggtgacggc cgccagcaat 900

gcagacgtcg agacgctgcg cgtttacggc gagcaccctt tttttaccct gttacagcag 960

cgagccgcgc ttcagtggcc acagctgcgc gtggaacaac ggccggcatt accggggctg 1020

gggtcggccg tccagatcaa tgacgctttc accgtcagca tcaccgatgg ccgcacggcg 1080

agccaactgg ccgagcagac ggcagcggat gcctttgtgg tcgatgtcgc cctgaactac 1140

gccgacacga cgtatctggc ggcggcgcac agccgccacg cctctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg ggggagaaaa acgtcaggac aacgctgagc 1440

aatctggctc agctgctgca cgcggcgcgc tatgcgccgc attacaccct tctgcacgcc 1500

gcgcaaccgg cgctgacgac cacgccttaa 1530

<210> 88
<211> 1530
<212> DNA
<213> Serratia nematodiphila DSM21420

<400> 88
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgcg ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcagtggtca agcccgacac gctgatcgcc 360

accaatacct cctcactgtc gctcaacaag ctggcaacgg cggtaacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aagccggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatctcgc cctgaactac 1140

gccgacacca cgtatctggt ggcggcgcac agccgccacg cttctgcggc caataaggcg 1200

ctgtttttac gcctgctgca cacggcaatc cctcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 89
<211> 1530
<212> DNA
<213> Serratia plymuthica NBRC102599

<400> 89
atggcagaga ataattcggc aatccgttca gccgccgtta ttggtgcggg gaccatgggc 60

agaggcatcg cctatctcct ggcgctgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggac 240

gtctttgacg ccataaccga cagcgatctg gtgattgaaa ccattgcgga agatgagcaa 300

accaagcatg aaatcctggc agccattgcg gcgacggtaa aaccggaggc gatcattgcg 360

accaatactt cctctctgtc gctgaacaaa ctggcggcgg gggtggaaaa caacccgcgc 420

tttatcggcc tgcatttttt caatccggcg ccgctgatga agttgatcga aattattccc 480

tcttatttca cctctcgcgc caccagtcta cgctgccagc agttggtaac agcgttgggt 540

aaacagtttg tggtctgcaa agccacgccg ggctttattg ttaaccgcat ggcgcggcct 600

ttctatctgg aagggttccg gctgctggag gaaaacgtgg cgctggcgcc acagatcgac 660

cgcgccctca aggccggcgg gcattttcgc atggggcctt tagaactgac tgattttatc 720

ggccaggata tcaactatca ggtcagcaag cagatttggc aggatatgca gttcgatccc 780

cgctataccc ccggccattt gcaacgctcg ctggtggatg ccgggctgct ggggaggaaa 840

aacgggcgct ctttttttgc ttcccaacct gctacaccac ccaacccaac cacagagggc 900

gacacgccaa cttcactgca tttttatggt gaacacgctt tattcgatca cctgcaacag 960

cgcgctttgg ccacctggcc tgcgctgcgc gttcagcggt tgccggaacg gccggaactg 1020

gggcgtttta tcctggtgaa taacaggctg gcgatcaaaa tcactgatgg cagaacggcg 1080

aacctgctcg ccggcttaac cgctctcgac accttcgtga ttgacgccgc gctgaactac 1140

gccgacaccg cctatctggt ggccgcccac aatcaacatg ccacagagac gaataaagcg 1200

ctgtttctga cgctgctgca aaccctcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aattgctgca ctccacgcgc tattacccgc attactcatt gctgaacgcg 1500

gcccggcctg agctggctgt agcgccctaa 1530

<210> 90
<211> 1530
<212> DNA
<213> Serratia proteamaculans 568

<400> 90
atggcagaga ataattcggc aatccattcg gtcgctgtta ttggtgccgg gaccatgggc 60

agaggtattg cctatcttct ggcgcaaaac ggcatacgaa ccctgcttta taatcgtagc 120

ggtaataatc tgaatcaggc cagggactat attattcgtg acctggataa gaaaatagat 180

ggcggaaaaa taagcccgca gaaaaaaggc gagatattgg ccaatctggt tttctctccc 240

attttcgagg ctattgccga cagcgatctg gtgattgaaa ccatcgcgga acatgaggca 300

accaagcatg agatcctcgc ggcgattgcg gccacggtga aaaaagaggc gattatcgcc 360

accaatacct cctcgctgtc attgaataag ctggccgcag gcgtcgaaaa taacgcccgg 420

tttatcggcc tgcacttctt caatccggcc ccgctgatga aactgatcga aattattccg 480

tcttacttta ccagccgggc caccagcctg cgttgccagc agttggtgac ggcgataggc 540

aaacagtttg tggtctgcaa agccacgccg ggctttatcg tcaaccggat ggcgcgacct 600

ttttatctgg aagggttccg gctgttggaa gagaacgtgg cgctggcgcc gcagatcgac 660

cgcgcactca aggccggtgg ccactttcgc atgggacctt tagagctgac cgattttatc 720

ggccaggata ttaactatca ggtcagcagc cagatttggc aggacatgca gtacgatccc 780

cgctataccc ccggccattt gcaacgttcg ctggtggatg ccgggctgct ggggaagaaa 840

aacggccgat cctttttttac cccctcttcc gccgaaccca gctccgccga tgcaggtagc 900

ggcacgccga cctcactgaa tttttatggt gaacatcccc tgttcgacct gttgcaacag 960

cgcgctttgg cgctctggcc aagggtgcag attaatcgcc aatcggaaca gccaacgctg 1020

ggccgcttta tccgggtgaa tgacgcaatg gccatcaaaa tcaccgatgg ccgcaccgcc 1080

aatctgctgg ctgaattgac cgaactcgat accttcgtga tcgacgccgc gctcaactac 1140

gccgataccg cctatctggc ggctgcccac agccaggatg ccagcgcggc caataaagcg 1200

ctgtttctga cgctgctgca aacgttgatc ccgcaggtgg agtttattaa agactccccg 1260

ggcctgattg tcgcccgcgt cctgagcagc ctgatcaatg agtcggtgat tatggtggag 1320

agcggggttt gcagccgggc ggacatcgat atcgccgcgg tggccggcgt taactatgcc 1380

gatggcatct ttagctggct ggcgcagctt gggcaaaaaa acgtgaagtc gacgctggac 1440

aatatggcgc aactgctgca ttccgcccgc tattacccgc attactcttt gctcaatacc 1500

ccccggccag agctggccgt cgcgccctaa 1530

<210> 91
<211> 1530
<212> DNA
<213> Serratia ureilytica Lr5/4

<400> 91
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgaactgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcctccgc cgcttgcggc cgccgtcgat 900

gcggaggtcg agacgctacg catttacggt gaacatcctc tctttactct gctacagcag 960

cgggccaccc tgcaatggcc gcggctgcgc gtggaacaac ggccgacatt accgggcctg 1020

ggcgccgcca ttcaggttaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcagc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 92
<211> 1530
<212> DNA
<213> Serratia sp. BW106

<400> 92
atggcagaga ataattcggc aatccattcg gtcgctgtta ttggtgccgg aaccatgggc 60

agaggtattg cctatcttct ggcgcagaac ggcatacgaa ccctgcttta taatcgtagc 120

ggtaataatc tggatcaggc ccgcgactat attatccgtg acctggataa gaaaatagac 180

aacgggaaaa taagccaaca gaaaaaaggc gaagtattgg ccaacctggt tttctctccc 240

attttcgacg ctatcgccga cagcgacctg gtgattgaaa ccatcgcgga gcatgagacc 300

accaagcatg aaatcctcgc ggcgattgcg gccacggtga aacaagaggc cattatcgcc 360

accaacacct catcgctgtc gttgaataag ctggcggcag gcgtcgaaaa caacgcccgt 420

tttatcggcc tgcacttctt caatccggcc ccgctgatga aactgatcga gattattccg 480

tcctatttta ccagccgggc caccagcctg cgctgccagc agttggtgac ggcgctaggc 540

aaacagtttg tggtctgcaa agccacgccg ggttttatcg tcaaccggat ggcacggcct 600

ttttatctgg aaggattccg gctgttggaa gaaaacgtag cattggcacc gcagatcgac 660

cgcgccctca aggccggtgg ccactttcgc atgggccctt tagagctgac cgactttatc 720

ggccaggata ttaactatca ggtcagcagc cagatttggc aggacatgca gtacgaccct 780

cgctataccc ccggccattt gcaacgttcg ctggtggatg ccgggttgtt ggggaagaaa 840

aacggccgat cctttttgc tgccccttct tccgaatcga accccctcga cgcaggcaac 900

ggcacgctga cttccctgca tttttatggc gaacataccc tgtttgacct gctgcaacag 960

cgcgccttgg ctatctggcc aacgctgcag attattcacc agccggaacg gccgacgctg 1020

ggacgcttta tccgggtgaa tgacgcattg gccgtcaaaa tcaccgatgg tcgcaccgcc 1080

aatctgctcg ctgaattgac cgatctcgac acctttgtga tcgacgccgc actgaattac 1140

agcgataccg cctatctggt ggccgcccac aatcaggacg ccgccgaggc caataaagcg 1200

ctgtttctgt cgctgctgca aacgttgatc ccgcaggtgg agtttattaa agactctcca 1260

ggcctgatcg tcgcccgggt tctgagcagt ctgatcaatg agtcggtgat catggtggag 1320

agcggggttt gcagccgggc agatatcgat attgccgccg tggcgggcgt taactatgcc 1380

gatggcatct ttgcctggct gacgcagctc gggcaaaaaa acgtgaaatc aacgctggat 1440

aatatggcgc aactgctgca ttccgcccgc tattacccgc attactcatt gctgaatgcc 1500

ccccggcccg aactggccgt cgcgccgtaa 1530

<210> 93
<211> 1530
<212> DNA
<213> Serratia liquefaciens FK01

<400> 93
atggcagaga ataatacggc aatagattcg gtcgccgtga ttggcgccgg aaccatgggc 60

agaggcattg cctatctttt ggcactgaac ggcatacgaa ccctgcttta taatcgaaac 120

ggtaatcatc ttaaccaggc ccgggattat attgtcggcg acctggataa aaaaatcgac 180

aacggaaaaa taagccaaca gaaaaagggc gaggtcctgg ccaatctggt tttctcgcca 240

gttttcgacg ccattgccga cagtgacctg gtgattgaaa ccatagcgga acatgaaccc 300

accaagcatg agatcctcgc ggccattgcc gccacggtaa aaaaagaggc gattatcgcc 360

accaatacct cttcgctgtc gctgaataaa ctggccgcag ggatagaaaa caactcgcgc 420

tttatcggcc tgcatttctt caacccggca ccgctgatga agctgatcga aattatcccg 480

tcctatttta ccgcccgggc caccacctta cgctgccagc agttggtcac ggcgataggc 540

aaaaaatttg tggtctgcaa agccacgccg ggctttatcg tcaatcgcat ggcgcggcct 600

ttttatctgg aaggttttcg cctgctggag gaaaacgtgg cgctggcacc gcaaatcgac 660

cgcgcgctca aggccggagg ccacttccgc atggggcctt tggaactgac tgattttatt 720

ggtcaggaca ttaattatca ggtcagcagc cagatttggc aggacatgca gtatgacccc 780

cgctataccc ccggccattt gcaacgttcg ctggtggatg ccgggctgtt ggggaagaaa 840

aacgggcgct cttttttttc cccctcggct gacgccgcca acccaccggc taccggcggc 900

ggtacgctga gctcgctgca tttttttggc gaacatccgc tgtttgatct gctgcaacag 960

cacgccttcg caacctgggc gcccctggca ataacacgtc agccggaaca cccggttctg 1020

ggccgtttta tccaggtgaa tgacagtctg gcagtcaaaa tcaccgacgg gcgaacggcc 1080

aatcagcttg ccgagctggc gggtctcgat accttcgtgg tcgacgttgc actgaactat 1140

gccaacaccg cttttctggt ggcggcccac agccaacagg ctaccgaggc gaataaagag 1200

ctgttcctca cgctgctgca aacggtgatc ccgcaggtgg agtttgttaa agattcccca 1260

ggcctgatcg tcgcccgggt tctgagcagc ctgatcaatg agtcggtgat catggtggag 1320

agcggggttt gtagccgaga agacatcgat atcgccgccg tggccggcgt caactatgcc 1380

gacggcattt ttgcctggct ggcgcagctc gggcagaaaa acgtgaaatc gacgctggat 1440

aatatggcgc aactgctgca ttccgcccgc tattacccgc actactcgtt gctccacgct 1500

accaggcccg agctggccgt cgcgccatga 1530

<210> 94
<211> 1530
<212> DNA
<213> Serratia sp. S119

<400> 94
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ctaatctggt gttcacctcg 240

gtgtttgaga ctatcgccga cagcgatcta gtgatagaaa ccatcgccga gcaagagcaa 300

accaaacttg aggtgctggc ggccatcgcc gcggcggtca agcccgacac gctgatcgcc 360

accaatacat cctcactgtc gcttaacaag ctggcaaccg cggtgacaca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcaat gtcgccgccg tcgccggcgt taactacgcc 1380

gacggcattt tcggcttgct cgatagcctg gggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 95

<211> 1530
<212> DNA
<213> Serratia sp. YD25

<400> 95
atggcagaac gtaatgcggc aattcaatcg gctgcgatta tcggcgcggg aacgatgggc 60

agaggcatcg cttatctttt cgcgcaaaaa ggcattcgta cggtgcttta taatcgcaac 120

ggcaataccc tcattcaggc tcgtgaatat atcgtgcaag acctggacaa aaaggtcgaa 180

cagggcaggc tcgcgccgca ggataaagac gcggtgctgg ccaatctgca gttctcatcg 240

gtgtttgaag ctatcgtcga cagcgatttg gtgctcgaaa ctatcgccga gcaagagcaa 300

gccaaactcg aggtgctggc cgccatcgct gcggcggtca aacccgacac gctgatcgcc 360

accaatactt cctcattgtc gctcaataag ctggcaacgg cggtgaccca cagcgaacgc 420

tttatcggtt tgcacttttt caatcccgca ccgctaatga agctgattga aatcattccg 480

gcctacttta ccacccaagc caccactgaa cgttgccgcc aactggtggc ggcattgggg 540

aaacgcgatg tcgtctgcca ggctacgccg gggttcatcg tcaaccgtat ggcccgcccc 600

tactacctgg aaggctttcg cctgttggaa gagcacgtgg cgcgcgcgcc gcagatcgac 660

cgcgccctca aggccggcgg acactttcgc atggggccgc tcgagctgac cgattttatc 720

ggccaggaca tcaactatca ggtcagccgg caaatttggc aggacatgca gtacgacccg 780

cgctataccc ccggccatct gcagcgttcg ctggtcgatg ccgggctgct ggggaaaaag 840

aacggtcgct cctatttttc cgccgaagaa accgccccgc cggttgaggc cgccgccgag 900

gcggatgtcg agacgctgcg catttacggc gaacaccctt tgtttaccct gctgcaacag 960

cgagccgcac tgcaatggcc gcagttgcgc gtggaacagc ggccggcctt gtcgggcctg 1020

ggagcggcca ttcaggtcaa tgacgctttc accgtcagcg tcaccgatgg ccgcacggca 1080

aatcagttgg ccgagcagac ggcggcggac gcctttgtgg tcgatgtcgc cctgaactac 1140

gccgacacgg cgtatctggt ggcggcgcac agccgccacg cctctgcggc taataaggcg 1200

ttgtttttgc gcctgctgca caccgcgctt ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt gaactacgcc 1380

gacggcattt tcggctggct cactcgcctg ggggagaaaa acgtcaggac gacgctgagc 1440

aacctggcgc agctgctgca cgcggcccgc tatgcgccgc attacaccct tctgcacgcc 1500

gcccaaccgg cgctgaccac cacgccttaa 1530

<210> 96
<211> 1530
<212> DNA
<213> Serratia sp. FS14

<400> 96
atggcagaaa gtaatgcggc aattcaatcg gctgcgatta tcggcgcggg aacgatgggc 60
agaggcatcg cttatctttt cgcgcaaaaa ggcattccca cgatgcttta taatcgcaac 120
ggcaataccc tcaatcaagc tcgcgaatat atcgcgcaag acctgaacaa gaaagtcgaa 180
cagggcaaga tcgcgttgca ggataaagac gcggtgctgg ccaatctgat gttcacttcc 240
gtgtttgagg ccatcgccga cagcgagctg gtgatagaaa ccatcgccga gcaagaacaa 300
accaaacttg aggtgctggc ggccatcgcc gcggtggtca agcccgacac gctgatcgcc 360
accaatacct cctcactgtc gcttaacaag ctggcaactg cggtgacgca cagcgagcgc 420
tttatcggtt tacatttttt caaccccgct ccgctgatga agctgattga aatcattccg 480
gcctacttta ccgcgcacgc caccacggaa cgctgccgcc aactggtggc ggcgttgggg 540
aaacgcgatg tcgtctgcca ggccacgccg gggttcatcg tcaatcgcat ggcccgcccc 600
tactacctgg aagggttccg cctgttggaa gaacatgtgg cgcgcgcggc gcagatcgac 660
cgcgccctca aggccggcgg gcgcttccgc atggggccgc tcgagctgac cgattttatc 720
ggccaagaca ttaactatca ggtcagccgg caaatctggc aggacatgca atacgacccg 780
cgctataccc ctggtcatct gcagcgttca ctggtcgatg ccggtctgtt ggggaaaaag 840
aacggccgct cctattttgc cgccgaagaa accgccccgc cggtgacggc cgccaacaat 900
gcagacgtcg agacgctgcg cgtttacggc gagcatcctt tttttaccct gttgcagcag 960
cgagccgcgc ttcagtggcc acagctgcgc gtggaacaac ggccggcatt accggggctg 1020
ggagcggccg tccagatcaa tgacgctttc accgtcagca tcaccgatgg ccgcacggcg 1080
agccaactgg ccgagcagac ggcagcggat gcctttgtgg tcgatgtcgc cctgaactac 1140
gccgacacca cgtacctggt ggcggcgcac agtcgccacg cttctgcggc caataaggcg 1200
ctgtttttac gcctgctgca cacggcaatc ccacaggttg aatttatcaa ggactctccg 1260
gcgcttatcg tcgcccgcgt cctcagcagc ctgatcaatg agtcggtgat catggtggaa 1320
agcggcgtct gcagccggga agacatcgat gtcgccgccg tcgcgggcgt taactacgcc 1380
ggcggcattt tcgactggct cggcaaactg gggagaaaa acgtcaggac gacgctgagc 1440
aatctggcac agctgctgca cgcggcgcgc tatgcgccgc attacaccct tctgcacgcc 1500
gcgcaaccgg cgctgacgac cacgccttaa 1530

<210> 97
<211> 1530
<212> DNA
<213> Serratia sp. HMSC15F11

<400> 97
atggcagaaa gtaatgcggc aattcagtcg gctgcgatta tcggcgcggg aacgatgggc 60
agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggtaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc ggccatcgcc gcggcagtca agcccgacac gctgatcgcc 360

accaatacct cctcactgtc gctgaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtggc cgcgttgggg 540

aaacgcgatg ttgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggcgg gcactttcgt atggggccgc tcgagctgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtttacggc gaacaccctt tttttaccct gttacaacaa 960

cgtgccgcgc ttcagtggcc acagctgcgc gtggaacaac ggccggcatt accgggcctg 1020

gggccggccg tccggatcaa tgacgctttc accgtcagca tcaccgatgg ccgcacggca 1080

aaccagctgg ccgagcagac ggcggcggat gcctttgtgg tcgatgtcgc cctgaattac 1140

gccgacacgg cgtatctggt ggcagcgcac agccgccacg cctctgcagc caataaagcg 1200

ctgttcttac gcctgctgca cacggcaatc ccgcaggttg aatttatcaa ggattccccg 1260

gcgctgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct agatagcctg gggggagaaaa acgtcaggac gacgctgagc 1440

aacctggcgc agctgctgca cgcagcacgc tatgcgccgc attacaccct tctgcacgcc 1500

gcgcaaccgg cgctgacgac cacgccttaa 1530

<210> 98
<211> 1530
<212> DNA
<213> Serratia sp. JKS000199

<400> 98
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccacg 240

gaatttgggg ccatcgccga cagcgatctg gtgatcgaaa ccatcgcaga gcatgagcaa 300

gccaaactcg aagtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttaggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaagtcg agacgctacg catttacggt gaacatcctc tctttaccct gctacagcag 960

cgggccgccc tgcaatggcc gcggctgcgc gtggaacaac ggccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcggc caataaggcg 1200

ctgtttttgc gcctgctgca caccgcgatc ccgctggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 99
<211> 1530
<212> DNA
<213> Serratia sp. TEL

<400> 99
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgaactgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcctccgc cgcttgcggc cgccgtcgat 900

gcggaggtcg agacgctacg catttacggt gaacatcctc tctttactct gctacagcag 960

cgggccaccc tgcaatggcc gcggctgcgc gtggaacaac ggccgacatt accgggcctg 1020

ggcgccgcca ttcaggttaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcagc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcagggc gacgctgagc 1440

aacctggcgc aattgctgca cgcggcgcgc tatgcgccgc attacaccct tctgcacgcc 1500

gcccaaccgg cgctgaccac cacgccttaa 1530

<210> 100
<211> 1530
<212> DNA
<213> Serratia sp. ISTD04

<400> 100
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgca 240

gaatttgggg tcatcgccga cagcgatctg gtgatcgaaa ccatcgcaga acatgagcaa 300

gccaaactcg aggtgctggc ggccatcgcc gcgacggtca agtccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttaggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaagtcg agacgctacg catttacggt gaacatcctc tctttactct gctacagcag 960

cgggccgccc tgcaatggcc gcggctgcgc gtggaacaac ggccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcacat agccgccatg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 101
<211> 1530
<212> DNA
<213> Serratia sp. SCBI

<400> 101
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt taccaccgaa cgttgccgtc aactggtggc ggcgttgggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc caccgaagaa tctccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggcattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 102
<211> 1530
<212> DNA
<213> Serratia sp. S4

<400> 102
atggcagaga ataattcggc aatccattcg gtcgctgtta ttggtgccgg gaccatgggc 60

agaggtattg cctatcttct ggcgcaaaac ggcatacgaa ccctgcttta taatcgtagc 120

ggtaataatc tgaatcaggc cagggactat attatccgtg acctggataa gaaaatagat 180

ggcggtaaaa taagcctgca gaagaaaggc gagatattgg ccaatctggt tttctctcct 240

attttcgagg ctattgccga cagcgacctg gtgattgaaa ccatcgcgga acatgagtca 300

accaagcatg agatcctctc ggcgattgcg gccacggtga aaaaagaggc gattatcgcc 360

accaatacct catcgctgtc attgaataag ctggcggcag gcgtcgaaaa taacgcccgt 420

tttatcggcc tgcacttctt caatccggcc ccgctgatga aactgatcga aattattccg 480

tcttacttta ccagccgggc caccagcctg cgttgccagc agttggtgac ggtgataggc 540

aaacagtttg tggtctgcaa agccacgccg ggctttatcg tcaaccggat ggcgcgacct 600

ttttatctgg aagggtttcg gctgttggaa gagaacgtgg cgctggcgcc gcagatcgac 660

cgcgcactca aggccggtgg ccacttccgc atgggacctt tagagctgac cgattttatc 720

ggccaggata ttaactatca ggtcagcagc cagatttggc aggacatgca gtacgacccc 780

cgctataccc ccggtcattt gcaacgttcg ctggtggatg ccgggctgct ggggaagaaa 840

aacggccgat cctttttac cccctcttcc gccacaccca gctccgccga cgcaggcagc 900

ggcacgccga cctcactgaa tttttatggt gaacatcccc tgttcgacct gttgcaacag 960

cgcgctttgg cgctctggcc aaggttgcag attaatcgcc aatcggaaca gccaacgctg 1020

ggccgcttta tccgggtgaa tgacgcaatg gccatcaaaa ttaccgatgg ccgcaccgcc 1080

aatctgctgg ctgaattgac cgaactcgat accttcgtga tcgacgccgc gctcaactac 1140

gccgataccg cctatctggc ggctgcccac agccaggatg ccagcacggc caataaagcg 1200

ctgtttctga cgctgctgca aacgttgatc ccacaggtgg agtttattaa agactccccg 1260

ggcctgattg tcgcccgcgt cctgagcagc ctgatcaatg agtcggtgat tatggtggag 1320

agcggggttt gcagccgggc ggacatcgat atcgccgcgg tggccggcgt taactatgcc 1380

gatggcatct ttagctggct ggcgcagctt gggcaaaaaa acgtgaagtc gacgctggac 1440

aatatggctc aactgctgca ttccgcccgc tattacccgc attactcttt gctcaatacc 1500

ccccggccag agctggccgt cgcgccctaa 1530

<210> 103
<211> 1530
<212> DNA
<213> Serratia sp. C-1

<400> 103
atggcagaga ataattcggc aatccgttca gccgccgtta ttggtgcggg gaccatgggc 60

agaggcatcg cctacctcct ggcactgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcagcag ggtggaaaa caacccgcgc 420

tttatcggcc tgcatttttt caatccggcg ccgctgatga agttgatcga aatcattccc 480

tcttatttta cctcccacgc caccagccta cgctgccaga agttggtaat agcattgggt 540

aaacagtttg tggtctgcaa agccacgccg ggctttattg ttaatcgcat ggcgcggcct 600

ttctatctgg aagggttccg gctgctggag gaaaacgtgg cgctggcgcc acagatcgac 660

cgcgccctca aggccggcgg gcattttcgc atgggccctt tagaactgac ggattttatc 720

ggtcaggata tcaactacca ggtcagcaag cagatttggc aggatatgca gttcgaccct 780

cgctataccc ctggtcattt gcaacgctcg ctggtggatg ccgggctgct ggggaggaaa 840

aacgggcgct ctttttttgc ttcccaaccg gcgacgccgc ccaccccgac cacagagagc 900

gacacgccaa cgtcactgca tttttatggg gagcatgctt tattcgatca tctgcaacag 960

cgtgctctgg ccgcctggcc tgcgctgcgc gttcagcggt tgccggaacg gcctgaactg 1020

gggcgattta tcctggtgaa taacgcgctg gcgatcaaaa tcaccgatgg cagaacggca 1080

aacctgctcg ccggcttaac cgctctcgac accttcgtga ttgacgctgc gctgaattac 1140

gccgacaccg cctatctggt ggcagcccac aatcaacatg ccacagagac gaataaagcg 1200

ctgtttctga cgctgctgca aaccgtcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aactgctgca ctccgcgcgc tattacccgc attactcttt gctgaacgcg 1500

gcccggcctg agctggccgt agcgccctaa 1530

<210> 104
<211> 1530
<212> DNA
<213> Serratia marcescens 532

<400> 104
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcggtgctgg ccaatctgat gttcacttca 240

gtgtttgagg ccatcaccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggctactg cggtgacgca cagcgaacgc 420

tttatcggtt tgcatttttt caaccccgcg ccgctgatga agctgattga aatcattccg 480

gcctacttta ccgcgcacgc caccaccgaa cgctgccgcc aactggtggc ggcgttgggg 540

aaacacgatg tcgtctgcca ggccacgccg gggttcatcg tcaatcgcat ggcccgcccc 600

tactacctgg aagggttccg tctgttggaa gaacacgtgg cgcgcgcggc gcagatcgac 660

cgcgctctca aggccggcgg gcgcttccgc atggggccgc tcgagctgac cgattttatc 720

ggccaagaca tcaactatca ggtcagtcgg caaatctggc aggacatgca atacgacccg 780

cgctataccc ccggtcatct gcagcgttca ctggtcgatg ccggtctgtt ggggaaaaag 840

aacggccgct cctattttgc cgccgaagaa accgccccgc cggtgacggc cgccagcaat 900

gcagacgtcg agacgctgcg cgtttacggc gagcaccctt tttttaccct gttacagcag 960

cgagccgcgc ttcagtggcc acagctgcgc gtggaacaac ggccggcatt accggggctg 1020

gggtcggccg tccagatcaa tgacgctttc accgtcagca tcaccgatgg ccgcacggcg 1080

agccaactgg ccgagcagac ggcagcagat gcctttgtgg tcgatgtcgc cctgaactac 1140

gccgacacga cgtatctggc ggcggcgcac agccgccacg cctctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggagaaaa acgtcaggac aacactgagc 1440

aatctggctc agctgctgca cgcggcgcgc tatgcgccgc attacaccct tctgcacgcc 1500

gcgcaaccgg cgctgacgac cacgccttaa 1530

<210> 105
<211> 1530
<212> DNA
<213> Serratia marcescens 2880STDY5683033

<400> 105
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcacg acctgaacaa gaaagtcgaa 180

caaggcaaga tcgcgctgca ggataaaggc gcggtgctgg ccaatctaat gttcacttca 240

gtgtttgagg ccatcgccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggctactg cggtgacgca cagcgaacgc 420

tttatcggtt tgcatttttt caaccccgcg ccgctgatga agctgattga aatcattccg 480

gcctacttta ccgcgcacgc caccaccgaa cgctgccgcc aactggtagc ggcgttgggg 540

aaacacgatg tcgtctgcca ggccacgccg gggttcatcg tcaatcgcat ggcccgcccc 600

tactacctgg aagggttccg cctgttggaa gaacacgtgg cgcgcgcggc gcagatcgac 660

cgcgccctca aggccggcgg gcgcttccgc atggggccgc tcgagctgac cgattttatc 720

ggccaagaca tcaactatca ggtcagtcgg caaatctggc aggatatgca atacgacccg 780

cgctataccc ccggtcatct gcagcgttca ctggtcgatg ccggtctgtt ggggaaaaag 840

aacggccgct cctattttgc caccgaagaa accgccccgc cggtgacggc cgccagcaat 900

gcagacgtcg agacgctgcg cgtttacggc gagcaccctt tttttaccct gttacagcag 960

cgagccgcgc ttcagtggcc acagctgcgc gtggaacaac ggccggcatt accggggctg 1020

gggtcggccg tccagatcaa tgacgctttc accgtcagca tcaccgatgg ccgcacggcg 1080

agccaactgg ccgagcagac ggcagcggat gcctttgtgg tcgatgtcgc cctgaactac 1140

gccgacacga cgtatctggc ggcggcgcac agccgccacg cctctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggagaaaa acgtcaggac aacgctgagc 1440

aatctggctc agctgctgca cgcggcgcgc tatgcgccgc attacaccct tctgcacgcc 1500

gcgcaaccgg cgctgacgac cacgccttaa 1530

<210> 106
<211> 1530
<212> DNA
<213> Serratia marcescens WW4

<400> 106
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaacaa gaaagtcgaa 180

cagggcaaga tcgcgctgca ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcagtggtca agcccgacac gctgatcgcc 360

accaatacct cctcactgtc gcttaacaag ctggcaactg cggtgacgca cagcgagcgc 420

tttatcggtt tgcatttttt caaccccgcg ccgctgatga agctgattga aatcattccg 480

gcctacttta ccgcacacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca ttaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtga tcgatgtcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactccccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg ggggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 107
<211> 1530
<212> DNA
<213> Serratia marcescens K27

<400> 107
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgca ggataaagac gcggtgctgg ccaatctgat gttcacttcc 240

gtgtttgagg ccatcgccga cagcgagctg gtaatagaaa ccatcgccga gcaagaacaa 300

accaaacttg aggtgctggc ggccatcgcc gcggtggtca agcccgacac gctgatcgcc 360

accaatacct cctcactgtc gctcaacaag ctggcaactg cggtgacgca cagcgaacgc 420

tttatcggtt tacatttttt caaccccgcg ccgctgatga agctgattga aatcattccg 480

gcctacttta ccgcgcacgc caccacggaa cgctgccgcc aactggtggc ggcgttaggg 540

aaacgcgatg tcgtctgcca ggccacgccg gggttcatcg tcaatcgcat ggcccgcccc 600

tactacctgg aagggttccg cctgttggaa gaacatgtgg cgcgctcggc gcagatcgac 660

cgcgccctca aggccggcgg gcgcttccgc atggggccgc tcgagctgac cgattttatc 720

ggccaagaca ttaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatgtcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg ggggagagaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 108
<211> 1530
<212> DNA
<213> Serratia marcescens 280

<400> 108
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgca ggataaagac gcggtgctgg ccaatctgat gttcacttcc 240

gtgtttgagg ccatcgccga cagcgagctg gtaatagaaa ccatcgccga gcaagaacaa 300

accaaacttg aggtgctggc ggccatcgcc gcagtggtca agcccgacac gctgatcgcc 360

accaatacct cctcactgtc gcttaacaag ctggcaactg cggtaacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggctggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcaccctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatgtcgc cctgaactac 1140

gccgacacca cgtatctggt ggcggcgcac agccgccacg cttctgctgc caataaggcg 1200

ctgtttttac tcctgctgca cacggcaatc ccgcaggttg aatttatcaa ggactccccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaagctg gggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 109
<211> 1530
<212> DNA
<213> Serratia marcescens 19F

<400> 109
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaacaa gaaagtcgaa 180

cagggcaaga tcgcgctgca ggataaggac gcggtgctgg ccaatctgat gttcacttcc 240

gtgtttgagg ccatcgccga cagcgagctg gtaatagaaa ccatcgccga gcaagaacaa 300

accaaacttg aggtgctggc ggccatcgcc gcggtggtca agcacgacac gctgatcgcc 360

accaatacct cctcactgtc gcttaacaag ctggcaactg cggtgacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca ttaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatgtcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactccccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 110
<211> 1530
<212> DNA
<213> Serratia marcescens 1185

<400> 110
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaacaa gaaagtcgaa 180

cagggcaaga tcgcgctgcg ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggctactg cggtgacgca cagcgaacgc 420

tttatcggtt tgcatttttt caaccccgcg ccgctgatga agctgattga aatcattccg 480

gcctacttta ccgcgcacgc caccaccgaa cgctgccgcc aactggtggc ggcgttgggg 540

aaacacgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cttattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatctcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactctccg 1260

gcgcttatcg tggcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcggcgcgc tatgcgccgc attacaccct tctgcacgcc 1500

gcgcaatcgg cgctgacgac cacgccttaa 1530

<210> 111
<211> 1530

<212> DNA
<213> Serratia marcescens S2I7

<400> 111
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag atctgaataa gaaagtcgaa 180

cagggcaaga tcgcgctgca ggataaggac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggcaactg cggtgacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgtgc gtggaacaac ggccggcatt accggggctg 1020

ggagcggccg tccagatcaa tgacgctttc accgtcagca tcaccgatgg ccgcacggcg 1080

agccaactgg ccgagcagac agcagcggat gcctttgtgg tcgatgtcgc cctgaactac 1140

gccgacacca cgtatctggt ggcagcgcac agccgccacg cttctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggggagagaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 112
<211> 1530
<212> DNA
<213> Serratia marcescens KHCo-24B

<400> 112

atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgcg ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcagtggtca agcccgacac gctgatcgcc 360

accaatacct cctcactgtc gctcaacaag ctggcaacgg cggtaacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggctggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcaggc ggcagcggat gcctttgtgg tcgatctcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 113
<211> 1530
<212> DNA
<213> Serratia marcescens Z6

<400> 113
atggcagaaa gtaatgcggc aattcaatcg gctgcgatta ttggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaacaa gaaagtcgaa 180

cagggcaaga tcgcgctgca ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcggtggtca agcccgacac gctgatcgcc 360

accaatacct cctcactgtc gcttaacaag ctggcaactg cggtgacgca cagagagcgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca ttaactatca ggtcagccgg caaatctggc aggacatgca atacgacccg 780

cgctataccc ctggtcattt gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcaccctt tttttaccct gttgcagcag 960

cgagccgcgc ttcagtggcc acagctgtgc gtggaacaac ggccggcatt accggggctg 1020

ggagcggccg tccagataaa tgacgctttc accgtcagca tcaccgatgg ccgcacggcg 1080

agccaactgg ccgagcagac ggcagcggat gcctttgtga tcgatgtcgc cctgaactac 1140

gccgacacca cgtatctggt ggcagcgcac agccgccacg cttctgcggc caataaggcg 1200

ctgtttttac gcctgctgca cacggcaatc ccgcaggttg aatttatcaa ggactccccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaagctg gggggagaaaa acgtcaggac gacgctgagc 1440

aatctggcgc agctgctgca cgcggcgcgc tatgcgccgc attacaccct cctgcacgcc 1500

gcgcaaccgg cgctgacgac cacgccttaa 1530

<210> 114
<211> 1530
<212> DNA
<213> Serratia marcescens 546

<400> 114
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc taaatcaagc tcgcgaatat atcgcgcaag acctgaacaa gaaagtcgaa 180

cagggcaaga tcgcgctgcg ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcagtggtca agcccgacac gctgatcgcc 360

accaatacct cctcactgtc gcttaacaag ctgtcaactg cggtaacgca caacgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcaccctt tttttaccct gttgcagcag 960

cgagccgcgc ttcagtggcc acagctgcgc gtggaacaac ggccggcatt accggggctg 1020

ggagcggccg tccggatcaa tgacgctttc accgtcagca tcaccgatgg ccgcacggcg 1080

agccaactgg ccgagcagac ggcagcggat gcctttgtgg tcgatgtcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 115
<211> 1530
<212> DNA
<213> Serratia nematodiphila MB307

<400> 115
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgcg ggataaagac gcggtgctgg ccaatctgat gttcacttcc 240

gtgtttgagg ccatcgccga cagcgagctg gtgatagaaa ccatcgccga gcaagaacaa 300

accaaacttg aggtgctggc ggccatcgcc gcagtggtca agcccgacac gctgatcgcc 360

accaatacct cctcactgtc gctcaacaag ctggcaacgg cggtaacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg ggtttcatcg tcaatcgcat ggcccgcccc 600

tactacctgg aagggttccg cctgttggaa gaacatgtgg cgcgcgcggc gcagatcgac 660

cgcgccctca aggctggcgg gcgcttccgc atggggccgc tcgagctgac cgattttatc 720

ggccaagaca tcaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc aggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtcagca tcaccgatgg ccgcacggcg 1080

agccaactgg ccgagcagac ggcagcggat gcctttgtgg tcgatctcgc cctgaactac 1140

gccgacacca cgtatctggt ggcggcgcac agccgccacg cttctgcggc caataaggcg 1200

ctgtttttac gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg ggggagaaaa acgtcaggac gacgctgagc 1440

aatctggcgc agctgctgca cgcggcgcgc tatgcgccgc attacaccct tctgcacgcc 1500

gcgcaaccgg cgctgacgac cacgccttaa 1530

<210> 116
<211> 1530
<212> DNA
<213> Serratia marcescens VGH107

<400> 116
atggcagaaa gtaatgcggc aattcaatcg gctgcgatta tcggcgcggg aacgatgggc 60

agaggcatcg cttatctttt cgcgcaaaaa ggcattccca cgatgcttta taatcgcaac 120

ggcaataccc tcaatcaagc tcgcgaatat atcgcgcaag acctgaataa gaaagtcgaa 180

cagggcaaga tcgcgctgca ggataaggac gcggtgctgg ccaatctgat gttcacttcc 240

gtgtttgagg ccatcgccga cagcgagctg gtaatagaaa ccatcgccga gcaagaacaa 300

accaaacttg aggtgctggc ggccatcgcc gcggtggtca agcacgacac gctgatcgcc 360

accaatacct cctcactgtc gctcaacaag ctggcaactg cggtgacgca cagcgaacgc 420

tttatcggtt tgcatttttt caaccccgcg ccgctgatga agctgattga aatcattccg 480

gcctacttta ccgcgcacgc caccacggaa cgctgccgcc aactggtggc ggcgttgggg 540

aaacgcgatg tcgtctgcca ggccacgccg gggttcatcg tcaatcgcat ggcccgcccc 600

tactacctgg aagggttccg cctgttggaa gaacatgtgg cgcgcgcggc gcagatcgac 660

cgcgccctca aggccggcgg gcgcttccgc atggggccgc tcgagctgac cgattttatt 720

ggccaagaca ttaactatca ggtcagccgg caaatctggc aggacatgca atacgacccg 780

cgctataccc ctggtcatct gcagcgttca ctggtcgatg ccggtctgtt ggggaaaaag 840

aacggccgct cctattttgc cgccgaagaa accgccccgc cggtgacggc cgccaacaat 900

gcagacgtcg agacgctgcg cgtttacggc gagcatcctt tttttaccct gttgcagcag 960

cgagccgcgc ttcagtggcc acagctgcgc gtggaacaac ggccggcatt accggggctg 1020

ggagcggccg tccagatcaa tgacgctttc accgtaagca tcaccgatgg ccgcacggcg 1080

agccaactgg ccgagcagac ggcagcggat gcctttgtgg tcgatgtcgc cctgaactac 1140

gccgacacca cgtatctggt ggcggcgcac agccgccacg cttctgcggc caataaggcg 1200

ctgtttttgc gcctgctgca cacggcaatc ccgcaggttg aatttatcaa ggactctccg 1260

gcgcttatcg tcgcccgcgt cctcagcagc ctgatcaatg agtcggtgat catggtggaa 1320

agcggcgtct gcagccggga agacatcgat gtcgccgccg tcgcgggcgt taactacgcc 1380

ggcggcattt tcgactggct cggcaaactg gggggagaaaa acgtcaggac gatgctgagc 1440

aatctggcgc agctgctgca cgcggcgcgc tatgcgccgc attacaacct tctgcacgcc 1500

gcgcaaccgg cgctgacgac cacgccttaa 1530

<210> 117
<211> 1530
<212> DNA
<213> Serratia marcescens MCB

<400> 117
atggcagaaa gtaatgcggc aattcaatcg gctgcgatta tcggcgcggg aacgatgggc 60

agaggcatcg cttatctttt cgcgcaaaag ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgagg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg ttaaccgcat ggcccgcccc 600

tactacctgg aagggttccg cctgttggaa gaacacgtgg cgcgcgcgcc gcagatcgac 660

cgtgccctca aggccggcgg acacttccgc atggggccgc tcgagctgac cgatttcatc 720

ggccaggaca tcaactatca ggtcagtcgg caaatctggc aggacatgca atacgacccg 780

cgctataccc ccggccatct gcagcgttcg ctggtcgatg ccggtctgtt ggggaaaaag 840

aacggccgtt cctattttac cgccgaagaa accgccccgc cggtgacggc tgccatcggt 900

gcagacgtcg agacgctgcg catttacggc gaacatcctt tttttaccct gttgcaacag 960

cgggccgcac ttcagtggcc acagctgcgc gtggaacaac ggccggcctt accaggcgtg 1020

gggccggccg tccagatcaa tgacgcattc accgtcagca tcaccgacgg ccgtacggcg 1080

agccagttag ccgagcagac gacggctgac gcttttgtgg tcgatatcgc cctgaactac 1140

gccgacacgg cgtacctggt tgcggcgcac aatcgccacg cctctgcggc caataaggcg 1200

ctgtttttac gcctgttgca caccgcaatt ccgcaggttg aatttatcaa agactccccg 1260

gcgctgatcg tcgctcgcgt cctcagcagc ctgatcaatg agtcggtgat catggtggaa 1320

agcggcgttt gcagccggga agacattgat gtcgccgccg tcgcgggcgt taactacgcc 1380

ggcggcattt tcgactggct cagcaagctg ggggagaaaa acgtcaggac gacgctgagc 1440

aatctggcgc agttgctgca cgcggcgcgt tatgcgccgc attacaccct tctgcacgcc 1500

gcgcaaccgg cgctgacgac cacgccttga 1530

<210> 118
<211> 1530
<212> DNA
<213> Serratia marcescens AH0650

<400> 118
atggcagaaa gtaatgcggc aattcaatcg gctgcgatta tcggcgcggg aacgatgggc 60

agaggcatcg cttatctttt cgcgcaaaaa ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgagg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg ttaaccgcat ggcccgcccc 600

tactacctgg aagggttccg cctgttggaa gaacacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

cgagccgcac gtcagtggcc acagctgcgc gtggaacaac ggccggcctt accgagcctg 1020

gggccggccg tccagatcaa tgacgcattc accgtcagca tcaccgatgg ccgcacggcg 1080

agccagttag ccgagcagac gacggctgac gcttttgtgg tcgacatcgc cctgaactac 1140

gccgacacgg cgtatctggt tgcggcgcac aatcgctatg cctctgcggc caataaggcg 1200

ctgtttttac gcctgctgca cactgcgatc ccgcaggttg aatttatcaa agactccccg 1260

gcgctgatcg tcgctcgtgt cctcagcagc ctgatcaatg agtcggtgat catggtggaa 1320

agcggcgttt gcagccggga agacattgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcagcgcgc tatgcgccgc attacaccct tctgcacgcc 1500

gcgcaaccgg cgctgacgac cacgccttga 1530

<210> 119
<211> 1530
<212> DNA
<213> Serratia marcescens UMH12

<400> 119
atggcagaaa gtaatgcgga aattcaatcg gctgcgatta tcggcgcggg aacgatgggc 60

agaggcatcg cttatctttt cgcgcaaaaa ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgagg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg ttaaccgcat ggcccgcccc 600

tactacctgg aagggttccg cctgttggaa gaacacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

cgagccgcac gtcagtggcc acagctgcgc gtggaacaac ggccggcctt accgagcctg 1020

gggccggccg tccagatcaa tgacgcattc accgtcagca tcaccgatgg ccgcacggcg 1080

agccagttag ccgagcagac gacggctgac gcttttgtgg tcgacatcgc cctgaactac 1140

gccgacacgg cgtatctggt tgcggcgcac aatcgctatg cctctgcggc caataaggcg 1200

ctgtttttac gcctgctgca cactgcgatc ccgcaggttg aatttatcaa agactccccg 1260

gcgctgatcg tcgctcgtgt cctcagcagc ctgatcaatg agtcggtgat catggtggaa 1320

agcggcgttt gcagccggga agacattgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcagcgcgc tatgcgccgc attacaccct tctgcacgcc 1500

gcgcaaccgg cgctgacgac cacgccttga 1530

<210> 120
<211> 1530
<212> DNA
<213> Serratia sp. OMLW3

<400> 120
atggcagaaa gtaatgcggc aattcaatcg gctgcgatta tcggcgcggg aacgatgggc 60

agaggcatcg cttatctttt cgcgcaaaaa ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgtgg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg tcaaccgcat ggcccgcccc 600

tactacctgg aagggttccg cctgttggaa gagcacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

cgagccgcac gtcagtggcc acagctgcgc gtggaacaac ggccggcctt accgagcctg 1020

gggccggccg tccagatcaa tgacgcattc accgtcagca tcaccgatgg ccgcacggcg 1080

agccagttag ccgagcagac gacggctgac gcttttgtgg tcgacatcgc cctgaactac 1140

gccgacacgg cgtatctggt tgcggcgcac aatcgctatg cctctgcggc caataaggcg 1200

ctgtttttac gcctgctgca cactgcgatc ccgcaggttg aatttatcaa agattccccg 1260

gcgctgatcg tcgctcgcgt cctcagcagc ctgatcaatg agtcggtgat catggtggaa 1320

agcggcgtct gcagccggga agacattgat gtcgccgccg tcgcgggcgt taactacgcc 1380

ggcggcattt tcgactggct cggcaaactg gggagaaaa acgtcaggac gacgctgagc 1440

aatctggcgc agctgctgca cgcagcgcgc tatgcgccgc attacaccct tctgcacgcc 1500

gcgcaaccgg cgctgacgac cacgccttga 1530

<210> 121
<211> 1530
<212> DNA
<213> Serratia marcescens UMH11

<400> 121
atggcagaaa gtaatgcggc aattcaatcg gctgcgatta tcggcgcggg aacgatgggc 60

agaggcatcg cttatctttt cgcgcaaaaa ggcattcgta cggtgcttta taatcgcagc 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgtgg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg tcaaccgcat ggcccgcccc 600

tactacctgg aagggttccg cctgttggaa gagcacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

cgagccgcac gtcagtggcc acagctgcgc gtggaacaac ggccggcctt accgagcctg 1020

gggccggccg tccagatcaa tgacgcattc accgtcagca tcaccgatgg ccgcacggcg 1080

agccagttag ccgagcagac gacggctgac gcttttgtgg tcgacatcgc cctgaactac 1140

gccgacacgg cgtatctggt tgcggcgcac aatcgctatg cctctgcggc caataaggcg 1200

ctgtttttac gcctgctgca cactgcgatc ccgcaggttg aatttatcaa agattccccg 1260

gcgctgatcg tcgctcgcgt cctcagcagc ctgatcaatg agtcggtgat catggtggaa 1320

agcggcgtct gcagccggga agacattgat gtcgccgccg tcgcgggcgt taactacgcc 1380

ggcggcattt tcgactggct cggcaaactg gggagaaaa acgtcaggac gacgctgagc 1440

aatctggcgc aactgctgca cgcagcgcgc tatgcgccgc attacaccct tctgcacgcc 1500

gcgcaaccgg cgctgacgac cacgccttga 1530

<210> 122
<211> 1530
<212> DNA
<213> Serratia marcescens UMH1

<400> 122
atggcagaaa gtaatgcggc aattcaatcg gctgcgatta tcggcgcggg aacgatgggc 60

agaggtatcg cttatctttt cgcgcaaaaa ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgagg ctatcgccga cagcgagttg gtgatagaaa ccatcgctga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacatgc caccacggaa cgttgccgtc aactggtggc cgcgttgggg 540

aaacgcgatg tcgtctgcca ggccacgccg gggtttatcg ttaaccgcat ggcccgcccc 600

tactacctgg aagggttccg cctgttggaa gaacacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

caagccgcac gtcagtggcc acagctgcgc gtggaacaac ggccggcctt accgagcctg 1020

gggccggccg tccagatcaa tggcgcattc accgtcagca tcaccgatgg ccgcacggcg 1080

agccagttag ccgagcagac gacggctgac gcttttgtga tcgacatcgc cctgaactac 1140

gccgacacgg cgtatctggt tgcggcgcac aatcgctatg cctctgcggc caataaggcg 1200

ctgtttttac gcctgctgca cactgcgatc ccgcaggttg aatttatcaa agactccccg 1260

gcgctgatcg tcgctcgtgt cctcagcagc ctgatcaatg agtcggtgat catggtggaa 1320

agcggcgttt gcagccggga agacattgat gtcgccgccg tcgcgggcgt taactacgcc 1380

ggcggcattt tcgactggct cggcaaactg gggagaaaa acgtcaggac gacgctgagc 1440

aatctggcgc agctgctgca cgcagcgcgc tatgcgccgc attacaccct tctgcacgcc 1500

gcgcaaccgg cgctgacgac cacgccttga 1530

<210> 123
<211> 1530
<212> DNA
<213> Serratia marcescens 2880STDY5683020

<400> 123
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagcagac ggcggcggat gcctttgtgg tcgatgtcgc cctgaattac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg ggggagaaaa acgtcaggac gacgctgagc 1440

aacctggcgc agctgctgca cgcggcacgc tatgcgccgc attacaccct tctgcacgcc 1500

gcgcaaccgg cgctgacgac cacgccttaa 1530

<210> 124
<211> 1530
<212> DNA
<213> Serratia marcescens 99

<400> 124
atggcagaaa gtaatgcggc aattcaatcg gctgcgatta tcggtgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg ggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgcctccaat 900

gcagacgtcg agacgctgcg cgtttacggc gaacaccctt tttttaccct gttgcaacag 960

cgggccgcgc ttcagtggcc acggctgcgc gtggagcaac ggccggcctt accgggtctg 1020

gggccggccg tccagatcaa tgaggctttc accgtcagcg tcaccgatgg ccgcacggcg 1080

agccaattgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg ggggagaaaa acgtcaggac gacgctgagc 1440

aacctggcgc agctgctgca cgcggcacgc tatgcgccgc attacaccct tctacacgcc 1500

gcgcaaccgg tgctgacgac cacgccttaa 1530

<210> 125
<211> 1530
<212> DNA
<213> Serratia marcescens 374

<400> 125
atggcagaaa gtaatgcggc aattcaatcg gctgcgatta tcggcgcggg aacgatgggc 60

agaggcatcg cttatctttt cgcgcaaaag ggcattcgta cggtgcttta taatcgcaac 120

ggcaataccc tcaatcaggc tcgcgaatat atcgcgcaag acctgaacaa gaaagtcgaa 180

cagggcaaga tcgcgctgca ggataaaagc tcagtgctgg ccaatctggt gttctcgacg 240

gcgcttgtgg ctatcgccga cagcgagttg gtgatagaaa ccatcgccga gcatgaacaa 300

accaaacttg aggtgttggc ggccatcgcc gcggtggtca agcccgatac gctgatcgcc 360

accaatacct cctcactgtc gcttaacaag ctggcaaccg cggtgacgca cagcgagcgg 420

tttatcggtt tgcacttttt caaccctgcg ccgctgatga aactgattga aatcatcccg 480

gcctacttta ccgcacacgc caccacggaa cgttgccgtc aactggtggc cgcgttgggg 540

aaacgcgatg tcgtctgtca ggccacgccg gggtttatcg tcaaccgcat ggcccgcccc 600

tactacctgg aagggttccg cctgttggaa gagcacgtgg cgcgcgcgcc gcagatcgac 660

cgtgccctca aggccggcgg acacttccgc atggggccgc tcgagctgac cgattttatc 720

ggccaggaca tcaactatca ggtcagccgg caaatctggc aggacatgca atacgacccg 780

cgctataccc ccggccatct gcagcgttcg ctggtcgatg ccggtctgtt ggggaaaaag 840

aacggccgct cctattttgc cgccgaagaa accgccccgc cagtgacggc cgccatcgat 900

gcagacgtcg agacgctgcg cgtttacggc gaacaccctt tttttaccct gttgcaacag 960

caagccgcac gtcagtggcc acagctgcgc gtggaacaac ggccggcctt accgagcctg 1020

gggccggccg tccagatcaa tggcgcattc accgtcagca tcaccgatgg ccgcacggcg 1080

agccagttag ccgagcagac gacggctgac gcttttgtga tcgacatcgc cctgaactac 1140

gccgacacgg cgtatctggt tgcggcgcac aatcgctatg cctctgcggc caataaggcg 1200

ctgtttttac gcctactgca cactgcgatc ccgcaggttg aatttatcaa agactccccg 1260

gcgctgatcg tcgctcgtgt cctcagcagc ctgatcaatg agtcggtgat catggtggaa 1320

agcggcgttt gcagccggga agacattgat gtcgccgccg tcgcgggcgt taactacgcc 1380

ggcggcattt tcgactggct cggcaaactg gggggagaaaa acgtcaggac gacgctgagc 1440

aatctggcgc agctgctgca cgcagcgcgc tatgcgccgc attacaccct tctgcacgcc 1500

gcgcaaccgg cgctgacgac cacgccttga 1530

<210> 126
<211> 1530
<212> DNA
<213> Serratia marcescens 2880STDY5683036

<400> 126
atggcagaaa gtaatgcggc aattcaatcg gctgcgatta tcggtgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgctcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg ggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgcctccaat 900

gcagacgtcg agacgctgcg cgtttacggc gaacaccctt tttttaccct gttgcaacag 960

cgggccgcgc ttcagtggcc acggctgcgc gtggagcaac ggccggcctt accgggtctg 1020

gggccggccg tccagatcaa tgaggctttc accgtcagcg tcaccgatgg ccgcacggcg 1080

agccaattgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg gggagaaaa acgtcaggac gacgctgagc 1440

aacctggcgc agctgctgca cgcggcacgc tatgcgccgc attacaccct tctacacgcc 1500

gcgcaaccgg tgctgacgac cacgccttaa 1530

<210> 127
<211> 1530
<212> DNA

<213>  Serratia marcescens 2880STDY5683034

<400>  127
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgctgccgtc agttggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg catttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgataacctg ggggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210>  128
<211>  1530
<212>  DNA
<213>  Serratia marcescens 2880STDY5682892

<400>  128
atggcagaaa gtaatgcggc aattcaatcg gctgcgatta tcggtgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg gggagaaaa acgtcaggac gacgctgagc 1440

aacctggcgc agctgctgca cgcggcacgc tatgcgccgc attacaccct tctgcacgcc 1500

gcgcaaccgg cgctgacgac cacgccttaa 1530

<210> 129
<211> 1530
<212> DNA
<213> Serratia marcescens SM39

<400> 129
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgtgcaag acctgaacaa gaaagtcgaa 180

cagggcaaga tcgcgctgca ggataaaggc gcagtgctgg ccaatctggt gttcacctcg 240

gtgtttgaga ctatcgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg catttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgataacctg gggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 130
<211> 1530
<212> DNA
<213> Serratia marcescens 189

<400> 130
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg ggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg catttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgataacctg gggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 131
<211> 1530
<212> DNA
<213> Serratia marcescens SMB2099

<400> 131
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgaggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg gggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 132
<211> 1530
<212> DNA
<213> Serratia marcescens 2880STDY5682862

<400> 132
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgtgcaag acctgaacaa gaaagtcgaa 180

cagggcaaga tcgcgctgca ggataaaggc gcagtgctgg ccaatctggt gttcacctcg 240

gtgtttgaga ccattgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgctggcgt taactacgcc 1380

gacggcattt tcggctggct cgatagcctg gggggagaaaa acgtcaggac gacgctgagc 1440

aacatggcgc agctgctgca cgcggcacgc tatgcgccgc attacaccct tctgcacgcc 1500

gcgcaaccgg cgctgatgac cacgccttaa 1530

<210> 133
<211> 1530
<212> DNA
<213> Serratia marcescens SE4145

<400> 133
atggcagaaa gtaatgcgga aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgtgcaag acctgaacaa gaaagtcgaa 180

cagggcaaga tcgcgctgca ggataaaggc gcagtgctgg ccaatctggt gttcacctcg 240

gtgtttgaga ctatcgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg catttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgataacctg gggggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 134
<211> 1530
<212> DNA
<213> Serratia marcescens 2880STDY5682876

<400> 134
atggcagaaa gtaatgcggc aattcaatcg gctgcgatta tcggtgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca gggtaaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg gggggagaaaa acgtcaggac gacgctgagc 1440

aacctggcgc agctgctgca cgcggcacgc tatgcgccgc attacaccct tctgcacgcc 1500

gcgcaaccgg cgctgacgac cacgccttaa 1530

<210> 135
<211> 1530
<212> DNA
<213> Serratia marcescens 709

<400> 135
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagtcgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacgac gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggctggct cgatagcctg gggggagaaaa acgtcaggac gacgctgagc 1440

aacctggcgc agctgctgca cgcggcacgc tatgcgccgc attacaccct tctgcacgcc 1500

gcgcaaccgg cgctgacgac cacgccttaa 1530

<210> 136
<211> 1530
<212> DNA
<213> Serratia marcescens MGH136

<400> 136
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

caggacaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtta agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgt caccaccgaa cgttgccgtc agctggtggc cgcgttgggg 540

aaacgcgatg tcgtctgcca ggccacgccg ggtttatcg tcaaccgcat ggcccgcccc 600

tactacctgg aagggttccg tctgttagaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgctg tcgccggcgt taactacgcc 1380

gacggcattt tcggctggct cgatagcctg gggagaaaa acgtcagaac gacgctgagc 1440

aacctggcgc agctgctgca cgcggcacgc tatgcgccgc attacaccct tctgcacgcc 1500

gcgcaaccgg cgctgacgac cacgccttaa 1530

<210> 137
<211> 1530
<212> DNA
<213> Serratia marcescens 2880STDY5682884

<400> 137
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgctgccgtc agttggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg catttacggc gaacaccctt tttttaccct gttgcaacag 960

cgggccacgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgat ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagtcggga agacatcgat gtcgccgccg tcgccggcgt taactacgcc 1380

gacggcattt tcggctggct cgatagcctg gggagaaaa acgtcaggat gacgctgagc 1440

aacctggcgc agctgctgca cgcggcacgc tatgcgccgc attacaccct tctacacgcc 1500

gcgcaaccgg cgctgacgac cacgccttaa 1530

<210> 138
<211> 1530
<212> DNA
<213> Serratia marcescens D-3

<400> 138
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccatcgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggcttgct cgatagcctg ggggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 139
<211> 1530
<212> DNA
<213> Serratia marcescens 2880STDY5682957

<400> 139
atggcagaaa gtaatgcggc aattcaatcg gctgcgatta tcggcgcggg aacgatgggc 60

agaggcatcg cttatctttt cgcacaaaaa ggcattcgca cggtgcttta taatcgcaac 120

ggcaataccc tcaatcaggc tcgcgaatat atcgagctag acctgaacaa gaaagtcgaa 180

cagggcaaga tcgcgctgca ggataaaggc gcggtgctgg ccaatctggt gttcacctcg 240

gcgtttgaga ccattgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggtattt tcggctggct cgatagcctg ggggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 140
<211> 1530
<212> DNA
<213> Serratia marcescens YDC563

<400> 140
atggcagaaa gtaatgcagc aattcagtcg gctgcgatta tcggcgcggg aacgatgggc 60

agaggcatcg cttatctttt cgcacaaaaa ggcattcgca cggtgcttta taatcgcaac 120

ggcaataccc tcaatcaggc tcgcgaatat atcgagctag acctgaacaa gaaagtcgaa 180

cagggcaaga tcgcgctgca ggataaaggc gcggtgctgg ccaatctggt gttcacctcg 240

gcgtttgaga ccattgccga cagcgagctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtagc cgcgttgggg 540

aaacgcgatg tcgtctgcca ggccacgccg ggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagcagac ggcggcggat gcctttgtgg tcgatgtcgc cctgaattac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgctggcgt taactacgcc 1380

gacggcattt tcggctggct cgatagcctg ggggagaaaa acgtcaggac gacgctgagc 1440

aacatggcgc agctgctgca cgcggcacgc tatgcgccgc attacaccct tctgcacgcc 1500

gcgcaaccgg cgctgatgac cacgccttaa 1530

<210> 141
<211> 1530
<212> DNA
<213> Serratia marcescens 2880STDY5683035

<400> 141
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacct cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg ggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccatcgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gtctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt taactacgcc 1380

gacggcattt tcggcttgct cgatagcctg gggagaaaa acgtcaggac gacgctgagc 1440

aacctggcgc agctgctgca cgcggcacgc tatgcgccgc attacaccct tctacacgcc 1500

gcgcaaccgg cgctgacgac cacgccttaa 1530

<210> 142
<211> 1530
<212> DNA
<213> Serratia marcescens 2880STDY5682930

<400> 142
atggcagaaa gtaatgcggc aattcaatcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgaatat atcgcgcaag acctgaacaa gaaagtcgaa 180

cagggcaaga tcgcgctgca ggataaaggc gcagtgctgg ccaatctggt gttcacctcg 240

gtgtttgaga ctatcgccga cagcgatctg gtgatagaaa ccatcgccga gcaagagcaa 300

accaaacttg aggtgctggc ggccatcgcc gcggcggtca agcccgacac gctgatcgcc 360

accaatacat cctcactgtc gcttaacaag ctggcaaccg cggtgacgca cagcgagcgg 420

tttatcggtt tgcacttttt caatcccgcg ccgctgatga aactgattga aatcatcccg 480

gcctacttta ccgcacgcgc caccaccgaa cgttgccgtc agctggtggc cgcgttgggg 540

aaacgcgatg tcgtctgcca ggccacgccg gggtttatcg tcaaccgcat ggcccgcccc 600

tactacctgg aagggttccg cctgttggaa gaacacgtgg cgcgcgcgcc gcagatcgac 660

cgcgccctca aggccggcgg gcactttcgc atggggccgc tcgagctgac cgattttatc 720

ggccaggaca tcaactatca ggtcagccgg caaatctggc aggacatgca atacgatccg 780

cgctataccc ccggccatct gcagcgttcg ctggtcgatg ccggtctgtt ggggaaaaag 840

aacggccgct cctattttgc cgccgaagaa aacgccttac cggtaacggc cgccaccgat 900

gcagacgtcg agacgctgcg cgtttacggc gaacaccctt tttttaccct gttgcaacag 960

cgggccgcgc ttcagtggcc acggctgcgc gtggagcaac ggccggcctt accgggcctg 1020

gggccggccg tccagatcaa tgaggctttc accgtcagcg tcaccgatgg ccgcacggcg 1080

agccagttgg cggagctgac ggcggcggat gcctttgtgg tcgatgtcgc cctgaactac 1140

gccgacacgg cgtatctggt ggcagcgcac aaccgccacg cctctgcggc caataaggcg 1200

ctgtttttac gcctgctgca cacggcaatc ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcaat gtcgccgccg tcgccggcgt taactacgcc 1380

gacggcattt tcggcttgct cgatagcctg gggagaaaa acgtcaggac gacgctgagc 1440

aacctggcgc agctgctgca cgcggcacgc tatgcgccgc attacaccct tctacacgcc 1500

gcgcaaccgg tgctgacgac cacgccttaa 1530

<210> 143
<211> 1530
<212> DNA
<213> Serratia marcescens 790

<400> 143
atggcagaaa gtaatgcggc aattcaatcg gctgcgatta tcggcgcggg aacgatgggc 60
agaggcatcg cttatctttt cgcgcaaaaa ggcattcgca cggtgcttta taatcgcaac 120
ggcaataccc tcaatcaggc tcgcgaatat atcgcgcaag acctgaacaa gaaagtcgaa 180
cagggcaaga tcgcgctgcg ggataaaggt gcagtgctag ccaatctggt gttcacctcg 240
gtgtttgaga ctatcgccga cagcgagctg gtgatagaaa ccatcgccga gcaagagcaa 300
accaaacttg aggtgctggc ggccatcgcc gcggcggtca agcccgacac gctgatcgcc 360
accaatacct cctcactgtc gcttaacaag ctggcaaccg cggtgacgca cagcgagcgg 420
tttatcggtt tgcacttttt caatcccgcg ccgctgatga aactgattga aatcatcccg 480
gcctacttta ccgcacgcgc caccaccgaa cgttgccgtc agctggtagc cgcgttgggg 540
aaacgcgatg tcgtctgcca ggccacgccg gggtttatcg tcaaccgcat ggcccgcccc 600
tactacctgg aagggttccg cctgttggaa gaacacgtgg cgcgcgcgcc gcagatcgac 660
cgcgccctca aggccggcgg gcactttcgc atggggccgc tcgagctgac cgattttatc 720
ggccaggaca tcaactatca ggtcagccgg caaatctggc aggacatgca atacgatccg 780
cgctataccc ccggccatct gcagcgttcg ctggtcgatg ccggtctgtt ggggaaaaag 840
aacggccgct cctattttgc cgccgaagaa aacgccttac cggtaacggc cgccaccgat 900
gcagacgtcg agacgctgcg cgtttacggc gaacaccctt tttttaccct gttgcaacag 960
cgggccgcgc ttcagtggcc acggctgcgc gtggagcaac ggccggcctt accgggcctg 1020
gggccggccg tccagaccaa tgaggctttc accgtcagcg tcaccgatgg ccgcacggcg 1080
aaccagttgg cggagctgac ggcggcggat gcctttgtgg tcgatgtcgc cctgaactac 1140
gccgacacgg cgtatctggt ggcagcgcac aaccgccacg cctctgcggc caataaggcg 1200
ctgtttttac gcctgctgca cacggcaatc ccgcaggttg aatttatcaa ggactccccg 1260
gcgctgatcg tcgcccgcgt cctcagcagc ctgatcaacg agtcggtgat catggtggaa 1320
agcggcgtct gcagccggga agacatcgat gtcgccgccg tcgctggcgt taactacgcc 1380
gacggcattt tcggctggct cgatagcctg gggagaaaa acgtcaggac gacgctgagc 1440
aacatggcgc agctgctgca cgcggcacgc tatgcgccgc attacaccct tctgcacgcc 1500
gcgcaaccgg cgctgatgac cacgccttaa 1530

<210> 144
<211> 1530
<212> DNA
<213> Serratia marcescens UMH5

<400> 144
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcagtcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc gaccatcgcc gctgcagtca agcccgacac gctgatcgcc 360

accaatacct cctcactgtc gctaaataag ctggcaaccg cggtgacgca cagcgagcgg 420

tttatcggtt tgcacttttt caaccccgcg ccgctgatga aactgattga aatcatcccg 480

gcctactgta ccgcacacgc caccacggag cgttgccgcc agctggtggc cgcgttgggg 540

aaacgtgacg tcgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggtgg gcactttcgt atggggccac tcgaactgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtttacggc gaacaccctt tttttaccct gttacaacag 960

cgggccgcgc ttcagtggcc acggctgcgc gtggaacaac ggcaggcatt accgggcctg 1020

gggccggccg tccggatcaa tgacgctttc accatcagca tcaccgatgg ccgcacggca 1080

aaccagctgg ccgagcagac ggcggcggat gcctttgtgg tcgatgttgc cctgaattac 1140

gccgacacgg cgtatctggt ggcagcgcac agccgccacg cctctggggc caataaagcg 1200

ctgttcttac gcctgctgca cacggcaatc ccgcaggttg aatttatcaa ggattccccg 1260

gccttgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct cgatagcctg ggggagaaaa acgtcaggac gacgctgagc 1440

aacctggcgc agttgctgca cgcggcgcgc tatgcgccgc attacaccct tctgcacgcc 1500

gcgcaaccgg cgctgacgac cacgccttaa 1530

<210> 145
<211> 1530
<212> DNA
<213> Serratia marcescens 2880STDY5682988

<400> 145
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcagtcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc gaccatcgcc gctgcagtca agcccgacac gctgatcgcc 360

accaatacct cctcactgtc gctaaataag ctggcaaccg cggtgacgca cagcgagcgg 420

tttatcggtt tgcacttttt caaccccgcg ccgctgatga aactgattga aatcatcccg 480

gcctactgta ccgcacacgc caccacggag cgttgccgcc agctggtggc cgcgttgggg 540

aaacgtgacg tcgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggtgg gcactttcgt atggggccac tcgaactgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtttacggc gaacaccctt tttttaccct gttacaacag 960

cgggccgcgc ttcagtggcc acggctgcgc gtggaacaac ggcaggcatt accgggcctg 1020

gggccggccg tccggatcaa tgacgctttc accatcagca tcaccgatgg ccgcacggca 1080

aaccagctgg ccgagcagac ggcgacggat gcctttgtgg tcgatgttgc cctgaattac 1140

gccgacacgg cgtatctggt ggcagcgcac agccgccacg cctctggggc caataaagcg 1200

ctgttcttac gcctgctgca cacggcaatc ccgcaggttg aatttatcaa ggattccccg 1260

gccttgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct cgatagcctg ggggagaaaa acgtcaggac gacgctgagc 1440

aacctggcgc agttgctgca cgcggcgcgc tatgcgccgc attacaccct tctgcacgcc 1500

gcgcaaccgg cgctgacgac cacgccttaa 1530

<210> 146
<211> 1530
<212> DNA
<213> Serratia marcescens 945154301

<400> 146
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc ggccatagcc gcggcagtca agcccgacac gctgatcgcc 360

accaatacct cctcactgtc gctgaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtggc cgcgttgggg 540

aaacgcgatg ttgtctgcca ggccacgccg ggtttatcg tcaaccgcat ggcccgcccc 600

tactacctgg aagggttccg cctattggaa gaacacgtgg cgcgcgcgcc gcagatcgac 660

cgtgccctca aggctggcgg gcactttcgt atggggccgc tcgagctgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgccgaagaa aacgccccgc cggtgatggc cgccaccgat 900

gcagacattg agacgctgca cgtttacggc gaacaccctt tttttaccct gttacaacaa 960

cgtgccgcgc ttcagtggcc acagctgcgc gtggaacaac ggccggcatt accgggcctg 1020

gggccggccg tccggatcaa tgacgctttc agcgtcagca tcaccgatgg ccgcacggca 1080

aaccagctgg ccgagcagac ggcggcggat gcctttgtgg tcgatgtcgc cctgaattac 1140

gccgacacgg cgtatctggt ggcagcgcac agccgccatg cctctgcagc caataaagcg 1200

ctgttcttac gcctgctgca cacggcaatc ccgcaggttg aatttatcaa ggattccccg 1260

gcgctgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct agatagcctg ggggagaaaa acgtcaggac gacgctgagc 1440

aacctggcgc agctgctgca cgcagcacgc tatgcgccgc attacaccct tctgcacgcc 1500

gcgcaaccgg cgctgacgac cacgccttaa 1530

<210> 147
<211> 1530
<212> DNA
<213> Serratia marcescens at10508

<400> 147
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc ggccatcgcc gcggcagtca agcccgacac gctgatcgcc 360

accaatacct cctcactgtc gctgaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtgac cgcgttgggg 540

aaacgcgatg ttgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggcgg gcactttcgt atggggccgc tcgagctgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtttacggc gaacaccctt tttttaccct gttacaacaa 960

cgtgccgcgc ttcagtggcc acagctgcgc gtggaacaac ggccggcatt accgggcctg 1020

gggccggccg tccggatcaa tgacgctttc accgtcagca tcaccgatgg ccgcacggca 1080

aaccagctgg ccgagcagac ggcggcggat gcctttgtgg tcgatgtcgc cctgaattac 1140

gccgacacgg cgtatctggt ggcagcgcac agccgccatg cctctgcagc caataaagcg 1200

ctgttcttac gcctgctgca cacggcaatc ccgcaggttg aatttatcaa ggattccccg 1260

gcgctgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct agatagcctg ggggagaaaa acgtcaggac gacgctgagc 1440

aacctggcgc agctgctgca cgcagcacgc tatgcgccgc attacaccct tctgcacgcc 1500

gcgcaaccgg cgctgacgac cacgccttaa 1530

<210> 148
<211> 1530
<212> DNA
<213> Serratia marcescens ML2637

<400> 148
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc ggccatcgcc gcggcagtca agcccgacac gctgatcgcc 360

accaatacct cctcactgtc gctgaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtgac cgcgttgggg 540

aaacgcgatg ttgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggcgg gcactttcgt atggggccgc tcgagctgac cgattttatc 720

ggccaggaca tcaactatca ggtcagtcgg caaatctggc aggacatgca atacgacgcg 780

cgctataccc ccggccacct acagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtttacggc gaacaccctt tttttaccct gttacaacaa 960

cgtgccgcgc ttcagtggcc acagctgcgc gtggaacaac ggccggcatt accgggcctg 1020

gggccggccg tccggatcaa tgacgctttc accgtcagca tcaccgatgg ccgcacggca 1080

aaccagctgg ccgagcagac ggcggcggat gcctttgtgg tcgatgtcgc cctgaattac 1140

gccgacacgg cgtatctggt ggcagcgcac agccgccacg cctctgcagc caataaagcg 1200

ctgttcttac gcctgctgca cacggcaatc ccgcaggttg aatttatcaa ggattccccg 1260

gcgctgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct agatagcctg ggggagaaaa acgtcaggac gacgctgagc 1440

aacctggcgc agctgctgca cgcagcacgc tatgcgccgc attacaccct tctgcacgcc 1500

gtgcaaccgg cgctgacgac cacgccttaa 1530

<210> 149
<211> 1530
<212> DNA
<213> Serratia marcescens SM1978

<400> 149
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggcaaga tcgcgctgca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc ggccatagcc gcggcagtca agcccgacac gctgatcgcc 360

accaatacct cctcactgtc gctgaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtgac cgcgttgggg 540

aaacgcgatg ttgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggcgg gcactttcgt atggggccgc tcgagctgac cgattttatc 720

ggccaggaca tcaactatca ggtcagtcgg caaatctggc aggacatgca atacgacgcg 780

cgctataccc ccggccacct gcagcgttcg ctggtcgatg ccggtctatt gggaaaaaag 840

aacggccgct cctattttgc cgccgaagaa aacgccccgc cggtgatggc ctccaccgat 900

gcagacattg agacgctgca cgtttacggc gaacaccctt tttttaccct gttacaacaa 960

cgtgccgcgc ttcagtggcc acagctgcgc gtggaacaac ggccggcatt accgggcctg 1020

gggccggccg tccggatcaa tgacgctttc agcgtcagca tcaccgatgg ccgcacggca 1080

aaccagctgg ccgagcagac gacggcggat gcctttgtgg tcgatgtcgc cctgaattac 1140

gccgacacgg cgtatctggt ggcagcgcac agccgccacg cctctgcagc caataaagcg 1200

ctgttcttac gcctgctgca cacggcaatc ccgcaggttg aatttatcaa ggattccccg 1260

gcgctgatcg tcgcccgcgt cctcagcagc ctaatcaacg agtcggtgat catggtggaa 1320

agcggcgtct gcagccggga agacatcgat gtcgccgccg tcgccggcgt taactacgcc 1380

gacggcattt tcggctggct agatagcctg ggggagaaaa acgtcaggac gacgctgagc 1440

aacctggcgc agctgctgca cgcagcacgc tatgcgccgc attacaccct tctgcacgcc 1500

gcgcaaccgg cgctgacgac cacgccttaa 1530

<210> 150
<211> 1530
<212> DNA
<213> Serratia marcescens PWN146

<400> 150
atggcagaaa gtaatgcggc aattcagtcg gctgcgatta tcggcgcggg aacgatgggc 60

agaggcatcg cttatctttt cgcgcaaaaa ggcattcgca cggtgcttta taatcgcaac 120

ggcaataccc tcaatcaggc tcgcgactat atcgagctag acctgaacaa gaaagtcgaa 180

cagggcaaga tcgcgctaca ggataaaggc gcggtgttgg ccaatctggt gttcacctcg 240

gtatttgaga ccattgccga cagcgagctg gtgatagaaa ccatcgccga gcaagagcaa 300

accaaacttg aggtgctggc gaccatcgcc gcggcagtca agcccgacac gctgatcgcc 360

accaatacct cctcactgtc gctaaataag ctggcaaccg cggtgacgca cagcgagcgg 420

tttatcggtt tgcacttttt taaccccgcg ccgctgatga aactgattga aatcatcccg 480

gcctacttta ccgcacacgc caccacggag cgttgccgtc aactggtggc cgcgttggga 540

aaacgcgacg ttgtctgcca ggccacgccg gggtttatcg tcaaccgcat ggcccgcccc 600

tactacctgg aagggttccg cctattggaa gaacacgtgg cgcgcgcgcc gcagatcgac 660

cgtgccctca aggccggcgg gcactttcgt atggggccgc tcgaactgac cgattttatc 720

ggccaggaca tcaactatca ggtcagccgg caaatctggc aggacatgca atacgacgcg 780

cgctataccc ccggccacct gcagcgttcg ctggtcgatg ccggtctatt gggaaaaaag 840

aacggccgct cctattttgc cgtcgaagaa aacgccccgc cggtgatggc cgccaccgat 900

gcagacattg agacgctgca cgtctgcggc gaacaccctt tttttactct gttgcaacag 960

cgagccgcac ttcagtggcc acggctgcgc gtggaacaac ggccggcctt gccgggcctg 1020

gggcccgccg tctggatcaa tgacgctttc accgtcagca tcaccgatgg ccgcacggca 1080

aaccagctgg ccgagcagac ggcggcggat gcctttgtgg tcgatgtcgc cctgaattac 1140

gccgacacgg cgtatctggt ggcagcacac agccgccacg cctctgcggc caataaagcg 1200

ctgttcttac gcctgctgca caccgcaatc ccgcaggttg aatttatcaa ggattccccg 1260

gcgctgatcg tcgctcgcgt cctcagcagc ctgatcaatg agtcggtgat catggtggaa 1320

agcggcgtct gcagccggga agacatcgat gtcgccgccg tcgccggcgt taactacgcc 1380

gacggcattt tcggctggct cgatagcctg gggggagaaaa acgtcagggc gacgctgagc 1440

aacttggcgc agctgctgca cgcggcgcgc tatgcgccgc attacaccct tctgcacgcc 1500

gcgcaaccgg cgctgacgac cacgccttaa 1530

<210> 151
<211> 1530
<212> DNA
<213> Serratia marcescens H1q

<400> 151
atggcagaac gtaatgcggc aattcaatcg gctgcgatta tcggcgcggg aacgatgggc 60

agaggcatcg cttatctttt cgcgcaaaaa ggcattcgta cggtgcttta taatcgcaac 120

ggcaataccc tcattcaggc tcgtgaatat atcgtgcaag acctggacaa aaaggtcgaa 180

cagggcaggc tcgcgccgca ggataaagac gcggtgctgg ccaatctgca gttctcatcg 240

gtgtttgaag ctatcgtcga cagcgatttg gtgctcgaaa ctatcgccga gcaagagcaa 300

gccaaactcg aggtgctggc cgccatcgct gcggcggtca aacccgacac gctgatcgcc 360

accaatacgt cctcattgtc gctcaataag ctggcaacgg cggtgaccca cagcgaacgc 420

tttatcggtt tgcacttttt caaccccgca ccgctaatga agctgattga aatcattccg 480

gcctacttta ccgcccaagc caccactgaa acttgccgcc aactggtggc ggcattgggg 540

aaacgcgatg tcgtctgcca ggctacgccg gggttcatcg tcaaccgtat ggcccgcccc 600

tactacctgg aaggctttcg cctgttggaa gagcacgtgg cgcgcgcgcc gcagatcgac 660

cgcgccctca aggccggcgg acactttcgc atggggccgc tcgagctgac cgattttatc 720

ggccaggaca tcaactatca ggtcagccgg caaatttggc aggacatgca gtacgacccg 780

cgctataccc ccggccatct gcagcgttcg ctggtcgatg ccgggctgct ggggaaaaag 840

aacggtcgct cctatttttc cgccgaagaa accgccccgc cggttgaggc cgccgtcgag 900

gcggatgtcg agacgctgcg catttacggc gaacaccctt tgtttaccct gctgcaacag 960

cgagccgcac tgcaatggcc gcagttgcgc gtggaacagc ggccggcctt gtcgggcctg 1020

ggagcggcca ttcaggtcaa tgacgctttc accgtcagca tcaccgatgg ccgcacggca 1080

aatcagttgg ccgagcagac ggcggcggac gcctttgtgg tcgatgtcgc cctgaactac 1140

gccgacacgg cgtatctggt ggcggcgcac agccgccacg cctctgcggc taataaggcg 1200

ttgtttttgc gcctgctgca caccgcgctt ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt gaactacgcc 1380

gacggcattt tcggctggct cactcgcctg gggagaaaa acgtcaggac gacgctgagc 1440

aacctggcgc agctgctgca cgcggcccgc tatgcgccgc attacaccct tctgcacgcc 1500

gcccaaccgg cgctgaccac cacgccttaa 1530

<210> 152
<211> 1530
<212> DNA
<213> Serratia marcescens UMH6

<400> 152
atggcagaaa gtaatgcggc aattcaatcg gctgcgatta tcggcgcggg aacgatgggc 60

agaggcatcg cttatctttt tgcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aagtgctggc ggccatcgcc gcgacggtca agcccgacac gctgatcgcc 360

accaatacct cctcactgtc gcttaataag ctggcgacag cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgcca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttctc cgccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gttgaacaac ggccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgttgc cctgaactac 1140

ggcgatacgg cgtacctggt ggcggcacat agccgccacg cctctgcggc caataaggcg 1200

ctgtttttac gcctgctgca caccgcgatc ccgcaggtgg aatttatcaa ggattccccg 1260

gccctgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 153
<211> 1530
<212> DNA
<213> Serratia nematodiphila WCU338

<400> 153
atggcagaaa gtaatgcggc aattcaatcg gctgcgatta tcggcgcggg aacgatgggc 60

agaggcatcg cttatctttt cgcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggataaagac gcggtgctgg ctaatctgac gttctccgcg 240

gaatttgggg ccatcgccga cagcgatctg gtgatcgaaa ccatcgcaga acatgagcaa 300

gccaaactcg aggtgctggc ggccatcgcc gcgacggtca agcccgacac gctgatcgcc 360

accaatacct cctcactgtc gctgaataag ctggcgacgg cggtgacgca cagcgaacgc 420

tttatcggtt tgcacttttt caaccccgcg ccgctgatga agctgattga aatcattccg 480

gcctatttta ccgcacagat caccaccgaa cgttgccgtc aactggtggc ggcattgggg 540

aaacgcgatg tcgtctgtca ggccacgccg gggtttatcg tcaaccgtat ggctcgcccc 600

tactatctgg aaggattccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtttgct ggggaaaaag 840

aacggccgct cctatttttc cgccgaagaa tctcccccgc cgcttgcagc cgccgtcgat 900

gcggaggtcg agacgctacg catttacggt gaacatcctc tctttaccct gctacagcag 960

cgggccgccc tgcaatggcc gcggctgcgc gtggaacagc ggccgacatt accaggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg catctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 154
<211> 1530
<212> DNA
<213> Serratia sp. OLEL1

<400> 154
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aagtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgcca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttctc cgccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accgggcctg 1020

ggcgccgcca ttcaggttaa tgacgctttc accgtcagcg ttaccgatgg ccgcacggca 1080

aaccagcttg ccgaacagac tgcggcggac gcctttgtcg tcgatgtcgc cctgaactac 1140

ggcgatacgg cgtacctggt ggcggcgcat agccgccacg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggcattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 155
<211> 1530
<212> DNA
<213> Serratia marcescens 7209

<400> 155
atggcagaaa gtaatgcggc aattcaatcg gctgcgatta tcggcgcggg aacgatgggc 60

agaggcatcg cttatctttt tgcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgca 240

gaatttgggg tcatcgccga cagcgatctg gtgatcgaaa ccatcgcaga acatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttaggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaagtcg agacgctacg catttacggt gaacatcctc tctttaccct gctacagcag 960

cgggccgccc tgcaatggcc gcggctgcgc gtggaacaac ggccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcacat agccgccatg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggttat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 156
<211> 1530
<212> DNA
<213> Serratia marcescens sicaria (Ss1)

<400> 156
atggcagaaa gtaatgcggc aattcaatcg gctgcgatta tcggcgcggg aacgatgggc 60

agaggcatcg cttatctttt tgcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgcca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc caccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accgggcttg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cctacctggt ggcggcgcat agccgccacg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgattaacg agtcggtgat catggtggaa 1320

agcggcgtct gtagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 157
<211> 1530
<212> DNA
<213> Serratia sp. OLFL2

<400> 157
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttgggg ccatcgccga cagcgatctg gtgatcgaaa ccatcgcaga gcatgagcaa 300

gccaaactcg acgtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgcca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttctc cgccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accgggcctg 1020

ggcgccgcca ttcaggttaa tgacgctttc accgtcagcg ttaccgatgg ccgcacggca 1080

aaccagcttg ccgaacagac tgcggcggac gcctttgtcg tcgatgtcgc cctgaactac 1140

ggcgatacgg cgtacctggt ggcggcgcat agccgccacg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggcattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 158

<211> 1530
<212> DNA
<213> Serratia marcescens BIDMC 81

<400> 158
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccgcg 240

gaatttggag ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtaacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgcca ggccacgccg ggatttatcg tcaaccgtat ggcccgcccc 600

tactatctgg aagggttccg cctgttggaa gaacacgtgg cgcgtgcgcc gcagatcgac 660

cgagccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accggacctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 159
<211> 1530
<212> DNA
<213> Serratia marcescens BIDMC 50

<400> 159
atggcagaaa gtaatgcggc aattcaatcg gctgcgatta tcggcgcggg aacgatgggc 60

agaggcatcg cttatctttt tgcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gtggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttaggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaggtcg agacgctacg catttacggt gaacatcctc tctttaccct gctacagcag 960

cgggccgccc tgcaatggcc gcggctgcgc gtggaacaac ggccgacatt accggacctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcacat agccgccatg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 160
<211> 1530
<212> DNA
<213> Serratia marcescens UMH7

<400> 160
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac acggtgctgg ctaatctgac gttctccgcg 240

gaatttgggg ccatcgccga cagcgatctg gtgatcgaaa ccatcgcaga gcatgagcaa 300

gccaaactcg aggtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgaactgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcctccgc cgcttgcggc cgccgtcgat 900

gcggaggtcg agacgctacg catttacggt gaacatcctc tctttactct gctacagcag 960

cgggccaccc tgcaatggcc gcggctgcgc gtggaacaac ggccgacatt accgggcctg 1020

ggcgccgcca ttcaggttaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcagc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 161
<211> 1530
<212> DNA
<213> Serratia marcescens RSC-14

<400> 161
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag acctgaacaa aaaggtggaa 180

cagggcaagc tcgcgctgca ggacaaagac gcggtgctgg ctaatctgac gttctccacg 240

gaatttgggg ccatcgccga cagcgatctg gtgatcgaaa ccatcgcaga gcatgagcaa 300

gccaaactcg aagtgctggc ggccatcgcc gcgacggtca agcccgacag gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttaggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcggc cgccgtcgat 900

gcggaagtcg agacgctacg catttacggt gaacatcctc tctttaccct gctacagcag 960

cgggccgccc tgcaatggcc gcggctgcgc gtggaacaac agccgacatt accgggcctg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcacat agccgccatg cctctgcggc caataaggcg 1200

ctgtttttac gcctgctgca caccgcgatc ccgcaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcgcctc ggggaggaaa atgtcaggac gacgctgagc 1440

aacctggcgc aattgctgca cgcggcgcgc tatgcgccgc attacaccct tctgcacgcc 1500

gcccaaccgg cgctgaccac cacgccttaa 1530

<210> 162
<211> 1530
<212> DNA
<213> Serratia marcescens SM03

<400> 162
atggcagaac gtaatgcggc aattcaatcg gctgcgatta tcggcgcggg aacgatgggc 60

agaggcatcg cttatctttt cgcgcaaaaa ggcattcgta cggtgcttta taatcgcaac 120

ggcaataccc tcattcaggc tcgtgaatat atcgtgcaag acctggacaa aaaggtcgaa 180

cagggcaggc tcgcgccgca ggataaagac gcggtgctgg ccaatctgca gttctcatcg 240

gtgtttgaag ctatcgtcga cagcgatttg gtgctcgaaa ctatcgccga gcaagagcaa 300

gccaaactcg aggtgctggc cgccatcgct gcggcggtca aacccgacac gctgatcgcc 360

accaatacgt cctcattgtc gctcaataag ctggcaacgg cggtgaccca cagcgaacgc 420

tttatcggtt tgcacttttt caaccccgca ccgctaatga agctgattga aatcattccg 480

gcctacttta ccgcccaagc caccactgaa acttgccgcc aactggtggc ggcattgggg 540

aaacgcgatg tcgtctgcca ggctacgccg gggttcatcg tcaaccgtat ggcccgcccc 600

tactacctgg aaggctttcg cctgttggaa gagcacgtgg cgcgcgcgcc gcagatcgac 660

cgcgccctca aggccggcgg acactttcgc atggggccgc tcgagctgac cgattttatc 720

ggccaggaca tcaactatca ggtcagccgg caaatttggc aggacatgca gtacgacccg 780

cgctataccc ccggccatct gcagcgttcg ctggtcgatg ccgggctgct ggggaaaaag 840

aacggtcgct cctatttttc cgccgaagaa accgccccgc cggttgaggc cgccgccgag 900

gcggatgtcg agacgctgcg catttacggc gaacaccctt tgtttaccct gctgcaacag 960

cgagccgcac tgcaatggcc gcagttgcgc gtggaacagc ggccggcctt gtcgggcctg 1020

ggagcggcca ttcaggtcaa tgacgctttc accgtcagcg tcaccgatgg ccgcacggca 1080

aatcagttgg ccgagcagac ggcggcggac gcctttgtgg tcgatgtcgc cctgaactac 1140

gccgacacgg cgtatctggt ggcggcgcac agccgccacg cctctgcggc taataaggcg 1200

ttgtttttgc gcctgctgca caccgcgctt ccgcaggttg aatttatcaa ggactccccg 1260

gcgctgatcg tcgcccgcgt cctcagcagc ctgatcaacg agtcggtgat catggtggaa 1320

agcggcgtct gcagccggga agacatcgat gtcgccgccg tcgccggcgt gaactacgcc 1380

gacggcattt tcggctggct cactcgcctg gggagaaaa acgtcaggac gacgctgagc 1440

aacctggcgc agctgctgca cgcggcccgc tatgcgccgc attacaccct tctgcacgcc 1500

gcccaaccgg cgctgaccac cacgccttaa 1530

<210> 163
<211> 1530
<212> DNA
<213> Serratia marcescens 90-166

<400> 163
atggcagaaa gtaatgcggc aattcaatcg gctgcaatta tcggcgcggg aacgatgggc 60

agaggcatcg cttatctttt cgcgcaaaaa ggcattcgta cggtgcttta taatcgcaac 120

ggcaataccc tcattcaggc tcgtgaatat atcgtgcaag acctggacaa aaagatcgaa 180

cagggcaggg tcgcgccgca ggataaagac gcggtgctgg ctaatctgac gttctccgcg 240

gaatttgggg ccatcgtcga cagcgatctg gtgatcgaaa ccattgcaga gcatgagcaa 300

gccaaactcg aggtgctggc ggccatcgcc gcgacggtca agcccgacac gctgatcgcc 360

accaatacct cctcactgtc gctgaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcattgggg 540

aaacgcgatg tcgtctgtca ggccacgccg gggtttatcg tcaaccgtat ggctcgcccc 600

tactatctgg aagggttccg cctgttggaa gaacacgtgg cacgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctcccccgc cgcttgcagc cgccgtcgat 900

gcggagatcg agacgctgcg catttacggt gaacatcctc tctttaccct gctacagcag 960

cgggccgccc tgcaatggcc gcggctgcgc gtggaacacc ggccgacatt accgggcctg 1020

ggcgccgcca ttcaggtcaa tgatgctttc accgtcagcg ttaccgatgg tcgcacggca 1080

aaccagcttg ccgaacagac cgcggcggac gcctttgtcg tcgatgtcgc cctgaactac 1140

ggcgatacgg cgtacctggc ggcggcgcat agccgccacg cctctgcggc caataaggcg 1200

ctgtttttac gcctgctgca caccgcgatc ccccaggtgg aatttatcaa ggattccccg 1260

gccttgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcgtctggct cactcgcctc ggggaggaaa atgtcaggac gacgctgagc 1440

aacctggcgc aattgctgca cgcggcgcgc tatgcaccgc attacaccct tctgcacgcc 1500

gcccaaccgg cgctgacctc cacgccttaa 1530

<210> 164
<211> 1530
<212> DNA
<213> Serratia marcescens UMH2

<400> 164
atggcagaaa gtaatgcggc aattcaatcg gctgcgatta tcggcgcggg aacgatgggc 60

agaggcatcg cttatctttt ggcgcaaaaa agcattcgta cggtgcttta taatcgcaac 120

ggcaataccc tcaatcaagc tcgcgacgct atcgtgcaag atctgaacaa aaaggtggaa 180

cagggcaagc tcgcgctgca ggacaaagac gcggtgctgg ctaatctgac gttctccacg 240

gaatttgggg ccatcgccga cagcgatctg gtgatcgaaa ccatcgcaga gcatgagcaa 300

gccaaactcg aagtgctggc ggccatcgcc gcgacggtca agcccgacac gctgatcgcc 360

accaatacct cctcactgtc gcttaataag ctggcgacgg cggtgacgca cagcgaacgc 420

tttatcggtt tgcacttttt caaccccgcg ccgctgatga agctgattga aatcattccg 480

gcctatttta ccgcacaggt caccaccgaa cgttgccgtc aactggtggc ggcgttgggg 540

aaacgcgatg tcgtctgtca ggccacgccg ggatttatcg tcaaccgtat ggcccgcccc 600

tactatctgg aagggttccg cctgttggaa gaacacgtgg cgcgtgcgcc gcagatcgac 660

cgcgccctca aagccggcgg acactttcgc atggggccgc tcgagctgac cgattttatc 720

ggtcaggaca tcaactatca ggtcagccgg caaatttggc aggacatgca gtacgatccg 780

cgctataccc cgggccatct gcagcgctcg ctggtcgatg ccggtctgtt gggcaaaaag 840

aacggccgct cctatttttc cgccgaagaa tctccccgc cgcttgcggc cgccgtcgat 900

gcggaggtcg aggcgctacg catttacggt gaacatcctc tctttaccct gctacagcag 960

cgggccgccc tgcaatggcc gcggctgcgc gtggaacaac ggccgacatt accgggcttg 1020

ggcgccgcca ttcaggtcaa tgacgctttc accgtcagcg ttaccgatgg ccgcacggca 1080

aaccagcttg ccgaacagac cgcggcggac gcctttgtcg tcgatgtcgc cctgaactac 1140

ggcgatacgg cgtacctggt ggcggcgcat agccgccacg cctctgcggc caataaggcg 1200

ctgtttttac gcctgctgca caccgcgatc ccgcaggtgg aatttatcaa ggattccccg 1260

gccctgatcg tcgcccgcgt gctcagcagc ctgatcaacg agtcggtgat catggtggaa 1320

agcggcgtct gcagccggga agacattgat gtcgccgccg tcgccggcgt gaactacgcc 1380

gacggtattt tcggctggct cactcacctc ggggaggaaa atgtcaggac gacgctgagc 1440

aacctggcgc aattactcca cgcggcgcgc tatgcgccgc attacaccct tctgcacgcc 1500

gcccaaccgg cgctgaccac cacgccttaa 1530

<210> 165
<211> 1530
<212> DNA
<213> Serratia plymuthica AS9

<400> 165
atggcagaga ataattcggc aatccgttca gccgccgtta ttggtgcggg aaccatgggc 60

agaggcatcg cctatctcct ggcgctgaac ggcatacgaa ccgtacttta taatcgcaat 120

ggtaataatc tcaatcaggc ccgtgactat attgtcagtg acctggacag aaaaatggat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccagaggc gatcattgcg 360

accaatacct cctcgttgtc gctgaacaaa ctggcggcgg gggtggaaaa caatccgcgc 420

tttatcggcc tgcatttttt caatccggcg ccgctgatga agttgatcga aatcattccc 480

tcttatttta cctcccacac caccagccta cgctgccagc agttggtaat agcgttgggt 540

aaacagtttg tggtctgcaa agccacgccg ggctttattg ttaatcgcat ggcgcgacct 600

ttctatctgg aagggttccg gctgctggag gaaaacgtgg cacaggctcc acagatcgac 660

cgcgccctca aggcaggcgg gcattttcgc atggggccct tagaactgac tgattttatt 720

ggccaggata tcaactatca ggtcagcaag cagatttggc aggatatgca gttcgactcc 780

cgctataccc ccggccattt gcaacgctcg ctggtggatg ccgggctgct ggggaggaag 840

aacgggcgct cttttttgc ttccctaccg gctacaccgc ccaccccggc cacagagagc 900

gacacaccaa cttcactgca tttttatggt gaacacgctt tattcgatca cctgcaacag 960

cgcgctttgg ccacctggcc tgcgctgcgc gttcagcggt tgccggaacg gccggaactg 1020

gggcgtctta tcctggtgaa taacacgctg gcgatcaaaa tcaccgatgg cagaacggcg 1080

aacctgctcg ccggcttaac cgctctcgac accttcgtga ttgacgctgc gctgaattac 1140

gccgacaccg cctatctggt ggccgcccac aatcagcatg ccacagaggc gaataaagcg 1200

ctgtttctgt cgctgctgca aaccgtcatc gctcaggtag agtttattaa agattcccct 1260

gccctgatcg ttgcccgcgt actgagcagc ctgatcaacg aatcggtgat catggtggag 1320

agcggcgttt gcagccgggc ggatatcgat atcgccgccg tggccggcgt gaactatgcc 1380

gacggcattt ttgcctggct ggcgcagctc gggcagaaaa acgtgaagtc gacgctggat 1440

aacatggcgc aattgctgca ctccgcgcgc tattacccgc attactcatt gctgaacgcg 1500

gcccggcctg agctggctgt agcgccctaa 1530

<210> 166
<211> 1530
<212> DNA
<213> Serratia plymuthica tumat 205

<400> 166
atggcagaga ataattcggc aatccgttca gccgccgtta ttggtgcggg gactatgggc 60

agaggcatcg cctacctctt ggcactgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcggcag gggtggaaaa caacccgcgc 420

tttatcggcc tgcatttttt caatccggcg ccgctgatga agttgatcga aatcattccc 480

tcttatttta cctcccacgc caccagccta cgctgccagc agttggtaat agcattgggt 540

aaacagtttg tggtctgcaa agccacaccg ggctttattg ttaatcgcat ggcgcggcct 600

ttctatctgg aagggttccg gctgctggag gaaaacgtgg cgctggcgcc acagatcgac 660

cgcgccctca aggccggcgg gcattttcgc atggggcctt tagaactgac ggattttatc 720

ggtcaggata tcaactacca ggtcagcaag cagatttggc aggatatgca gttcgaccct 780

cgctataccc ctggtcattt gcaacgctcg ctggtggatg ccgggctgct ggggaggaaa 840

aacgggcgct cttttttttgc ttcccaaccg gtgacgccac ccaccccgac cacagagagc 900

gacacgccaa cttcactgca tttttatggg gagcatgctt tattcgatca tctgcaacag 960

cgtgctctgg ccacctggcc tgcgctgcgc gttcagcggt tgccggaacg gcctgaactg 1020

gggcgattta tcctggtgaa taacgcgatg gcgatcaaaa tcaccgatgg cagaacggca 1080

aacctgctcg ccggcttaac cgctctcgac accttcgtga ttgacgctgc gctgaattac 1140

gccgacaccg cctatctggt ggccgcccac aatcaacatg ccacagagac gaataaagcg 1200

ctgtttctga cgctgctgca aaccgtcatc gctcaggtgg agtttattaa agattcccct 1260

gctctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aactgctgca ctccgcgcgc tattacccgc attactcatt gctgaacgcg 1500

gcccggcctg agctggccgt agcgccctaa 1530

<210> 167
<211> 1530
<212> DNA
<213> Serratia plymuthica A30

<400> 167
atggcagaga ataattcggc aatccgttca gccgccgtta ttggtgcggg gaccatgggc 60

agaggcatcg cctacctcct ggcactgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcggcag gggtggaaaa caacccgcgc 420

tttatcggcc tgcatttttt caatccggcg ccactgatga agttgatcga aatcattccc 480

tcttatttta cctcccacgc caccagccta cgctgccaga agttggtaat agcattgggt 540

aaacagtttg tggtctgcaa agccacgccg ggctttattg ttaatcgcat ggcgcggcct 600

ttctatctgg aagggttccg gctgctggag gagaacgtgg cgctggcgcc acagatcgac 660

cgcgccctca aggccggcgg gcattttcgc atggggcctt tagaactgac ggattttatc 720

ggccaggata tcaactacca ggtcagcaag cagatttggc aggatatgca gttcgaccct 780

cgctataccc ctggtcattt gcaacgctcg ctggtggatg ccgggctgct ggggaggaaa 840

aacgggcgct cttttttttgc ttcccaaccg gcgacgccgc ccaacccgac cactgagggc 900

gacacgccaa cttcactgca tttttatggt gaacacgctt tattcgatca cctgcaacag 960

cgcgctttgg ccacctggcc tgcgttgcgc gttcagcggt tgccggaacg gccggaactg 1020

gggcgtttta tcctgatgaa taacaggctg gcgatcaaaa tcactgatgg cagaacggcg 1080

aacctgctcg ccggcttaac cgctctcgac accttcgtga ttgacgccgc gctgaactac 1140

gccgacaccg cctatctggt ggccgcccac aatcaacatg ccacagagac gaataaagcg 1200

ctgtttctga cgctgctgca aaccctcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggtattt ttgcctggtt ggcgcagctc gggcagaaaa acgtgaaatc gacgctggat 1440

aacatggcgc aactgctgca ctccgcgcgc tattacccgc attactcatt gctgaacgcg 1500

gcccggcctg agctggccgt agcgccctaa 1530

<210> 168
<211> 1530
<212> DNA
<213> Serratia plymuthica 4Rx13

<400> 168
atggcagaga ataattcggc aatccgttca gccgccgtta ttggtgcggg gaccatgggc 60

agaggcatcg cctacctcct ggcgctgaac ggcatacgaa ccgtacttta taatcgcaat 120

ggtaatagtc tcaatcaggc ccgtgactat attgtcagcg acctggacag aaaaatagat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcggcag ggtggaaaa caacccgcgc 420

tttatcggcc tgcatttttt caatccggcg ccgctgatga agttgatcga aatcattccc 480

tcttatttta cctcccacgc caccagccta cgctgccaga agttggtaat agcattgggt 540

aaacagtttg tggtctgcaa agccacgccg ggctttattg ttaatcgcat ggcgcggcct 600

ttctatctgg aagggttccg gctgctggag gagaacgtgg cgctggcgcc acagatcgac 660

cgcgccctca aggccggcgg gcattttcgc atggggcctt tagaactgac ggattttatc 720

ggtcaggata tcaactacca ggtcagcaag cagatttggc aggatatgca gttcgaccct 780

cgctataccc ccggccactt gcaacgctcg ctggtggatg ccgggctgct ggggaggaaa 840

aacgggcgtt cttttttgc ttcccaaccg gcgacgccac ccaccccgac cacagagagc 900

gacacgccaa cttcactgca tttttatggt gagcacgctt tattcgatca tctgcaacag 960

cgcgctctgg ccacctggcc tgcgctgagc gttcagcggt tgccggaacg gcctgaactg 1020

gggcgattta tcctggtgaa taacgcgctg gcgatcaaaa tcaccgatgg cagaacggca 1080

aacctgctcg ccggcttaac cgctctcgac accttcgtga ttgacgctgc gctgaattac 1140

gccgacaccg cctatctggt ggccgcccac aatcaacatg ccacagagac gaataaagcg 1200

ctgtttctga cgctgctgca aaccgtcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aactgctgca ttccgcgcgc tattacccgc attactcatt gctgaacgcg 1500

gcccggcctg agctggccgt agcgccctaa 1530

<210> 169
<211> 1530
<212> DNA
<213> Serratia plymuthica V4

<400> 169
atggcagaga ataattcggc aatccgttca gccgccgtta ttggtgcggg gaccatgggc 60

agaggcatcg cctacctcct ggcactgaac ggcatacgaa ccgtacttta taatcgcaat 120

ggtaataatc tcaatcaggc ccgtgactat attgtcagcg acctggacag aaaaatagat 180

aacggaaaaa taacccttca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcggcag gggtggaaaa caacccgcgc 420

tttatcggcc tgcatttttt caatccggcg ccgctgatga agttgatcga aatcattccc 480

tcttatttta cctcccacgc caccagccta cgctgccaga agttggtaat agcattgggt 540

aaacagtttg tggtctgcaa agccacgccg ggctttattg ttaatcgcat ggcgcggcct 600

ttctatctgg aagggttccg gctgctggag gaaaacgtgg cgctggcgcc acagatcgac 660

cgcgccctca aggccggcgg gcattttcgc atgggccctt tagaactgac ggattttatc 720

ggccaggata tcaactacca ggtcagcaag cagatttggc aggatatgca gttcgaccct 780

cgctataccc ctggtcattt gcaacgctcg ctggtggatg ccgggctgct ggggaggaaa 840

aacgggcgct cttttttttgc ttcccaaccg gcgacgccac ccacccgac cacagagagc 900

gacacgccaa cttcactgca tttttatggt gaacacgctt tattcgatca cctgcaacag 960

cgcgctttgg ccacctggcc tgcgttgcgc gttcagcggt tgccggaacg gccggaactg 1020

gggcgtttta tcctggtgaa taacaggctg gcgatcaaaa tcaccgatgg cagaacggca 1080

aacctgctcg ccggcttaac cgctctcgac accttcgtga ttgacgctgc gctgaattac 1140

gccgacaccg cctatctggt ggccgcccac aatcaacatg ccacagagac gaataaagcg 1200

ctgtttctga cgctgctgca aaccgtcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggtattt ttgcctggtt ggcgcagctc gggcagaaaa acgtgaaatc gacgctggat 1440

aacatggcgc aactgctgca ctccgcgcgc tattacccgc attactcatt gctgaacgcg 1500

gcccggcctg agctggccgt agcgccctaa 1530

<210> 170
<211> 1530
<212> DNA
<213> Serratia plymuthica 3Rp8

<400> 170
atggcagaga ataattcggc aatccgttca gccgccgtta ttggtgcggg gaccatgggc 60

agaggcatcg cctacctcct ggcactgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggcc 240

gtctttgacg ccataaccga cagcgatctg gtgattgaaa ccattgcgga agatgagcaa 300

accaagcatg aaatcctggc agccattgcg gctacggtaa aaccggaggc gatcattgcg 360

accaatacct cctcgttgtc gctgaacaaa ctggcggcag ggtggaaaa caacccgcgc 420

tttatcggcc tgcatttttt caatccggcg ccgctgatga agttgatcga aatcattccc 480

tcttatttta cctcccacgc caccagccta cgctgccaga agttggtaat agcattgggt 540

aaacagtttg tggtctgcaa agccacgccg ggctttattg ttaatcgcat ggcgcggcct 600

ttctatctgg aagggttccg gctgctggag gagaacgtgg cgctggcgcc acagatcgac 660

cgcgccctca aggccggcgg gcattttcgc atggggcctt tagaactgac ggattttatc 720

ggccaggata tcaactacca ggtcagcaag cagatttggc aggatatgca gttcgaccct 780

cgctataccc ctggtcattt gcaacgctcg ctggtggatg ccgggctgct ggggaggaaa 840

aacgggcgct ctttttttgc ttcccaaccg gcgacgccac ccaccccgac cacagagagc 900

gacacgccaa cgtcactgca tttttatggt gaacacgctt tattcgatca cctgcaacag 960

cgcgctttgg ccacctggcc tgcgttgcgc gttcagcggt tgccggaacg gccggaactg 1020

gggcgtttta tcctggtgaa taacaggctg gcgatcaaaa tcaccgatgg cagaacggcg 1080

aacctgctcg ccggcttaac cgctctcgac accttcgtga ttgacgccgc gctgaactac 1140

gccgacaccg cctatctggt ggccgcccac aatcaacatg ccacagagac gaataaagcg 1200

ctgtttctga cgctgctgca aaccctcatc gctcaggtgg agtttattaa agattcccct 1260

gctctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aactgctgca ctccgcgcgc tattacccgc attactcatt gctgaacgcg 1500

gcccggcctg agctggccgt agcgccctaa 1530

<210> 171
<211> 1530
<212> DNA
<213> Serratia proteamaculans MFPA44A14

<400> 171
atggcagaga ataattcggc aatccattcg gtcgctgtta ttggtgccgg aaccatgggc 60

agaggtattg cctatcttct ggcgcagaac ggcatacgaa ccctgcttta taatcgtagc 120

ggtaataatc tggatcaggc ccgcgactat attatccgtg acctggataa gaaaatagac 180

aacgggaaaa taagcccaca gaaaaaaggc gaagtattgg ccaacctggt tttctctccc 240

attttcgatg ctatcgccga cagcgacctg gtgattgaaa ccatcgcgga gcatgagacc 300

accaagcatg aaatcctcgc ggcgattgcg gccacggtga aacaagaggc cattatcgcc 360

accaacacct catcgctgtc gttgaataag ctggcggcag gcgtcgaaaa caacgcccgt 420

tttatcggcc tgcacttctt caatccggcc ccgctgatga aactgatcga gattattccg 480

tcctatttta ccagccgggc caccagcctg cgctgccagc agttggtgac ggcgctaggc 540

aaacagtttg tggtctgcaa agccacgccg ggttttatcg tcaaccggat ggcacggcct 600

ttttatctgg aaggattccg gctgttggaa gaaaacgtgg cattagcacc gcagatcgac 660

cgcgccctca aggccggtgg ccactttcgc atgggccctt tggagctgac cgactttatc 720

ggccaggata ttaactatca ggtcagcagc cagatttggc aggacatgca atacgacccc 780

cgctataccc ccggtcattt gcaacgttcg ctggtggatg ccgggttgct ggggaagaaa 840

aacggccgat cctttttgc tgccccttct gccgaatcca acctcctcga cgcaggcaac 900

ggtacgctga cttccctgca tttttatggc gaacataccc tgtttgacct gctgcaacag 960

cgcgccttgg ctacttggcc aacgctgcag attattcacc agccggaacg gccaacgctg 1020

ggacgcttta tccgggtgaa tgacgcattg gccgtcaaaa tcaccgacgg ccgcaccgcc 1080

aatctgctcg ctgaattgac cgatctcgac acctttgtga tcgacgccgc actgaattac 1140

agcgatacca cctatctggt ggccgcccac aatcaggacg ccgccgaggc caataaagcg 1200

ctgtttctgt cgctgctgca aacgttgatc ccgcaggtgg agtttattaa agactctcca 1260

ggcctgatcg tcgcccgggt tctgagcagt ctgatcaatg agtcggtgat catggtggag 1320

agcggggttt gcagccgggc agatatcgat attgccgccg tggcgggcgt taactatgcc 1380

gatggcatct ttgcctggct gacgcagctc gggcaaaaaa acgtgaaatc aacgctggat 1440

aatatggcgc aactgctgca ttccgcccgc tattacccgc attactcatt gctgaatatc 1500

ccccggcccg agctggccgt cgcgccgtaa 1530

<210> 172
<211> 1530
<212> DNA
<213> Serratia plymuthica A153

<400> 172
atggcagaga ataattcggc aatccgttca gccgccgtta ttggtgcggg gaccatgggc 60

agaggcatcg cctatctcct ggcgctgaac ggcatacgaa ccgtacttta taatcgcaat 120

ggtaataatc tcaatcaggc ccgtgactat attgtcagcg acctggacag aaaaatagat 180

aacggaaaaa taaccctgca gaaaaaaggc cagatattag ccaatattat tttctcggac 240

gtctttgacg ccataaccga cagcgatctg gtgattgaaa ccattgcgga agatgagcaa 300

accaagcatg aaatcctggc agccattgcg gcgacggtaa aaccggaggc gatcattgcg 360

accaatactt cctcgctgtc gctgaacaaa ctggcggcgg gggtggaaaa caacccgcgc 420

tttatcggcc tgcatttttt caatccggcg ccgctgatga agttgatcga aattattccc 480

tcttatttca cctctcgcgc caccagtcta cgctgccagc agttggtaac agcgttgggt 540

aaactgtttg tggtctgcaa agccacgccg ggctttattg ttaatcgcat ggcgcggcct 600

ttctatctgg aagggttccg gctgctggag gaaaacgtgg cgctggctcc acagatcgac 660

cgcgccctca aggccggcgg gcattttcgc atggggcctt tagaactgac tgattttatc 720

ggccaggata tcaactatca ggtcagcaaa cagatttggc aggatatgca gttcgatccc 780

cgctataccc ccggccattt gcaacgctcg ctggtggatg ccgggctgct ggggaggaaa 840

aacgggcgct ctttttttgc ttcccaacct gctacaccgc ccaacccgac cactgagggc 900

cacacgccaa cttcactgct tttttatggt gaacacgctt tattcgatca cctgcaacag 960

cgcgctttgg ccacctggcc tgcgctgcgc gttcagcggt tgccggaacg gccggaactg 1020

ggacgtttta tcctggtgaa taacaggctg gcgatcaaaa tcaccgatgg cagaacggcg 1080

aacctgctcg ccggcttaac cgctctcgac accttcgtga ttgacgccgc gctgaactac 1140

gccgacaccg cctatctggt ggccgcccac aatcaacatg ccacagagcc gaataaagcg 1200

ctgtttctga cgctgctgca aaccctcatc gctcaggtgg agtttattaa agattcccct 1260

gccctgatcg ttgcccgcgt actgagcagc ctgatcaatg aatcggtgat catggtggag 1320

agcggcgttt gcagccgggc agatatcgat atcgccgccg tggccggcgt gaactatgcc 1380

gacggcattt ttgcctggtt ggcgcagctc gggcagaaaa acgtgaaatc gacgctggat 1440

aacatggcgc aattgctgca ctccacgcgc tattacccgc attactcatt gctgaacgcg 1500

gcccggcctg agctggctgt agcgccctaa 1530

<210> 173
<211> 24
<212> PRT
<213> Artificial Sequence

<220>
<223> consensus sequence 1

<220>
<221> misc_feature
<222> (13)..(13)
<223> Xaa can be any naturally occurring amino acid

<220>
<221> misc_feature
<222> (15)..(17)
<223> Xaa can be any naturally occurring amino acid

<220>
<221> misc_feature
<222> (19)..(19)
<223> Xaa can be any naturally occurring amino acid

<220>
<221> misc_feature
<222> (21)..(21)
<223> Xaa can be any naturally occurring amino acid

<400> 173

```
Gly Ala Gly Thr Met Gly Arg Gly Ile Ala Tyr Leu Xaa Ala Xaa Xaa
1               5                   10                  15


Xaa Ile Xaa Thr Xaa Leu Tyr Asn
            20
```

<210> 174
<211> 774
<212> DNA
<213> Pseudomonas putida KT2440

<400> 174

```
atgccgcgat atatcgatgt gcaggcgccc gaacatggcg ttcagctcat taccctgcaa      60

cggcccgagg ccttgaatgc cctgtgcacc gagctactgg cagaactggc cgctgcgctg     120

caggctgccg ggaacgacga gcatgtccgt gccacagtga ttaccggcag cgccaaggca     180

ttcgccgcag gcgccgacat ccgcgagatg gccgatcgcg acctggtcgg catcctcaat     240

gacccgcgcg tagcgcattg gcaaagcatc gccgcattcg ccaaaccgct gattgctgca     300

gtcaacggct atgccctggg tggcggttgc gaactggcaa tgtgcgccga catcgtcatc     360

gccagtaccg acgcccgttt cggccagccg gaaatcaacc ttggcatcat ccccggtgct     420

ggcggcaccc agcgcctgtt acgtgccgtc ggtaagccgt tggccatgca gatggtgctg     480
```

acgggggaag ccatcactgc cctccgcgcc cagcaggccg gcctggtcag cgaaatcacc 540

cagcccgaac tcaccgtaga acgcgccatg caggttgccc gcagcatcgc cgccaaagcg 600

ccgctggctg tgcgcctggc caaggaggcg ttactgaagg ccggtgatac cgacctggcc 660

agcggcctgc gcttcgagcg ccatgccttc accctgctgg cgggcaccgc cgaccgcgat 720

gaaggcatcc gcgccttcca ggaaaagcgc caggcccgct tccaagggcg ctga 774

<210> 175
<211> 696
<212> DNA
<213> Pseudomonas putida KT2440

<400> 175
ttgatcaata aaacgtacga gtccatcgcc agcgcggtgg aagggattac cgacggttcg 60

accatcatgg tcggtggctt cggcacggct ggcatgccgt ccgagctgat cgatggcctc 120

attgccaccg gtgcccgcga cctgaccatc atcagcaaca acgccggcaa cggcgagatc 180

ggcctggccg ccctgctcat ggcaggcagc gtgcgcaagg tggtctgctc gttcccgcgc 240

cagtccgact cctacgtgtt cgacgaactg taccgcgccg gcaagatcga gctggaagtg 300

gtcccgcagg gcaacctggc cgagcgtatc cgcgccgcag gctccggcat tggtgcgttc 360

ttctcgccaa ccggctacgg caccctgctg gccgagggca aggaaacccg tgagatcgat 420

ggccgcatgt acgtgctgga aatgccgctg cacgccgact tcgcactgat caaggcgcac 480

aagggtgacc gttggggcaa cctgacctac cgcaaggccg cccgcaactt cggcccgatc 540

atggccatgg ctgccaagac cgccatcgcc caggtcgacc aggtcgtcga actcggtgaa 600

ctggacccgg aacacatcat caccccgggt atcttcgtcc agcgcgtggt cgccgtcacc 660

ggtgctgccg cttcttcgat tgccaaagct gtctga 696

<210> 176
<211> 642
<212> DNA
<213> Pseudomonas putida KT2440

<400> 176
atgaccatca ccaaaaagct ctcccgcacc gagatggccc aacgcgtggc cgcagacatc 60

caggaaggcg cgtacgtaaa cctgggcatc ggcgcaccga ccctggtggc caactacctg 120

ggcgacaagg aagtgttcct gcacagcgag aacggcctgc tgggcatggg cccaagccct 180

gcgccgggcg aggaagacga tgacctgatc aacgccggca agcagcacgt caccctgctg 240

accggtggtg ccttcttcca ccatgccgat tcgttctcga tgatgcgtgg cggccacctg 300

gacatcgctg tactgggcgc cttccaggtg tcggtcaagg gcgacctggc caactggcac 360

acgggtgccg aaggctcgat cccggccgta ggcggtgcaa tggacctggc caccggcgcc 420

cgccaggtgt tcgtgatgat ggaccacctg accaagaccg gcgaaagcaa gctggtgccc 480

gagtgcacct acccgctgac cggtatcgct tgcgtcagcc gcatctacac cgacctggcc 540

gtactggaag tgacacctga agggctgaaa gtggtcgaaa tctgcgcgga catcgacttt 600

gacgagctgc agaaactcag tggcgtgccg ctgatcaagt ga 642

<210> 177
<211> 774
<212> DNA
<213> Pseudomonas putida KT2440

<400> 177
atgccgcgat atatcgatgt gcaggcgccc gaacatggcg ttcagctcat taccctgcaa 60

cggccccgagg ccttgaatgc cctgtgcacc gagctactgg cagaactggc cgctgcgctg 120

caggctgccg ggaacgacga gcatgtccgt gccacagtga ttaccggcag cgccaaggca 180

ttcgccgcag gcgccgacat ccgcgagatg gccgatcgcg acctggtcgg catcctcaat 240

gacccgcgcg tagcgcattg gcaaagcatc gccgcattcg ccaaaccgct gattgctgca 300

gtcaacggct atgccctggg tggcggttgc gaactggcaa tgtgcgccga catcgtcatc 360

gccagtaccg acgcccgttt cggccagccg gaaatcaacc ttggcatcat ccccggtgct 420

ggcggcaccc agcgcctgtt acgtgccgtc ggtaagccgt tggccatgca gatggtgctg 480

acgggggaag ccatcactgc cctccgcgcc cagcaggccg gcctggtcag cgaaatcacc 540

cagcccgaac tcaccgtaga acgcgccatg caggttgccc gcagcatcgc cgccaaagcg 600

ccgctggctg tgcgcctggc caaggaggcg ttactgaagg ccggtgatac cgacctggcc 660

agcggcctgc gcttcgagcg ccatgccttc accctgctgg cgggcaccgc cgaccgcgat 720

gaaggcatcc gcgccttcca ggaaaagcgc caggcccgct tccaagggcg ctga 774

<210> 178
<211> 505
<212> PRT
<213> Pseudomonas putida KT2440

<400> 178

Met Ala Ala Leu Ala Ser Asn Val Gln Val Ala Val Ile Gly Ala Gly
1               5                   10                  15

Ala Met Gly Ala Gly Ile Ala Gln Val Ala Ala Gln Ala Gly His Pro
            20                  25                  30

Val Lys Leu Tyr Asp Asn Arg Pro Gly Ala Ala Ala Gln Ala Val Thr
        35                  40                  45

Gly Ile Asp Arg Gln Leu Ala Arg Leu Val Asp Lys Gly Lys Leu Leu
    50                  55                  60

Ala Ala Glu Arg Glu Thr Ile Asn Ala Arg Leu Cys Pro Val Asp Thr
65 70 75 80

Leu Glu Ala Leu Ala Asp Ala Gly Leu Val Ile Glu Ala Ile Val Glu
85 90 95

Asn Leu Gln Val Lys Gln Ala Leu Phe Ser Gln Leu Glu Thr Leu Cys
100 105 110

Ala Ala Asp Cys Ile Leu Ala Ser Asn Thr Ser Ser Leu Ser Ile Thr
115 120 125

Ser Leu Ala Ala Gly Leu Glu Arg Pro Gln His Val Val Gly Met His
130 135 140

Phe Phe Asn Pro Ala Pro Leu Met Ala Leu Val Glu Val Val Ser Gly
145 150 155 160

Leu Ala Thr Asp Pro Ala Val Ala Ala Cys Ile Tyr Ala Thr Ala Gln
165 170 175

Ala Trp Gly Lys Gln Pro Val His Ala Arg Ser Thr Pro Gly Phe Ile
180 185 190

Val Asn Arg Val Ala Arg Pro Phe Tyr Ala Glu Ser Leu Arg Leu Leu
195 200 205

Gln Glu Gly Ala Ala Asp Cys Ala Ser Leu Asp Ala Leu Met Arg Asp
210 215 220

Ser Gly Gly Phe Arg Met Gly Ala Phe Glu Leu Thr Asp Leu Ile Gly
225 230 235 240

His Asp Val Asn Tyr Ala Val Thr Cys Ser Val Phe Asp Ala Phe Tyr
245 250 255

Gly Asp Phe Arg Phe Gln Pro Ser Leu Val Gln Lys Glu Leu Val Asp
260 265 270

Ala Gly His Leu Gly Arg Lys Thr Gly Gln Gly Phe Tyr Arg Tyr Ala
275 280 285

Glu Gly Val Glu Arg Pro Gln Pro Ala Glu Leu His Ser Ser Ala Cys
290 295 300

Ala Glu Ala Cys Val Val Glu Gly Asn Leu Gly Val Met Gln Pro Leu
305 310 315 320

Val Glu Arg Leu Arg Gln Ser Gly Ile Ala Val Thr Gln Arg Ala Gly
325 330 335

Ser Gly Leu Ile Gln Val Gly Asp Ala Thr Leu Ala Leu Ser Asp Gly
340 345 350

Arg Leu Ala Ser Gln Arg Ala Arg Glu Asp Gly Leu Arg Asn Leu Val
355 360 365

Leu Leu Asp Leu Ala Leu Asp Tyr Ser Ser Ala Thr Arg Ile Ala Ile
370 375 380

Ser Trp Ser Ala Asp Thr Ser Asp Ser Ala Arg Asp Gln Ala Val Ala
385 390 395 400

Leu Leu Gln Arg Ala Gly Leu Lys Val Thr Gly Val Ala Asp Leu Pro
405 410 415

Gly Leu Val Val Leu Arg Thr Val Ala Met Leu Ala Asn Glu Ala Ala
420 425 430

Asp Ala Val Leu Gln Gly Val Gly Ser Ala Ala Asp Ile Asp Leu Ala
435 440 445

Met Arg Ala Gly Val Asn Tyr Pro Cys Gly Pro Leu Ala Trp Ala Ala
450 455 460

Asn Ile Gly Ile Ala His Thr Leu Arg Val Leu Asp Asn Leu Gln Cys
465 470 475 480

Ser Tyr Gly Glu Ser Arg Tyr Arg Pro Ser Leu Leu Leu Arg Arg Cys
485 490 495

Glu Ala Lys Gly Gly Thr Leu His Asp
500 505

<210> 179
<211> 475
<212> PRT
<213> Escherichia coli str. K-12 substr. MG1655

<400> 179

Met Met Ile Asn Val Gln Thr Val Ala Val Ile Gly Ser Gly Thr Met
1 5 10 15

Gly Ala Gly Ile Ala Glu Val Ala Ala Ser His Gly His Gln Val Leu
20 25 30

```
Leu Tyr Asp Ile Ser Ala Glu Ala Leu Thr Arg Ala Ile Asp Gly Ile
        35                  40                  45

His Ala Arg Leu Asn Ser Arg Val Thr Arg Gly Lys Leu Thr Ala Glu
    50                  55                  60

Thr Cys Glu Arg Thr Leu Lys Arg Leu Ile Pro Val Thr Asp Ile His
65                  70                  75                  80

Ala Leu Ala Ala Ala Asp Leu Val Ile Glu Ala Ala Ser Glu Arg Leu
                85                  90                  95

Glu Val Lys Lys Ala Leu Phe Ala Gln Leu Ala Glu Val Cys Pro Pro
            100                 105                 110

Gln Thr Leu Leu Thr Thr Asn Thr Ser Ser Ile Ser Ile Thr Ala Ile
        115                 120                 125

Ala Ala Glu Ile Lys Asn Pro Glu Arg Val Ala Gly Leu His Phe Phe
    130                 135                 140

Asn Pro Ala Pro Val Met Lys Leu Val Glu Val Val Ser Gly Leu Ala
145                 150                 155                 160

Thr Ala Ala Glu Val Val Glu Gln Leu Cys Glu Leu Thr Leu Ser Trp
                165                 170                 175

Gly Lys Gln Pro Val Arg Cys His Ser Thr Pro Gly Phe Ile Val Asn
            180                 185                 190

Arg Val Ala Arg Pro Tyr Tyr Ser Glu Ala Trp Arg Ala Leu Glu Glu
        195                 200                 205

Gln Val Ala Ala Pro Glu Val Ile Asp Ala Ala Leu Arg Asp Gly Ala
    210                 215                 220

Gly Phe Pro Met Gly Pro Leu Glu Leu Thr Asp Leu Ile Gly Gln Asp
225                 230                 235                 240

Val Asn Phe Ala Val Thr Cys Ser Val Phe Asn Ala Phe Trp Gln Glu
                245                 250                 255

Arg Arg Phe Leu Pro Ser Leu Val Gln Gln Glu Leu Val Ile Gly Gly
            260                 265                 270

Arg Leu Gly Lys Lys Ser Gly Leu Gly Val Tyr Asp Trp Arg Ala Glu
```

275 280 285

Arg Glu Ala Val Val Gly Leu Glu Ala Val Ser Asp Ser Phe Ser Pro
290 295 300

Met Lys Val Glu Lys Lys Ser Asp Gly Val Thr Glu Ile Asp Asp Val
305 310 315 320

Leu Leu Ile Glu Thr Gln Gly Glu Thr Ala Gln Ala Leu Ala Ile Arg
325 330 335

Leu Ala Arg Pro Val Val Val Ile Asp Lys Met Ala Gly Lys Val Val
340 345 350

Thr Ile Ala Ala Ala Ala Val Asn Pro Asp Ser Ala Thr Arg Lys Ala
355 360 365

Ile Tyr Tyr Leu Gln Gln Gln Gly Lys Thr Val Leu Gln Ile Ala Asp
370 375 380

Tyr Pro Gly Met Leu Ile Trp Arg Thr Val Ala Met Ile Ile Asn Glu
385 390 395 400

Ala Leu Asp Ala Leu Gln Lys Gly Val Ala Ser Glu Gln Asp Ile Asp
405 410 415

Thr Ala Met Arg Leu Gly Val Asn Tyr Pro Tyr Gly Pro Leu Ala Trp
420 425 430

Gly Ala Gln Leu Gly Trp Gln Arg Ile Leu Arg Leu Leu Glu Asn Leu
435 440 445

Gln His His Tyr Gly Glu Glu Arg Tyr Arg Pro Cys Ser Leu Leu Arg
450 455 460

Gln Arg Ala Leu Leu Glu Ser Gly Tyr Glu Ser
465 470 475

<210> 180
<211> 505
<212> PRT
<213> Acinetobacter baylyi ADP1

<400> 180

Met Thr His Pro Ile Lys Lys Ile Ala Ile Ile Gly Val Gly Val Met
1 5 10 15

Gly Ser Gly Ile Ala Gln Ile Ala Ala Gln Ser Gly His Ile Thr Tyr

20 25 30

Leu Tyr Asp Ala Lys Ala Gly Ala Ala Gln Gln Ala Lys Gln Gln Leu
35 40 45

Ala Ile Thr Phe Gln Lys Leu Leu Asp Lys Asn Lys Ile Thr Thr Glu
50 55 60

Tyr Ala Asp Ala Ala Asn Ala Asn Leu Leu Ile Ala Asn Glu Leu His
65 70 75 80

Asp Leu Lys Asp Cys Asp Leu Ile Val Glu Ala Ile Val Glu Arg Leu
85 90 95

Asp Ile Lys Gln Ser Leu Met Ser Gln Leu Glu Ala Ile Val Pro Glu
100 105 110

Thr Thr Ile Leu Ala Ser Asn Thr Ser Ser Leu Ser Ile Thr Ala Ile
115 120 125

Ala Ser Asn Cys Lys His Pro Glu Arg Val Ala Gly Tyr His Phe Phe
130 135 140

Asn Pro Val Pro Leu Met Lys Val Val Glu Val Ile Gln Gly Leu Lys
145 150 155 160

Thr Asp Pro Lys His Ile Glu Thr Leu Asn Gln Leu Ser Arg Val Leu
165 170 175

Gly His Arg Pro Val Val Ala Lys Asp Thr Pro Gly Phe Ile Ile Asn
180 185 190

His Ala Gly Arg Ala Tyr Gly Thr Glu Ala Leu Lys Ile Leu Asn Glu
195 200 205

Asn Val Thr Asp Ile Ser Glu Ile Asp Arg Ile Leu Arg Asp Gly Val
210 215 220

Gly Phe Arg Met Gly Pro Phe Glu Leu Met Asp Leu Thr Gly Leu Asp
225 230 235 240

Val Ser His Pro Val Met Glu Ser Ile Tyr His Gln Tyr Tyr Glu Glu
245 250 255

Ala Arg Tyr Arg Pro Asn Ser Leu Thr Lys Gln Met Leu Glu Ala Lys
260 265 270

```
Gln Leu Gly Arg Lys Val Gly Gln Gly Phe Tyr Asp Tyr Arg Thr Gly
        275                 280                 285

Ser Lys Thr Gly Glu Thr Ser Ala Lys Val Ala Glu Arg Leu Thr Leu
    290                 295                 300

Tyr Pro Lys Val Trp Ile Ala Ala Asp Phe Glu Asp Asp Lys Gln Leu
305                 310                 315                 320

Leu Ile Asn Tyr Leu Thr Thr His Asn Ile Gln Leu Asp Val Gly Ala
                325                 330                 335

Lys Pro Gln Ala Asp Ser Leu Cys Leu Leu Ala Cys Tyr Gly Glu Asp
            340                 345                 350

Thr Thr His Ala Ala Leu Arg Leu Asn Val Asn Pro Ala His Ser Val
        355                 360                 365

Ala Ile Asp Met Leu Tyr Gly Ile Glu Lys His Arg Thr Leu Met Pro
    370                 375                 380

Ser Leu Ile Thr Glu Val Thr Tyr Ser His Ala Ala His Ser Ile Phe
385                 390                 395                 400

Asn Leu Asp Gly Ala Met Val Ser Thr Ile Gly Glu Ser Ile Gly Phe
                405                 410                 415

Val Ala Gln Arg Ile Leu Ala Met Val Ile Asn Leu Gly Cys Asp Ile
            420                 425                 430

Ala Gln Gln Ala Ile Ala Ser Val Asp Asp Ile Asn Ala Ala Val Arg
        435                 440                 445

Leu Gly Leu Gly Tyr Pro Phe Gly Pro Ile Glu Trp Gly Asp Glu Ile
    450                 455                 460

Gly Ser Asn Lys Ile Leu Leu Ile Leu Asn Arg Ile Thr Ala Leu Thr
465                 470                 475                 480

Ser Asp Pro Arg Tyr Arg Pro Ser Pro Trp Leu Gln Arg Arg Val Ala
                485                 490                 495

Leu Asn Leu Pro Leu Thr Phe Thr Thr
            500                 505


<210>  181
<211>  506
<212>  PRT
```

<213> Serratia plymuthica NBRC102599

<400> 181

Met Ser Leu Pro Asn Phe Asn Gly Thr Val Ala Val Ile Gly Ala Gly
1 5 10 15

Thr Met Gly Ile Gly Ile Ala Gln Val Ala Ala His Ala Gly His Gln
20 25 30

Val Lys Leu Phe Asp Ile Ala Ala Thr Ala Ala Gln Asn Ala Leu Gly
35 40 45

Ala Leu Ser Leu Arg Leu Arg Gln Arg Val Ala Ala Gly Lys Ala Asp
50 55 60

Ala Asp Ala Thr Glu Ala Leu Leu Ala Arg Ile Gln Pro Val Asp Thr
65 70 75 80

Leu Glu Gln Leu Ala Asp Ser Thr Leu Ile Ile Glu Ala Val Ala Glu
85 90 95

Lys Leu Ala Ile Lys Gln Ser Leu Phe Arg Glu Leu Glu Ala Leu Cys
100 105 110

Ser Pro Ala Thr Leu Phe Ala Ser Asn Thr Ser Ser Leu Ser Ile Thr
115 120 125

Ala Ile Gly Gly Ala Leu Gln His Pro Gln Arg Leu Ala Gly Leu His
130 135 140

Phe Phe Asn Pro Ala Pro Leu Met Thr Leu Val Glu Ile Val Ser Gly
145 150 155 160

Leu Asp Thr Gly Ala Asp Thr Val Ala Thr Leu Gln Thr Leu Ala Arg
165 170 175

Gln Trp Gly Lys Gln Ser Val Leu Cys Arg Ser Thr Pro Gly Phe Ile
180 185 190

Val Asn Arg Val Ala Arg Pro Phe Tyr Ala Glu Thr Leu Arg Ala Leu
195 200 205

Glu Glu Arg Val Ala Asp Val Ala Thr Leu Asp Ala Val Met Arg Asp
210 215 220

Ala Gly Cys Phe Ala Met Gly Pro Leu Gln Leu Thr Asp Leu Ile Gly
225 230 235 240

```
Gln Asp Ile Asn Tyr Ala Val Thr Glu Ser Val Phe Gln Ala Phe Phe
                245                 250                 255

Gln Asp Pro Arg Phe Thr Pro Ser Leu Val Gln Gln Glu Leu Val Ala
            260                 265                 270

Ala Gly Arg Leu Gly Arg Lys Ser Gly Cys Gly Phe Tyr Arg Tyr Asp
        275                 280                 285

Gly Glu Gln Thr Ser Ser Thr Ala Val Cys Leu Pro Leu Ser Gln Ala
    290                 295                 300

Glu Pro Pro Arg Ser Ile Gln Leu His Gly Asp Glu Ala Gly Val Ala
305                 310                 315                 320

Phe Leu Ala Gly Leu Leu Thr Gly Asn Ala Glu Ala Ile Ile Gln Pro
                325                 330                 335

Gly Gln Thr Ser Ala Phe Ala Arg Ile Asp Glu Val Thr Phe Met Leu
            340                 345                 350

Thr Asn Gly Lys Thr Ala Ser Gln Ile Ala Glu Glu Thr Gly Thr Pro
        355                 360                 365

Val Val Leu Phe Asp Leu Ser Ala Asn Tyr Ser Gln Ala Pro Cys Val
    370                 375                 380

Ala Ile Ser Cys Ala Met Gln Asn Asp Ala Arg His Asn Asp Lys Val
385                 390                 395                 400

Val Arg Leu Leu Gln Ser Phe Gly Lys Gln Val Ile Leu Leu Pro Asp
                405                 410                 415

Tyr Pro Gly Leu Leu Val Met Arg Thr Leu Ala Met Leu Ser Asn Glu
            420                 425                 430

Ala Leu Asp Ala Val Asn Lys Gly Val Ala Ser Ala Glu Asp Ile Asp
        435                 440                 445

Ser Ala Leu Arg Cys Gly Val Asn Tyr Pro Arg Gly Pro Leu Glu Trp
    450                 455                 460

Gly Ala Ala Leu Gly Trp Arg Gln Ile Leu Ala Thr Leu Glu Asn Leu
465                 470                 475                 480

His Arg Tyr Tyr Gly Glu Pro Arg Tyr Arg Pro Met Pro Leu Leu Arg
                485                 490                 495
```

His Tyr Ala Phe Leu Ser Ser Gly Ala Glu
500 505

<210> 182
<211> 470
<212> PRT
<213> Escherichia coli str. K-12 substr. MG1655

<400> 182

Met Lys Lys Thr Lys Ile Val Cys Thr Ile Gly Pro Lys Thr Glu Ser
1 5 10 15

Glu Glu Met Leu Ala Lys Met Leu Asp Ala Gly Met Asn Val Met Arg
20 25 30

Leu Asn Phe Ser His Gly Asp Tyr Ala Glu His Gly Gln Arg Ile Gln
35 40 45

Asn Leu Arg Asn Val Met Ser Lys Thr Gly Lys Thr Ala Ala Ile Leu
50 55 60

Leu Asp Thr Lys Gly Pro Glu Ile Arg Thr Met Lys Leu Glu Gly Gly
65 70 75 80

Asn Asp Val Ser Leu Lys Ala Gly Gln Thr Phe Thr Phe Thr Thr Asp
85 90 95

Lys Ser Val Ile Gly Asn Ser Glu Met Val Ala Val Thr Tyr Glu Gly
100 105 110

Phe Thr Thr Asp Leu Ser Val Gly Asn Thr Val Leu Val Asp Asp Gly
115 120 125

Leu Ile Gly Met Glu Val Thr Ala Ile Glu Gly Asn Lys Val Ile Cys
130 135 140

Lys Val Leu Asn Asn Gly Asp Leu Gly Glu Asn Lys Gly Val Asn Leu
145 150 155 160

Pro Gly Val Ser Ile Ala Leu Pro Ala Leu Ala Glu Lys Asp Lys Gln
165 170 175

Asp Leu Ile Phe Gly Cys Glu Gln Gly Val Asp Phe Val Ala Ala Ser
180 185 190

Phe Ile Arg Lys Arg Ser Asp Val Ile Glu Ile Arg Glu His Leu Lys
195 200 205

```
Ala His Gly Gly Glu Asn Ile His Ile Ile Ser Lys Ile Glu Asn Gln
    210                 215                 220

Glu Gly Leu Asn Asn Phe Asp Glu Ile Leu Glu Ala Ser Asp Gly Ile
225                 230                 235                 240

Met Val Ala Arg Gly Asp Leu Gly Val Glu Ile Pro Val Glu Glu Val
                245                 250                 255

Ile Phe Ala Gln Lys Met Met Ile Glu Lys Cys Ile Arg Ala Arg Lys
            260                 265                 270

Val Val Ile Thr Ala Thr Gln Met Leu Asp Ser Met Ile Lys Asn Pro
        275                 280                 285

Arg Pro Thr Arg Ala Glu Ala Gly Asp Val Ala Asn Ala Ile Leu Asp
    290                 295                 300

Gly Thr Asp Ala Val Met Leu Ser Gly Glu Ser Ala Lys Gly Lys Tyr
305                 310                 315                 320

Pro Leu Glu Ala Val Ser Ile Met Ala Thr Ile Cys Glu Arg Thr Asp
                325                 330                 335

Arg Val Met Asn Ser Arg Leu Glu Phe Asn Asn Asp Asn Arg Lys Leu
            340                 345                 350

Arg Ile Thr Glu Ala Val Cys Arg Gly Ala Val Glu Thr Ala Glu Lys
        355                 360                 365

Leu Asp Ala Pro Leu Ile Val Val Ala Thr Gln Gly Gly Lys Ser Ala
    370                 375                 380

Arg Ala Val Arg Lys Tyr Phe Pro Asp Ala Thr Ile Leu Ala Leu Thr
385                 390                 395                 400

Thr Asn Glu Lys Thr Ala His Gln Leu Val Leu Ser Lys Gly Val Val
                405                 410                 415

Pro Gln Leu Val Lys Glu Ile Thr Ser Thr Asp Asp Phe Tyr Arg Leu
            420                 425                 430

Gly Lys Glu Leu Ala Leu Gln Ser Gly Leu Ala His Lys Gly Asp Val
        435                 440                 445

Val Val Met Val Ser Gly Ala Leu Val Pro Ser Gly Thr Thr Asn Thr
```

450 455 460

Ala Ser Val His Val Leu
465 470

<210> 183
<211> 480
<212> PRT
<213> Escherichia coli str. K-12 substr. MG1655

<400> 183

Met Ser Arg Arg Leu Arg Arg Thr Lys Ile Val Thr Thr Leu Gly Pro
1 5 10 15

Ala Thr Asp Arg Asp Asn Asn Leu Glu Lys Val Ile Ala Ala Gly Ala
20 25 30

Asn Val Val Arg Met Asn Phe Ser His Gly Ser Pro Glu Asp His Lys
35 40 45

Met Arg Ala Asp Lys Val Arg Glu Ile Ala Ala Lys Leu Gly Arg His
50 55 60

Val Ala Ile Leu Gly Asp Leu Gln Gly Pro Lys Ile Arg Val Ser Thr
65 70 75 80

Phe Lys Glu Gly Lys Val Phe Leu Asn Ile Gly Asp Lys Phe Leu Leu
85 90 95

Asp Ala Asn Leu Gly Lys Gly Glu Gly Asp Lys Glu Lys Val Gly Ile
100 105 110

Asp Tyr Lys Gly Leu Pro Ala Asp Val Val Pro Gly Asp Ile Leu Leu
115 120 125

Leu Asp Asp Gly Arg Val Gln Leu Lys Val Leu Glu Val Gln Gly Met
130 135 140

Lys Val Phe Thr Glu Val Thr Val Gly Gly Pro Leu Ser Asn Asn Lys
145 150 155 160

Gly Ile Asn Lys Leu Gly Gly Gly Leu Ser Ala Glu Ala Leu Thr Glu
165 170 175

Lys Asp Lys Ala Asp Ile Lys Thr Ala Ala Leu Ile Gly Val Asp Tyr
180 185 190

Leu Ala Val Ser Phe Pro Arg Cys Gly Glu Asp Leu Asn Tyr Ala Arg

195 200 205

Arg Leu Ala Arg Asp Ala Gly Cys Asp Ala Lys Ile Val Ala Lys Val
210 215 220

Glu Arg Ala Glu Ala Val Cys Ser Gln Asp Ala Met Asp Asp Ile Ile
225 230 235 240

Leu Ala Ser Asp Val Val Met Val Ala Arg Gly Asp Leu Gly Val Glu
245 250 255

Ile Gly Asp Pro Glu Leu Val Gly Ile Gln Lys Ala Leu Ile Arg Arg
260 265 270

Ala Arg Gln Leu Asn Arg Ala Val Ile Thr Ala Thr Gln Met Met Glu
275 280 285

Ser Met Ile Thr Asn Pro Met Pro Thr Arg Ala Glu Val Met Asp Val
290 295 300

Ala Asn Ala Val Leu Asp Gly Thr Asp Ala Val Met Leu Ser Ala Glu
305 310 315 320

Thr Ala Ala Gly Gln Tyr Pro Ser Glu Thr Val Ala Ala Met Ala Arg
325 330 335

Val Cys Leu Gly Ala Glu Lys Ile Pro Ser Ile Asn Val Ser Lys His
340 345 350

Arg Leu Asp Val Gln Phe Asp Asn Val Glu Glu Ala Ile Ala Met Ser
355 360 365

Ala Met Tyr Ala Ala Asn His Leu Lys Gly Val Thr Ala Ile Ile Thr
370 375 380

Met Thr Glu Ser Gly Arg Thr Ala Leu Met Thr Ser Arg Ile Ser Ser
385 390 395 400

Gly Leu Pro Ile Phe Ala Met Ser Arg His Glu Arg Thr Leu Asn Leu
405 410 415

Thr Ala Leu Tyr Arg Gly Val Thr Pro Val His Phe Asp Ser Ala Asn
420 425 430

Asp Gly Val Ala Ala Ala Ser Glu Ala Val Asn Leu Leu Arg Asp Lys
435 440 445

Gly Tyr Leu Met Ser Gly Asp Leu Val Ile Val Thr Gln Gly Asp Val
450 455 460

Met Ser Thr Val Gly Ser Thr Asn Thr Thr Arg Ile Leu Thr Val Glu
465 470 475 480

<210> 184
<211> 470
<212> PRT
<213> Serratia grimesii NBRC13537

<400> 184

Met Lys Lys Thr Lys Ile Val Cys Thr Ile Gly Pro Lys Thr Glu Ser
1 5 10 15

Glu Glu Met Leu Thr Asn Leu Leu Asn Ala Gly Met Asn Val Met Arg
20 25 30

Leu Asn Phe Ser His Gly Asp Tyr Glu Glu His Gly Asn Arg Ile Lys
35 40 45

Asn Met Arg Ala Val Met Ala Lys Thr Gly Gln Asn Ala Gly Ile Leu
50 55 60

Leu Asp Thr Lys Gly Pro Glu Ile Arg Thr Met Lys Leu Glu Gly Gly
65 70 75 80

Lys Asp Ala Ala Leu Val Ala Gly Gln Thr Phe Thr Phe Thr Thr Asp
85 90 95

Gln Ser Val Ile Gly Asn Asn Glu Arg Val Ala Val Thr Tyr Ala Gly
100 105 110

Phe Ser Ala Asp Leu Lys Ile Gly Asn Thr Val Leu Val Asp Asp Gly
115 120 125

Leu Ile Gly Met Glu Val Thr Asn Val Thr Glu Asn Glu Val Val Cys
130 135 140

Lys Val Leu Asn Ser Gly Asp Leu Gly Glu Asn Lys Gly Val Asn Leu
145 150 155 160

Pro Gly Val Ser Ile Gln Leu Pro Ala Leu Ala Glu Lys Asp Lys Arg
165 170 175

Asp Leu Ile Phe Gly Cys Glu Gln Gly Val Asp Phe Val Ala Ala Ser
180 185 190

Phe Ile Arg Lys Arg Ser Asp Val Leu Glu Ile Arg Glu His Leu Lys
195 200 205

Ala His Gly Gly Glu Gln Ile Gln Ile Ile Ser Lys Ile Glu Asn Gln
210 215 220

Glu Gly Leu Asn Asn Phe Asp Glu Ile Leu Glu Ala Ser Asp Gly Ile
225 230 235 240

Met Val Ala Arg Gly Asp Leu Gly Val Glu Ile Pro Val Glu Glu Val
245 250 255

Ile Phe Ala Gln Lys Met Met Ile Glu Lys Cys Asn Arg Ala Arg Lys
260 265 270

Val Val Ile Thr Ala Thr Gln Met Leu Asp Ser Met Ile Lys Asn Pro
275 280 285

Arg Pro Thr Arg Ala Glu Ala Gly Asp Val Ala Asn Ala Ile Leu Asp
290 295 300

Gly Thr Asp Ala Val Met Leu Ser Gly Glu Ser Ala Lys Gly Lys Tyr
305 310 315 320

Pro Leu Glu Ala Val Thr Ile Met Ala Thr Ile Cys Glu Arg Thr Asp
325 330 335

Arg Val Met Pro Ser Arg Ile Asp Ser Leu Asn Asp Asn Arg Lys Leu
340 345 350

Arg Ile Thr Glu Ala Val Cys Arg Gly Ala Val Glu Thr Ala Glu Lys
355 360 365

Leu Asp Ala Pro Leu Ile Val Val Ala Thr Ser Gly Gly Lys Ser Ala
370 375 380

Lys Ser Val Arg Lys Tyr Phe Pro Asn Ala Val Ile Leu Ala Leu Thr
385 390 395 400

Thr Asn Glu Val Thr Ala His Gln Leu Ile Leu Ser Lys Gly Val Ile
405 410 415

Pro Gln Met Val Lys Glu Ile Ala Ser Thr Asp Asp Phe Tyr Arg Ile
420 425 430

Gly Lys Glu Ala Ala Leu Ala Ser Gly Leu Ala Gln Lys Gly Asp Val
435 440 445

Val Val Met Val Ser Gly Ala Leu Val Pro Ser Gly Thr Thr Asn Thr
450 455 460

Ala Ser Val His Val Leu
465 470

<210> 185
<211> 480
<212> PRT
<213> Serratia grimesii NBRC13537

<400> 185

Met Ser Arg Arg Leu Arg Arg Thr Lys Ile Val Thr Thr Leu Gly Pro
1 5 10 15

Ala Thr Asp Arg Asp Asn Asn Leu Glu Lys Ile Ile Ala Ala Gly Ala
20 25 30

Asn Val Val Arg Leu Asn Phe Ser His Gly Ser Ala Glu Asp His Gln
35 40 45

Ala Arg Ala Asp Lys Val Arg Glu Ile Ala Ala Lys Leu Gly Arg His
50 55 60

Val Ala Ile Leu Gly Asp Leu Gln Gly Pro Lys Ile Arg Val Ser Thr
65 70 75 80

Phe Lys Glu Gly Lys Ile Phe Leu Asn Ile Gly Asp Lys Phe Leu Leu
85 90 95

Asp Ala Asn Met Ser Lys Gly Glu Gly Asp Lys Glu Lys Val Gly Ile
100 105 110

Asp Tyr Lys Gly Leu Pro Ala Asp Val Val Pro Gly Asp Val Leu Leu
115 120 125

Leu Asp Asp Gly Arg Val Gln Leu Lys Val Leu Glu Val Gln Gly Met
130 135 140

Lys Val Phe Thr Glu Val Thr Val Gly Gly Pro Leu Ser Asn Asn Lys
145 150 155 160

Gly Ile Asn Lys Leu Gly Gly Gly Leu Ser Ala Glu Ala Leu Thr Glu
165 170 175

Lys Asp Lys Ala Asp Ile Val Thr Ala Ala Lys Ile Gly Val Asp Tyr
180 185 190

Leu Ala Val Ser Phe Pro Arg Thr Gly Glu Asp Leu Asn Tyr Ala Arg
195 200 205

Arg Leu Ala Arg Asp Ala Gly Cys Asn Ala Lys Ile Val Ser Lys Val
210 215 220

Glu Arg Ala Glu Ala Val Cys Ser Asp Glu Ala Met Asp Asp Ile Ile
225 230 235 240

Leu Ala Ser Asp Val Val Met Val Ala Arg Gly Asp Leu Gly Val Glu
245 250 255

Ile Gly Asp Pro Glu Leu Val Gly Ile Gln Lys Lys Leu Ile Arg Arg
260 265 270

Ala Arg Thr Leu Asn Arg Ala Val Ile Thr Ala Thr Gln Met Met Glu
275 280 285

Ser Met Ile Thr Asn Pro Met Pro Thr Arg Ala Glu Val Met Asp Val
290 295 300

Ala Asn Ala Val Leu Asp Gly Thr Asp Ala Val Met Leu Ser Ala Glu
305 310 315 320

Thr Ala Ala Gly Gln Tyr Pro Ala Glu Thr Val Ala Ala Met Ala Arg
325 330 335

Val Cys Leu Gly Ala Glu Lys Ile Pro Ser Ile Asn Val Ser Lys His
340 345 350

Arg Leu Asp Val Gln Phe Asp Asn Ile Glu Glu Ala Ile Ala Met Ser
355 360 365

Ser Met Tyr Ala Ala Asn His Leu Lys Gly Val Thr Ala Leu Ile Ala
370 375 380

Met Thr Glu Ser Gly Arg Thr Ala Leu Met Met Ser Arg Ile Ser Ser
385 390 395 400

Gly Leu Pro Ile Phe Ala Met Ser Arg His Glu His Thr Leu Asn Leu
405 410 415

Thr Ala Leu Tyr Arg Gly Val Thr Pro Val Tyr Phe Asp Ser His Glu
420 425 430

Asp Gly Val Ile Ala Ala Asn Asp Ala Val Asn Arg Leu Arg Asp Lys
435 440 445

Gly Phe Leu Val Ser Gly Asp Leu Val Ile Val Thr Gln Gly Asp Val
    450                 455                 460

Met Glu Thr Val Gly Thr Thr Asn Thr Ser Arg Ile Leu Arg Val Glu
465                 470                 475                 480

<210> 186
<211> 200
<212> DNA
<213> Escherichia coli str. K-12 substr. MG1655

<400> 186
cgtaattgcc ctttaaaatt cggggcgccg accccatgtg gtctcaagcc caaaggaaga 60

gtgaggcgag tcagtcgcgt aatgcttagg cacaggattg atttgtcgca atgattgaca 120

cgattccgct tgacgctgcg taaggttttt gtaattttac aggcaacctt ttattcacta 180

acaaatagct ggtggaatat 200

<210> 187
<211> 30
<212> DNA
<213> Artificial Sequence

<220>
<223> primer

<400> 187
taccgtcgac ctcgacgtaa ttgcccttta 30

<210> 188
<211> 41
<212> DNA
<213> Artificial Sequence

<220>
<223> primer

<400> 188
ggcccccct cgagtcatta agtactatat tccaccagct a 41

<210> 189
<211> 774
<212> DNA
<213> Pseudomonas putida KT2440

<400> 189
atgccgcgat atatcgatgt gcaggcgccc gaacatggcg ttcagctcat taccctgcaa 60

cggcccgagg ccttgaatgc cctgtgcacc gagctactgg cagaactggc cgctgcgctg 120

caggctgccg ggaacgacga gcatgtccgt gccacagtga ttaccggcag cgccaaggca 180

ttcgccgcag gcgccgacat ccgcgagatg gccgatcgcg acctggtcgg catcctcaat 240

gacccgcgcg tagcgcattg gcaaagcatc gccgcattcg ccaaaccgct gattgctgca 300

gtcaacggct atgccctggg tggcggttgc gaactggcaa tgtgcgccga catcgtcatc 360

gccagtaccg acgcccgttt cggccagccg gaaatcaacc ttggcatcat ccccggtgct 420

ggcggcaccc agcgcctgtt acgtgccgtc ggtaagccgt tggccatgca gatggtgctg 480

acgggggaag ccatcactgc cctccgcgcc cagcaggccg gcctggtcag cgaaatcacc 540

cagcccgaac tcaccgtaga acgcgccatg caggttgccc gcagcatcgc cgccaaagcg 600

ccgctggctg tgcgcctggc caaggaggcg ttactgaagg ccggtgatac cgacctggcc 660

agcggcctgc gcttcgagcg ccatgccttc accctgctgg cgggcaccgc cgaccgcgat 720

gaaggcatcc gcgccttcca ggaaaagcgc caggcccgct tccaagggcg ctga 774

<210> 190
<211> 32
<212> DNA
<213> Artificial Sequence

<220>
<223> primer

<400> 190
ctggtggaat atatgcacga cgtattcatc tg 32

<210> 191
<211> 41
<212> DNA
<213> Artificial Sequence

<220>
<223> primer

<400> 191
ctcgagtcat taagtgttaa ctcaaacccg ctcgatggcc a 41

<210> 192
<211> 696
<212> DNA
<213> Pseudomonas putida KT2440

<400> 192
ttgatcaata aaacgtacga gtccatcgcc agcgcggtgg aagggattac cgacggttcg 60

accatcatgg tcggtggctt cggcacggct ggcatgccgt ccgagctgat cgatggcctc 120

attgccaccg gtgcccgcga cctgaccatc atcagcaaca acgccggcaa cggcgagatc 180

ggcctggccg ccctgctcat ggcaggcagc gtgcgcaagg tggtctgctc gttcccgcgc 240

cagtccgact cctacgtgtt cgacgaactg taccgcgccg gcaagatcga gctggaagtg 300

gtcccgcagg gcaacctggc cgagcgtatc cgcgccgcag gctccggcat tggtgcgttc 360

ttctcgccaa ccggctacgg caccctgctg gccgagggca aggaaacccg tgagatcgat 420

ggccgcatgt acgtgctgga aatgccgctg cacgccgact tcgcactgat caaggcgcac 480

aagggtgacc gttggggcaa cctgacctac cgcaaggccg cccgcaactt cggcccgatc 540

atggccatgg ctgccaagac cgccatcgcc caggtcgacc aggtcgtcga actcggtgaa 600

ctggacccgg aacacatcat caccccgggt atcttcgtcc agcgcgtggt cgccgtcacc 660

ggtgctgccg cttcttcgat tgccaaagct gtctga 696

<210> 193
<211> 642
<212> DNA
<213> Pseudomonas putida KT2440

<400> 193
atgaccatca ccaaaaagct ctcccgcacc gagatggccc aacgcgtggc cgcagacatc 60

caggaaggcg cgtacgtaaa cctgggcatc ggcgcaccga ccctggtggc caactacctg 120

ggcgacaagg aagtgttcct gcacagcgag aacggcctgc tgggcatggg cccaagccct 180

gcgccgggcg aggaagacga tgacctgatc aacgccggca agcagcacgt caccctgctg 240

accggtggtg ccttcttcca ccatgccgat tcgttctcga tgatgcgtgg cggccacctg 300

gacatcgctg tactgggcgc cttccaggtg tcggtcaagg gcgacctggc caactggcac 360

acgggtgccg aaggctcgat cccggccgta ggcggtgcaa tggacctggc caccggcgcc 420

cgccaggtgt tcgtgatgat ggaccacctg accaagaccg gcgaaagcaa gctggtgccc 480

gagtgcacct acccgctgac cggtatcgct tgcgtcagcc gcatctacac cgacctggcc 540

gtactggaag tgacacctga agggctgaaa gtggtcgaaa tctgcgcgga catcgacttt 600

gacgagctgc agaaactcag tggcgtgccg ctgatcaagt ga 642

<210> 194
<211> 55
<212> DNA
<213> Artificial Sequence

<220>
<223> primer

<400> 194
gagcgggttt gagttcaatt gtagctggtg gaatatttga tcaataaaac gtacg 55

<210> 195
<211> 34
<212> DNA
<213> Artificial Sequence

<220>
<223> primer

<400> 195
gagtcattaa gtgtttcact tgatcagcgg cacg 34

<210> 196
<211> 35
<212> DNA
<213> Artificial Sequence

<220>
<223> primer

<400> 196
ctggtggaat atagtactat ggcagaaagt aatgc 35

<210> 197
<211> 39
<212> DNA
<213> Artificial Sequence

<220>
<223> primer

<400> 197
ttccaccagc taagtgctag cttaaggcgt ggtcgtcag 39

<210> 198
<211> 35
<212> DNA
<213> Artificial Sequence

<220>
<223> primer

<400> 198
ctggtggaat atagtactat ggcagaaagt aatgc 35

<210> 199
<211> 39
<212> DNA
<213> Artificial Sequence

<220>
<223> primer

<400> 199
ttccaccagc taagtgctag cttaaggcgt ggtcgtcag 39

<210> 200
<211> 35
<212> DNA
<213> Artificial Sequence

<220>
<223> primer

<400> 200
ctggtggaat atagtactat ggcagagaat aattc 35

<210> 201
<211> 39

<212> DNA
<213> Artificial Sequence

<220>
<223> primer

<400> 201
ttccaccagc taagtgctag cttagggcgc tacagccag 39

<210> 202
<211> 35
<212> DNA
<213> Artificial Sequence

<220>
<223> primer

<400> 202
ctggtggaat atagtactat ggcagagaat aattc 35

<210> 203
<211> 38
<212> DNA
<213> Artificial Sequence

<220>
<223> primer

<400> 203
ttccaccagc taagtgctag cttagggcgc gacggcca 38

<210> 204
<211> 35
<212> DNA
<213> Artificial Sequence

<220>
<223> primer

<400> 204
ctggtggaat atagtactat ggcagaaagt aatgc 35

<210> 205
<211> 40
<212> DNA
<213> Artificial Sequence

<220>
<223> primer

<400> 205
ttccaccagc taagtgctag cttaaggcgt ggtggtcagc 40

<210> 206
<211> 35
<212> DNA
<213> Artificial Sequence

<220>
<223> primer

<400> 206
ctggtggaat atagtactat ggcagagaat aattc 35

<210> 207
<211> 40
<212> DNA
<213> Artificial Sequence

<220>
<223> primer

<400> 207
ttccaccagc taagtgctag cttacggcgc gacggccagt 40

<210> 208
<211> 35
<212> DNA
<213> Artificial Sequence

<220>
<223> primer

<400> 208
ctggtggaat atagtactat ggcagagaat aatac 35

<210> 209
<211> 40
<212> DNA
<213> Artificial Sequence

<220>
<223> primer

<400> 209
ttccaccagc taagtgctag ctcatggcgc gacggccagc 40

<210> 210
<211> 40
<212> DNA
<213> Artificial Sequence

<220>
<223> primer

<400> 210
cggccagtga attcgagctc cgtaattgcc ctttaaaatt 40

<210> 211
<211> 41
<212> DNA
<213> Artificial Sequence

<220>
<223> primer

<400> 211
gatccccggg taccgcatgc tatattccac cagctatttg t 41

<210> 212
<211> 34
<212> DNA
<213> Artificial Sequence

<220>
<223> primer

<400> 212
gctggtggaa tatagatgcc gcgatatatc gatg 34

<210> 213
<211> 34
<212> DNA
<213> Artificial Sequence

<220>
<223> primer

<400> 213
attacgccaa gcttgtcagc gcccttggaa gcgg 34

<210> 214
<211> 672
<212> DNA
<213> Acinetobacter baylyi ADP1 dcaI

<400> 214
atgattaata aaatcattaa tgatattgag cccattttaa aatcgattcc agatggttca 60

acgattatga caagtggttt tggtacgaca ggtcaacctg aggcactact tgaagcatta 120

attgatttcg ctccaaaaga gttaactatt atcaataata atgcttcatc tggcccgaat 180

ggtttgactc agcttttcac tgctggattg gtcaaaaagc tcatttgctc gtatccaaaa 240

tcaatcagtt cgactgtttt tccagattta tatcgtgcgg gaaaaattga acttgaactg 300

gttcctcagg gaaatcttgc atgtcgtatt caagcagcag gtgctggtct gggagcggta 360

tttacccccca caggctacgg gaccaaaatt gctgaaggca aagaaacgcg cattattaac 420

ggtaaaaatt atgtacttga atatccactt gaagccgatt atgcattcat ttatgcagat 480

aaggcagatc gctgggggaa tttaacttat cgtaaagccg ccagaaattt tggtccgatt 540

atggccaaag ctgcaaaaac aacaattgct caagtcaatc aaactgttga attgggtgat 600

cttgatcccg aatgcattat tacacccggg atatttgtac agcatgtggt gcgattagga 660

gatataaaat ga 672

<210> 215
<211> 675
<212> DNA
<213> Acinetobacter baylyi ADP1 dcaJ

<400> 215
atgacgattc agaaaagaag tcgagaagat atcgcaatca tgatagcaaa ggatattcct 60

gatggttcgt atgtcaatct tggaattgga ttaccaaccc atgtcgcaaa atacttgcca 120

aaagataagg aaatttttct gcactcagag aatggtgtac tggcatttgg tccacctcct 180

gctgaaggtg aagaagatca ggacttagtc aatgcaggta aagagctggt gactttacta 240

tcaggtggat gttttatgca tcatggagat tcatttgaca ttatgcgtgg tggacatctg 300

gatatttgcg ttattggggc atttcaagtc gcgctcaatg gtgatttggc taactggcat 360

acaggtaaag acgatgatgt gcctgctgtt gggggagcaa tggatttggc ggttggggcc 420

aagcgtattt ttgtgtatat ggaacatacc accaaaaaag gcgaacctaa aattgttaaa 480

catctgactt atccaatcac tggtgagcag tgtgttgatc gtatttatac agatttatgt 540

acgattgagc tgaaagatgg acaggcatat gtgattgaaa tggtcgacgg attagatttt 600

gatactttgc aagccttaac tgaatgccca ttaattgatc actgtactta ttcatcgttg 660

attcagctta gataa 675

<210> 216
<211> 55
<212> DNA
<213> Artificial Sequence

<220>
<223> primer

<400> 216
gagcgggttt gagttcaatt gtagctggtg gaatatatga ttaataaaat catta 55

<210> 217
<211> 34
<212> DNA
<213> Artificial Sequence

<220>
<223> primer

<400> 217
gagtcattaa gtgttttatc taagctgaat caac 34

<210> 218
<211> 1155
<212> DNA
<213> Acinetobacter baylyi ADP1 dcaA

<400> 218
atgattcgcg atgaagggat gttgcaacaa ttactttcga caatacgaga ttttgtaaaa 60

aatgaattga ttcctcgaga gcatgaagtt gcagaaaagg attgtattcc tgaagatatt 120

attcagcaaa tgcgagaact aggcctattt ggtttaacca ttcccgaaga atacggtgga 180

cttggaatca caatggaaga agaggtgaat gtcgcgtttg aacttggtca gacatcccca 240

gcatttcgtt cattgattgg cacaaataat ggcattggtt caagtggttt aatcattgat 300

ggaactgagg agcaaaaaca gaaatatttg ccacgttatg ccagtggaga aattattggt 360

tcattttgct taactgaacc agaagcgggt tcagatgctg cctctttaaa aacgacagcg 420

gtaaaagatg gtgatttcta catattaaat ggaaccaagc gttttattac caatgcaccg 480

catgcagcaa catttaccgt aatggcacgc accaatccag caattaaagg ggcaggtgga 540

atttctgctt ttttggtaga agccaataca ccaggtatca cactaggcaa aatagatcag 600

aaaatgggac aaaaaggctc tcatacctgt gatgtgattt ttgaaaattg tcgagtacct 660

gcatctgcat taattggtgg cgttgaaggc gttggtttta aaacagcaat gaaagtgctg 720

gataaaggcc gtctacatat tggtgcatat agtgtaggtg ttgcagagcg tatgttgaat 780

gatgcactac attatgctgt cgagcgtaag cagtttggtc aacccattgc aaattttcaa 840

ttgattcaag ccatgctggc agactctaaa gccgaaattt atgcggctaa atgtatggtt 900

ttggatgcag cacgtcgccg tgatgaaggc caaaatatta gtacagaagc ctcatgtgcc 960

aagatgtttg caacagaaat gtgtggtcga gtcgcagatc gctgtgtgca aattcatggt 1020

ggggcaggtt acatcagtga atattcgatt gagcgttttt atcgtgacgt gcgtttattc 1080

cgtctttatg agggtacgac ccaagttcag caaattatta ttgccaaaaa tatgattaag 1140

gaagtgacgt cctaa 1155

<210> 219
<211> 50
<212> DNA
<213> Artificial Sequence

<220>
<223> primer

<400> 219
gggcgctgac aagcttagct ggtggaatat atgattcgcg atgaagggat 50

<210> 220
<211> 36
<212> DNA
<213> Artificial Sequence

<220>
<223> primer

<400> 220
tgattacgcc aagcttttag gacgtcactt ccttaa 36

<210> 221
<211> 70
<212> DNA
<213> Artificial Sequence

<220>
<223>  primer

<400>  221
gctggtttta ttcgtttctt tatccatcac aacttgtaga aaaatccgtc gtgtaggctg      60

gagctgcttc                                                              70

<210>  222
<211>  70
<212>  DNA
<213>  Artificial Sequence

<220>
<223>  primer

<400>  222
aaacgccgca aagcggcgtt tttgacaaaa gaattatgtc acgttttata atgggaatta      60

gccatggtcc                                                              70

<210>  223
<211>  20
<212>  DNA
<213>  Artificial Sequence

<220>
<223>  primer

<400>  223
cggtgccctg aatgaactgc                                                   20

<210>  224
<211>  20
<212>  DNA
<213>  Artificial Sequence

<220>
<223>  primer

<400>  224
cgccggtgaa accgtcacga                                                   20

<210>  225
<211>  20
<212>  DNA
<213>  Artificial Sequence

<220>
<223>  primer

<400>  225
gtgctgcgtc gtctttagct                                                   20

<210>  226
<211>  70
<212>  DNA

<213> Artificial Sequence

<220>
<223> primer

<400> 226
gacgttatcg caacaccaat aatttgcttc accagtcaac ggagtaatac gtgtaggctg 60

gagctgcttc 70

<210> 227
<211> 70
<212> DNA
<213> Artificial Sequence

<220>
<223> primer

<400> 227
cagtcaggtt cagtgtgtgc tcatgacgtg acatggcgaa gataggcagg atgggaatta 60

gccatggtcc 70

<210> 228
<211> 20
<212> DNA
<213> Artificial Sequence

<220>
<223> primer

<400> 228
tggtggaaat ggcgcagctt 20

<210> 229
<211> 20
<212> DNA
<213> Artificial Sequence

<220>
<223> primer

<400> 229
gcgtaaccga cccaattcga 20

<210> 230
<211> 67
<212> DNA
<213> Artificial Sequence

<220>
<223> primer

<400> 230
gaaagcaagt ttctcccatc cttctcaact taaagactaa gactgtcgtg taggctggag 60

ctgcttc 67

<210> 231
<211> 70
<212> DNA
<213> Artificial Sequence

<220>
<223> primer

<400> 231
cgcccatcag ggcgcttcga tatacaaatt aattcacaaa agcaatatta atgggaatta 60

gccatggtcc 70

<210> 232
<211> 18
<212> DNA
<213> Artificial Sequence

<220>
<223> primer

<400> 232
ccggatgaac tttcactt 18

<210> 233
<211> 18
<212> DNA
<213> Artificial Sequence

<220>
<223> primer

<400> 233
gagctgcgtc atctttag 18

<210> 234
<211> 70
<212> DNA
<213> Artificial Sequence

<220>
<223> primer

<400> 234
ggatttcatg ttcaagcaac acctggttgt ttcagtcaac ggagtattac gtgtaggctg 60

gagctgcttc 70

<210> 235
<211> 70
<212> DNA
<213> Artificial Sequence

<220>
<223> primer

<400> 235
tgaactgtag gccggatgtg gcgttttcgc cgcatccggc aacgtactta atgggaatta 60

gccatggtcc 70

<210> 236
<211> 18
<212> DNA
<213> Artificial Sequence

<220>
<223> primer

<400> 236
acgcatgagt tgtatgaa 18

<210> 237
<211> 18
<212> DNA
<213> Artificial Sequence

<220>
<223> primer

<400> 237
ttacccgatc aggatcat 18

<210> 238
<211> 1434
<212> DNA
<213> Serratia grimesii NBRC13537

<400> 238
atgttcaaga acgcatttgc aaacctgcaa aaagtaggta agtcgctaat gctgccggtg 60

tccgtattgc ctatcgcagg tatcctgctg ggcgtcggtt ccgccaactt tagctggcta 120

cctgcggtag tctcccacgt gatggcggaa gccggcggtt ctgttttcgc caatatgccg 180

ttaattttcg ccatcggtgt tgccctgggc ttcaccaaca acgacggtgt atccgcactg 240

gctgcagtcg tggcttacgg catcatggtg aaaaccatgg cggtggttgc acctttggtg 300

ctgcacttgc cggctgaaga gattgcggcc aaacatttgg cggataccgg tgtgctcggg 360

gggattatct ccggctccat cgctgcctat atgttcaacc gcttcttccg cattcaattg 420

ccggaatacc tgggcttctt tgcaggtaag cgttttgtgc cgattatctc cggcctggcg 480

gcgatcgttc tgggcgtagt attgtccttc atctggcctc caatcggtac ggctatccag 540

accttctccc agtgggctgc ttatcagaac ccggtagtgg cgtttggtat ttacggcgtg 600

gttgaacgtg cgctggtgcc atttggtctg caccatatct ggaacgtacc gttccaaatg 660

cagattggtg agtacaccaa cgcagcaggt caggtattcc acggcgatat cccacgttat 720

atggcgggtg acccaactgc gggtaaactg tccggtggct tcctgttcaa aatgtacggt 780

ctgcctgctg ctgcgattgc catctggcac tcagccaagc cggaaaaccg cgctaaagtc 840

ggcggtatca tgatctccgc tgccttgacc tcgttcctga ccggtatcac cgaaccgatc 900

gagttctcat tcatgttcgt cgcgccgatc ctgtacgcaa tccatgccat cctggctggc 960

ctggcgttcc cgatctgtat cctgctgggt atgcgtgacg gcaccagctt ctcacacggt 1020

ttgatcgact ttatcgtact gagcggtaac agcagcaaaa tttggctgtt cccaatcgtc 1080

ggtatcatct acggtctggt gtactacacc atcttccgtg tgctgattgc caagctggat 1140

ctgaaaaccc caggtcgcga agacactgtt tctgagcagg ttgcacaggg cggttctgaa 1200

atgtctgcgg cgctggttca ggctttcggc ggtaaagaaa acatcaccaa cctggatgcc 1260

tgtatcaccc gtctgcgtgt tagcgtggcg gacgtgagca aggttgacca ggctggcctg 1320

aaaaaactgg gtgctgccgg cgtagttgtc gcaggctctg gtgttcaggc tatctttggt 1380

accaaatctg ataacctgaa aacggatatg gacgaataca tccgtaacca ttaa 1434

<210> 239
<211> 70
<212> DNA
<213> Artificial Sequence

<220>
<223> primer

<400> 239
aagaatcgcg gaaagtagca aagcactatt actcaggagc acactcaact gtgtaggctg 60

gagctgcttc 70

<210> 240
<211> 70
<212> DNA
<213> Artificial Sequence

<220>
<223> primer

<400> 240
gccaataaaa aagggaggct ttcgcctccc ttaaaactcc cctgcctgaa atgggaatta 60

gccatggtcc 70

<210> 241
<211> 20
<212> DNA
<213> Artificial Sequence

<220>
<223> primer

<400> 241
gtaccgcttg atcgcattct 20

<210> 242
<211> 19
<212> DNA
<213> Artificial Sequence

<220>
<223>  primer

<400>  242
gaatatggtt acaagcgca                                                    19

<210>  243
<211>  70
<212>  DNA
<213>  Artificial Sequence

<220>
<223>  primer

<400>  243
cacgcgtgag aacgtaaaaa aagcacccat actcaggagc actctcaatt gtgtaggctg       60

gagctgcttc                                                              70

<210>  244
<211>  70
<212>  DNA
<213>  Artificial Sequence

<220>
<223>  primer

<400>  244
agccatctgg ctgccttagt ctccccaacg tcttacggat tagtggttac atgggaatta       60

gccatggtcc                                                              70

<210>  245
<211>  20
<212>  DNA
<213>  Artificial Sequence

<220>
<223>  primer

<400>  245
atcggttact ggtggaaact                                                   20

<210>  246
<211>  20
<212>  DNA
<213>  Artificial Sequence

<220>
<223>  primer

<400>  246
gtggatggga cagtcagtaa                                                   20